(19) Europäisches Patentamt
European Patent Office
Office européen des brevets

(11) EP 3 395 945 A1

(12) EUROPÄISCHE PATENTANMELDUNG

(43) Veröffentlichungstag:
**31.10.2018 Patentblatt 2018/44**

(51) Int Cl.:
***C12N 9/02*** *(2006.01)*

(21) Anmeldenummer: **18155821.4**

(22) Anmeldetag: **16.07.2004**

(84) Benannte Vertragsstaaten:
**AT BE BG CH CY CZ DE DK EE ES FI FR GB GR HU IE IT LI LU MC NL PL PT RO SE SI SK TR**

(30) Priorität: **01.08.2003 DE 10335992**
**24.09.2003 DE 10344557**
**10.10.2003 DE 10347869**
**18.12.2003 DE 10359593**
**27.02.2004 DE 102004009457**
**13.03.2004 DE 102004012370**
**14.05.2004 DE 102004024014**

(62) Dokumentnummer(n) der früheren Anmeldung(en) nach Art. 76 EPÜ:
**04763291.4 / 1 654 344**

(71) Anmelder: **BASF Plant Science GmbH**
**67056 Ludwigshafen (DE)**

(72) Erfinder:
- **ZANK, Thorsten**
**67056 Ludwigshafen (DE)**
- **BAUER, Joerg**
**27713 Durham (US)**
- **CIRPUS, Petra**
**67117 Limburgerhof (DE)**
- **ABBADI, Amine**
**24214 Gettorf (DE)**
- **HEINZ, Ernst**
**22609 Hamburg (DE)**
- **QIU, Xiao**
**S7N 3S5 Saskatoon, SK (CA)**
- **VRINTEN, Patricia**
**Saskatoon, Sk. S7J 4H5 (CA)**
- **SPERLING, Petra**
**22609 Hamburg (DE)**
- **DOMERGUE, Frederic**
**33076 Bordeaux Cedex (FR)**
- **MEYER, Astrid**
**50737 Köln (DE)**
- **KIRSCH, Jelena**
**22547 Hamburg (DE)**

(74) Vertreter: **BASF IP Association**
**BASF SE**
**G-FLP-C006**
**67056 Ludwigshafen (DE)**

Bemerkungen:
•Diese Anmeldung ist am 08-02-2018 als Teilanmeldung zu der unter INID-Code 62 erwähnten Anmeldung eingereicht worden.
•Die Patentansprüche wurden nach dem Anmeldetag / dem Tag des Eingangs der Teilanmeldung eingereicht (R. 68(4) EPÜ).

# (54) VERFAHREN ZUR HERSTELLUNG MEHRFACH UNGESÄTTIGTER FETTSÄUREN IN TRANSGENEN ORGANISMEN

(57) Die vorliegende Erfindung betrifft ein Verfahren zur Herstellung von mehrfach ungesättigten Fettsäuren in einem Organismus, indem Nukleinsäuren in den Organismus eingebracht werden, die für Polypeptide mit Δ-5-Elongaseaktivität codieren. Vorteilhaft können diese Nukleinsäuresequenzen gegebenenfalls zusammen mit weiteren Nukleinsäuresequenzen, die für Polypeptide der Biosynthese des Fettsäure- oder Lipidstoffwechels codieren, in dem Organismus exprimiert werden. Besonders vorteilhaft sind Nukleinsäuresequenzen, die für eine Δ-6-Desaturase-, eine Δ-5-Desaturase-, Δ-4-Desaturase- und/oder Δ-6-Elongaseaktivität codieren. Vorteilhaft stammen diese Desaturasen und Elongasen aus Thalassiosira, Euglena oder Ostreococcus. Weiterhin betrifft die Erfindung ein Verfahren zur Herstellung von Ölen und/oder Triacylglyceriden mit einem erhöhten Gehalt an langkettigen mehrfach ungesättigten Fettsäuren.

Die vorliegende Erfindung betrifft außerdem in einer bevorzugten Ausführungsform ein Verfahren zur Herstellung von ungesättigten ω-3 Fettsäuren sowie ein Verfahren zur Herstellung von Triglyceriden mit einem erhöhten Gehalt an ungesättigten Fettsäuren, besonders von ω-3 Fettsäuren mit mehr als drei Doppelbindungen. Die Erfindung betrifft die Herstellung eines transgenen Organismus bevorzugt einer transgenen Pflanze oder eines transgenen Mikroorganismus mit erhöhtem Gehalt an ungesättigten ω-3-Fettsäuren, Ölen oder Lipiden mit ω-3-Doppelbindungen aufgrund der Expression der im erfindungsgemäßen Verfahren verwendeten Elongasen und Desaturasen vorteilhaft in Verbindung mit ω-3-De-

EP 3 395 945 A1

saturasen z.B. einer ω-3-Desaturase aus Pilzen der Familie Pythiaceae wie der Gattung Phytophtora beispielsweise der Gattung und Art Phytophtora infestans oder einer ω-3-Desaturase aus Algen wie der Familie der Prasinophyceae z.B. der Gattung Ostreococcus speziell der Gattung und Art Ostreococcus tauri oder Diatomeen wie der Gattung Thalassiosira speziell der Gattung und Art Thalassiosira pseudonana.

Die Erfindung betrifft weiterhin die Nukleinsäuresequenzen, Nukleinsäurekonstrukte, Vektoren und Organismen enthaltend die erfindungsgemäßen Nukleinsäuresequenzen, Vektoren enthaltend die Nukleinsäuresequenzen und/oder die Nukleinsäurekonstrukte sowie transgene Organismen enthalten die vorgenannten Nukleinsäuresequenzen, Nukleinsäurekonstrukte und/oder Vektoren.

Ein weiterer Teil der Erfindung betrifft Öle, Lipide und/oder Fettsäuren hergestellt nach dem erfindungsgemäßen Verfahren und deren Verwendung. Außerdem betrifft die Erfindung ungesättigte Fettsäuren sowie Triglyceride mit einem erhöhten Gehalt an ungesättigten Fettsäuren und deren Verwendung.

## Beschreibung

Die vorliegende Erfindung betrifft ein Verfahren zur Herstellung von mehrfach ungesättigten Fettsäuren in einem Organismus, indem Nukleinsäuren in den Organismus eingebracht werden, die für Polypeptide mit Δ-5-Elongaseaktivität codieren. Vorteilhaft können diese Nukleinsäuresequenzen gegebenenfalls zusammen mit weiteren Nukleinsäuresequenzen, die für Polypeptide der Biosynthese des Fettsäure- oder Lipidstoffwechels codieren, in dem Organismus exprimiert werden. Besonders vorteilhaft sind Nukleinsäuresequenzen, die für eine Δ-6-Desaturase-, eine Δ-5-Desaturase-, Δ-4-Desaturase-, Δ-12-Desaturase- und/oder Δ-6-Elongaseaktivität codieren. Vorteilhaft stammen diese Desaturasen und Elongasen aus Thalassiosira, Euglena oder Ostreococcus. Weiterhin betrifft die Erfindung ein Verfahren zur Herstellung von Ölen und/oder Triacylglyceriden mit einem erhöhten Gehalt an langkettigen mehrfach ungesättigten Fettsäuren.

Die vorliegende Erfindung betrifft außerdem in einer bevorzugten Ausführungsform ein Verfahren zur Herstellung von ungesättigten ω-3 Fettsäuren sowie ein Verfahren zur Herstellung von Triglyceriden mit einem erhöhten Gehalt an ungesättigten Fettsäuren, besonders von ω-3 Fettsäuren mit mehr als drei Doppelbindungen. Die Erfindung betrifft die Herstellung eines transgenen Organismus bevorzugt einer transgenen Pflanze oder eines transgenen Mikroorganismus mit erhöhtem Gehalt an ungesättigten ω-3-Fettsäuren, Ölen oder Lipiden mit ω-3-Doppelbindungen aufgrund der Expression der im erfindungsgemäßen Verfahren verwendeten Elongasen und Desaturasen vorteilhaft in Verbindung mit ω-3-Desaturasen z.B. einer ω-3-Desaturase aus Pilzen der Familie Pythiaceae wie der Gattung Phytophtora beispielsweise der Gattung und Art Phytophtora infestans oder einer ω-3-Desaturase aus Algen wie der Familie der Prasinophyceae z.B. der Gattung Ostreococcus speziell der Gattung und Art Ostreococcus tauri oder Diatomeen wie der Gattung Thalassiosira speziell der Gattung und Art Thalassiosira pseudonana.

Die Erfindung betrifft weiterhin die Nukleinsäuresequenzen, Nukleinsäurekonstrukte, Vektoren und Organismen enthaltend die erfindungsgemäßen Nukleinsäuresequenzen, Vektoren enthaltend die Nukleinsäuresequenzen und/oder die Nukleinsäurekonstrukte sowie transgene Organismen enthalten die vorgenannten Nukleinsäuresequenzen, Nukleinsäurekonstrukte und/oder Vektoren.

Ein weiterer Teil der Erfindung betrifft Öle, Lipide und/oder Fettsäuren hergestellt nach dem erfindungsgemäßen Verfahren und deren Verwendung. Außerdem betrifft die Erfindung ungesättigte Fettsäuren sowie Triglyceride mit einem erhöhten Gehalt an ungesättigten Fettsäuren und deren Verwendung.

Fettsäuren und Triacylglyceride haben eine Vielzahl von Anwendungen in der Lebensmittelindustrie, der Tierernährung, der Kosmetik und im Pharmabereich.

Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfachungesättigte Fettsäuren wie Linol- und Linolensäure sind für Säugetiere essentiell, da sie nicht von diesen selbst hergestellt werden können. Deshalb stellen mehrfach ungesättigte ω-3-Fettsäuren und ω-6-Fettsäuren einen wichtigen Bestandteil der tierischen und menschlichen Nahrung dar.

Mehrfach ungesättigte langkettige ω-3-Fettsäuren wie Eicosapentaensäure (= EPA, $C20{:}5^{\Delta5,8,11,14,17}$) oder Docosahexaensäure (= DHA, $C22{:}6^{\Delta4,7,10,13,16,19}$) sind wichtige Komponenten der menschlichen Ernährung aufgrund ihrer verschiedenen Rollen in der Gesundheit, die Aspekte wie die Entwicklung des kindlichen Gehirns, der Funktionalität des Auges, der Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, $C22{:}6^{\Delta4,7,10,13,16,19}$) oder Eisosapentaensäure (= EPA, $C20{:}5^{\Delta5,8,11,14,17}$) Babynahrung zur Erhöhung des Nährwertes zugesetzt. Der ungesättigten Fettsäure DHA wird dabei ein positiver Effekt auf die Entwicklung und Aufrechterhaltung von Gehirnfunktionen zugeschrieben.

Im folgenden werden mehrfach ungesättigte Fettsäuren als PUFA, PUFAs, LCPUFA oder LCPUFAs bezeichnet (**p**oly **u**nsaturated **f**atty **a**cids, **PUFA,** mehrfach ungesättigte Fettsäuren; **l**ong **c**hain **p**oly **u**nsaturated **f**atty **a**cids, **LCPUFA,** langkettige mehrfach ungesättigte Fettsäuren).

Hauptsächlich werden die verschiedenen Fettsäuren und Triglyceride aus Mikroorganismen wie Mortierella oder Schizochytrium oder aus Öl-pröduzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie DHA, EPA, Arachidonsäure (= ARA, $C20{:}4^{\Delta5,8,11,14}$), Dihomo-γ-linolensäure ($C20{:}3^{\Delta8,11,14}$) oder Docosapentaensäure (DPA, $C22{:}5^{\Delta7,10,13,16.19}$) werden in Ölfruchtpflanzen wie Raps, Soja, Sonnenblume, Färbersaflor nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander

oder Thunfisch oder Algen.

Je nach Anwendungszweck werden Öle mit gesättigten oder ungesättigten Fettsäuren bevorzugt. So werden z.B. in der humanen Ernährung Lipide mit ungesättigten Fettsäuren speziell mehrfach ungesättigten Fettsäuren bevorzugt Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche speziell chronisch entzündliche Prozesse im Rahmen immunologischer Erkrankungen wie rheumatroider Arthritis lassen sich durch ω-3-Fettsäuren positiv beeinflussen. Sie werden deshalb Lebensmitteln speziell diätischen Lebensmitteln zugegeben oder finden in Medikamenten Anwendung. ω-6-Fettsäuren wie Arachidonsäure haben bei diesen rheumatischen Erkrankungen aufgrund unserer üblichen Nahrungsmittelzusammensetzung eher einen negativen Effekt auf diese Krankheiten.

ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, den Thromoxanen und Leukotrienen, die sich von der Arachidonsäure und der Eicosapentaensäure ableiten. Eicosanoide (sog. $PG_2$-Serie), die aus ω-6-Fettsäuren gebildet werden fördern in der Regel Entzündungsreaktionen, während Eicosanoide (sog. $PG_3$-Serie) aus ω-3-Fettsäuren geringe oder keine entzündungsfördernde Wirkung haben.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben und auch die Anwendung zur Produktion in transgenen Organismen beschrieben wie in WO98/46763 WO98/46764, WO9846765. Dabei wird auch die Expression verschiedener Desaturasen wie in WO99/64616 oder WO98/46776 und Bildung polyungesättigter Fettsäuren beschrieben und beansprucht. Bzgl. der Effektivität der Expression von Desaturasen und ihren Einfluss auf die Bildung polyungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden. Weiterhin wurde in der Regel ein Gemisch aus ω-3- und ω-6-Fettsäuren erhalten.

Besonders geeignete Mikroorganismen zur Herstellung von PUFAs sind Mikroorganismen wie Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moosen wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wann immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Wobei diese in der Regel je nach verwendeten Mikroorganismus als Fettsäuregemische aus beispielsweise EPA, DPA und ARA anfallen.

Für die Synthese von Arachidonsäure, Eicosapentaensäure (EPA) und Docosahexaensäure (DHA) werden verschiedene Synthesewege diskutiert (Figur. 1). So erfolgt die Produktion von EPA bzw. DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731, 1997).

Ein alternative Strategie verläuft über die wechselnde Aktivität von Desaturasen und Elongasen (Zank, T.K. et al. Plant Journal 31:255-268, 2002; Sakuradani, E. et al. Gene 238:445-453, 1999). Eine Modifikation des beschriebenen Weges über Δ6-Desaturase, Δ6-Elongase, Δ5-Desaturase, Δ5-Elongase, Δ4-Desaturase ist der Sprecher-Syntheseweg (Sprecher 2000, Biochim. Biophys. Acta 1486:219-231) in Säugetieren. Anstelle der Δ4-Desaturierung erfolgt hier ein weiterer Elongationsschritt auf $C_{24}$, eine weitere Δ6-Desaturierung und abschliessend eine β-Oxidation auf die $C_{22}$-Kettenlänge. Für die Herstellung in Pflanzen und Mikroorganismen ist der sogenannte Sprecher-Syntheseweg (siehe Figur 1) allerdings nicht geeignet, da die Regulationsmechanismen nicht bekannt sind.

Die polyungesättigten Fettsäuren können entsprechend ihrem Desaturierungsmuster in zwei große Klassen, in ω-6- oder ω-3-Fettsäuren eingeteilt werden, die metabolisch und funktionell unterschiedlich Aktivitäten haben (Fig. 1).

Als Ausgangsprodukt für den w-6-Stoffwechselweg fungiert die Fettsäure Linolsäure ($18:2^{\Delta 9,12}$), während der ω-3-Weg über Linolensäure ($18:3^{\Delta 9,12,15}$) abläuft. Linolensäure wird dabei durch Aktivität einer ω-3-Desaturase gebildet (Tocher et al. 1998, Prog. Lipid Res. 37, 73-117 ; Domergue et al. 2002, Eur. J. Biochem. 269, 4105-4113).

Säugetiere und damit auch der Mensch verfügen über keine entsprechende Desaturaseaktivität (Δ-12- und ω-3-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen polyungesättigten Fettsäuren Arachidonsäure (= ARA, $20:4^{\Delta 5,8,11,14}$), eine ω-6-Fettsäure und die beiden ω-3-Fettsäuren Eicosapentaen- (= EPA, $20:5^{\Delta 5,8,11,14,17}$) und Docosahexaensäure (DHA, $22:6^{\Delta 4,7,10,13,17,19}$) synthetisiert. Die Applikation von ω-3-Fettsäuren zeigt dabei die wie oben beschrieben therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthridis (Cleland und James 2000, J. Rheumatol. 27, 2305-2307).

Aus ernährungsphysiologischer Sicht ist es deshalb wichtig bei der Synthese mehrfach ungesättigter Fettsäuren eine Verschiebung zwischen dem ω-6-Syntheseweg und dem ω-3-Syntheseweg (siehe Figur 1) zu erreichen, so dass mehr ω-3-Fettsäuren hergestellt werden. In der Literatur wurden die enzymatischen Aktivitäten verschiedener ω-3-Desaturasen beschrieben, die $C_{18:2}$-, $C_{22:4}$- oder $C_{22:5}$-Fettsäuren desaturieren (siehe Figur 1). Keine der biochemisch beschriebenen Desaturasen setzt jedoch ein breites Substratspektrum des ω-6-Synthesewegs zu den entsprechenden Fettsäuren des ω-3-Syntheseweg um.

Es besteht daher weiterhin ein großer Bedarf an einer ω-3-Desaturase, die zur Herstellung von ω-3-polyungesättigte Fettsäuren geeignet ist. Alle bekannten pflanzlichen und cyanobakteriellen ω-3-Desaturasen desaturieren $C_{18}$-Fettsäuren mit Linolsäure als Substrat, können aber keine $C_{20}$- oder $C_{22}$-Fettsäuren desaturieren.

Von dem Pilz Saprolegnia dicilina ist eine ω-3-Desaturase bekannt [Pereira et al. 2004, Biochem. J. 378(Pt 2):665-71], die $C_{20}$-mehrfach ungesättigte Fettsäuren desaturieren kann. Von Nachteil ist jedoch, dass diese ω-3-Desaturase keine $C_{18}$- oder $C_{22}$-PUFAs, wie den wichtigen Fettsäuren $C_{18:2}$-, $C_{22:4}$- oder $C_{22:5}$-Fettsäuren des ω-6-Syntheseweg desaturieren kann. Ein weiterer Nachteil dieses Enzyms ist, dass es keine Fettsäuren desaturieren kann, die an Phospholipide gebunden sind. Es werden nur die CoA-Fettsäureester umgesetzt.

Die Verlängerung von Fettsäuren durch Elongasen um 2 bzw. 4 C-Atome ist für die Produktion von $C_{20}$- bzw. $C_{22}$-PUFAs von entscheidender Bedeutung. Dieser Prozess verläuft über 4 Stufen. Der erste Schritt stellt die Kondensation von Malonyl-CoA an das Fettsäure-Acyl-CoA durch die Ketoacyl-CoA-Synthase (KCS, im weiteren Text als Elongase bezeichnet). Es folgt dann ein Reduktionschritt (Ketoacyl-CoA-Reduktase, KCR), ein Dehydratationsschritt (Dehydratase) und ein abschliessender Reduktionsschritt (enoyl-CoA-Reduktase). Es wurde postuliert, dass die Aktivität der Elongase die Spezifität und Geschwindigkeit des gesamten Prozesses beeinflussen (Millar and Kunst, 1997 Plant Journal 12:121-131).

In der Vergangenheit wurden zahlreiche Versuche unternommen, Elongase Gene zu erhalten. Millar and Kunst, 1997 (Plant Journal 12:121-131) und Millar et al. 1999, (Plant Cell 11:825-838) beschreiben die Charakterisierung von pflanzlichen Elongasen zur Synthese von einfachungesättigten langkettigen Fettsäuren (C22:1) bzw. zur Synthese von sehr langkettigen Fettsäuren für die Wachsbildung in Pflanzen ($C_{28}$-$C_{32}$). Beschreibungen zur Synthese von Arachidonsäure und EPA finden sich beispielsweise in WO0159128, WO0012720, WO02077213 und WO0208401. Die Synthese von mehrfachungesättigter C24 Fettsäuren ist beispielsweise in Tvrdik et al 2000, JCB 149:707-717 oder WO0244320 beschrieben.

Zur Herstellung von DHA (C22:6 n-3) in Organismen, die diese Fettsäure natürlicherweise nicht produzieren, wurde bisher keine spezifische Elongase beschrieben. Bisher wurden nur Elongasen beschrieben, die $C_{20}$- bzw. $C_{24}$-Fettsäuren bereitstellen. Eine Δ-5-Elongase-Aktivität wurde bisher noch nicht beschrieben.

Höhere Pflanzen enthalten mehrfach ungesättigte Fettsäuren wie Linolsäure (C18:2) und Linolensäure (C18:3). ARA, EPA und DHA kommen im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Hierzu müssen vorteilhaft über gentechnische Methoden Gene kodierend für Enzyme der Biosynthese von LCPUFAs in Ölsaaten eingeführt und exprimiert werden. Dies sind Gene, die beispielsweise für Δ-6-Desaturasen, Δ-6-Elongasen, Δ-5-Desaturasen oder Δ-4-Desaturasen codieren. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert.

Erste transgene Pflanzen, die Gene kodierend für Enzyme der LCPUFA-Biosynthese enthalten und exprimieren und LCPUFAs produzieren wurden beispielsweise in DE 102 19 203 (Verfahren zur Herstellung mehrfach ungesättigter Fettsäuren in Pflanzen) erstmals beschrieben. Diese Pflanzen produzieren allerdings LCPUFAs in Mengen, die für eine

Aufarbeitung der in den Pflanzen enthaltenen Öle noch weiter optimiert werden müssen.

Um eine Anreicherung der Nahrung und/oder des Futters mit diesen mehrfach ungesättigten Fettsäuren zu ermöglichen, besteht daher ein großer Bedarf an einem einfachen, kostengünstigen Verfahren zur Herstellung dieser mehrfach ungesättigten Fettsäuren speziell in eukaryontischen Systemen.

Es bestand daher die Aufgabe weitere Gene bzw. Enzyme, die für die Synthese von LCPUFAs geeignet sind, speziell Gene, die eine Δ-5-Elongase-, eine Δ-5-Desaturase-, Δ-4-Desaturase-, Δ-12-Desaturase- oder Δ-6-Desaturaseaktivität aufweisen, für die Herstellung von mehrfach ungesättigten Fettsäuren zur Verfügung zu stellen. Eine weitere Aufgabe dieser Erfindung war die Bereitstellung von Genen bzw. Enzymen, die eine Verschiebung von den ω-6-Fettsäuren zu den ω-3-Fettsäuren hin ermöglichen. Weiterhin bestand die Aufgabe ein Verfahren zur Herstellung von mehrfach ungesättigten Fettsäuren in einem Organismus vorteilhaft in einem eukaryontischen Organismus bevorzugt in einer Pflanze oder einem Mikroorganismus zu entwickeln. Diese Aufgabe wurde durch das erfindungsgemäße Verfahren zur Herstellung von Verbindungen der allgemeinen Formel I

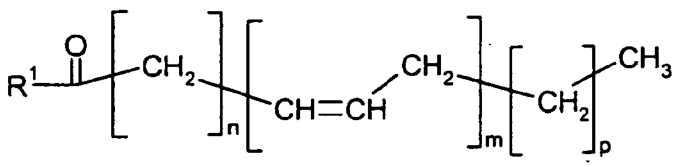 (I)

in transgenen Organismen mit einem Gehalt von mindestens 1 Gew.-% dieser Verbindungen bezogen auf den Gesamtlipidgehalt des transgenen Organismus, dadurch gekennzeichnet, dass es folgende Verfahrensschritte umfasst:

a) Einbringen mindestens einer Nukleinsäuresequenz in den Organismus, welche für eine Δ-9-Elongase- und/oder eine Δ-6-Desaturase-Aktivität codiert, und

b) Einbringen mindestens einer Nukleinsäuresequenz in den Organismus, welche für eine Δ-8-Desaturase- und/oder eine Δ-6-Elongase-Aktivität codiert, und

c) Einbringen mindestens einer Nukleinsäuresequenz in den Organismus, welche für eine Δ-5-Desaturase-Aktivität codiert, und

d) Einbringen mindestens einer Nukleinsäuresequenz in den Organismus, welche für eine Δ-5-Elongase-Aktivität codiert, und

e) Einbringen mindestens einer Nukleinsäuresequenz in den Organismus, welche für eine Δ-4-Desaturase-Aktivität codiert, und

wobei die Variablen und Substituenten in der Formel I die folgende Bedeutung haben:

$R^1$ = Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

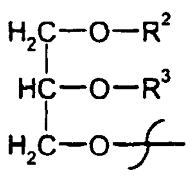 (II)

$R^2$ = Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes $C_2$-$C_{24}$-Alkylcarbonyl-,

$R^3$ = Wasserstoff-, gesättigtes oder ungesättigtes $C_2$-$C_{24}$-Alkylcarbonyl-, oder $R^2$ oder $R^3$ unabhängig voneinander einen Rest der allgemeinen Formel Ia:

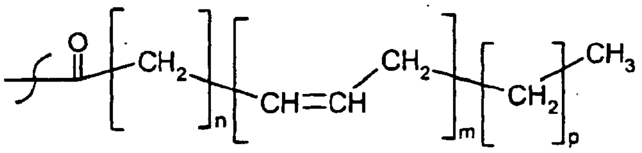 (Ia)

n = 2, 3, 4, 5, 6, 7 oder 9, m = 2, 3, 4, 5 oder 6 und p = 0 oder 3, gelöst.

$R^1$ bedeutet in der allgemeinen Formel I Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

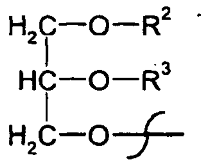 (II)

Die oben genannten Reste von $R^1$ sind immer in Form ihrer Thioester an die Verbindungen der allgemeinen Formel I gebunden.

$R^2$ bedeutet in der allgemeinen Formel II Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes $C_2$-$C_{24}$-Alkylcarbonyl-,
Als Alkylreste seien substituiert oder unsubstituiert, gesättigt oder ungesättigte $C_2$-$C_{24}$-Alkylcarbonyl-Ketten wie Ethylcarbonyl-, n-Propylcarbonyl-, n-Butylcarbonyl-, n-Pentylcarbonyl-, n-Hexylcarbonyl-, n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- or n-Tetracosanylcarbonyl- genannt, die ein oder mehrere Doppelbindungen enthalten. Gesättigte oder ungesättigte $C_{10}$-$C_{22}$-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl-., die ein oder mehrere Doppelbindungen enthalten, sind bevorzugt. Besonders bevorzugt sind gesättigte und/oder ungesättigte $C_{10}$-$C_{22}$-Alkylcarbonylreste wie $C_{10}$-Alkylcarbonyl-, $C_{11}$-Alkylcarbonyl-, $C_{12}$-Alkylcarbonyl-, $C_{13}$-Alkylcarbonyl-, $C_{14}$-Alkylcarbonyl-, $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Ganz besonders bevorzugt sind gesättigte oder ungesättigte $C_{16}$-$C_{22}$-Alkylcarbonylreste wie $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt fünf oder sechs Doppelbindungen. Alle genannten Reste leiten sich von den entsprechenden Fettsäuren ab.

$R^3$ bedeutet in der allgemeinen Formel II Wasserstoff-, gesättigtes oder ungesättigtes $C_2$-$C_{24}$-Alkylcarbonyl.

Als Alkylreste seien substituiert oder unsubstituiert, gesättigt oder ungesättigte $C_2$-$C_{24}$-Alkylcarbonyl-Ketten wie Ethylcarbonyl-, n-Propylcarbonyl-, n-Butylcarbonyl-, n-Pentylcarbonyl-, n-Hexylcarbonyl-,n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- or n-Tetracosanylcarbonyl- genannt, die ein oder mehrere Doppelbindungen enthalten. Gesättigte oder ungesättigte $C_{10}$-$C_{22}$-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- oder n-Tetracosanylcarbonyl-, die ein oder mehrere Doppelbindungen enthalten, sind bevorzugt. Besonders bevorzugt sind gesättigte und/oder ungesättigte $C_{10}$-$C_{22}$-Alkylcarbonylreste wie $C_{10}$-Alkylcarbonyl-, $C_{11}$-Alkylcarbonyl-, $C_{12}$-Alkylcarbonyl-, $C_{13}$-Alkylcarbonyl-, $C_{14}$-Alkylcarbonyl-, $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Ganz besonders bevorzugt sind gesättigte oder unge sättigte $C_{16}$-$C_{22}$-Alkylcarbonylreste wie $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Diese vorteilhaften Reste können zwei, drei,

vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt fünf oder sechs Doppelbindungen. Alle genannten Reste leiten sich von den entsprechenden Fettsäuren ab.

Die oben genannten Reste von $R^1$, $R^2$ and $R^3$ können mit Hydroxyl- und/oder Epoxygruppen substituierte sein und/oder können Dreifachbindungen enthalten.

Vorteilhaft enthalten die im erfindungsgemäßen Verfahren hergestellten mehrfach ungesättigten Fettsäuren mindestens zwei vorteilhaft drei, vier, fünf oder sechs Doppelbindungen. Besonders vorteilhaft enthalten die Fettsäuren vier fünf oder sechs Doppelbindungen. Im Verfahren hergestellte Fettsäuren haben vorteilhaft 18-, 20- oder 22-C-Atome in der Fettsäurekette, bevorzugt enthalten die Fettsäuren 20 oder 22 Kohlenstoffatome in der Fettsäurekette. Vorteilhaft werden gesättigte Fettsäuren mit den im Verfahren verwendeten Nukleinsäuren wenig oder gar nicht umgesetzt. Unter wenig ist zu verstehen, das im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 %, ganz besonders bevorzugt mit weniger als 1; 0,5; 0,25 oder 0,125 % umgesetzt werden. Diese hergestellten Fettsäuren können als einziges Produkt im Verfahren hergestellt werden oder in einem Fettsäuregemisch vorliegen.

Bei den im erfindungsgemäßen Verfahren verwendeten Nukleinsäuresequenzen handelt es sich um isolierte Nukleinsäuresequenzen, die für Polypeptide mit Δ-9-Elongase-, Δ-6-Desaturase-, Δ-8-Desaturase-, Δ-6-Elongase-, Δ-5-Desaturase-, Δ-5-Elongase- und/oder Δ-4-Desaturaseaktivität codieren.

Vorteilhaft werden im erfindungsgemäßen Verfahren Nukleinsäuresequenzen, die für Polypeptide mit Δ-9-Elongase-, Δ-6-Desaturase-, Δ-8-Desaturase-, Δ-6-Elongase-, Δ-5-Desaturase-, Δ-5-Elongase- oder Δ-4-Desaturaseaktivität codieren, verwendet ausgewählt aus der Gruppe bestehend aus:

a) einer Nukleinsäuresequenz mit der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 dargestellten Sequenz, oder

b) Nukleinsäuresequenzen, die sich als Ergebnis des degenerierten genetischen Codes von den in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138 oder SEQ ID NO: 184 dargestellten Aminosäuresequenzen ableiten lassen, oder

c) Derivate der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 dargestellten Nukleinsäuresequenz, die für Polypeptide mit mindestens 40 % Identität auf Aminosäureebene mit SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO:

44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138 oder SEQ ID NO: 184 codieren und eine Δ-9-Elongase-, Δ-6-Desaturase-, Δ-8-Desaturase-, Δ-6-Elongase-, Δ-5-Desaturase-, Δ-5-Elongase- oder Δ-4-Desaturaseaktivität aufweisen.

Vorteilhaft bedeuten die Substituenten $R^2$ oder $R^3$ in den allgemeinen Formeln I und II unabhängig voneinander gesättigtes oder ungesättigtes $C_{18}$-$C_{22}$-Alkylcarbonyl-, besonders vorteilhaft bedeuten sie unabhängig voneinander ungesättigtes $C_{18}$-, $C_{20}$- oder $C_{22}$-Alkylcarbonyl- mit mindestens zwei Doppelbindungen.

Eine bevorzugte Ausführungsform des Verfahrens ist dadurch gekennzeichnet, dass eine Nukleinsäuresequenz zusätzlich in den Organismus eingebracht wird, die für Polypeptide mit ω-3-Desaturase-Aktivität codiert, ausgewählt aus der Gruppe bestehend aus:

a) einer Nukleinsäuresequenz mit der in SEQ ID NO: 87 oder SEQ ID NO: 105 dargestellten Sequenz, oder

b) Nukleinsäuresequenzen, die sich als Ergebnis des degenerierten genetischen Codes von der in SEQ ID NO: 88 oder SEQ ID NO: 106 dargestellten Aminösäuresequenz ableiten lassen, oder

c) Derivate der in SEQ ID NO: 87 oder SEQ ID NO: 105 dargestellten Nukleinsäuresequenz, die für Polypeptide mit mindestens 60 % Identität auf Aminosäureebene mit SEQ ID NO: 88 oder SEQ ID NO: 106 codieren und eine ω3-Desaturaseaktivität aufweisen.

In einer weiteren bevorzugten Ausführungsform ist das Verfahren dadurch gekennzeichnet, dass eine Nukleinsäuresequenz zusätzlich in den Organismus eingebracht wird, die für Polypeptide mit Δ-12-Desaturaseaktivität codiert, ausgewählt aus der Gruppe bestehend aus:

a) einer Nukleinsäuresequenz mit der in SEQ ID NO: 107 oder SEQ ID NO: 109 dargestellten Sequenz, oder

b) Nukleinsäuresequenzen, die sich als Ergebnis des degenerierten genetischen Codes von der in SEQ ID NO: 108 oder SEQ ID NO: 110 dargestellten Aminosäuresequenz ableiten lassen, oder

c) Derivate der in SEQ ID NO: 107 oder SEQ ID NO: 109 dargestellten Nukleinsäuresequenz, die für Polypeptide mit mindestens 60 % Identität auf Aminosäureebene mit SEQ ID NO: 108 oder SEQ ID NO: 110 codieren und eine Δ-12-Desaturaseaktivität aufweisen.

Diese vorgenannten Δ-12-Desaturasesequenzen können allein oder in Kombination mit den ω3-Desaturasesequenzen mit den im Verfahren verwendeten Nukleinsäuresequenzen, die für Δ-9-Elongasen, Δ-6-Desaturasen, Δ-8-Desaturasen, Δ-6-Elongasen, Δ -5-Desaturasen, Δ-5-Elongasen und/oder Δ-4-Desaturasen codieren verwendet werden.

Tabelle 1 gibt die Nukleinsäuresequenzen, den Herkunftsorganismus und die Sequenz-D-Nummer wieder.

| Nr. | Organismus | Aktivität | Sequenznummer |
|---|---|---|---|
| 1. | Euglena gracilis | Δ-8-Desaturase | SEQ ID NO: 1 |
| 2. | Isochrysis galbana | Δ-9-Elongase | SEQ ID NO: 3 |
| 3. | Phaeodactylum tricornutum | Δ-5-Desaturase | SEQ ID NO: 5 |
| 4. | Ceratodon purpureus | Δ-5-Desaturase | SEQ ID NO: 7 |
| 5. | Physcomitrella patens | Δ-5-Desaturase | SEQ ID NO: 9 |
| 6. | Thraustrochytrium sp. | Δ-5-Desaturase | SEQ ID NO: 11 |
| 7. | Mortierella alpina | Δ-5-Desaturase | SEQ ID NO: 13 |
| 8. | Caenorhabditis elegans | Δ-5-Desaturase | SEQ ID NO: 15 |
| 9. | Borago officinalis | Δ-6-Desaturase | SEQ ID NO: 17 |
| 10. | Ceratodon purpureus | Δ-6-Desaturase | SEQ ID NO: 19 |

(fortgesetzt)

| Nr. | Organismus | Aktivität | Sequenznummer |
|---|---|---|---|
| 11. | Phaeodactylum tricornutum | Δ-6-Desaturase | SEQ ID NO: 21 |
| 12. | Physcomitrella patens | Δ-6-Desaturase | SEQ ID NO: 23 |
| 13. | Caenorhabditis elegans | Δ-6-Desaturase | SEQ ID NO: 25 |
| 14. | Physcomitrella patens | Δ-6-Elongase | SEQ ID NO: 27 |
| 15. | Thraustrochytrium sp. | Δ-6-Elongase | SEQ ID NO: 29 |
| 16. | Phytophtora infestans | Δ-6-Elongase | SEQ ID NO: 31 |
| 17. | Mortierella alpina | Δ-6-Elongase | SEQ ID NO: 33 |
| 18. | Mortierella alpina | Δ-6-Elongase | SEQ ID NO: 35 |
| 19. | Caenorhabditis elegans | Δ-6-Elongase | SEQ ID NO: 37 |
| 20. | Euglena gracilis | Δ-4-Desaturase | SEQ ID NO: 39 |
| 21. | Thraustrochytrium sp. | Δ-4-Desaturase | SEQ ID NO: 41 |
| 22. | Thalassiosira pseudonana | Δ-5-Elongase | SEQ ID NO: 43 |
| 23. | Thalassiosira pseudonana | Δ-6-Elongase | SEQ ID NO: 45 |
| 24. | Crypthecodinium cohnii | Δ-5-Elongase | SEQ ID NO: 47 |
| 25. | Crypthecodinium cohnii | Δ-5-Elongase | SEQ ID NO: 49 |
| 26. | Oncorhynchus mykiss | Δ-5-Elongase | SEQ ID NO: 51 |
| 27. | Oncorhynchus mykiss | Δ-5-Elongase | SEQ ID NO: 53 |
| 28. | Thalassiosira pseudonana | Δ-5-Elongase | SEQ ID NO: 59 |
| 29. | Thalassiosira pseudonana | Δ-5-Elongase | SEQ ID NO: 61 |
| 30. | Thalassiosira pseudonana | Δ-5-Elongase | SEQ ID NO: 63 |
| 31. | Thraustrochytrium aureum | Δ-5-Elongase | SEQ ID NO: 65 |
| 32. | Ostreococcus tauri | Δ-5-Elongase | SEQ ID NO: 67 |
| 33. | Ostreococcus tauri | Δ-6-Elongase | SEQ ID NO: 69 |
| 34. | Primula farinosa | Δ-6-Desaturase | SEQ ID NO: 71 |
| 35. | Primula vialii | Δ-6-Desaturase | SEQ ID NO: 73 |
| 36. | Ostreococcus tauri | Δ-5-Elongase | SEQ ID NO: 75 |
| 37. | Ostreococcus tauri | Δ-5-Elongase | SEQ ID NO: 77 |
| 38. | Ostreococcus tauri | Δ-5-Elongase | SEQ ID NO: 79 |
| 39. | Ostreococcus tauri | Δ-6-Elongase | SEQ ID NO: 81 |
| 40. | Thraustrochytrium sp. | Δ-5-Elongase | SEQ ID NO: 83 |
| 41. | Thalassiosira pseudonana | Δ-5-Elongase | SEQ ID NO: 85 |
| 42. | Phytophtora infestans | ω-3-Desaturase | SEQ ID NO: 87 |
| 43. | Ostreococcus tauri | Δ-6-Desaturase | SEQ ID NO: 89 |
| 44. | Ostreococcus tauri | Δ-5-Desaturase | SEQ ID NO: 91 |
| 45. | Ostreococcus tauri | Δ-5-Desaturase | SEQ ID NO: 93 |
| 46. | Ostreococcus tauri | Δ-4-Desaturase | SEQ ID NO: 95 |
| 47. | Thalassiosira pseudonana | Δ-6-Desaturase | SEQ ID NO: 97 |
| 48. | Thalassiosira pseudonana | Δ-5-Desaturase | SEQ ID NO: 99 |

(fortgesetzt)

| Nr. | Organismus | Aktivität | Sequenznummer |
|---|---|---|---|
| 49. | Thalassiosira pseudonana | Δ-5-Desaturase | SEQ ID NO: 101 |
| 50. | Thalassiosira pseudonana | Δ-4-Desaturase | SEQ ID NO: 103 |
| 51. | Thalassiosira pseudonana | ω-3-Desaturase | SEQ ID NO: 105 |
| 52. | Ostreococcus tauri | Δ-12-Desaturase | SEQ ID NO: 107 |
| 53. | Thalassiosira pseudonana | Δ-12-Desaturase | SEQ ID NO: 109 |
| 54. | Ostreococcus tauri | Δ-6-Elongase | SEQ ID NO: 111 |
| 55. | Ostreococcus tauri | Δ-5-Elongase | SEQ ID NO: 113 |
| 56. | Xenopus laevis (BC044967) | Δ-5-Elongase | SEQ ID NO: 117 |
| 57. | Ciona intestinalis (AK112719) | Δ-5-Elongase | SEQ ID NO: 119 |
| 58. | Euglena gracilis | Δ-5-Elongase | SEQ ID NO: 131 |
| 59. | Euglena gracilis | Δ-5-Elongase | SEQ ID NO:133 |
| 60. | Arabidopsis thaliana | Δ-5-Elongase | SEQ ID NO: 135 |
| 61. | Arabidopsis thaliana | Δ-5-Elongase | SEQ ID NO: 137 |
| 62. | Phaeodactylum tricornutum | Δ-6-Elongase | SEQ ID NO: 183 |

Die im Verfahren hergestellten mehrfach ungesättigten Fettsäuren sind vorteilhaft in Membranlipiden und/oder Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Glyceride vorliegen. Die in den Triacylglyceriden gebundenen verschieden Fettsäuren lassen sich dabei von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind die langkettigen Fettsäuren besonders bevorzugt sind die langkettigen Fettsäuren LCPUFAs von $C_{18}$-. $C_{20}$- und/oder $C_{22}$-Fettsäuren.

Im erfindungsgemäßen Verfahren werden vorteilhaft Fettsäureester mit mehrfach ungesättigten $C_{18}$-, $C_{20}$- und/oder $C_{22}$-Fettsäuremolekülen mit mindestens zwei Doppelbindungen im Fettsäureester, vorteilhaft mit mindestens drei, vier, fünf oder sechs Doppelbindungen im Fettsäureester, besonders vorteilhaft von mindestens fünf oder sechs Doppelbindungen im Fettsäureester hergestellt und führen vorteilhaft zur Synthese von Linolsäure (=LA, C18:2$^{\Delta 9,12}$). γ-Linolensäure (= GLA, C18:3$^{\Delta 6,9,12}$), Stearidonsäure (= SDA, C18:4$^{\Delta 6,9,12,15}$). Dihomo-γ-Linolensäure (= DGLA, 20:3$^{\Delta 8,11,14}$), ω -3-Eicosatetraensäure (= ETA, C20:4$^{\Delta 5,8,11,14}$),Arachidonsäure (ARA, C20:4$^{\Delta 5,8,11,14}$), Eicosapentaensäure (EPA, C20:5$^{\Delta 5,8,11,14,17}$), ω-6-Docosapentaensäure (C22:5$^{\Delta 4,7,10,13,16}$), ω-6-Docosatetraensäure (C22:4$^{\Delta ,7,10,13,16}$), ω-3-Docosapentaensäure (= DPA, C22:5$^{\Delta 7,10,13,16,19}$), Docosahexaensäure (= DHA, C22:6$^{\Delta 4,7,10,13,16,19}$) oder deren Mischungen, bevorzugt ARA, EPA und/oder DHA. Ganz besonders bevorzugt werden, ω -3-Fettsäuren wie EPA und/oder DHA hergestellt.

Die Fettsäureester mit mehrfach ungesättigten $C_{18}$-, $C_{20}$- und/oder $C_{22}$-Fettsäuremolekülen können aus den Organismen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipide, Phospholipide wie Phosphatidylethanolamin, Phosphatidylcholin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens zwei, drei, vier, fünf oder sechs bevorzugt fünf oder sechs Doppelbindungen enthalten, isoliert werden, vorteilhaft werden sie in der Form ihrer Diacylglyceride, Triacylglyceride und/oder in Form des Phosphatidylcholin isoliert, besonders bevorzugt in der Form der Triacylglyceride. Neben diesen Estern sind die mehrfach ungesättigten Fettsäuren auch als freie Fettsäuren oder gebunden in anderen Verbindungen in den Organismen vorteilhaft den Pflanzen enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren werden die hergestellten LCPUFAs mit einem Gehalt von mindestens 3

Gew.-%, vorteilhaft von mindestens 5 Gew.-%, bevorzugt von mindestens 8 Gew.-%, besonders bevorzugt von mindestens 10 Gew.-%, ganz besonders bevorzugt von mindestens 15 Gew.-% bezogen auf die gesamten Fettsäuren in den transgenen Organismen vorteilhaft in einer transgenen Pflanze hergestellt. Dabei werden vorteilhaft $C_{18}$- und/oder $C_{20}$-Fettsäuren, die in den Wirtsorganismen vorhanden sind, zu mindestens 10 %, vorteilhaft zu mindestens 20 %, besonders vorteilhaft zu mindestens 30 %, ganz besonders vorteilhaft zu mindestens 40 % in die entsprechenden Produkte wie DPA oder DHA, um nur zwei beispielhaft zu nennen, umgesetzt. Vorteilhaft werden die Fettsäuren in gebundener Form hergestellt. Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA), Eicosapentaensäure (EPA), ω-6-Docosapentaensäure oder DHA nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA, EPA oder DHA als Mischungen vor. Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endprodukts betragen. Vorteilhaft werden in einer transgenen Pflanze als Endprodukte nur ARA, EPA oder nur DHA im erfindungsgemäßen Verfahren gebunden oder als freie Säuren hergestellt. Werden die Verbindungen ARA, EPA und DHA gleichzeitig hergestellt, werden sie vorteilhaft in einem Verhältnis von mindesten 1:1:2 (EPA:ARA:DHA), vorteilhaft von mindestens 1:1:3, bevorzugt von 1:1:4, besonders bevorzugt von 1:1:5 hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopentendodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure), 6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Buttersäure, kein Cholesterin, keine Clupanodonsäure (= Docosapentaensäure, $C22:5^{\Delta 4,8,12,15,21}$) sowie keine Nisinsäure (Tetracosahexaensäure, $C23:6^{\Delta 3,8,12,15,18,21}$).

Durch die erfindungsgemäßen Nukleinsäuresequenzen bzw. im erfindungsgemäßen Verfahren verwendeten Nukleinsäuresequenzen kann eine Steigerung der Ausbeute an mehrfach ungesättigten Fettsäuren von mindestens 50 %, vorteilhaft von mindestens 80 %, besonders vorteilhaft von mindestens 100 %, ganz besonders vorteilhaft von mindestens 150 % gegenüber den nicht transgenen Ausgangsorganismus beispielsweise einer Hefe, einer Alge, einem Pilz oder einer Pflanze wie Arabidopsis oder Lein beim Vergleich in der GC-Analyse siehe Beispiele erreicht werden.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus dem Organismus wie den Mikroorganismen oder den Pflanzen oder dem Kulturmedium, in dem oder auf dem die Organismen angezogen wurden, oder aus dem Organismus und dem Kulturmedium in bekannter Weise beispielsweise über Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Als Organismus für die Herstellung im erfindungsgemäßen Verfahren kommen prinzipiell alle Organismen wie Mikroorganismen, nicht-humane Tiere oder Pflanzen in Frage.

Als Pflanzen kommen prinzipiell alle Pflanzen in Frage, die in der Lage sind Fettsäuren zu synthetisieren wie alle dicotylen oder monokotylen Pflanzen, Algen oder Moose. Vorteilhaft Pflanzen sind ausgewählt aus der Gruppe der Pflanzenfamilien Adelotheciaceae, Anacardiaceae, Asteraceae, Apiaceae, Betulaceae, Boraginaceae, Brassicaceae, Bromeliaceae, Caricaceae, Cannabaceae, Convolvulaceae, Chenopodiaceae, Crypthecodiniaceae, Cucurbitaceae, Ditrichaceae, Elaeagnaceae, Ericaceae, Euphorbiaceae, Fabaceae, Geraniaceae, Gramineae, Juglandaceae, Lauraceae, Leguminosae, Linaceae, Euglenaceae, Prasinophyceae oder Gemüsepflanzen oder .Zierpflanzen wie Tagetes in Betracht.

Beispielhaft seien die folgenden Pflanzen genannt ausgewählt aus der Gruppe: Adelotheciaceae wie die Gattungen Physcomitrella z.B. die Gattung und Arten *Physcomitrella patens,* Anacardiaceae wie die Gattungen Pistacia, Mangifera, Anacardium z.B. die Gattung und Arten *Pistacia vera* [Pistazie], *Mangifer indica* [Mango] oder *Anacardium occidentale* [Cashew], Asteraceae wie die Gattungen Calendula, Carthamus, Centaurea, Cichorium, Cynara, Helianthus, Lactuca, Locusta, Tagetes, Valeriana z.B. die Gattung und Arten Calendula officinalis [Garten-Ringelblume], Carthamus tinctorius [Färberdistel, safflower], *Centaurea cyanus* [Komblume], *Cichorium intybus* [Wegwarte], Cynara scolymus [Artichoke], *Helianthus annus* [Sonnenblume], *Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [Salat], *Tagetes lucida, Tagetes erecta* oder *Tagetes tenuifolia* [Studentenblume], Apiaceae wie die Gattung Daucus z.B. die Gattung und Art *Daucus carota* [Karotte], Betulaceae wie die Gattung Corylus z.B. die Gattungen und Arten *Corylus avellana* oder *Corylus colurna* [Haselnuss], Boraginaceae wie die Gattung Borago z.B. die Gattung und Art *Borago officinalis* [Borretsch], Brassicaceae wie die Gattungen Brassica, Camelina, Melanosinapis, Sinapis, Arabadopsis z.B. die Gattungen und Arten *Brassica napus, Brassica rapa* ssp. [Raps], *Sinapis arvensis Brassica juncea, Brassica juncea var. juncea, Brassica juncea var. crispifolia, Brassica juncea var. foliosa, Brassica nigra, Brassica sinapioides,* Camelina sativa, *Melanosinapis communis* [Senf], *Brassica oleracea* [Futterrübe] oder Arabidopsis thaliana, Bromeliaceae wie die Gattungen Anana, Bromelia (Ananas) z.B. die Gattungen und Arten *Anana comosus, Ananas ananas* oder *Bromelia comosa* [Ananas], Caricaceae wie die Gattung Carica wie die Gattung und Art *Carica papaya* [Papaya], Cannabaceae wie die Gattung Cannabis wie die Gattung und Art *Cannabis sative* [Hanf], Convolvulaceae wie die Gattungen Ipomea, Convolvulus z.B. die Gattungen und Arten *Ipomoea batatus, Ipomoea pandurata, Convolvulus batatas, Convolvulus tiliaceus, Ipomoea fastigiata, Ipomoea tiliacea, Ipomoea triloba* oder *Convolvulus panduratus* [Süßkartoffel, Batate], Chenopodiaceae wie die Gattung Beta wie die Gattungen und Arten *Beta vulgaris, Beta vulgaris var. altissima, Beta vulgaris var. Vulgaris, Beta maritima, Beta vulgaris var. perennis, Beta vulgaris var. conditiva* oder *Beta vulgaris var. esculenta* [Zuckerrübe], Crypthecodiniaceae wie die Gattung Crypthecodinium z.B. die Gattung und Art *Cryptecodinium cohnii,* Cucurbitaceae wie die Gattung Cucubita z.B. die Gattungen und Arten *Cucurbita maxima, Cucurbita mixta, Cucurbita pepo* oder *Cucurbita moschata* [Kürbis], Cymbellaceae wie die Gattungen Amphora, Cymbella, Okedenia, Phaeodactylum, Reimeria z.B. die Gattung und Art *Phaeodactylum tricornutum,* Ditrichaceae wie die Gattungen Ditrichaceae, Astomiopsis, Ceratodon, Chrysoblastella, Ditrichum, Distichium, Eccremidium, Lophidion, Philibertiella, Pleuridium, Saelania, Trichodon, Skottsbergia z.B. die Gattungen und Arten *Ceratodon antarcticus, Ceratodon columbiae, Ceratodon heterophyllus, Ceratodon purpurascens, Ceratodon purpureus, Ceratodon purpureus ssp. convolutus, Ceratodon purpureus ssp. stenocarpus, Ceratodon purpureus var. rotundifolius, Ceratodon ratodon, Ceratodon stenocarpus, Chrysoblastella chilensis, Ditrichum ambiguum, Ditrichum brevisetum, Ditrichum crispatissimum, Ditrichum difficile, Ditrichum falcifolium, Ditrichum flexicaule, Ditrichum giganteum, Ditrichum heteromallum, Ditrichum lineare, Ditrichum lineare, Ditrichum montanum, Ditrichum montanum, Ditrichum pallidum, Ditrichum punctulatum, Ditrichum pusillum, Ditrichum pusillum var. tortile, Ditrichum rhynchostegium, Ditrichum schimperi, Ditrichum tortile, Distichium capillaceum, Distichium hagenii, Distichium inclinatum, Distichium macounii, Eccremidium floridanum, Eccremidium whiteleggei, Lophidion strictus, Pleuridium acuminatum, Pleuridium alternifolium, Pleuridium holdridgei, Pleuridium mexicanum, Pleuridium ravenelii, Pleuridium subulatum, Saelania glaucescens, Trichodon borealis, Trichodon cylindricus oder Trichodon cylindricus var. oblongus,* Elaeagnaceae wie die Gattung Elaeagnus z.B. die Gattung und Art *Olea europaea* [Olive], Ericaceae wie die Gattung Kalmia z.B. die Gattungen und Arten *Kalmia latifolia, Kalmia angustifolia, Kalmia microphylla, Kalmia polifolia, Kalmia occidentalis, Cistus chamaerhodendros* oder *Kalmia lucida* [Berglorbeer], Euglenaceae wie die Gattungen Ascoglena, Astasia, Colacium, Cyclidiopsis, Euglena, Euglenopsis, Hyalaphacus, Khawkinea, Lepocinclis, Phacus, Strombomonas, Trachelomonas z.B. die Gattung und Art Euglena gracilis; Euphorbiaceae wie die Gattungen Manihot, Janipha, Jatropha, Ricinus z.B. die Gattungen und Arten *Manihot utilissima, Janipha manihot,, Jatropha manihot., Manihot aipil, Manihot dulcis, Manihot manihot, Manihot melanobasis, Manihot esculenta* [Manihot] oder *Ricinus communis* [Rizinus], Fabaceae wie die Gattungen Pisum, Albizia, Cathormion, Feuillea, Inga, Pithecolobium, Acacia, Mimosa, Medicajo, Glycine, Dolichos, Phaseolus, Soja z.B. die Gattungen und Arten *Pisum sativum, Pisum arvense, Pisum humile* [Erbse], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithe-*

*cellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [Seidenbaum], *Medicago sativa, Medicago falcata, Medicago varia* [Alfalfa] *Glycine max Dolichos soja, Glycine gracilis, Glycine hispida, Phaseolus max, Soja hispida* oder *Soja max* [Sojabohne], Funariaceae wie die Gattungen Aphanorrhegma, Entosthodon, Funaria, Physcomitrella, Physcomitrium z.B. die Gattungen und Arten *Aphanorrhegma serratum, Entosthodon attenuatus, Entosthodon bolanderi, Entosthodon bonplandii, Entosthodon californicus, Entosthodon drummondii, Entosthodon jamesonii, Entosthodon leibergii, Entosthodon neoscoticus, Entosthodon rubrisetus, Entosthodon spathulifolius, Entosthodon tucsoni, Funaria americana, Funaria bolanderi, Funaria calcarea, Funaria californica, Funaria calvescens, Funaria convoluta, Funaria flavicans, Funaria groutiana, Funaria hygrometrica, Funaria hygrometrica var. arctica, Funaria hygrometrica var. calvescens, Funaria hygrometrica var. convoluta, Funaria hygrometrica var. muralis, Funaria hygrometrica var. utahensis, Funaria microstoma, Funaria microstoma var. obtusifolia, Funaria muhlenbergii, Funaria orcuttii, Funaria plano-convexa, Funaria polaris, Funaria ravenelii, Funaria rubriseta, Funaria serrata, Funaria sonorae, Funaria sublimbatus, Funaria tucsoni, Physcomitrella californica, Physcomitrella patens, Physcomitrella readeri, Physcomitrium australe, Physcomitrium californicum, Physcomitrium collenchymatum, Physcomitrium coloradense, Physcomitrium cupuliferum, Physcomitrium drummondii, Physcomitrium eurystomum, Physcomitrium flexifolium, Physcomitrium hookeri, Physcomitrium hookeri var. serratum, Physcomitrium immersum, Physcomitrium kellermanii, Physcomitrium megalocarpum, Physcomitrium pyriforme, Physcomitrium pyriforme var. serratum, Physcomitrium rufipes, Physcomitrium sandbergii, Physcomitrium subsphaericum, Physcomitrium washingtoniense,* Geraniaceae wie die Gattungen Pelargonium, Cocos, Oleum z.B. die Gattungen und Arten Cocos *nucifera, Pelargonium grossularioides* oder *Oleum cocois* [Kokusnuss], Gramineae wie die Gattung Saccharum z.B. die Gattung und Art Saccharum officinarum, Juglandaceae wie die Gattungen Juglans, Wallia z.B. die Gattungen und Arten *Juglans regia, Juglans ailanthifolia, Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* oder *Wallia nigra* [Walnuss], Lauraceae Wie die Gattungen Persea, Laurus z.B. die Gattungen und Arten *Laurus nobilis* [Lorbeer], *Persea americana, Persea gratissima* oder *Persea persea* [Avocado], Leguminosae wie die Gattung Arachis z.B. die Gattung und Art *Arachis hypogaea* [Erdnuss], Linaceae wie die Gattungen Linum, Adenolinum z.B. die Gattungen und Arten *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* oder *Linum trigynum* [Lein], Lythrarieae wie die Gattung Punica z.B. die Gattung und Art *Punica granatum* [Granatapfel], Malvaceae wie die Gattung Gossypium z.B. die Gattungen und Arten *Gossypium hirsutum, Gossypium arboreum, Gossypium barbadense, Gossypium herbaceum* oder *Gossypium thurberi* [Baumwolle], Marchantiaceae wie die Gattung Marchantia z.B. die Gattungen und Arten *Marchantia berteroana, Marchantia foliacea, Marchantia macropora,* Musaceae wie die Gattung Musa z.B. die Gattungen und Arten *Musa nana, Musa acuminata, Musa paradisiaca, Musa* spp. [Banane], Onagraceae wie die Gattungen Camissonia, Oenothera z.B. die Gattungen und Arten *Oenothera biennis* oder *Camissonia brevipes* [Nachtkerze], Palmae wie die Gattung Elacis z.B. die Gattung und Art *Elaeis guineensis* [Ölpalme], Papaveraceae wie die Gattung Papaver z.B. die Gattungen und Arten *Papaver orientale, Papaver rhoeas, Papaver dubium* [Mohn], Pedaliaceae wie die Gattung Sesamum z.B. die Gattung und Art *Sesamum indicum* [Sesam], Piperaceae wie die Gattungen Piper, Artanthe, Peperomia, Steffensia z.B. die Gattungen und Arten *Piper aduncum, Piper amalago, Piper angustifolium, Piper auritum, Piper betel, Piper cubeba, Piper longum, Piper nigrum, Piper retrofractum, Artanthe adunca; Artanthe elongata, Peperomia elongata, Piper elongatum, Steffensia elongata.* [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [Gerste], *Secale cereale* [Roggen], Avena sativa, *Avena fatua, Avena byzantina, Avena fatua var. sativa, Avena hybrida* [Hafer], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia* [Reis], Zea *mays* [Mais] *Triticum aestivum, Triticum durum, Triticum turgidum, Triticum hybernum, Triticum macha, Triticum sativum* oder *Triticum vulgare* [Weizen], Porphyridiaceae wie die Gattungen Chroothece, Flintiella, Petrovanella, Porphyridium, Rhodella, Rhodosorus, Vanhoeffenia z.B. die Gattung und Art *Porphyridium cruentum,* Proteaceae wie die Gattung Macadamia z.B. die Gattung und Art *Macadamia intergrifolia* [Macadamia], Prasinophyceae wie die Gattungen Nephroselmis, Prasinococcus, Scherffelia, Tetraselmis, Mantoniella, Ostreococcus z.B. die Gattungen und Arten *Nephroselmis olivacea, Prasinococcus capsulatus, Scherffelia dubia, Tetraselmis chui, Tetraselmis suecica,* Mantoniella squamata, Ostreococcus tauri, Rubiaceae wie die Gattung Coffea z.B. die Gattungen und Arten Cofea spp., *Coffea arabica, Coffea canephora* oder *Coffea liberica* [Kaffee], Scrophulariaceae wie die Gattung Verbascum z:B. die Gattungen und Arten *Verbascum blattaria, Verbascum*

*chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* oder *Verbascum thapsus* [*Königskerze*], Solanaceae wie die Gattungen Capsicum, Nicotiana, Solanum, Lycopersicon z.B. die Gattungen und Arten *Capsicum annuum, Capsicum annuum var. glabriusculum, Capsicum frutescens* [Pfeffer], *Capsicum annuum* [Paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [Tabak], *Solanum tuberosum* [Kartoffel], *Solanum melongena* [Aubergine] *Lycopersicon esculentum, Lycopersicon lycopersicum., Lycopersicon pyriforme, Solanum integrifolium* oder *Solanum lycopersi*cum [Tomate], Sterculiaceae wie die Gattung Theobroma z.B. die Gattung und Art *Theobroma cacao* [Kakao] oder Theaceae wie die Gattung Camellia z.B. die Gattung und Art *Camellia sinensis* [Tee].

Vorteilhafte Mikroorganismen sind beispielweise Pilze ausgewählt aus der Gruppe der Familien Chaetomiaceae, Choanephoraceae, Cryptococcaceae, Cunninghamellaceae, Demetiaceae, Moniliaceae, Mortierellaceae, Mucoraceae, Pythiaceae, Sacharomycetaceae, Saprolegniaceae, Schizosacharomycetaceae, Sodariaceae oder Tuberculariaceae.

Beispielhaft seien die folgenden Mikroorganismen genannt ausgewählt aus der Gruppe: Choanephoraceae wie den Gattungen Blakeslea, Choanephora z.B. die Gattungen und Arten *Blakeslea trispora, Choanephora cucurbitarum, Choanephora infundibulifera* var. *cucurbitarum,* Mortierellaceae wie der Gattung Mortierella z.B. die Gattungen und Arten *Mortierella isabellina, Mortierella polycephala*, *Mortierella ramanniana*, *Mortierella vinacea, Mortierella zonata,* Pythiaceae wie den Gattungen Phytium, Phytophthora z.B. die Gattungen und Arten *Pythium debaryanum, Pythium intermedium, Pythium irregulare, Pythium megalacanthum, Pythium paroecandrum, Pythium sylvaticum, Pythium ultimum, Phytophthora cactorum, Phytophthora cinnamomi, Phytophthora citricola, Phytophthora citrophthora, Phytophthora cryptogea, Phytophthora drechsleri, Phytophthora erythroseptica, Phytophthora lateralis, Phytophthora megasperma, Phytophthora nicotianae, Phytophthora nicotianae var. parasitica, Phytophthora palmivora, Phytophthora parasitica, Phytophthora syringae,* Saccharomycetaceae wie den Gattungen Hansenula, Pichia, Saccharomyces, Saccharomycodes, Yarrowia z.B. die Gattungen und Arten *Hansenula anomala, Hansenula californica, Hansenula canadensis, Hansenula capsulata, Hansenula ciferrii, Hansenula glucozyma, Hansenula henricii, Hansenula holstii, Hansenula minuta, Hansenula nonfermentans, Hansenula philodendri, Hansenula polymorpha, Hansenula saturnus, Hansenula subpelliculosa, Hansenula wickerhamii, Hansenula wingei, Pichia alcoholophila, Pichia angusta, Pichia anomala, Pichia bispora, Pichia burtonii, Pichia canadensis, Pichia capsulata, Pichia carsonii, Pichia cellobiosa, Pichia ciferrii, Pichia farinosa, Pichia fermentans, Pichia finlandica, Pichia glucozyma, Pichia guilliermondii, Pichia haplophila, Pichia henricii, Pichia holstii, Pichia jadinii, Pichia lindnerii, Pichia membranaefaciens, Pichia methanolica, Pichia minuta var. minuta, Pichia minuta var. nonfermentans, Pichia norvegensis, Pichia ohmeri, Pichia pastoris, Pichia philodendri, Pichia pini, Pichia polymorpha, Pichia quercuum, Pichia rhodanensis, Pichia sargentensis, Pichia stipitis, Pichia strasburgensis, Pichia subpelliculosa, Pichia toletana, Pichia trehalophila, Pichia vini, Pichia xylosa, Saccharomyces aceti, Saccharomyces bailii, Saccharomyces bayanus, Saccharomyces bisporus, Saccharomyces capensis, Saccharomyces carlsbergensis, Saccharomyces cerevisiae, Saccharomyces cerevisiae var. ellipsoideus, Saccharomyces chevalieri, Saccharomyces delbrueckii, Saccharomyces diastaticus, Saccharomyces drosophilarum, Saccharomyces elegans, Saccharomyces ellipsoideus, Saccharomyces fermentati, Saccharomyces florentinus, Saccharomyces fragilis, Saccharomyces heterogenicus, Saccharomyces hienipiensis, Saccharomyces inusitatus, Saccharomyces italicus, Saccharomyces kluyveri, Saccharomyces krusei, Saccharomyces lactis, Saccharomyces marxianus, Saccharomyces microellipsoides, Saccharomyces montanus, Saccharomyces norbensis, Saccharomyces oleaceus, Saccharomyces paradoxus, Saccharomyces pastorianus, Saccharomyces pretoriensis, Saccharomyces rosei, Saccharomyces rouxii, Saccharomyces uvarum, Saccharomycodes ludwigii, Yarrowia lipolytica,* Schizosacharomycetaceae such as the genera Schizosaccharomyces e.g. the species *Schizosaccharomyces japonicus var. japonicus, Schizosaccharomyces japonicus var. versatilis, Schizosaccharomyces malidevorans, Schizosaccharomyces octosporus, Schizosaccharomyces pombe var. malidevorans, Schizosaccharomyces pombe var. pombe,* Thraustochytriaceae such as the genera Althornia, Aplanochytrium, Japonochytrium, Schizochytrium, Thraustochytrium e.g. the species *Schizochytrium aggregatum, Schizochytrium limacinum, Schizochytrium mangrovei, Schizochytrium minutum, Schizochytrium octosporum, Thraustochytrium aggregatum, Thraustochytrium amoeboideum, Thraustochytrium antacticum, Thraustochytrium arudimentale, Thraustochytrium aureum, Thraustochytrium benthicola, Thraustochytrium globosum, Thraustochytrium indicum, Thraustochytrium kerguelense, Thraustochytrium kinnei, Thraustochytrium motivum, Thraustochytrium multirudimentale, Thraustochytrium pachydermum, Thraustochytrium proliferum, Thraustochytrium roseum, Thraustochytrium rossii, Thraustochytrium striatum oder Thraustochytrium visurgense.*

Weitere vorteilhafte Mikroorganismen sind beispielweise Bakterien ausgewählt aus der Gruppe der Familien Bacillaceae, Enterobacteriacae oder Rhizobiaceae.

Beispielhaft seien die folgenden Mikroorganismen genannt ausgewählt aus der Gruppe: Bacillaceae wie die Gattung Bacillus z.B die Gattungen und Arten *Bacillus acidocaldarius, Bacillus acidoterrestris, Bacillus alcalophilus, Bacillus amyloliquefaciens, Bacillus amylolyticus, Bacillus brevis, Bacillus cereus, Bacillus circulans, Bacillus coagulans,*

*Bacillus sphaericus subsp. fusiformis, Bacillus galactophilus, Bacillus globisporus, Bacillus globisporus subsp. marinus, Bacillus halophilus, Bacillus lentimorbus, Bacillus lentus, Bacillus licheniformis, Bacillus megaterium, Bacillus polymyxa, Bacillus psychrosaccharolyticus, Bacillus pumilus, Bacillus sphaericus, Bacillus subtilis subsp. spizizenii, Bacillus subtilis subsp. subtilis* oder *Bacillus thuringiensis;* Enterobacteriacae wie die Gattungen Citrobacter, Edwardsiella, Enterobacter, Erwinia, Escherichia, Klebsiella, Salmonella oder Serratia z.B die Gattungen und Arten *Citrobacter amalonaticus, Citrobacter diversus, Citrobacter freundii, Citrobacter genomospecies, Citrobacter gillenii, Citrobacter intermedium, Citrobacter koseri, Citrobacter murliniae, Citrobacter sp., Edwardsiella hoshinae, Edwardsiella ictaluri, Edwardsiella tarda, Erwinia alni, Erwinia amylovora, Erwinia ananatis, Erwinia aphidicola, Erwinia billingiae, Erwinia cacticida, Erwinia cancerogena, Erwinia carnegieana, Erwinia carotovora* subsp. *atroseptica, Erwinia carotovora* subsp. *betavasculorum, Erwinia carotovora* subsp. *odorifera, Erwinia carotovora* subsp. *wasabiae, Erwinia chrysanthemi, Erwinia cypripedii, Erwinia dissolvens, Erwinia herbicola, Erwinia mallotivora, Erwinia milletiae, Erwinia nigrifluens, Erwinia nimipressuralis, Erwinia persicina, Erwinia psidii, Erwinia pyrifoliae, Erwinia quercina, Erwinia rhapontici, Erwinia rubrifaciens, Erwinia salicis, Erwinia stewartii, Erwinia tracheiphila, Erwinia uredovora, Escherichia adecarboxylata, Escherichia anindolica, Escherichia aurescens, Escherichia blattae, Escherichia coli, Escherichia coli var. communior, Escherichia coli-mutabile, Escherichia fergusonii, Escherichia hermannii, Escherichia sp., Escherichia vulneris, Klebsiella aerogenes, Klebsiella edwardsii subsp. atlantae, Klebsiella ornithinolytica, Klebsiella oxytoca, Klebsiella planticola, Klebsiella pneumoniae, Klebsiella pneumoniae subsp. pneumoniae, Klebsiella sp., Klebsiella terrigena, Klebsiella trevisanii, Salmonella abony, Salmonella arizonae, Salmonella bongori, Salmonella choleraesuis subsp. arizonae, Salmonella choleraesuis subsp. bongori, Salmonella choleraesuis subsp. cholereasuis, Salmonella choleraesuis subsp. diarizonae, Salmonella choleraesuis subsp. houtenae, Salmonella choleraesuis subsp. indica, Salmonella choleraesuis subsp. salamae, Salmonella daressalaam, Salmonella enterica subsp. houtenae, Salmonella enterica subsp. salamae, Salmonella enteritidis, Salmonella gallinarum, Salmonella heidelberg, Salmonella panama, Salmonella senftenberg, Salmonella typhimurium, Serratia entomophila, Serratia ficaria, Serratia fonticola, Serratia grimesii, Serratia liquefaciens, Serratia marcescens, Serratia marcescens subsp. marcescens, Serratia marinorubra, Serratia odorifera, Serratia plymouthensis, Serratia plymuthica, Serratia proteamaculans, Serratia proteamaculans subsp. quinovora, Serratia quinivorans* oder *Serratia rubidaea;* Rhizobiaceae wie die Gattungen Agrobacterium, Carbophilus, Chelatobacter, Ensifer, Rhizobium, Sinorhizobium z.B. die Gattungen und Arten *Agrobacterium atlanticum, Agrobacterium ferrugineum, Agrobacterium gelatinovorum, Agrobacterium larrymoorei, Agrobacterium meteori, Agrobacterium radiobacter, Agrobacterium rhizogenes, Agrobacterium rubi, Agrobacterium stellulatum, Agrobacterium tumefaciens, Agrobacterium vitis, Carbophilus carboxidus, Chelatobacter heintzii, Ensifer adhaerens, Ensifer arboris, Ensifer fredii, Ensifer kostiensis, Ensifer kummerowiae, Ensifer medicae, Ensifer meliloti, Ensifer saheli, Ensifer terangae, Ensifer xinjiangensis, Rhizobium ciceri Rhizobium etli, Rhizobium fredii, Rhizobium galegae, Rhizobium gallicum, Rhizobium giardinii, Rhizobium hainanense, Rhizobium huakuii, Rhizobium huautlense, Rhizobium indigoferae, Rhizobium japonicum, Rhizobium leguminosarum, Rhizobium loessense, Rhizobium loti, Rhizobium lupini, Rhizobium mediterraneum, Rhizobium meliloti, Rhizobium mongolense, Rhizobium phaseoli, Rhizobium radiobacter, Rhizobium rhizogenes, Rhizobium rubi, Rhizobium sullae, Rhizobium tianshanense, Rhizobium trifolii, Rhizobium tropici, Rhizobium undicola, Rhizobium vitis, Sinorhizobium adhaerens, Sinorhizobium arboris, Sinorhizobium fredii, Sinorhizobium kostiense, Sinorhizobium kummerowiae, Sinorhizobium medicae, Sinorhizobium meliloti, Sinorhizobium morelense, Sinorhizobium saheli* oder *Sinorhizobium xinjiangense.*

Weitere vorteilhafte Mikroorganismen für das erfindungsgemäße Verfahren sind beispielweise Protisten oder Diatomeen ausgewählt aus der Gruppe der Familien Dinophyceae, Turaniellidae oder Oxytrichidae wie die Gattungen und Arten: *Crypthecodinium cohnii, Phaeodactylum tricornutum, Stylonychia mytilus, Stylonychia pustulata, Stylonychia putrina, Stylonychia notophora, Stylonychia sp.,* Colpidium campylum oder Colpidium sp.

Vorteilhaft werden im erfindungsgemäßen Verfahren transgene Organismen wie Pilze wie Mortierella oder Traustochytrium, Hefen wie Saccharomyces oder Schizosaccharomyces, Moose wie Physcomitrella oder Ceratodon, nicht-humane Tiere wie Caenorhabditis, Algen wie Nephroselmis, Pseudoscourfielda, Prasinococcus, Scherffelia, Tetraselmis, Mantoniella, Ostreococcus, Crypthecodinium oder Phaeodactylum oder Pflanzen wie zweikeimblättrige oder einkeimblättrige Pflanzen verwendet. Besonders vorteilhaft werden Organismen im erfindungsgemäßen Verfahren verwendet, die zu den Öl-produzierenden Organismen gehören, das heißt die für die Herstellung von Ölen verwendet werden, wie Pilze wie Mortierella oder Thraustochytrium, Algen wie Nephroselmis, Pseudoscourfielda, Prasinococcus, Scherffelia, Tetraselmis, Mantoniella, Ostreococcus, Crypthecodinium, Phaeodactylum oder Pflanzen, insbesondere Pflanzen bevorzugt Ölfruchtpflanzen, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte. Bevorzugte erfindungsgemäße Pflanzen sind Ölfruchtpflanzen, wie Erdnuss, Raps, Canola, Sonnenblume, Saflor , Mohn, Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche Pflanzen wie Sonnenblume, Färberdistel, Tabak, Königskerze, Sesam, Baumwolle, Kürbis, Mohn, Nachtkerze. Walnuss, Lein, Hanf, Distel oder Färberdistel. Ganz besonders bevorzugt sind Pflanzen wie Färberdistel, Sonnenblume, Mohn, Nachtkerze, Walnuss, Lein oder Hanf.

Für das erfindungsgemäße beschriebene Verfahren ist es vorteilhaft in den Organismus zusätzlich zu den unter Verfahrensschritt (a) bis (d) eingebrachten Nukleinsäuren sowie den ggf. eingebrachten Nukleinsäuresequenzen, die für die ω-3-Desaturasen codieren, zusätzlich weitere Nukleinsäuren einzubringen, die für Enzyme des Fettsäure- oder Lipidstoffwechsels codieren.

Im Prinzip können alle Gene des Fettsäure- oder Lipidstoffwechsels vorteilhaft in Kombination mit der(den) erfinderischen Δ-5-Elongase(n), Δ-6-Elongase(n) und/oder w-3-Desaturase(n) [im Sinne dieser Anmeldung soll der Plural den Singular und umgekehrt beinhalten] im Verfahren zur Herstellung mehrfach ungesättigter Fettsäuren verwendet werden vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetytenasen, Lipoxygenasen, Triacylglycerol-Lipasen, Allenoxid-Synthasen, Hydroperoxid-Lyasen oder Fettsäure-Elongase(n) in Kombination mit der Δ-5-Elongase, Δ-6-Elongase und/oder ω-3-Desaturase verwendet. Besonders bevorzugt werden Gene ausgewählt aus der Gruppe der Δ-4-Desaturasen, Δ -5-Desaturasen, Δ-6-Desaturasen. Δ-8-Desatuasen, Δ-9-Desaturasen, Δ-12-Desaturasen, Δ-6-Elongasen oder Δ-9-Elongasen in Kombination mit den vorgenannten Genen für die Δ-5-Elongase, Δ-6-Elongase und/oder ω-3-Desaturase verwendet, wobei einzelne Gene oder mehrere Gene in Kombination verwendet werden können.

Die erfindungsgemäßen Δ-5-Elongasen haben gegenüber den humanen Elongasen oder Elongasen aus nicht-humanen Tieren wie denen aus Oncorhynchus, Xenopus oder Ciona die vorteilhafte Eigenschaft, dass sie $C_{22}$-Fettsäuren nicht zu den entsprechenden C24-Fettsäuren elongieren. Weiterhin setzen sie vorteilhaft keine Fettsäuren mit einer Doppelbindung in Δ-6-Position um, wie sie von den humanen Elongasen oder den Elongasen aus nicht-humanen Tieren umgesetzt werden. Besonders vorteilhafte Δ -5-Elongasen setzen bevorzugt nur ungesättigte $C_{20}$-Fettsäuren um. Diese vorteilhaften Δ-5-Elongasen weisen einige putative Transmembran-Helixes (5 - 7) auf. Vorteilhaft werden nur $C_{20}$-Fettsäuren mit einer Doppelbindung in Δ-5-Position umgesetzt, wobei ω-3-$C_{20}$ Fettsäuren bevorzugt werden (EPA). Weiterhin haben sie in einer bevorzugten Ausführungsform der Erfindung die Eigenschaft, dass sie neben der Δ-5-Elongaseaktivität vorteilhaft keine oder nur eine relativ geringe Δ-6-Elongaseaktivität aufweisen. Im Gegensatz dazu weisen die humanen Elongasen oder nicht-humanen Tier-Elongasen eine annäherend gleiche Aktivität gegenüber Fettsäuren mit einer Δ-6- oder Δ-5-Doppelbindung auf. Diese vorteilhaften Elongasen werden als sogenannte monofunktionelle Elongasen bezeichnet. Die humanen Elongasen oder die nicht-humanen Tierelongasen werden dem gegenüber als multifunktionelle Elongasen bezeichnet, die neben den vorgenannten Substraten auch monoungesättigte $C_{16}$- und $C_{18}$-Fettsäuren beispielsweise mit Δ-9- oder Δ-11-Doppelbindung umsetzen. Vorteilhaft setzen die monofunktionellen Elongasen in einem Hefefütterungstext, in dem als Substrat EPA den Hefen zugesetzt wurde, mindestens 15 Gew.-% des zugesetzten EPAs zu Docosapentaensäure (DPA, $C22{:}5^{\Delta 7,10,13,16,19}$), vorteilhaft mindestens 20 Gew.-%, besonders vorteilhaft mindestens 25 Gew.-% um. Wird als Substrat γ-Linolensäure (= GLA, $C18{:}3^{\Delta 6,9,12}$) gegeben, so wird diese vorteilhaft gar nicht elongiert. Ebenfalls wird auch $C18{:}3^{\Delta 5,9,2}$ nicht elongiert. In einer anderen vorteilhaften Ausführungsform werden weniger als 60 Gew.-% des zugesetzten GLA zu Dihomo-γ-linolensäure (= $C20{:}3^{\Delta 8,11,14}$) umgesetzt, vorteilhaft weniger als 55 Gew.-%, bevorzugt weniger als 50 Gew.-%, besonders vorteilhaft weniger als 45 Gew.-%, ganz besonders vorteilhaft weniger als 40 Gew.-%. In einer weiteren ganz bevorzugten Ausführungsform der erfindungsgemäßen Δ-5-Elongaseaktivität wird GLA nicht umgesetzt.

Die Figuren 27 und 28 geben die gemessenen Substratspezifitäten der verschiedenen Elongasen wieder. In Figur 27 sind die Spezifitäten der multifunktnonellen Elongasen von Xenopus laevis (Fig. 27 A), Ciona intestinalis (Fig. 27 B) und Oncorhynchus mykiss (Fig. 27 C) wiedergegeben. Alle diese Elongasen setzen ein breites Spektrum an Substraten um. Dies kann im erfindungsgemäßen Verfahren zu Nebenprodukten führen, die durch weitere enzymatische Aktivitäten umgesetzt werden müssen. Diese Enzyme sind deshalb im erfindungsgemäßen Verfahren weniger bevorzugt. Die bevorzugten monofunktionellen Elongasen und ihre Substratspezifität werden in Figur 28 wiedergegeben. Figur 28A zeigt die Spezifität der Ostreococcus tauri Δ-5-Elongase. Dies setzt nur Fettsäuren mit einer Doppelbindung in Δ-5-Position um. Vorteilhaft werden nur C20-Fettsäuren umgesetzt Eine ähnlich hohe Substratspezifität weist die Δ-5-Elongase von Thalassiosira pseudonana (Fig. 28. C) auf. Sowohl die Δ-6-Elongase von Ostreococcus tauri (Fig. 28 B) als auch die von Thalassiosira pseudonana (Fig. 28 D) setzen vorteilhaft nur Fettsäuren mit einer Doppelbindung in Δ-6-Position um. Vorteilhaft werden nur C18-Fettsäuren umgesetzt. Auch die Δ-5-Elongasen aus Arabidopsis thaliana und Euglena gracilis zeichnen sich durch ihre Spezifität aus.

Vorteilhafte erfindungsgemäße Δ-6-Elongasen zeichnen sich ebenfalls durch eine hohe Spezifität aus, das heißt bevorzugt werden $C_{18}$-Fettsäuren elongiert. Vorteilhaft setzen sie Fettsäuren mit einer Doppelbindung in Δ-6-Position um. Besonders vorteilhafte Δ-6-Elongasen setzen vorteilhaft $C_{18}$-Fettsäuren mit drei oder vier Doppelbindungen

im Molekül um, wobei diese eine Doppelbindung in Δ-6-Position enthalten müssen. Weiterhin haben sie in einer bevorzugten Ausführungsform der Erfindung die Eigenschaft, dass sie neben der Δ-6-Elongaseaktivität vorteilhaft keine oder nur eine relativ geringe Δ-5-Elongaseaktivität aufweisen. Im Gegensatz dazu weisen die humanen Elongasen oder nicht-humanen Tier-Elongasen eine annäherend gleiche Aktivität gegenüber Fettsäuren mit einer Δ-6- oder A-5-Doppelbindung auf. Diese vorteilhaften Elongasen werden als sogenannte monofunktionelle Elongasen bezeichnet. Die humanen Elongasen öder die nicht-humanen Tierelongasen werden, wie oben beschrieben, dem gegenüber als multifunktionelle Elongasen bezeichnet, die neben den vorgenannten Substraten auch monoungesättigte $C_{16}$- und $C_{18}$-Fettsäuren beispielsweise mit Δ-9- oder Δ-11-Doppelbindung umsetzen. Vorteilhaft setzen die monofunktionellen Elongasen in einem Hefefütterungstext, in dem als Substrat EPA den Hefen zugesetzt wurde, mindestens 10 Gew.-% der zugesetzten α-Linolensäure (= ALA, $C18:3^{\Delta 9,12,15}$) bzw. mindestens 40 Gew.-% der zugesetzten γ-Linolensäure (= GLA, $C18:3^{\Delta 6,9,12}$), vorteilhaft mindestens 20 Gew.-% bzw. 50 Gew.-%, besonders vorteilhaft mindestens 25 Gew.-% bzw. 60 Gew.-% um. Besonders vorteilhaft wird auch $C18:4^{\Delta 6,9,12,15}$ (Stearidonsäure) elongiert. SDA wird dabei zu mindestens 40 Gew.-%, vorteilhaft zu mindestens 50 Gew.-%, besonders vorteilhaft zu mindestens 60 Gew.-%, ganz besonders vorteihaft zu mindestens 70 Gew.-% umgesetzt. Besonders vorteilhafte Δ-6-Elongasen zeigen keine oder nur eine sehr geringe Aktivität (weniger als 0,1 Gew-% Umsatz) gegenüber den folgenden Substraten: $C18:1^{\Delta 6}$, $C18:1^{\Delta 9}$, $C18:1^{\Delta 11}$, $C20:2^{\Delta 11,14}$, $C20:3^{\Delta 11,14,17}$, $C20:3^{\Delta 8,11,14}$, $C20:4^{\Delta 5,8.11,14}$, $C20:5^{\Delta 5,8,11,14,17}$ oder $C22:4^{\Delta 7,10,13,16}$.

Die Figuren 29 und 30 sowie die Tabelle 18 geben die gemessenen Substratspezifitäten der verschiedenen Elongasen wieder.

Die erfindungsgemäße ω-3-Desaturase hat gegenüber den bekannten ω-3-Desaturase die vorteilhafte Eigenschaft, dass sie ein breites Spektrum an ω-6-Fettsäuren desaturieren kann, bevorzugt werden $C_{20}$- und $C_{22}$-Fettsäuren wie $C_{20:2}$-, $C_{20:3}$-, $C_{20:4}$-, $C_{22:4}$- oder $C_{22:5}$-Fettsäuren desaturiert. Aber auch die kürzeren $C_{18}$-Fettsäuren wie $C_{18:2}$- oder $C_{18:3}$-Fettsäuren werden vorteilhaft desaturiert. Durch diese Eigenschaften der ω-3-Desaturase ist es vorteilhaft möglich das Fettsäurespektrum innerhalb eines Organismus vorteilhaft innerhalb einer Pflanze oder einem Pilz von den ω-6-Fettsäuren zu den ω-3-Fettsäuren hin zu verschieben. Bevorzugt werden von der erfindungsgemäßen ω-3-Desaturase $C_{20}$-Fettsäuren desaturiert. Innerhalb des Organismus werden diese Fettsäuren aus dem vorhandenen Fettsäurepool zu mindestens 10%, 15%, 20%, 25% oder 30% zu den entsprechenden ω-3-Fettsäuren umgesetzt. Gegenüber den $C_{18}$-Fettsäuren weist die ω-3-Desaturase eine um den Faktor 10 geringere Aktivität auf, das heißt es werden nur ca. 1,5 bis 3% der im Fettsäurepool vorhandenen Fettsäuren zu den entsprechenden ω-3-Fettsäuren umgesetzt. Bevorzugtes Substrat der erfindungsgemäßen ω-3-Desaturase sind die in Phospholipiden gebundenen ω-6-Fettsäuren. Figur 19 zeigt deutlich am Beispiel der Desaturierung von Dihomo-γ-linolensäure [$C_{20:4}{}^{\Delta 8,11,14}$], dass die ω-3-Desaturase bei der Desaturierung vorteilhaft nicht zwischen an sn1- oder sn2-Position gebundenen Fettsäuren unterscheidet. Sowohl an sn1- oder sn2-Position in den Phospholipide gebundene Fettsäuren werden desaturiert. Weiterhin ist vorteilhaft, dass die ω-3-Desaturase eine breite Palette von Phospholipiden wie Phosphatidylcholin (= PC), Phosphatidylinositol (= PIS) oder Phosphatidylethanolamin (= PE) umsetzt. Schließlich lassen sich auch Desaturierungsprodukte in den Neutrallipiden (= NL), das heißt in den Triglyceriden finden.

Die erfingungsgemäßen Δ-4-Desaturasen, Δ-5-Desaturasen und Δ-6-Desaturasen haben gegenüber den bekannten Δ-4-Desaturasen, Δ-5-Desaturasen und Δ-6-Desaturasen den Vorteil, dass sie Fettsäuren gebunden an Phospholipide oder CoA-Fettsäureester, vorteilhaft CoA-Fettsäureester umsetzen können.

Vorteilhaft setzen die im erfingungsgemäßen Verfahren verwendeten Δ-12-Desaturasen Ölsäure ($C18:1^{\Delta 9}$) zu Linolsäure ($C18:2^{\Delta 9,12}$) oder $C18:2^{\Delta 6,9}$ zu $C18:3^{\Delta 6,9,12}$ (= GLA) um. Vorteilhaft setzen die verwendeten Δ-12-Desaturasen Fettsäuren gebunden an Phospholipide oder CoA-Fettsäureester, vorteilhaft gebunden an CoA-Fettsäureester um.

Durch die enzymatische Aktivität der im erfindungsgemäßen Verfahren verwendeten Nukleinsäuren, die für Polypeptide mit Δ-5-Elongase-, Δ-6-Elongase- und/oder ω-3-Desaturaseaktivität codieren, vorteilhaft in Kombination mit Nukleinsäuresequenzen, die für Polypeptide des Fettsäure- oder Lipidstoffwechsels wie weiteren Polypeptiden mit Δ-4-, Δ-5-, Δ-6-, Δ-8-, Δ-12-Desaturase- oder Δ-5-, Δ-6-oder Δ-9-Elongaseaktivität codieren, können unterschiedlichste mehrfach ungesättigte Fettsäuren im erfindungsgemäßen Verfahren hergestellt werden. Je nach Auswahl der für das erfindungsgemäße Verfahren verwendeten Organismen wie den vorteilhaften Pflanzen lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder, die sich von C18:3-Fettsäuren ableiten, wie SDA, ETA oder EPA. Liegt in der für das Verfahren verwendeten Pflanze als ungesättigte Fettsäure nur Linolsäure (= LA, $C18:2^{\Delta 9,12}$) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, $C18:3^{\Delta 9,12,15}$) beispielsweise wie in Lein, so können als Produkte des Verfahrens nur SDA, ETA, EPA und/oder DHA entstehen, die wie oben beschrieben als freie Fettsäuren oder gebunden vorliegen können. Durch Modifikation der Aktivität des an der Synthese beteiligten Enzyms Δ-5-Elongase vorteilhaft in Kombination mit der Δ-4-, Δ-5-, Δ-6-, Δ-12-Desaturase und/oder Δ-6-Elongase, oder der Δ-4-, Δ-5-, Δ-8-, Δ-12-Desaturase, und/oder Δ-9-Elongase lassen sich gezielt in den vorgenannten

Organismen vorteilhaft in den vorgenannten Pflanzen nur einzelne Produkte herstellten. Durch die Aktivität der Δ-6-Desaturase und Δ-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Werden die Δ-5-Desaturase, die Δ-5-Elongase und die Δ-4-Desaturase zusätzlich in die Organismen vorteilhaft in die Pflanze eingebracht, so entstehen zusätzlich ARA, EPA und/oder DHA. Dies gilt auch für Organismen in die vorher die Δ-8-Desaturase und Δ-9-Elongase eingebracht wurde. Vorteilhaft werden nur ARA, EPA oder DHA oder deren Mischungen synthetisiert, abhängig von der in im Organismus bzw. in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen bzw. ARA, EPA, DHA oder deren Mischungen vorteilhaft EPA oder DHA oder deren Mischungen.

Das von der erfindungsgemäßen Nukleinsäure kodierte Protein zeigt ein hohe Spezifität für die beiden Vorstufen $C18{:}4^{\Delta 6,9,12,15}$- und $C20{:}5^{\Delta 5,8,11,14,17}$-Fettsäuren zur Synthese von DHA (Vorstufen und Synthese von DHA siehe Figur 1). Das von SEQ NO: 53 kodierte Protein hat damit eine Spezifität für Δ6- und Δ5-Fettsäuren mit zusätzlich einer ω 3-Doppelbindung (Figur 2). Die Δ-5-Elongase hat eine keto-Acyl-CoA-Synthase-Aktivität, die vorteilhaft Fettsäurereste von Acyl-CoA-Estern um 2 Kohlenstoffatome verlängert.

Mittels der Δ-5-Elongase-Gene, der Δ5-Desaturase aus Phaeodacylum sowie der Δ 4-Desaturase aus Euglena konnte die Synthese von DHA in Hefe (Saccharomyces cerevisiae) nachgewiesen werden (Figur 3).

Neben der Produktion der Ausgangsfettsäuren für die erfindungsgemäße Δ-5-Elongase, Δ-6-Elongase und/oder ω-3-Desaturase direkt im Organismus können die Fettsäuren auch von außen gefüttert werden. Aus Kostengründen ist die Produktion im Organismus bevorzugt. Bevorzugt Substrate der ω-3-Desaturase sind die Linolsäure ($C18{:}2^{\Delta 9,12}$), die γ-Linolensäure ($C18{:}3^{\Delta 6,9,12}$), die Eicosadiensäure ($C20{:}2^{\Delta 11,14}$), die Dihomo-γ-linolensäure ($C20{:}3^{\Delta 8,11,14}$), die Arachidonsäure ($C20{:}4^{\Delta 5,8,11,14}$), die Docosatetraensäure ($C22{:}4^{\Delta 7,10,13,16}$) und die Docosapentaensäure ($C22{:}5^{\Delta 4,7,10,13,15}$).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Dies ist besonders vorteilhaft in Öl-produzierenden Organismen wie der Familie der Brassicaceae wie der Gattung Brassica z.B. Raps; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max,* die einen hohen ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten Δ-12-Desaturasen zur Herstellung des Ausgangsprodukts Linolsäure vorteilhaft.

Im erfindungsgemäßen Verfahren verwendeten Nukleinsäuren stammen vorteilhaft aus Pflanzen wie Algen beispielsweise Algen der Familie der Prasinophyceae wie aus den Gattungen Heteromastix, Mammella, Mantoniella, Micromonas, Nephroselmis, Ostreococcus, Prasinocladus, Prasinococcus, Pseudoscourfielda, Pycnococcus, Pyramimonas, Scherffelia oder Tetraselmis wie den Gattungen und Arten Heteromastix longifillis, Mamiella gilva, Mantoniella squamata, Micromonas pusilla, Nephroselmis olivacea, Nephroselmis pyriformis, Nephroselmis rotunda, Ostreococcus tauri, Ostreococcus sp. Prasinocladus ascus, Prasinocladus lubricus, Pycnococcus provasolii, Pyramimonas amylifera, Pyramimonas disomata, Pyramimonas obovata, Pyramimonas orientalis, Pyramimonas parkeae, Pyramimonas spinifera, Pyramimonas sp., Tetraselmis apiculata, Tetraselmis carteriaformis, Tetraselmis chui, Tetraselmis convolutae, Tetraselmis desikacharyi, Tetraselmis gracilis, Tetraselmis hazeni, Tetraselmis impellucida, Tetraselmis inconspicua, Tetraselmis levis, Tetraselmis maculata, Tetraselmis marina, Tetraselmis striata, Tetraselmis subcordiformis, Tetraselmis suecica, Tetraselmis tetrabrachia, Tetraselmis tetrathele, Tetraselmis verrucosa, Tetraselmis verrucosa fo. rubens oder Tetraselmis sp. oder aus Algen der Familie Euglenaceae wie aus den Gattungen Ascoglena, Astasia, Colacium, Cyclidiopsis, Euglena, Euglenopsis, Hyalophacus, Khawkinea, Lepocinclis, Phacus, Strombomonas oder Trachelomonas wie die Gattungen und Art Euglena acus, Euglena geniculata, Euglena gracilis, Euglena mixocylindracea, Euglena rostrifera, Euglena viridis, Colacium stentorium, Trachelomonas cylindrica oder Trachelomonas volvocina. Vorteilhaft stammen die verwendeten Nukleinsäuren aus Algen der Gattungen Euglena, Mantoniella oder Ostreococcus.

Weitere vorteilhafte Pflanzen sind Algen wie Isochrysis oder Crypthecodinium, Algen/ Diatomeen wie Thalassiosira oder Phaeodactylum, Moose wie Physcomitrella oder Ceratodon oder höheren Pflanzen wie den Primulaceae wie Aleuritia, Calendula stellata, Osteospermum spinescens oder Osteospermum hyoseroides, Mikroorganismen wie Pilzen wie Aspergillus, Thraustochytrium, Phytophthora, Entomophthora, Mucor oder Mortierella, Bakterien wie Shewanella, Hefen oder Tieren wie Nematoden wie Caenorhabditis, Insekten, Fröschen, Seegurken oder Fischen. Vorteilhaft stammen die erfindungsgemäßen isolierten Nukleinsäuresequenzen aus einem Tier aus der Ordnung der Vertebraten. Bevorzugt stammen die Nukleinsäuresequenzen aus der Klasse der Vertebrata; Euteleostomi, Actinopterygii; Neopterygii; Teleostei; Euteleostei, Protacanthopterygii, Salmoniformes; Salmonidae bzw. Oncorhynchus oder Vertebrata, Amphibia, Anura, Pipidae, Xenopus oder Evertebrata wie Protochordata, Tunicata, Holothuroidea, Cionidae wie Amaroucium constellatum, Botryllus schlosseri, Ciona intestinalis, Molgula citrina, Molgula manhattensis, Perophora viridis oder Styela partita. Besonders vorteilhaft stammen die Nukleinsäuren aus Pilzen, Tieren oder aus Pflanzen wie Algen oder Moosen, bevorzugt aus der Ordnung der Salmoniformes wie der Familie der Salmonidae wie der Gattung Salmo beispielsweise aus den Gattungen und Arten Oncorhynchus mykiss, Trutta trutta oder Salmo trutta fario, aus Algen wie den Gattungen Mantoniella oder Ostreococcus oder aus den Diatomeen wie den Gattungen Thalassiosira oder Phaeodactylum oder aus Algen wie Crypthecodinium.

Vorteilhaft werden im erfindungsgemäßen Verfahren die vorgenannten Nukleinsäuresequenzen oder deren Derivat oder Homologe, die für Polypeptide codieren, die noch die enzymatische Aktivität der durch Nukleinsäuresequenzen codierten Proteine besitzen. Diese Sequenzen werden einzeln oder in Kombination mit den für die Δ-12-Desaturase, Δ-4-Desaturase, Δ-5-Desaturase, Δ-6-Desaturase, Δ-5-Elongase, Δ-6-Elongase und/oder ω-3-Desaturase codierenden Nukleinsäuresquenzen in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder eines ganzen Organismus, der die im Verfahren verwendeten Nukleinsäuresequenzen enthält, wobei die Zelle und/oder der Organismus mit einer erfindungsgemäßen Nukleinsäuresequenz, die für die Δ-12-Desaturase, Δ-4-Desaturase, Δ-5-Desaturase, Δ-6-Desaturase, Δ-5-Elongase, Δ-6-Elongase und/oder ω -3-Desaturase codiert, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Öle, Lipide oder freien Fettsäuren aus dem Organismus oder aus der Kultur. Bei der Kultur kann es sich beispielsweise um eine Fermentationskultur beispielsweise im Falle der Kultivierung von Mikroorganismen wie z.B. Mortierella, Thalassiosira, Mantoniella, Ostreococcus, Saccharomyces oder Thraustochytrium oder um eine Treibhaus oder Feldkultur einer Pflanze handeln. Die so hergestellte Zelle oder der so hergestellte Organismus ist vorteilhaft eine Zelle eines Öl-produzierenden Organismus wie einer Ölfruchtpflanze wie beispielsweise Erdnuss, Raps, Canola, Lein, Hanf, Erdnuss, Soja, Safflower, Hanf, Sonnenblumen oder Borretsch.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

"Transgen" bzw. "Rekombinant" im Sinne der Erfindung bedeutet bezüglich zum Beispiel einer Nukleinsäuresequenz, einer Expressionskassette (= Genkonstrukt) oder einem Vektor enthaltend die erfindungsgemäße Nukleinsäuresequenz oder einem Organismus transformiert mit den erfindungsgemäßen Nukleinsäuresequenzen, Expressionskassette oder Vektor alle solche durch gentechnische Methoden zustandegekommenen Konstruktionen, in denen sich entweder

a) die erfindungsgemäße Nukleinsäuresequenz, oder

b) eine mit der erfindungsgemäßen Nukleinsäuresequenz funktionell verknüpfte genetische Kontrollsequenz, zum Beispiel ein Promotor, oder

c) (a) und (b)

sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der erfindungsgemäßen Nukleinsäuresequenzen mit den entsprechenden Δ-12-Desaturase-, Δ-4-Desaturase-, Δ-5-Desaturase-, Δ-6-Desaturase-, Δ-8-Desaturase-, ω-3-Desaturase-, Δ -9-Elongase-, Δ-6-Elongase- und/oder Δ-5-Elongasegenen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beispielsweise beschrieben in US 5,565,350 oder WO 00/15815.

Unter transgenen Organismus bzw. transgener Pflanze im Sinne der Erfindung ist wie vorgenannt zu verstehen, dass die im Verfahren verwendeten Nukleinsäuren nicht an ihrer natürlichen Stelle im Genom eines Organismus sind,

dabei können die Nukleinsäuren homolog oder heterolog exprimiert werden. Transgen bedeutet aber auch wie genannt, dass die erfindungsgemäßen Nukleinsäuren an ihrem natürlichen Platz im Genom eines Organismus sind, dass jedoch die Sequenz gegenüber der natürlichen Sequenz verändert wurde und/oder das die Regulationssequenzen, der natürlichen Sequenzen verändert wurden. Bevorzugt ist unter transgen die Expression der erfindungsgemäßen Nukleinsäuren an nicht natürlicher Stelle im Genom zu verstehen, das heißt eine homologe oder bevorzugt heterologe Expression der Nukleinsäuren liegt vor. Bevorzugte transgene Organismen sind Pilze wie Mortierella oder Phytophtora, Moose wie Physcomitrella, Algen wie Mantoniella, Euglena, Crypthecodinium oder Ostreococcus, Diatomeen wie Thalassiosira oder Phaeodyctylum oder Pflanzen wie die Ölfruchtpflanzen.

Als Organismen bzw. Wirtsorganismen für die im erfindungsgemäßen Verfahren verwendeten Nukleinsäuren, die Expressionskassette oder den Vektor eignen sich prinzipiell vorteilhaft alle Organismen, die in der Lage sind Fettsäuren speziell ungesättigte Fettsäuren zu synthetisieren bzw. für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne, Mikroorganismen wie Pilze beispielsweise die Gattung Mortierella, Thraustochytrium, Saprolegnia, Phytophtora oder Pythium, Bakterien wie die Gattung Escherichia oder Shewanella, Hefen wie die Gattung Saccharomyces, Cyanobakterien, Ciliaten, Algen wie Mantoniella, Euglena, Thalassiosira oder Ostreococcus oder Protozoen wie Dinoflagellaten wie Crypthecodinium genannt. Bevorzugt werden Organismen, die natürlicherweise Öle in größeren Mengen synthetisieren können wie Pilze wie Mortierella alpina, Pythium insidiosum, Phytophtora infestans oder Pflanzen wie Soja, Raps, Kokosnuss, Ölpalme, FärberSaflor, Flachs, Hanf, Rizinus, Calendula, Erdnuss, Kakaobohne oder Sonnenblume oder Hefen wie Saccharomyces cerevisiae, besonders bevorzugt werden Soja, Flachs, Raps, FärberSaflor, Sonnenblume, Calendula, Mortierella oder Saccharomyces cerevisiae. Prinzipiell sind als Wirtsorganismen neben den vorgenannten transgenen Organismen auch transgene Tiere vorteilhaft nicht-humane Tiere geeignet beispielsweise C. elegans, Ciona intestinalis oder Xenopus laevis.

Nutzbare Wirtszellen sind weiterhin genannt in: Goeddel, Gene Expression Technology: Methods in Enzymology 185, Academic Press, San Diego, CA (1990).

Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128.

Hierzu gehören Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Transgene Pflanzen, die die im erfindungsgemäßen Verfahren synthetisierten mehrfach ungesättigten Fettsäuren enthalten, können vorteilhaft direkt vermarktet werden ohne dass die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe. Die im erfindungsgemäßen Verfahren hergestellten Verbindungen können aber auch aus den Organismen vorteilhaft Pflanzen in Form ihrer Öle, Fett, Lipide und/oder freien Fettsäuren isoliert werden. Durch dieses Verfahren hergestellte mehrfach ungesättigten Fettsäuren lassen sich durch Ernten der Organismen entweder aus der Kultur, in der sie wachsen, oder vom Feld ernten. Dies kann über Pressen oder Extraktion der Pflanzenteile bevorzugt der Pflanzensamen erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt. Im Falle von Mikroorganismen werden diese nach Ernte beispielsweise direkt ohne weitere Arbeitsschritte extrahiert oder aber nach Aufschluss über verschiedene dem Fachmann bekannte Methoden extrahiert. Auf diese Weise können mehr als 96 % der im Verfahren hergestellten Verbindungen isoliert werden. Anschließend werden die so erhaltenen Produkte weiter bearbeitet, das heißt raffiniert. Dabei werden zunächst beispielsweise die Pflanzenschleime und Trübstoffe entfernt. Die sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf noch desodoriert.

Vorzugsweise sind die durch dieses Verfahren produzierten PUFAs bzw. LCPUFAs $C_{18}$-, $C_{20}$- oder $C_{22}$-Fett-

säuremoleküle vorteilhaft $C_{20}$- oder $C_{22}$-Fettsäuremoleküle mit mindestens zwei Doppelbindungen im Fettsäuremolekül, vorzugsweise drei, vier, fünf oder sechs Doppelbindungen. Diese $C_{18}$-, $C_{20}$- oder $C_{22}$-Fettsäuremoleküle lassen sich aus dem Organismus in Form eines Öls, Lipids oder einer freien Fettsäure isolieren.

Geeignete Organismen sind beispielsweise die vorstehend erwähnten. Bevorzugte Organismen sind transgene Pflanzen.

Eine Ausführungsform der Erfindung sind deshalb Öle, Lipide oder Fettsäuren oder Fraktionen davon, die durch das oben beschriebene Verfahren hergestellt worden sind, besonders bevorzugt Öl, Lipid oder eine Fettsäurezusammensetzung, die PUFAs umfassen und von transgenen Pflanzen herrühren.

Diese Öle, Lipide oder Fettsäuren enthalten wie oben beschrieben vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octädecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure),Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-:Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %,3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Butterbuttersäure, kein Cholesterin, keine Clupanodonsäure (= Docosapentaensäure, $C22:5^{\Delta 4,8,12,15,21}$) sowie keine Nisinsäure (Tetracosahexaensäure, $C23:6^{\Delta 3,8,12,15,18,21}$).

Vorteilhaft enthalten die erfindungsgemäßen Öle, Lipide oder Fettsäuren mindestens 0,5%, 1%, 2%, 3%, 4% oder 5%, vorteilhaft mindestens 6%, 7%, 8%, 9% oder 10%, besonders vorteilhaft mindestens 11%, 12%, 13%, 14% oder 15% ARA oder mindestens 0,5%, 1%, 2%, 3%, 4% oder 5%, vorteilhaft mindestens 6%, oder 7%, besonders vorteilhaft mindestens 8%, 9% oder 10% EPA und/oder DHA bezogen auf den Gesamtfettsäuregehalt des Produktionsorganismus vorteilhaft einer Pflanze, besonders vorteilhaft einer Ölfruchtpflanze wie Soja, Raps, Kokosnuss, Ölpalme, Färbersafflor, Flachs, Hanf, Rizinus, Calendula, Erdnuss, Kakaobohne, Sonnenblume oder den oben genannten weiteren ein- oder zweikeimblättrigen Ölfruchtpflanzen.

Eine weitere erfindungsgemäße Ausführungsform ist die Verwendung des Öls, Lipids, der Fettsäuren und/oder der Fettsäurezusammensetzung in Futtermitteln, Nahrungsmitteln, Kosmetika oder Pharmazeutika. Die erfindungsgemäßen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, $\gamma$-Linolensäure, Dihomoy-linolensäure, Arachidonsäure, $\alpha$-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 % oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach

Ausgangsorganismus der Anteil der verschiedenen Fettsäuren in dem Öl oder Fett schwanken.

Bei den im Verfahren hergestellten mehrfach ungesättigte Fettsäuren mit vorteilhaft mindestens zwei Doppelbindungen enthalten, handelt es sich wie oben beschrieben beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren mit vorteilhaft mindestens fünf oder sechs Doppelbindungen lassen sich die enthaltenden mehrfach ungesättigten Fettsäuren beispielsweise über eine Alkalibehandlung beispielsweise wäßrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. $H_2SO_4$. Die Freisetzung der Fettsäuren kann auch direkt ohne die vorhergehend beschriebene Aufarbeitung erfolgen.

Die im Verfahren verwendeten Nukleinsäuren können nach Einbringung in einem Organismus vorteilhaft einer Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid- und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb vorteilhaft im erfindungsgemäßen Verfahren verwendbar.

Als Substrate der im erfindungsgemäßen Verfahren verwendeten Nukleinsäuren, die für Polypeptide mit Δ-12-Desaturase-, Δ-5-Desaturase-, Δ-4-Desaturase-, Δ-6-Desaturase-, Δ-8-Desaturase-, A-9-Elongase-, Δ-5-Elongase-, Δ-6-Elongase- und/oder ω -3-Desaturase-Aktivität codieren, und/oder den weiteren verwendeten Nukleinsäuren wie den Nukleinsäuren, die für Polypeptide des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) codieren eignen sich vorteilhaft $C_{16}$-, $C_{18}$- oder $C_{20}$-Fettsäuren. Bevorzugt werden die im Verfahren als Substrate umgesetzten Fettsäuren in Form ihrer Acyl-CoA-Ester und/oder ihrer Phospholipid-Ester umgesetzt.

Zur Herstellung der erfindungsgemäßen langkettigen PUFAs müssen die mehrfach ungesättigten $C_{18}$-Fettsäuren zunächst durch die enzymatische Aktivität einer Desaturase zunächst desaturiert und anschließend über eine Elongase um mindestens zwei Kohlenstoffatome verlängert werden. Nach einer Elongationsrunde führt diese Enzymaktivität zu $C_{20}$-Fettsäuren, und nach zwei Elongationsrunden zu $C_{22}$-Fettsäuren. Die Aktivität der erfindungsgemäßen Verfahren verwendeten Desaturasen und Elongasen führt vorzugsweise zu $C_{18}$-, $C_{20}$- und/oder $C_{22}$-Fettsäuren vorteilhaft mit mindestens zwei Doppelbindungen im Fettsäuremolekül, vorzugsweise mit drei, vier, fünf oder sechs Doppelbindungen, besonders bevorzugt zu $C_{20}$- und/oder $C_{22}$-Fettsäuren mit mindestens zwei Doppelbindungen im Fettsäuremolekül, vorzugsweise mit drei, vier, fünf oder sechs Doppelbindungen, ganz besonders bevorzugt mit fünf oder sechs Doppelbindungen im Molekül. Nachdem eine erste Desaturierung und die Verlängerung stattgefunden hat, können weitere Desaturierungs- und Elongierungsschritte wie z.B. eine solche Desaturierung in Δ-5- und Δ-4-Position erfolgen. Besonders bevorzugt als Produkte des erfindungsgemäßen Verfahrens sind Dihomo-γ-linolensäure, Arachidonsäure, Eicosapentaensäure, Docosapetaensäure und/oder Docosahesaensäure. Die $C_{20}$-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Werden im erfindungsgemäßen Verfahren als Organismen Mikroorganismus wie Hefen wie Saccharomyces oder Schizosaccharomyces, Pilze wie Mortierella, Aspergillus, Phytophtora, Entomophthora, Mucor oder Thraustochytrium Algen wie Isochrysis, Mantoniella, Euglena, Ostreococcus, Phaeodactylum oder Crypthecodinium verwendet, so werden diese Organismen vorteilhaft fermentativ angezogen.

Durch die Verwendung der erfindungsgemäßen Nukleinsäuren, die für eine Δ-5-Elongase codieren, können im Verfahren die hergestellten mehrfach ungesättigten Fettsäuren mindestens um 5 % , bevorzugt mindestens um 10 %, besonders bevorzugt mindestens um 20 %, ganz besonders bevorzugt um mindestens 50 % gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Organismen prinzipiell auf zwei Arten erhöht werden. Es kann vorteilhaft der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Werden im erfindungsgemäßen Verfahren als Organismen Mikroorganismen verwendet, so werden sie je nach Wirtsorganismus in dem Fachmann bekannter Weise angezogen bzw. gezüchtet. Mikroorganismen werden in der Regel in einem flüssigen Medium, das eine Kohlenstoffquelle meist in Form von Zuckern, eine Stickstoffquelle meist in Form von organischen Stickstoffquellen wie Hefeextrakt oder Salzen wie Ammoniumsulfat, Spurenelemente wie Eisen-, Mangan-, Magnesiumsalze und gegebenenfalls Vitamine enthält, bei Temperaturen zwischen 0°C und 100°C, bevorzugt zwischen 10°C bis 60°C unter Sauerstoffbegasung angezogen. Dabei kann der pH der Nährflüssigkeit auf einen festen Wert gehalten werden, das heißt während der Anzucht reguliert werden oder nicht. Die Anzucht kann batch weise, semi batch weise oder kontinuierlich erfolgen. Nährstoffe können zu Beginn der Fermentation vorgelegt oder semikontinuierlich oder kontinuierlich nachgefüttert werden. Die hergestellten mehrfach ungesättigten Fettsäuren können nach dem Fachmann bekannten Verfahren wie oben beschrieben aus den Organismen isoliert werden. Beispielsweise über Extraktion, Destillation, Kristallisation, ggf. Salzfällung und/oder Chromatographie. Die Organismen können dazu vorher noch vorteilhaft aufgeschlossen werden.

Das erfindungsgemäße Verfahren wird, wenn es sich bei den Wirtsorganismen um Mikroorganismen handelt, vorteilhaft bei einer Temperatur zwischen 0°C bis 95°, bevorzugt zwischen 10°C bis 85°C, besonders bevorzugt zwischen 15°C bis 75°C, ganz besonders bevorzugt zwischen 15°C bis 45°C durchgeführt.

Der pH-Wert wird dabei vorteilhaft zwischen pH 4 und 12, bevorzugt zwischen pH 6 und 9, besonders bevorzugt zwischen pH 7 und 8 gehalten.

Das erfindungsgemäße Verfahren kann batchweise, semi-batchweise oder kontinuierlich betrieben werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der merican Society für Bacteriology (Washington D. C., USA, 1981) enthalten.

Diese erfindungsgemäß einsetzbaren Medien umfassen wie oben beschrieben gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z.B. Sojaöl, Sonnenblumenöl, Erdnussöl und/oder Kokosfett, Fettsäuren wie z.B. Palmitinsäure, Stearinsäure und/oder Linolsäure, Alkohole und/oder Polyalkohole wie z. B. Glycerin, Methanol und/oder Ethanol und/oder organische Säuren wie z.B. Essigsäure und/oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak in flüssiger- oder gasform oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor- oder Sulfatsalze von Calcium, Magnesium, Natrium, Kobalt, Molybdän, Kalium, Mangan, Zink, Kupfer und Eisen.

Als Schwefelquelle für die Herstellung von schwefelhaltigen Feinchemikalien, insbesondere von Methionin, können anorganische schwefelhaltige Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder

die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die erfindungsgemäß zur Kultivierung von Mikroorganismen eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht lässt sich während der Anzucht durch Zugabe von basischen Verbindungen wie Natriumhydroxid, Kaliumhydroxid, Ammoniak bzw. Ammoniakwasser oder sauren Verbindungen wie Phosphorsäure oder Schwefelsäure kontrollieren. Zur Kontrolle der Schaumentwicklung können Antischaummittel wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z.B. Umgebungsluft, in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen, insbesondere mehrfach ungesättigte Fettsäuren enthaltenden, Fermentationsbrühen haben üblicherweise eine Trockenmasse von 7,5 bis 25 Gew.-%.

Die Fermentationsbrühe kann anschließend weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden. Vorteilhaft wird die Biomasse nach Abtrennung aufgearbeitet.

Die Fermentationsbrühe kann aber auch ohne Zellabtrennung mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann schließlich zur Gewinnung der darin enthaltenen Fettsäuren aufgearbeitet werden.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Ein weiterer erfindungsgemäßer Gegenstand sind isolierte Nukleinsäuresequenzen, die für Polypeptide mit Δ-5-Elongase codieren, wobei die durch die Nukleinsäuresequenzen codierten Δ-5-Elongasen $C_{20}$-Fettsäuren mit mindestens vier Doppelbindungen im Fettsäuremolekül umsetzen; die vorteilhaft letztlich in Diacylglyceride und/oder Triacylglyceride eingebaut werden.

Vorteilhafte isolierte Nukleinsäuresequenzen sind Nukleinsäuresequenzen, die für Polypeptide mit Δ-5-Elongaseaktivität codieren und die eine Aminosäuresequenz enthalten ausgewählt aus der Gruppe einer Aminosäuresequenz mit der in SEQ ID NO: 115, SEQ ID NO: 116, SEQ ID NO: 139, SEQ ID NO: 140, SEQ ID NO: 141 oder SEQ ID NO: 142 dargestellten Sequenz.

Weitere vorteilhafte isolierte Nukleinsäuresequenzen sind Nukleinsäuresequenzen, die für Polypeptide mit Δ-5-Elongaseaktivität codieren und die eine Kombination der Aminosäuresequenzen enthalten ausgewählt aus der Gruppe:

a) SEQ ID NO: 115 und SEQ ID NO: 139, SEQ ID NO: 115 und SEQ ID NO: 140 oder SEQ ID NO: 139 und SEQ ID NO: 140; oder

b) SEQ ID NO: 116 und SEQ ID NO: 141, SEQ ID NO: 116 und SEQ ID NO: 142 oder SEQ ID NO: 141 und SEQ ID NO: 142; oder

c) SEQ ID NO: 115, SEQ ID NO: 139 und SEQ ID NO: 140 oder SEQ ID NO: 116, SEQ ID NO: 141 und SEQ ID NO: 142.

Die in den Sequenzen SEQ ID NO: 115 (NXXXHXXMYXYYX), SEQ ID NO: 116 (HHXXXXWAWW), SEQ ID NO: 139 (LHXXHH), SEQ ID NO: 140 (TXXQXXQF), SEQ ID NO: 141 (DTXFMV) und SEQ ID NO: 142 (TQAQXXQF) wiedergegebenen Sequenzen stellen konservierte Bereiche der verschiedenen Elongasen wieder. Tabelle 2 gibt die Bedeutung der in den genannten Nukleinsäuresequenzen enhaltenen mit X bezeichneten Aminosäuren wieder (Spalte 3). Auch die bevorzugten Aminosäuren in den verschiedenen Positionen sind der Tabelle zu entnehmen (Spalte 3). Spalte 1 gibt die SEQ ID NO wieder, Spalte 2 die Position in der Sequenz.

Tabelle 2: Bedeutung der mit X bezeichneten Aminosäure in den Konsensus-Sequenzen.

| SEQ ID NO: | Position des X in der Sequenz | Aminosäure | bevorzugte Aminosäure |
|---|---|---|---|
| 115 (NXXXHXXMYXYYX) | 2 | Ser, Cys, Leu, Gly | Cys, Leu |
| 115 | 3 | Thr, Phe, Ile, Ser, Val, Trp, Gly | Phe, Trp |
| 115 | 4 | Val, Ile | Val, Ile |
| 115 | 6 | Val, Ile, Thr | Val, Ile |
| 115 | 7 | Ile, Phe, Val, Leu, Cys | Cys, Val |
| 115 | 10 | Ser, Gly, Tyr, Thr, Ala | Thr, Ser |
| 115 | 13 | Phe, Met, Thr, Leu, Ala, Gly | Leu |
| 116 (HHXXXXWAWW) | 3 | Ala, Ser, Thr | Ala, Ser besonders bevorzugt Ala |
| 116 | 4 | Thr, Met, Val, Leu, Ile, Ser | Leu, Thr besonders bevorzugt Leu |
| 116 | 5 | Val, Thr, Met, Leu, Ile | Ile, Ser besonders bevorzugt Ile |
| 116 | 6 | Val, Met, Leu, Ile, Ala, Pro, Ser, Phe | Ile, Ser besonders bevorzugt Ile |
| 139 LHXXHH | 3 | Val, Tyr, Ile | Val, Thr |
| 139 | 4 | Tyr, Phe | Tyr |
| 140 TXXQXXQF | 2 | Asn, Asp, Thr, Gln, Met, Ser, Ala | Gln |
| 140 | 3 | Thr, Cys, Leu, Met, Ala, Ile, Val, Phe | Ala, Met |
| 140 | 5 | Met, Ile, Leu | Met |
| 140 | 6 | Val, Ile, Leu, Thr, Phe | Leu |
| 141 DTXFMV | 3 | Leu, Ile, Val, Tyr, Phe, Ala | Phe |
| 142 TQAQXXQF | 5 | Met, Ile, Leu | Met, Leu besonders bevorzugt Met |
| 142 | 6 | Val, Ile, Leu, Thr, Phe | Leu |

Besonders vorteilhafte Δ-5-Elongasen enthalten mindestens eine der Sequenzen SEQ ID NO: 116, SEQ ID NO: 141 und/oder SEQ ID NO: 142.

Besonders vorteilhafte isolierte Nukleinsäuresequenzen sind Sequenzen ausgewählt aus der Gruppe:

a) einer Nukleinsäuresequenz mit der in SEQ ID NO: 43, SEQ ID NO: 45, SEQ ID NO: 47, SEQ ID NO: 49, SEQ ID NO: 59, SEQ ID NO: 61, SEQ ID NO: 63; SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 131 oder SEQ ID NO: 133

dargestellten Sequenz,

b) Nukleinsäuresequenzen, die sich als Ergebnis des degenerierten genetischen Codes von der in SEQ ID NO: 44, SEQ ID NO: 46, SEQ ID NO: 48, SEQ ID NO: 50, SEQ ID NO: 60, SEQ ID NO: 62, SEQ ID NO: 64, SEQ ID NO: 66, SEQ ID NO: 68, SEQ ID NO: 76, SEQ ID NO: 78, SEQ ID NO: 80, SEQ ID NO: 84, SEQ ID NO: 86, SEQ ID NO: 114, SEQ ID NO: 132 oder SEQ ID NO: 134 dargestellten Aminosäuresequenz ableiten lassen, oder

c) Derivate der in SEQ ID NO: 43, SEQ ID NO: 45, SEQ ID NO: 47, SEQ ID NO: 49, SEQ ID NO: 59, SEQ ID NO: 61, SEQ ID NO: 63; SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 131 oder SEQ ID NO: 133 dargestellten Nukleinsäuresequenz, die für Polypeptide mit mindestens 40 % Homologie auf Aminosäureebene mit SEQ ID NO: 44, SEQ ID NO: 46, SEQ ID NO: 48, SEQ ID NO: 50, SEQ ID NO: 60, SEQ ID NO: 62, SEQ ID NO: 64, SEQ ID NO: 66, SEQ ID NO: 68, SEQ ID NO: 76, SEQ ID NO: 78, SEQ ID NO: 80, SEQ ID NO: 84, SEQ ID NO: 85, SEQ ID NO: 113, SEQ ID NO: 131 oder SEQ ID NO: 133 codieren und eine Δ-5-Elongaseaktivität aufweisen.

Ein weiterer Erfindungsgegenstand sind isolierte Nukleinsäuresequenzen, die für Polypeptide mit Δ-6-Elongaseaktivität codieren, ausgewählt aus der Gruppe:

a) einer Nukleinsäuresequenz mit der in SEQ ID NO: 69, SEQ ID NO: 81, SEQ ID NO: 111 oder SEQ ID NO: 183 dargestellten Sequenz,

b) Nukleinsäuresequenzen, die sich als Ergebnis des degenerierten genetischen Codes von der in SEQ ID NO: 70, SEQ ID NO: 82, SEQ ID NO: 112 oder SEQ ID NO: 184 dargestellten Aminosäuresequenz ableiten lassen, oder

c) Derivate der in SEQ ID NO: 69, SEQ ID NO: 81, SEQ ID NO: 111 oder SEQ ID NO: 183 dargestellten Nukleinsäuresequenz, die für Polypeptide mit mindestens 40 % Homologie auf Aminosäureebene mit SEQ ID NO: 70, SEQ ID NO: 82, SEQ ID NO: 112 oder SEQ ID NO: 184 codieren und eine Δ-6-Elongaseaktivität aufweisen.

Ein weiterer Erfindungsgegenstand sind isolierte Nukleinsäuresequenzen, die für Polypeptide mit ω-3-Desaturaseaktivität codieren, ausgewählt aus der Gruppe:

a) einer Nukleinsäuresequenz mit der in SEQ ID NO: 87 oder SEQ ID NO: 105dargestellten Sequenz,

b) Nukleinsäuresequenzen, die sich als Ergebnis des degenerierten genetischen Codes von der in SEQ ID NO: 88 oder SEQ ID NO: 106 dargestellten Aminosäuresequenz ableiten lassen, oder

c) Derivate der in SEQ ID NO: 87 oder SEQ ID NO: 105 dargestellten Nukleinsäuresequenz, die für Polypeptide mit mindestens 60 % Identität auf Aminosäureebene mit SEQ ID NO: 88 oder SEQ ID NO: 106 aufweisen und eine ω-3-Desaturaseaktivität aufweisen.

Ein weiterer Erfindungsgegenstand sind isolierte Nukleinsäuresequenzen, die für Polypeptide mit Δ-6-Desaturaseaktivität codieren, ausgewählt aus der Gruppe:

a) einer Nukleinsäuresequenz mit der in SEQ ID NO: 89 oder in SEQ ID NO: 97 dargestellten Sequenz,

b) Nukleinsäuresequenzen, die sich als Ergebnis des degenerierten genetischen Codes von der in SEQ ID NO: 90 oder SEQ ID NO: 98 dargestellten Aminosäuresequenz ableiten lassen, oder

c) Derivate der in SEQ ID NO: 89 oder SEQ ID NO: 97 dargestellten Nukleinsäuresequenz, die für Polypeptide mit mindestens 40 % Homologie auf Aminosäureebene mit SEQ ID NO: 90 oder SEQ ID NO: 98 codieren und eine Δ-6-Desaturaseaktivität aufweisen.

Ein weiterer Erfindungsgegenstand sind isolierte Nukleinsäuresequenzen, die für Polypeptide mit Δ-5-Desaturaseaktivität codieren, ausgewählt aus der Gruppe:

a) einer Nukleinsäuresequenz mit der in SEQ ID NO: 91, SEQ ID NO: 93, SEQ ID NO: 99 oder in SEQ ID NO: 101 dargestellten Sequenz,

b) Nukleinsäuresequenzen, die sich als Ergebnis des degenerierten genetischen Codes von der in SEQ ID NO: 92, SEQ ID NO: 94, SEQ ID NO: 100 oder in SEQ ID NO: 102 dargestellten Aminosäuresequenz ableiten lassen, oder

c) Derivate der in SEQ ID NO: 91, SEQ ID NO: 93, SEQ ID NO: 99 oder in SEQ ID NO: 101 dargestellten Nukleinsäuresequenz, die für Polypeptide mit mindestens 40 % Homologie auf Aminosäureebene mit SEQ ID NO: 92, SEQ ID NO: 94, SEQ ID NO: 100 oder in SEQ ID NO: 102 codieren und eine Δ-5-Desaturaseaktivität aufweisen.

Ein weiterer Erfindungsgegenstand sind isolierte Nukleinsäuresequenzen, die für Polypeptide mit Δ-4-Desaturaseaktivität codieren, ausgewählt aus der Gruppe:

a) einer Nukleinsäuresequenz mit der in SEQ ID NO: 95 oder in SEQ ID NO: 103 dargestellten Sequenz,

b) Nukleinsäuresequenzen, die sich als Ergebnis des degenerierten genetischen Codes von der in SEQ ID NO: 96 oder SEQ ID NO: 104 dargestellten Aminosäuresequenz ableiten lassen, oder

c) Derivate der in SEQ ID NO: 95 oder SEQ ID NO: 103 dargestellten Nukleinsäuresequenz, die für Polypeptide mit mindestens 40 % Homologie auf Aminosäureebene mit SEQ ID NO: 96 oder SEQ ID NO: 104 codieren und eine Δ-4-Desaturaseaktivität aufweisen.

Ein weiterer Erfindungsgegenstand sind isolierte Nukleinsäuresequenzen, die für Polypeptide mit Δ-12-Desaturaseaktivität codieren, ausgewählt aus der Gruppe:

a) einer Nukleinsäuresequenz mit der in SEQ ID NO: 107 oder in SEQ ID NO: 109 dargestellten Sequenz,

b) Nukleinsäuresequenzen, die sich als Ergebnis des degenerierten genetischen Codes von der in SEQ ID NO: 108 oder SEQ ID NO: 110 dargestellten Aminosäuresequenz ableiten lassen, oder

c) Derivate der in SEQ ID NO: 107 oder SEQ ID NO: 109 dargestellten Nukleinsäuresequenz, die für Polypeptide mit mindestens 50 % Homologie auf Aminosäureebene mit SEQ ID NO: 108 oder SEQ ID NO: 110 codieren und eine Δ-12-Desaturaseaktivität aufweisen.

Ein weiterer Erfindungsgegenstand sind Genkonstrukte, die die erfindungsgemäßen Nukleinsäuresequenzen SEQ ID NO: 43, SEQ ID NO: 45, SEQ ID NO: 47, SEQ ID NO: 49, SEQ ID NO: 59, SEQ ID NO: 61, SEQ ID NO: 63; SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 enthalten, wobei die Nukleinsäure funktionsfähig mit einem oder mehreren Regulationssignalen verbunden ist. Zusätzlich können weitere Biosynthesegene des Fettsäure- oder Lipidstoffwechsels enthält ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) im Genkonstrukt enthalten sein. Vorteilhaft sind zusätzlich Biosynthesegene des Fettsäure- oder Lipidstoffwechsels ausgewählt aus der Gruppe der Δ-4-Desaturase, Δ-5-Desaturase, Δ-6-Desaturase, Δ-8-Desatuase, Δ-9-Desaturase, Δ-12-Desaturase, A-6-Elongase, A-9-Elongase oder ω-3-Desaturase enthalten.

Vorteilhaft stammen alle die im erfindungsgemäßen Verfahren verwendeten Nukleinsäuresequenzen aus einem eukaryontischen Organismus wie einer Pflanze, einem Mikroorganismus oder einem Tier. Bevorzugt stammen die Nukleinsäuresequenzen aus der Ordnung Salmoniformes, Xenopus oder Ciona, Algen wie Mantoniella, Crypthecodinium, Euglena oder Ostreococcus, Pilzen wie der Gattung Phytophtora oder von Diatomeen wie den Gattungen Thalassiosira oder Phaeodactylum.

Die im Verfahren verwendeten Nukleinsäuresequenzen, die für Proteine mit ω-3-Desaturase-, Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase- oder Δ-9-Elongase-Aktivität codieren, werden vorteilhaft allein oder bevorzugt in Kombination in einer Expressionskassette (= Nukleinsäurekonstrukt), die die Expression der Nukleinsäuren in einem Organismus vorteilhaft einer Pflanze oder einem Mikroorganismus ermöglicht, eingebracht. Es kann im Nukleinsäurekonstrukt mehr als eine Nukleinsäuresequenz einer enzymatischen Aktivität wie z.B. einer Δ-12-Desaturase, Δ-4-Desaturase, Δ-5-Desaturase, Δ-6-Desaturase, Δ-5-Elongase, Δ-6-Elongase und/oder ω-3-Desaturase enthalten sein.

Zum Einbringen werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden in Anlehnung an die zu amplifizierende Sequenz gewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann die Analyse nach gelelektrophoretischer Auftrennung hinsichtlich Qualität und Quantität erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung. Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilze gewährleisten, und die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cis-regulatorische Regionen wie Promotoren und Terminatoren und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter T-DNA, jedoch kein vir-Gen trägt. Dadurch sind letztere Vektoren relativ klein, leicht zu manipulieren und sowohl in E.-coli als auch in Agrobacterium zu replizieren. Zu diesen binären Vektoren gehören Vektoren der Serien pBIB-HYG, pPZP, pBecks, pGreen. Erfindungsgemäß bevorzugt verwendet werden Bin19, pBI101, pBinAR, pGPTV und pCAMBIA. Eine Übersicht über binäre Vektoren und ihre Verwendung gibt Hellens et al, Trends in Plant Science (2000) 5, 446-451. Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittenen und erforderlichenfalls gereinigten Amplifikat mit ähnlich präparierten Vektorfragmenten mit Einsatz von Ligase kloniert. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie die oben beschriebenen Promotoren und Terminatoren. Die Konstrukte lassen sich vorteilhafterweise in Mikroorganismen, insbesondere Escherichia coli und Agrobacterium tumefaciens, unter selektiven Bedingungen stabil propagieren und ermöglichen einen Transfer von heterologer DNA in Pflanzen oder Mikroorganismen.

Unter der vorteilhaften Verwendung von Klonierungsvektoren können die im Verfahren verwendeten Nukleinsäuren, die erfinderischen Nukleinsäuren und Nukleinsäurekonstrukte in Organismen wie Mikroorganismen oder vorteilhaft Pflanzen eingebracht werden und damit bei der Pflanzentransformation verwendet werden, wie denjenigen, die veröffentlicht sind in und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225)). Die im Verfahren verwendeten Nukleinsäuren, die erfinderischen Nukleinsäuren und Nukleinsäurekonstrukte und/oder Vektoren lassen sich damit zur gentechnologischen Veränderung eines breiten Spektrums an Organismen vorteilhaft an Pflanzen verwenden, so dass diese bessere und/oder effizientere Produzenten von PUFAs werden.

Es gibt eine Reihe von Mechanismen, durch die eine Veränderung des erfindungsgemäßen Δ-12-Desaturase-, Δ-5-Elongase-, A-6-Elongase, Δ-5-Desaturase-, Δ-4-Desaturase-, Δ-6-Desaturase- und/oder ω-3-Desaturase-Proteins sowie der weiteren im Verfahren verwendeten Proteine wie die Δ-12-Desaturase-, Δ-9-Elongase-, Δ-6-Desaturase-, Δ-8-Desaturase-, Δ-6-Elongase-, Δ-5-Desaturase- oder Δ-4-Desaturase-Proteine möglich ist, so dass die Ausbeute, Produktion und/oder Effizienz der Produktion der vorteilhaft mehrfach ungesättigten Fettsäuren in einer Pflanze bevorzugt in einer Ölfruchtpflanze oder einem Mikroorganismus aufgrund dieses veränderten Proteins direkt beeinflusst werden kann. Die Anzahl oder Aktivität der Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Proteine oder -Gene kann erhöht werden, so dass größere Mengen der Genprodukte und damit letztlich größere Mengen der Verbindungen der allgemeinen Formel I hergestellt werden. Auch eine de novo Synthese in einem Organismus, dem die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Genes in einen Organismus allein oder in Kombination mit anderen Genen in eine Zelle kann nicht nur den Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Durch Optimierung der Aktivität oder Erhöhung der Anzahl einer oder mehrerer Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Gene, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die im erfindungsgemäßen Verfahren verwendeten isolierten Nukleinsäuremoleküle codieren für Proteine oder Teile von diesen, wobei die Proteine oder das einzelne Protein oder Teile davon eine Aminosäuresequenz enthält, die ausreichend homolog zu einer Aminosäuresequenz ist, die in den Sequenzen SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138 oder SEQ ID NO: 184 dargestellt ist, so dass die Proteine oder Teile davon noch eine Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Aktivität aufweisen. Vorzugsweise haben die Proteine oder Teile davon, die von dem Nukleinsäuremolekül/den Nukleinsäuremolekülen kodiert wird/werden, noch seine wesentliche enzymatische Aktivität und die Fähigkeit, am Stoffwechsel von zum Aufbau von Zellmembranen oder Lipidkörperchen in Organismen vorteilhaft in Pflanzen notwendigen Verbindungen oder am Transport von Molekülen über diese Membranen teilzunehmen. Vorteilhaft sind die von den Nukleinsäuremolekülen kodierten Proteine zu mindestens etwa 50 %, vorzugsweise mindestens etwa 60 % und stärker bevorzugt mindestens etwa 70 %, 80 % oder 90 % und am stärksten bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr identisch zu den in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106 SEQ ID NO: 108, SEQ ID NO: 110, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138 oder SEQ ID NO: 184 dargestellten Aminosäuresequenzen. Im Sinne der Erfindung ist unter Homologie oder homolog, Identität oder identisch zu verstehen.

Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25,351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Diese Einstellungen wurden, falls nicht anders angegeben, immer als Standardeinstellungen für Sequenzvergleiche verwendet wurden.

Unter wesentlicher enzymatischer Aktivität der im erfindungsgemäßen Verfahren verwendeten Δ-12-Desaturase-, ω-3-Desaturase-, Δ-9-Elongase, Δ-6-Desaturase, Δ-8-Desaturase, Δ-6-Elongase, Δ-5-Desaturase, Δ-5-Elongase oder Δ-4-Desaturase ist zu verstehen, dass sie gegenüber den durch die Sequenz mit SEQ ID NO: 1, SEQ ID NO: 3,

SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 und deren Derivate codierten Proteinen/Enzymen im Vergleich noch mindestens eine enzymatische Aktivität von mindestens 10 %, bevorzugt 20 %, besonders bevorzugt 30 % und ganz besonders 40 % aufweisen und damit am Stoffwechsel von zum Aufbau von Fettsäuren, Fettsäureester wie Diacylglyceride und/oder Triacylglyceride in einem Organismus vorteilhaft einer Pflanze oder Pflanzenzelle notwendigen Verbindungen oder am Transport von Molekülen über Membranen teilnehmen können, wobei $C_{18}$-, $C_{20}$- oder $C_{22}$-Kohlenstoffketten im Fettsäuremolekül mit Doppelbindungen an mindestens zwei, vorteilhaft drei, vier, fünf oder sechs Stellen gemeint sind.

Vorteilhaft im Verfahren verwendbare Nukleinsäuren stammen aus Bakterien, Pilzen, Diatomeen, Tieren wie Caenorhabditis oder Oncorhynchus oder Pflanzen wie Algen oder Moosen wie den Gattungen Shewanella, Physcomitrella, Thraustochytrium, Fusarium, Phytophthora, Ceratodon, Mantoniella, Ostreococcus, Isochrysis, Aleurita, Muscarioides, Mortierella, Borago, Phaeodactylum, Crypthecodinium, speziell aus den Gattungen und Arten Oncorhynchus mykiss, Xenopus laevis, Ciona intestinalis, Thalassiosira pseudonona, Mantoniella squamata, Ostreococcus sp., Ostreococcus tauri, Euglena gracilis, Physcomitrella patens, Phytophtora infestans, Fusarium graminaeum, Cryptocodinium cohnii, Ceratodon purpureus, Isochrysis galbana, Aleurita farinosa, Thraustochytrium sp., Muscarioides viallii, Mortierella alpina, Borago officinalis, Phaeodactylum tricornutum, Caenorhabditis elegans oder besonders vorteilhaft aus Oncorhynchus mykiss, Euglena gracilis, Thalassiosira pseudonana oder Crypthecodinium cohnii.

Alternativ können im erfindungsgemäßen Verfahren Nukleotidsequenzen verwendet werden, die für eine Δ-12-Desaturase, ω-3-Desaturase, Δ-9-Elongase, Δ-6-Desaturase, Δ-8-Desaturase, A-6-Elongase, Δ-5-Desaturase, Δ-5-Elongase oder Δ-4-Desaturase codieren und die an eine Nukleotidsequenz, wie in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 dargestellt, vorteilhaft unter stringenten Bedingungen hybridisieren.

Die im Verfahren verwendeten Nukleinsäuresequenzen werden vorteilhaft in einer Expressionskassette, die die Expression der Nukleinsäuren in Organismen wie Mikroorganismen oder Pflanzen ermöglicht, eingebracht.

Dabei werden die Nukleinsäuresequenzen, die für die Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase codieren, mit einem oder mehreren Regulationssignalen vorteilhafterweise zur Erhöhung der Genexpression funktionell verknüpft. Diese regulatorischen Sequenzen sollen die gezielte Expression der Gene und der Proteinexpression ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette (= Expressionskonstrukt = Genkonstrukt) kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren.

Die Δ-12-Desaturase-, ω-3-Desaturase-, Δ-4-Desaturase-, Δ5-Desaturase-, Δ-6-Desaturase-, Δ-8-Desaturase-, Δ-5-Elongase-, Δ-6-Elongase- und/oder Δ-9-Elongase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen im Wirtsorganismus exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Eine weitere Ausführungsform der Erfindung sind ein oder mehrere Genkonstrukte, die eine oder mehrere Sequenzen enthalten, die durch SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105 SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 oder dessen Derivate definiert sind und für Polypeptide gemäß SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138 oder SEQ ID NO: 184 kodieren. Die genannten Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Proteine führen dabei vorteilhaft zu einer Desaturierung oder Elongierung von Fettsäuren, wobei das Substrat vorteilhaft ein, zwei, drei, vier, fünf oder sechs Doppelbindungen aufweist und vorteilhaft 18, 20 oder 22 Kohlenstoffatome im Fettsäuremolekül aufweist. Gleiches gilt für ihre Homologen, Derivate oder Analoga, die funktionsfähig mit einem oder mehreren Regulationssignalen, vorteilhafterweise zur Steigerung der Genexpression, verbunden sind.

Vorteilhafte Regulationssequenzen für das neue Verfahren liegen beispielsweise in Promotoren vor, wie dem cos-, tac-, trp-, tet-, trp-tet-, lpp-, lac-, lpp-lac-, laclq-, T7-T5-, T3-, gal-, trc-, ara-, SP6-, λ-PR- oder A-PL-Promotor und werden vorteilhafterweise in Gram-negativen Bakterien angewendet. Weitere vorteilhafte Regulationssequenzen liegen beispielsweise in den Gram-positiven Promotoren amy und SP02, in den Hefe- oder Pilzpromotoren ADC1, MFα, AC, P-60, CYC1, GAPDH, TEF, rp28, ADH oder in den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclininduzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der ausführungsgemäße USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen lpt-2- oder lpt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren die im Embryo und/oder im Endosperm aktiv sind. Samen-spezifische Promotoren können prinzipiell sowohl aus dikotolydonen als auch aus monokotolydonen Pflanzen isoliert werden. Im folgenden sind vorteilhafte bevorzugte Promotoren aufgeführt: USP (= unknown seed protein) und Vicilin (Vicia faba) [Bäum-lein et al., Mol. Gen Genet., 1991, 225(3)], Napin (Raps) [US 5,608,152], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und Ipt1(Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder $\alpha$-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der Biosynthesegene in die transgene Pflanze über mehrere Generation sicherzustellen, sollte jede der im Verfahren verwendeten Nukleinsäuren, die für die $\Delta$-12-Desaturase, $\omega$-3-Desaturase, $\Delta$-9-Elongase, $\Delta$-6-Desaturase, $\Delta$-8-Desaturase, $\Delta$-6-Elongase, $\Delta$-5-Desaturase, $\Delta$-5-Elongase und/oder $\Delta$-4-Desaturase codieren, unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Die Expressionskassette ist dabei vorteilhaft so aufgebaut, dass einem Promotor eine geeignete Schnittstelle zur Insertion der zu exprimierenden Nukleinsäure folgt vorteilhaft in einem Polylinker anschließend gegebenenfalls ein Terminator hinter dem Polylinker liegt. Diese Abfolge wiederholt sich mehrfach bevorzugt drei-, vier- oder fünfmal, so dass bis zu fünf Gene in einem Konstrukt zusammengeführt werden und so zur Expression in die transgene Pflanze eingebracht werden können. Vorteilhaft wiederholt sich die Abfolge bis zu dreimal. Die Nukleinsäuresequenzen werden zur Expression über die geeignete Schnittstelle beispielsweise im Polylinker hinter den Promotor inseriert. Vorteilhaft hat jede Nukleinsäuresequenz ihren eigenen Promotor und gegebenenfalls ihren eigenen Terminator. Derartige vorteilhafte Konstrukte werden beispielsweise in DE 10102337 oder DE 10102338 offenbart. Es ist aber auch möglich mehrere Nukleinsäuresequenzen hinter einem Promotor und ggf. vor einem Terminator zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch die Expression wesentlich beeinflusst wird. Es können in der Expressionskassette vorteilhaft unterschiedliche Promotoren wie beispielsweise der USP-, LegB4 oder DC3-Promotor und unterschiedliche Terminatoren verwendet werden. Es ist aber auch möglich nur einen Promotortyp in der Kassette zu verwenden. Dies kann jedoch zu unerwünschten Rekombinationsereignissen führen.

Wie oben beschrieben sollte die Transkription der eingebrachten Gene vorteilhaft durch geeignete Terminatoren am 3'-Ende der eingebrachten Biosynthesegene (hinter dem Stoppcodon) abgebrochen werden. Verwendet werden kann hier z.B. der OCS1 Terminator. Wie auch für die Promotoren, so sollten hier für jedes Gen unterschiedliche Terminatorsequenzen verwendet werden.

Das Genkonstrukt kann, wie oben beschrieben, auch weitere Gene umfassen, die in die Organismen eingebracht werden sollen. Es ist möglich und vorteilhaft, in die Wirtsorganismen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und darin zu exprimieren. Diese Gene können heterologen oder homologen Ursprungs sein. Weiterhin können vorteilhaft im Nukleinsäurekonstrukt bzw. Genkonstrukt weitere Biosynthesegene des Fettsäure- oder Lipidstoffwechsels enthalten sein oder aber diese Gene können auf einem weiteren oder mehreren weiteren Nukleinsäurekonstrukten liegen. Vorteilhaft werden als Biosynthesegene des Fettsäure- oder Lipidstoffwechsels ein Gen ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) oder deren Kombinationen verwendet. Besonders vorteilhafte Nukleinsäuresequenzen sind Biosynthesegene des Fettsäure- oder Lipidstoffwechsels ausgewählt aus der Gruppe der Acyl-CoA:Lysophospholipid-Acyltransferase, ω-3-Desaturase, Δ-4-Desaturase, Δ-5-Desaturase, Δ-6-Desaturase, Δ-8-Desatuase, Δ-9-Desaturase, Δ-12-Desaturase, Δ-5-Elongase, Δ-6-Elongase und/oder Δ-9-Elongase.

Dabei können die vorgenannten Nukleinsäuren bzw. Gene in Kombination mit anderen Elongasen und Desaturasen in Expressionskassetten, wie den vorgenannten, kloniert werden und zur Transformation von Pflanzen Mithilfe von Agrobakterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Die Expressionskassetten können prinzipiell direkt zum Einbringen in die Pflanze verwendet werden oder aber in einen Vektoren eingebracht werden.

Diese vorteilhaften Vektoren, vorzugsweise Expressionsvektoren, enthalten die im Verfahren verwendeten Nukleinsäuren, die für die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen oder Δ-4-Desaturasen codieren, oder ein Nukleinsäurekonstrukt, die die verwendeten Nukleinsäure allein oder in Kombination mit weiteren Biosynthesegenen des Fettsäure- oder Lipidstoffwechsels wie den Acyl-CoA:Lysophospholipid-Acyltransferasen, ω-3-Desaturasen, Δ-4-Desaturasen, Δ-5-Desaturasen, Δ-6-Desaturasen, Δ-8-Desatuasen, Δ-9-Desaturasen, Δ-12-Desaturasen, w3-Desaturasen, Δ-5-Elongasen, Δ-6-Elongasen und/oder Δ-9-Elongasen. Wie hier verwendet, betrifft der Begriff "Vektor" ein Nukleinsäuremolekül, das eine andere Nukleinsäure transportieren kann, an welche es gebunden ist. Ein Vektortyp ist ein "Plasmid", was für eine zirkuläre doppelsträngige DNA-Schleife steht, in die zusätzlichen DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch diese anderen Expressionsvektorformen, wie virale Vektoren, die ähnliche Funktionen ausüben, umfassen. Ferner soll der Begriff Vektor auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die unten beschriebenen Nukleinsäuren oder das oben beschriebene Genkonstrukt in einer Form, die sich zur Expression der verwendeten Nukleinsäuren in einer Wirtszelle eignen, was bedeutet, dass die rekombinanten Expressionsvektoren eine oder mehrere Regulationssequenzen, ausgewählt auf der Basis der zur Expression zu verwendenden Wirtszellen, die mit der zu exprimierenden Nukleinsäuresequenz funktionsfähig verbunden ist, umfasst. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird). Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, welche die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, dem Ausmaß der Expression des gewünschten Proteins usw., abhängen kann.

Die verwendeten rekombinanten Expressionsvektoren können zur Expression von Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in prokaryotischen oder eukaryotischen Zellen gestaltet sein. Dies ist vorteilhaft, da häufig Zwischenschritte der Vektorkonstruktion der Einfachheithalber in Mikroorganismen durchgeführt werden. Beispielsweise können die Δ-12-Desaturase-, ω -3-Desaturase-, Δ-9-Elongase-, Δ-6-Desaturase-, Δ-8-Desaturase-, Δ-6-Elongase-, Δ -5-Desaturase-, Δ-5-Elongase- und/oder Δ-4-Desaturase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene

expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185, Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-111113-B1, λ gt11 or pBdCl, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Bei einer weiteren Ausführungsform des Verfahrens können die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in einzelligen Pflanzenzellen (wie Algen), siehe Falciatore et al., 1999, Marine Biotechnology 1 (3):239-251 und

darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Prometoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814.) oder der durch Wunden induzierbare pinll-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der ipt2- oder ipt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Insbesondere kann die multiparallele Expression der im Verfahren verwendeten Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektiön, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Wirtszellen, die im Prinzip zum Aufnehmen der erfindungsgemäßen Nukleinsäure, des erfindungsgemäßen Genproduktes oder des erfindungsgemäßen Vektors geeignet sind, sind alle prokaryotischen oder eukaryotischen Organismen. Die vorteilhafterweise verwendeten Wirtsorganismen sind Mikroorganismen, wie Pilze oder Hefen oder Pflanzenzellen vorzugsweise Pflanzen oder Teile davon. Pilze, Hefen oder Pflanzen werden vorzugsweise verwendet, besonders bevorzugt Pflanzen, ganz besonders bevorzugt Pflanzen, wie Ölfruchtpflanzen, die große Mengen an Lipidverbindungen enthalten, wie Raps, Nachtkerze, Hanf, Diestel, Erdnuss, Canola, Lein, Soja, Saflor, Sonnenblume, Borretsch, oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) sowie ausdauernde Gräser und Futterfeldfrüchte. Besonders bevorzugte erfindungsgemäße Pflanzen sind Ölfruchtpflanzen, wie Soja, Erdnuss, Raps, Canola, Lein, Hanf, Nachtkerze, Sonnenblume, Saflor, Bäume (Ölpalme, Kokosnuss).

Ein weiterer erfindungsgemäßer Gegenstand sind wie oben beschrieben isolierte Nukleinsäuresequenz, die für Polypeptide mit Δ-5-Elongase-Aktivität codieren, wobei die durch die Nukleinsäuresequenzen codierten Elongase $C_{16}$- und $C_{18}$- Fettsäuren mit einer Doppelbindung und vorteilhaft mehrfach ungesättigte $C_8$-Fettsäuren mit einer Δ6-Doppelbindung und mehrfach ungesättigte $C_{20}$-Fettsäuren mit einer Δ5-Doppelbindung umsetzt. $C_{22}$-Fettsäuren werden nicht elongiert.

Vorteilhafte isolierte Nukleinsäuresequenzen sind Nukleinsäuresequenzen, die für Polypeptide mit Δ-5-Elongaseaktivität codieren und die eine Aminosäuresequenz enthalten ausgewählt aus der Gruppe einer Aminosäuresequenz mit der in SEQ ID NO: 115, SEQ ID NO: 116, SEQ ID NO: 139, SEQ ID NO: 140, SEQ ID NO: 141 oder SEQ ID NO: 142 dargestellten Sequenz.

Weitere vorteilhafte isolierte Nukleinsäuresequenzen sind Nukleinsäuresequenzen, die für Polypeptide mit Δ-5-Elongaseaktivität codieren und die eine Kombination der Aminosäuresequenzen enthalten ausgewählt aus der Gruppe:

a) SEQ ID NO: 115 und SEQ ID NO: 139, SEQ ID NO: 115 und SEQ ID NO: 140 oder SEQ ID NO: 139 und SEQ ID NO: 140; oder

b) SEQ ID NO: 116 und SEQ ID NO: 141, SEQ ID NO: 116 und SEQ ID NO: 142 oder SEQ ID NO: 141 und SEQ ID NO: 142; oder

c) SEQ ID NO: 115, SEQ ID NO: 139 und SEQ ID NO: 140 oder SEQ ID NO: 116, SEQ ID NO: 141 und SEQ ID NO: 142.

Bevorzugte Nukleinsäuresequenzen, die für Polypeptide mit Δ-5-Elongaseaktivität codieren enthalten vorteilhaft die vorgenannten Aminosäuresequenzen. Diese werden in Tabelle 2 näher beschrieben.

Besonders vorteilhafte isolierte Nukleinsäuresequenzen sind Sequenzen ausgewählt aus der Gruppe:

a) einer Nukleinsäuresequenz mit der in SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 131 oder SEQ ID NO: 133 dargestellten Sequenz,

b) Nukleinsäuresequenzen, die sich als Ergebnis des degenerierten genetischen Codes von der in SEQ ID NO: 44, SEQ ID NO: 46, SEQ ID NO: 48, SEQ ID NO: 50, SEQ ID NO: 60, SEQ ID NO: 62, SEQ ID NO: 64, SEQ ID NO: 66, SEQ ID NO: 68, SEQ ID NO: 76, SEQ ID NO: 78, SEQ ID NO: 80, SEQ ID NO: 84, SEQ ID NO: 86, SEQ ID NO: 114, SEQ ID NO: 132 oder SEQ ID NO: 134 dargestellten Aminosäuresequenz ableiten lassen, oder

c) Derivate der in SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 131 oder SEQ ID NO: 133 dargestellten Nukleinsäuresequenz,

die für Polypeptide mit mindestens 40 % Homologie auf Aminosäureebene mit SEQ ID NO: 44, SEQ ID NO: 46, SEQ ID NO: 48, SEQ ID NO: 50, SEQ ID NO: 60, SEQ ID NO: 62, SEQ ID NO: 64, SEQ ID NO: 66, SEQ ID NO: 68, SEQ ID NO: 76, SEQ ID NO: 78, SEQ ID NO: 80, SEQ ID NO: 84, SEQ ID NO: 86, SEQ ID NO: 114, SEQ ID NO: 132 oder SEQ ID NO: 134 aufweisen und eine Δ-5-Elongaseaktivität aufweisen.

Weitere Erfindungsgegenstände sind die im folgenden aufgezählten Nukleinsäuresequenzen, die für Δ-6-Elongasen, ω-3-Desaturasen, Δ-6-Desaturasen, Δ-5-Desaturasen, Δ-4-Desaturasen oder Δ-12-Desaturasen codieren.

Weitere vorteilhafte isolierte Nukleinsäuresequenzen sind Sequenzen ausgewählt aus der Gruppe:

a) einer Nukleinsäuresequenz mit der in SEQ ID NO: 69, SEQ ID NO: 81, SEQ ID NO: 111 oder SEQ ID NO: 183 dargestellten Sequenz,

b) Nukleinsäuresequenzen, die sich als Ergebnis des degenerierten genetischen Codes von der in SEQ ID NO: 70, SEQ ID NO: 82, SEQ ID NO: 112 oder SEQ ID NO: 184 dargestellten Aminosäuresequenz ableiten lassen, oder

c) Derivate der in SEQ ID NO: 69, SEQ ID NO: 81, SEQ ID NO: 111 oder SEQ ID NO: 183 dargestellten Nukleinsäuresequenz, die für Polypeptide mit mindestens 40 % Homologie auf Aminosäureebene mit SEQ ID NO: 70, SEQ ID NO: 82, SEQ ID NO: 112 oder SEQ ID NO: 184 codieren und eine Δ-6-Elongaseaktivität aufweisen.

Isolierte Nukleinsäuresequenzen, die für Polypeptide mit ω-3-Desaturaseaktivität codieren, ausgewählt aus der Gruppe:

a) einer Nukleinsäuresequenz mit der in SEQ ID NO: 87 oder SEQ ID NO: 105 dargestellten Sequenz,

b) Nukleinsäuresequenzen, die sich als Ergebnis des degenerierten genetischen Codes von der in SEQ ID NO: 88 oder SEQ ID NO: 106 dargestellten Aminosäuresequenz ableiten lassen, oder

c) Derivate der in SEQ ID NO: 87 oder SEQ ID NO: 105 dargestellten Nukleinsäuresequenz, die für Polypeptide mit mindestens 60 % Identität auf Aminosäureebene mit SEQ ID NO: 88 oder SEQ ID NO: 106 aufweisen und eine ω-3-Desaturaseaktivität aufweisen.

Isolierte Nukleinsäuresequenzen, die für Polypeptide mit Δ-6-Desaturaseaktivität codieren, ausgewählt aus der Gruppe:

a) einer Nukleinsäuresequenz mit der in SEQ ID NO: 89 oder in SEQ ID NO: 97 dargestellten Sequenz,

b) Nukleinsäuresequenzen, die sich als Ergebnis des degenerierten genetischen Codes von der in SEQ ID NO: 90 oder SEQ ID NO: 98 dargestellten Aminosäuresequenz ableiten lassen, oder

c) Derivate der in SEQ ID NO: 89 oder SEQ ID NO: 97 dargestellten Nukleinsäuresequenz, die für Polypeptide mit mindestens 40 % Homologie auf Aminosäureebene mit SEQ ID NO: 90 oder SEQ ID NO: 98 codieren und eine Δ-6-Desaturaseaktivität aufweisen.

Isolierte Nukleinsäuresequenzen, die für Polypeptide mit Δ-5-Desaturaseaktivität codieren, ausgewählt aus der Gruppe:

a) einer Nukleinsäuresequenz mit der in SEQ ID NO: 91, SEQ ID NO: 93, SEQ ID NO: 99 oder in SEQ ID NO: 101 dargestellten Sequenz,

b) Nukleinsäuresequenzen, die sich als Ergebnis des degenerierten genetischen Codes von der in SEQ ID NO: 92, SEQ ID NO: 94, SEQ ID NO: 100 oder in SEQ ID NO: 102 dargestellten Aminosäuresequenz ableiten lassen, oder

c) Derivate der in SEQ ID NO: 91, SEQ ID NO: 93, SEQ ID NO: 99 oder in SEQ ID NO: 101 dargestellten Nukleinsäuresequenz, die für Polypeptide mit mindestens 40 % Homologie auf Aminosäureebene mit SEQ ID NO: 92, SEQ ID NO: 94, SEQ ID NO: 100 oder in SEQ ID NO: 102 codieren und eine Δ-5-Desaturaseaktivität aufweisen.

Isolierte Nukleinsäuresequenzen, die für Polypeptide mit Δ-4-Desaturaseaktivität codieren, ausgewählt aus der Gruppe:

a) einer Nukleinsäuresequenz mit der in SEQ ID NO: 95 oder in SEQ ID NO: 103 dargestellten Sequenz,

b) Nukleinsäuresequenzen, die sich als Ergebnis des degenerierten genetischen Codes von der in SEQ ID NO: 96 oder SEQ ID NO: 104 dargestellten Aminosäuresequenz ableiten lassen, oder

c) Derivate der in SEQ ID NO: 95 oder SEQ ID NO: 103 dargestellten Nukleinsäuresequenz, die für Polypeptide mit mindestens 40 % Homologie auf Aminosäureebene mit SEQ ID NO: 96 oder SEQ ID NO: 104 codieren und eine Δ-6-Desaturaseaktivität aufweisen.

Isolierte Nukleinsäuresequenzen, die für Polypeptide mit Δ-12-Desaturaseaktivität codieren, ausgewählt aus der Gruppe:

a) einer Nukleinsäuresequenz mit der in SEQ ID NO: 107 oder in SEQ ID NO: 109 dargestellten Sequenz,

b) Nukleinsäuresequenzen, die sich als Ergebnis des degenerierten genetischen Codes von der in SEQ ID NO: 108 oder SEQ ID NO: 110 dargestellten Aminosäuresequenz ableiten lassen, oder

c) Derivate der in SEQ ID NO: 107 oder SEQ ID NO: 109 dargestellten Nukleinsäuresequenz, die für Polypeptide mit mindestens 50 % Homologie auf Aminosäureebene mit SEQ ID NO: 108 oder SEQ ID NO: 110 codieren und eine Δ-12-Desaturaseaktivität aufweisen.

Die oben genannte erfindungsgemäßen Nukleinsäuren stammen von Organismen, wie nicht-humanen Tieren, Ciliaten, Pilzen, Pflanzen wie Algen oder Dinoflagellaten, die PUFAs synthetisieren können.

Vorteilhaft stammen die isolierten oben genannten-Nukleinsäuresequenzen aus der Ordnung Salmoniformes, Xenopus oder Ciona, den Diatomeengattungen Thalassiosira oder Crythecodinium oder aus der Familie der Prasinophyceae wie der Gattung Ostreococcus oder der Familie Euglenaceae wie der Gattung Euglena oder Pythiaceae wie der Gattung Phytophtora stammt.

Ein weiterer erfindungsgemäßer Gegenstand sind wie oben beschrieben isolierte Nukleinsäuresequenz, die für Polypeptide mit ω-3-Desaturase-Aktivität codieren, wobei die durch die Nukleinsäuresequenzen codierten ω-3-Desaturasen $C_{18}$-, $C_{20}$- und $C_{22}$-Fettsäuren mit zwei, drei, vier oder fünf Doppelbindungen und vorteilhaft mehrfach ungesättigte $C_{18}$-Fettsäuren mit zwei oder drei Doppelbindungen und mehrfach ungesättigte $C_{20}$-Fettsäuren mit zwei, drei oder vier Doppelbindungen umsetzt. Auch $C_{22}$-Fettsäuren mit vier oder fünf Doppelbindungen werden desaturiert.

Zu den erfindungsgemäßen Gegenständen gehören außerdem, wie oben beschrieben, isolierte Nukleinsäuresequenz, die für Polypeptide mit Δ-12-Desaturasen, Δ-4-Desaturasen, Δ-5-Desaturasen und Δ-6-Desaturasen codieren, wobei die durch diese Nukleinsäuresequenzen codierten Δ-12-Desaturasen. Δ-4-Desaturasen, Δ-5-Desaturasen oder Δ-6-Desaturasen $C_{18}$-, $C_{20}$- und $C_{22}$-Fettsäuren mit ein, zwei, drei, vier oder fünf Doppelbindungen und vorteilhaft mehrfach ungesättigte $C_{18}$-Fettsäuren mit ein, zwei oder drei Doppelbindungen wie C18:$1^{\Delta 9}$, C18:$2^{\Delta 9,12}$oder C18:3 $^{\Delta 9.12,15}$, mehrfach ungesättigte $C_{20}$-Fettsäuren mit drei oder vier Doppelbindungen wie C20:$3^{\Delta 8,11,14}$ oder C20:$46^{\Delta 8,11,14,17}$ oder mehrfach ungesättigte $C_{22}$-Fettsäuren mit vier oder fünf Doppelbindungen wie C22:$4^{\Delta 7,10,13,16}$ oder C22:$5^{\Delta 7,10,13,16,19}$ umsetzen. Vorteilhaft werden die Fettsäuren in den Phospholipiden oder CoA-Fettsäureestern desaturiert, vorteilhaft in den CoA-Fettsäureester.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturasemolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt flankieren.

Die im Verfahren verwendeten Nukleinsäuremoleküle, z.B. ein Nukleinsäuremolekül mit einer Nukleotidsequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ

ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 oder eines Teils davon, kann unter Verwendung molekularbiologischer Standardtechniken und der hier bereitgestellten Sequenzinformation isoliert werden. Auch kann Mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lässt sich ein Nukleinsäuremolekül, umfassend eine vollständige Sequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 oder einen Teil davon, durch Polymerasekettenreaktion isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105,.SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 gezeigten Sequenzen oder Mithilfe der in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ 10 NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138 oder SEQ ID NO: 184 dargestellten Aminosäuresequenzen erstellen. Eine erfindungsgemäße Nukleinsäure kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Homologe der verwendeten Δ-12-Desaturase-, ω-3-Desaturase-, Δ-9-Etongase-, Δ-6-Desaturase-, Δ-8-Desaturase-, Δ-6-Elongase-, Δ-5-Desaturase-, Δ-5-Elongase- oder Δ-4-Desaturase-Nukleinsäuresequenzen mit der Sequenz SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID

NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO:. 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 bedeutet beispielsweise allelische Varianten mit mindestens etwa 50 oder 60 %, vorzugsweise mindestens etwa 60 oder 70 %, stärker bevorzugt mindestens etwa 70 oder 80 %, 90 % oder 95 % und noch stärker bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr Identität bzw. Homologie zu einer in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 gezeigten Nukleotidsequenzen oder ihren Homologen, Derivaten oder Analoga oder Teilen davon. Weiterhin sind isolierte Nukleinsäuremoleküle einer Nukleotidsequenz, die an eine der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ -ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 gezeigten Nukleotidsequenzen oder einen Teil davon hybridisieren, z.B. unter stringenten Bedingungen hybridisiert. Unter einem Teil gemäß der Erfindung ist dabei zu verstehen, dass mindestens 25 Basenpaare (= bp), 50 bp, 75 bp, 100 bp, 125 bp oder.150 bp, bevorzugt mindestens 175 bp, 200 bp, 225 bp, 250 bp, 275 bp oder 300 bp, besonders bevorzugt 350 bp, 400 bp, 450 bp, 500 bp oder mehr Basenpaare für die Hybridisierung verwendet werden. Es kann auch vorteilhaft die Gesamtsequenz verwendet werden. Allelische Varianten umfassen insbesondere funktionelle Varianten, die sich durch Deletion, Insertion oder Substitution von Nukleotiden aus/in der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der A-12-Desaturase, w-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO:

183 kodierten Protein. Die Homologie wurde über den gesamten Aminosäure- bzw. Nukleinsäuresequenzbereich berechnet. Für das Vergleichen verschiedener Sequenzen stehen dem Fachmann eine Reihe von Programmen,die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Die falls nicht anders angegeben als Standardeinstellungen immer für Sequenzvergleiche verwendet wurden.

Homologen der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 bedeuten beispielsweise auch bakterielle, Pilz- und Pflanzenhomologen, verkürzte Sequenzen, einzelsträngige DNA oder RNA der kodierenden und nicht-kodierenden DNA-Sequenz.

Homologen der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 183 bedeutet auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die vorgenannten Nukleinsäuren und Proteinmoleküle mit Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Aktivität, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Organismen vorteilhaft in Pflanzen, wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekt Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat. Da mehrfach ungesättigte Fettsäuren (= PUFAs) nicht nur einfach in Triacylglycerin sondern auch in Membranlipide eingebaut werden.

Besonders zur Herstellung von PUFAs, beispielsweise Stearidonsäure , Eicosapentaensäure und Docosahexaensäure eignen sich Brasicaceae, Boraginaceen, Primulaceen, oder Linaceen. Besonders vorteilhaft eignet sich Lein (Linum usitatissimum) zur Herstellung von PUFAS mit dem erfindungsgemäßen Nukleinsäuresequenzen vorteilhaft, wie

beschrieben, in Kombination mit weiteren Desaturasen und Elongasen.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weitere elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese $C_{18}$-Kohlenstoff-Fettsäuren müssen auf $C_{20}$ und $C_{22}$ verlängert werden, damit Fettsäuren vom Eicosa- und Docosa-Kettentyp erhalten werden. Mithilfe der im Verfahren verwendeten Desaturasen wie der $\Delta$-12-, $\omega$3-, $\Delta$-4-, $\Delta$-5-, $\Delta$-6- und $\Delta$-8-Desaturasen und/oder der $\Delta$-5-, $\Delta$-6-, $\Delta$-9-Elongasen können Arachidonsäure, Eicosapentaensäure, Docosapentaensäure oder Docosahexaensäure vorteilhaft Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden. Mit den genannten Enzymen können $C_{20}$- und/oder $C_{22}$-Fettsäuren mit mindestens zwei vorteilhaft mindestens drei, vier, fünf oder sechs Doppelbindungen im Fettsäuremolekül, vorzugsweise $C_{20}$- oder $C_{22}$-Fettsäuren mit vorteilhaft vier, fünf oder sechs Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von $C_{20}$ zu $C_{22}$-Fettsäuren,zu Fettsäuren wie $\gamma$-Linolensäure, Dihomo-$\gamma$-linolensäure, Arachidonsäure, Stearidonsäure, Eicosatetraensäure oder Eicosapentaensäure. Substrate der verwendeten Desaturasen und Elongasen im erfindungsgemäßen Verfahren sind $C_{16}$-, $C_{18}$- oder $C_{20}$-Fettsäuren wie zum Beispiel Linolsäure, $\gamma$-Linolensäure, $\alpha$-Linolensäure, Dihomo-$\gamma$-linolensäure, Eicosatetraensäure oder Stearidonsäure. Bevorzugte Substrate sind Linolsäure, $\gamma$-Linotensäure und/oder $\alpha$-Linolensäure, Dihomo-$\gamma$-linolensäure bzw. Arachidonsäure, Eicosatetraensäure oder Eicosapentaensäure. Die synthetisierten $C_{20}$- oder $C_{22}$-Fettsäuren mit mindestens zwei, drei, vier, fünf oder sechs Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden, beispielsweise können Katzen Arachidonsäure nicht mehr synthetisieren.

Unter Phospholipiden im Sinne der Erfindung sind zu verstehen Phosphatidylcholin, Phosphatidylethanolamin, Phosphatidylserin, Phosphatidylglycerin und/oder Phosphatidylinositol vorteilhafterweise Phosphatidylcholin. Die Begriffe Produktion oder Produktivität sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Es umfasst auch die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff Ausbeute oder Produkt/Kohlenstoff-Ausbeute ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht. Die Begriffe Biosynthese oder Biosyntheseweg sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess. Die Begriffe Abbau oder Abbauweg sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess. Der Begriff Stoffwechsel ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Bei einer weiteren Ausführungsform kodieren Derivate des erfindungsgemäßen Nukleinsäuremoleküls wiedergegeben in SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 131, SEQ ID NO: 133 oder SEQ ID NO: 183 Proteine mit mindestens 40 %, vorteilhaft etwa 50 oder 60 %, vorzugsweise mindestens etwa 60 oder 70 % und stärker bevorzugt mindestens etwa 70 oder 80 %, 80 bis 90 %, 90 bis 95 % und am stärksten bevorzugt mindestens etwa 96 %, 97 %, 98 %, 99 % oder mehr Homologie (= Identität) zu einer vollständigen Aminosäuresequenz der SEQ ID NO: 44, SEQ ID NO: 46, SEQ ID NO: 48, SEQ ID NO: 50, SEQ ID NO: 60, SEQ ID NO: 62, SEQ ID NO: 64, SEQ ID NO: 66, SEQ ID NO: 68, SEQ ID NO: 70, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ 10 NO: 110, SEQ ID NO: 112, SEQ ID NO: 114, SEQ ID NO: 132, SEQ ID NO: 134 oder SEQ ID NO: 184. Die Homologie wurde über den gesamten Aminosäure- bzw. Nukleinsäuresequenzbereich berechnet. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm BestFit über den gesamten Sequenzbereich mit folgenden Einstellungen ermittelt: Gap Weight: 50, Length Weight: 3, Average Match: 10.000 und Average Mismatch: 0.000. Die falls nicht anders angegeben als Standardeinstellungen immer für Sequenzvergleiche verwendet wurden.

Die Erfindung umfasst zudem Nukleinsäuremoleküle, die sich von einer der in SEQ ID NO: 43, SEQ ID NO: 45, SEQ ID NO: 47, SEQ ID NO: 49, SEQ ID NO: 59, SEQ ID NO:61, SEQ ID NO: 63, SEQ ID NO: 65, SEQ ID NO: 67, SEQ ID NO: 69, SEQ ID NO: 75, SEQ ID NO: 77; SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 111, SEQ ID NO: 113, SEQ ID NO: 131, SEQ ID NO: 133 oder SEQ ID NO: 183 gezeigten Nukleotidsequenzen (und Teilen davon) aufgrund des degenerierten genetischen Codes unterscheiden und somit die gleiche Δ-12-Desaturase, ω-3-Desaturase, Δ-6-Desaturase, Δ-5-Desaturase, Δ-4-Desaturase, Δ-6-Elongase oder Δ-5-Elongase codieren wie diejenige, die von den in SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81,

SEQ ID NO: 83, SEQ ID NO:85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 113, SEQ ID NO: 131, SEQ ID NO: 133 oder SEQ ID NO: 183 gezeigten Nukleotidsequenzen kodiert wird.

Zusätzlich zu den in SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 113, SEQ ID NO: 131, SEQ ID NO: 133 oder SEQ ID NO: 183 gezeigten Δ-12-Desaturasen, ω-3-Desaturasen, Δ-5-Elongasen, Δ-6-Desaturasen, Δ-5-Desaturasen, Δ-4-Desaturasen oder Δ-6-Elongasen erkennt der Fachmann, dass DNA-Sequenzpolymorphismen, die zu Änderungen in den Aminosäuresequenzen der Δ-12-Desaturase, ω-3-Desaturase, Δ-5-Elongase, Δ-6-Desaturase, Δ-5-Desaturase, Δ-4-Desaturase und/oder Δ-6-Elongase führen, innerhalb einer Population existieren können. Diese genetischen Polymorphismen im Δ-12-Desaturase-, ω-3-Desaturase-, Δ-5-Elongase-, Δ-6-Desaturase-, Δ-5-Desaturase-, Δ-4-Desaturase- und/oder Δ-6-Elongase-Gen können zwischen Individuen innerhalb einer Population aufgrund von natürlicher Variation existieren. Diese natürlichen Varianten bewirken üblicherweise eine Varianz von 1 bis 5 % in der Nukleotidsequenz des Δ-12-Desaturase-, ω-3-Desaturase-, Δ-5-Elongase-, Δ-6-Desaturase-, Δ-5-Desaturase-, Δ-4-Desaturase- und/oder Δ-6-Elongase-Gens. Sämtliche und alle dieser Nukleotidvariationen und daraus resultierende Aminosäurepolymorphismen in der Δ-12-Desaturase, ω-3-Desaturase, Δ-5-Elongase, Δ-6-Desaturase, Δ-5-Desaturase, Δ-4-Desaturase und/oder Δ-6-Elongase, die das Ergebnis natürlicher Variation sind und die funktionelle Aktivität von nicht verändern, sollen im Umfang der Erfindung enthalten sein.

Für das erfindungsgemäße Verfahren vorteilhafte Nukleinsäuremoleküle können auf der Grundlage ihrer Homologie zu den hier offenbarten Δ-12-Desaturase-, ω-3-Desaturase-, Δ-5-Elongase-, Δ-6-Desaturase-, Δ-5-Desaturase-, Δ-4-Desaturase- und/oder Δ-6-Elongase-Nukleinsäuren unter Verwendung der Sequenzen oder eines Teils davon als Hybridisierungssonde gemäß Standard-Hybridisierungstechniken unter stringenten Hybridisierungsbedingungen isoliert werden. Dabei können beispielsweise isolierte Nukleinsäuremoleküle verwendet werden, die mindestens 15 Nukleotide lang sind und unter stringenten Bedingungen mit dem Nukleinsäuremolekülen, die eine Nukleotidsequenz der SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 113, SEQ ID NO: 131, SEQ ID NO: 133 oder SEQ ID NO: 183 umfassen, hybridisieren. Es können auch Nukleinsäuren mindestens 25, 50, 100, 250 oder mehr Nukleotide verwendet werden. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, stärker bevorzugt mindestens etwa 70 % und noch stärker bevorzugt mindestens etwa 75 % oder stärker zueinander homolog sind, gewöhnlich aneinander hybridisiert bleiben. Diese stringenten Bedingungen sind dem Fachmann bekannt und lassen sich in Current Protocols in Molecular Biology, John Wiley & Sons, N. Y. (1989), 6.3.1-6.3.6., finden. Ein bevorzugtes, nicht einschränkendes Beispiel für stringente Hybridisierungsbedingungen sind Hybridisierungen in 6 x Natriumchlorid/Natriumcitrat (sodium chloride/sodiumcitrate = SSC) bei etwa 45°C, gefolgt von einem oder mehreren Waschschritten in 0,2 x SSC, 0,1 % SDS bei 50 bis 65°C. Dem Fachmann ist bekannt, dass diese Hybridisierungsbedingungen sich je nach dem Typ der Nukleinsäure und, wenn beispielsweise organische Lösungsmittel vorliegen, hinsichtlich der Temperatur und der Konzentration des Puffers unterscheiden. Die Temperatur unterscheidet sich beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel im obengenannten Puffer vorliegt, zum Beispiel 50 % Formamid, ist die Temperatur unter Standardbedingungen etwa 42°C. Vorzugsweise sind die Hybridisierungsbedingungen für DNA:DNA-Hybride zum Beispiel 0,1 x SSC und 20°C bis 45°C, vorzugsweise zwischen 30°C und 45°C. Vorzugsweise sind die Hybridisierungsbedingungen für DNA:RNA-Hybride zum Beispiel 0,1 x SSC und 30°C bis 55°C, vorzugsweise zwischen 45°C und 55°C. Die vorstehend genannten Hybridisierungstemperaturen sind beispielsweise für eine Nukleinsäure mit etwa 100 bp (= Basenpaare) Länge und einem G + C-Gehalt von 50 % in Abwesenheit von Formamid bestimmt. Der Fachmann weiß, wie die erforderlichen Hybridisierungsbedingungen anhand von Lehrbüchern, wie dem vorstehend erwähnten oder aus den folgenden Lehrbüchern Sambrook et al., "Molecular Cloning", Cold Spring Harbor Laboratory, 1989; Harnes und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Zur Bestimmung der prozentualen Homologie (= Identität) von zwei Aminosäuresequenzen (z.B. einer der Sequenzen der SEQ ID NO: 44, SEQ ID NO: 46, SEQ ID NO: 48, SEQ ID NO: 50, SEQ ID NO: 60, SEQ ID NO: 62,

SEQ ID NO: 64, SEQ ID NO: 66, SEQ ID NO: 68, SEQ ID NO: 70, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 114, SEQ ID NO: 132, SEQ ID NO: 134 oder SEQ ID NO: 184) oder von zwei Nukleinsäuren (z.B. SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 113, SEQ ID NO: 131, SEQ ID NO: 133 oder SEQ ID NO: 183) werden die Sequenzen zum Zweck des optimalen Vergleichs untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an identischen Positionen, die den Sequenzen gemeinsam sind (d.h. % Homologie = Anzahl der identischen Positionen/Gesamtanzahl der Positionen x 100). Die Begriffe Homologie und Identität sind damit als Synonym anzusehen. Die verwendeten Programme bzw. Algorithmen sind oben beschrieben.

Ein isoliertes Nukleinsäuremolekül, das für eine Δ-12-Desaturase, ω-3-Desaturase, Δ-6-Desaturase, Δ-5-Desaturase, Δ-4-Desaturase, Δ-5-Elongase und/oder Δ-6-Elongase kodiert, die zu einer Proteinsequenz der SEQ ID NO: 44, SEQ ID NO: 46, SEQ ID NO: 48, SEQ ID NO: 50, SEQ ID NO: 60, SEQ ID NO: 62, SEQ ID NO: 64, SEQ ID NO: 66, SEQ ID NO: 68, SEQ ID NO: 70, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, SEQ ID NO: 108, SEQ ID NO: 110, SEQ ID NO: 114, SEQ ID NO: 132, SEQ ID NO: 134 oder SEQ ID NO: 184 homolog ist, kann durch Einbringen einer oder mehrerer Nukleotidsubstitutionen, - additionen oder-deletionen in eine Nukleotidsequenz der SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 113, SEQ ID NO: 131, SEQ ID NO: 133 oder SEQ ID NO: 183 erzeugt werden, so dass eine oder mehrere Aminosäuresubstitutionen, - additionen oder -deletionen in das kodierte Protein eingebracht werden. Mutationen können in eine der Sequenzen der SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 113, SEQ ID NO: 131, SEQ ID NO: 133 oder SEQ ID NO: 183 durch Standardtechniken, wie stellenspezifische Mutagenese und PCR-vermittelte Mutagenese, eingebracht werden. Vorzugsweise werden konservative Aminosäuresubstitutionen an einer oder mehreren der vorhergesagten nicht-essentiellen Aminosäureresten hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer Δ-12-Desaturase, ω-3-Desaturase, Δ-6-Desaturase, Δ-5-Desaturase, Δ-4-Desaturase, Δ-5-Elongase oder Δ-6-Elongase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der Δ-12-Desaturase, ω-3-Desaturase, Δ-6-Desaturase, Δ-5-Desaturase, Δ-4-Desaturase, Δ-5-Elongase oder Δ-6-Elongase kodierenden Sequenz eingebracht werden, z.B. durch Sättigungsmutagenese, und die resultierenden Mutanten können nach der hier beschriebenen Δ-12-Desaturase-, ω-3-Desaturase-, Δ-6-Desaturase-, Δ-5-Desaturase-, Δ-4-Desaturase-, Δ-5-Elongase- oder Δ-6-Elongase-Aktivität durchmustert werden, um Mutanten zu identifizieren, die die Δ-12-Desaturase-, ω-3-Desaturase-, Δ-6-Desaturase-, Δ-5-Desaturase-, Δ-4-Desaturase-, Δ-5-Elongase- oder Δ-6-Elongase-Aktivität beibehalten haben. Nach der Mutagenese einer der Sequenzen SEQ ID NO: 43, SEQ ID NO: 45, SEQ ID NO: 47,SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85,

SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 113, SEQ ID NO: 131, SEQ ID NO: 133 oder SEQ ID NO: 183 kann das kodierte Protein rekombinant exprimiert werden, und die Aktivität des Proteins kann z.B. unter Verwendung der hier beschriebenen Tests bestimmt werden.

Weitere Erfindungsgegenstände sind transgene nicht-humane Organismen, die die erfindungsgemäßen Nukleinsäuren SEQ ID NO: 43, SEQ ID NO: 45, SEQ ID NO: 47, SEQ ID NO: 49, SEQ ID NO: 59, SEQ ID NO: 61, SEQ ID NO: 63, SEQ ID NO: 65, SEQ ID NO: 67, SEQ ID NO: 69, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 105, SEQ ID NO: 107, SEQ ID NO: 109, SEQ ID NO: 113, SEQ ID NO: 131, SEQ ID NO: 133 oder SEQ ID NO: 183 enthalten oder ein Genkonstrukt oder einen Vektor, die diese erfindungsgemäßen Nukleinsäuresequenzen enthalten. Vorteilhaft handelt es sich bei dem nicht-humanen Organismus um einen Mikroorganismus, ein nicht-humanes Tier oder eine Pflanze, besonders bevorzugt um eine Pflanze.

Diese Erfindung wird durch die nachstehenden Beispiele weiter veranschaulicht, die nicht als beschränkend aufgefasst werden sollten. Der Inhalt sämtlicher in dieser Patentanmeldung zitierten Literaturstellen, Patentanmeldungen, Patente und veröffentlichten Patentanmeldungen ist hier durch Bezugnahme aufgenommen.

Beispiele

Beispiel 1: Allgemeine Klonierungsverfahren:

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

Beispiel 2: Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

Beispiel 3: Klonierung von Genen aus Oncorhynchus mykiss

Durch Suche nach konservierten Bereichen in den Proteinsequenzen entsprechend der in der Anmeldung aufgeführten Elongase-Gene wurden zwei Sequenzen mit entsprechenden Motiven in der Sequenzdatenbank von Genbank identifiziert.

| Gen-Name | Genbank No | Aminosäuren |
|---|---|---|
| OmELO2 | CA385234, CA364848, CA366480 | 264 |
| OmELO3 | CA360014, CA350786 | 295 |

Gesamt-RNA von Oncoryhnchus mykiss wurde mit Hilfe des RNAeasy Kits der Firma Qiagen (Valencia, CA, US) isoliert. Aus der Gesamt-RNA wurde mit Hilfe von oligo-dT-Cellulose poly-A+ RNA (mRNA) isoliert (Sambrook et al., 1989). Die RNA wurde mit dem Reverse Transcription System Kit von Promega revers transcribiert und die synthetisierte cDNA in den lambda ZAP Vektor (lambda ZAP Gold, Stratagene) kloniert. Entsprechend Herstellerangaben wurde die cDNA zur Plasmid-DNA entpackt. Die cDNA-Plasmid-Bank wurde dann für die PCR zur Klonierung von Expressionsplasmiden verwendet.

Beispiel 4: Klonierung von Expressionsplasmiden zur heterologen Expression in Hefen

Für die Klonierung der zwei Sequenzen zur heterologen Expression in Hefen wurden folgende Oligonukleotide für die PCR-Reaktion verwendet:

| Primer | Nukleotidsequenz |
|---|---|
| 5' f* OmELO2 | 5' aagcttacataatggcttcaacatggcaa (SEQ ID NO: 179) |
| 3' r* OmELO2 | 5' ggatccttatgtcttcttgctcttcctgtt (SEQ ID NO: 180) |
| 5' f OmELO3 | 5' aagcttacataatggagacttttaat (SEQ ID NO: 181) |
| 3' r OmELO3 | 5' ggatccttcagtcccccctcactttcc (SEQ ID NO: 182) |
| * f: forward, r: reverse | |

Zusammensetzung des PCR-Ansatzes (50 µL):

5,00 µL Template cDNA
5,00 µL 10x Puffer (Advantage-Polymerase)+ 25mM MgCl2
5,00 µL 2mM dNTP
1,25 µL je Primer (10 pmol/µL)
0,50 µL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR Produkt wurde für 2 h bei 37 °C mit den Restriktionsenzymen HindIII und BamHI inkubiert. Der Hefe-Expressionsvektor pYES3 (Invitrogen) wurde in gleicherweise inkubiert. Anschliessend wurde das 812 bp bzw. 905 bp große PCR Produkt sowie der Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und Elongase cDNA ligiert. Dazu wurde das Rapid Ligation Kit von Roche verwendet. Die entstandenen Plasmide pYES3-OmELO2 und pYES3-OmELO3 wurden durch Sequenzierung verifiziert und in den Saccharomyces Stamm INVSc1 (Invitrogen) durch Elektroporation (1500 V) transformiert. Zur Kontrolle wurde pYES3 parallel transformiert. Anschliessend wurden die Hefen auf Komplett-Minimalmedium ohne Tryptophan mit 2 % Glucose ausplattiert. Zellen, die auf ohne Tryptophan im Medium wachstumsfähig waren, enthalten damit die entsprechenden Plasmide pYES3, pYES3-OmELO2 (SEQ ID NO: 51) und pYES3-OmELO3 (SEQ ID NO: 53). Nach der Selektion wurden je zwei Transformaten zur weiteren funktionellen Expression ausgewählt.

Beispiel 5: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende des kodierenden Sequenz eingefügt:

PSUN-OmELO2
Forward: 5'-GCGGCCGCATAATGGCTTCAACATGGCAA (SEQ ID NO: 175)
Reverse: 3'-GCGGCCGCTTATGTCTTCTTGCTCTTCCTGTT (SEQ ID NO: 176)
PSUN-OMELO3
Forward: 5'-GCGGCCGCataatggagacttttaat (SEQ ID NO: 177)
Reverse: 3'-GCGGCCGCtcagtcccccctcactttcc (SEQ ID NO: 178)

Zusammensetzung des PCR-Ansatzes (50 µL):

5,00 µL Template cDNA
5,00 µL 10x Puffer (Advantage-Polymerase)+ 25mM MgCl2
5,00 µL 2mM dNTP
1,25 µL je Primer (10 pmol/µL)
0,50 µL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und PCR-Produkte ligiert. Dazu wurde das Rapid Ligation Kit von Roche verwendet. Die entstandenen Plasmide pSUN-OmELO2 und pSUN-OmELO3 wurde durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standard-primer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 174). Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel 6: Lipidextraktion aus Hefen und Samen:

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) -16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F.

Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester, GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan 1h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

Hefen, die wie unter Beispiel 4 mit den Plasmiden pYES3, pYES3-OmELO2 und pYES3-OmELO3 transformiert wurden, wurden folgendermaßen analysiert:

Die Hefezellen aus den Hauptkulturen wurden durch Zentrifugation (100 x g, 10 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1N methonolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 $\mu$l PE aufgenommen. Die Proben wurden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma).

Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 7: Funktionelle Charakterisierung von OmELO2 und OmELO3:

OmELO2 zeigt keine Elongase-Aktivität, während für OmELO3 eine deutliche Aktivität mit verschiedenen Substraten nachgewiesen werden konnte. Die Substratspezifität der OmElo3 konnte nach Expression und Fütterung verschiedener Fettsäuren ermittelt werden (Figur 2). Die gefütterten Substrate sind in großen Mengen in allen transgenen Hefen nachzuweisen. Alle transgene Hefen zeigen die Synthese neuer Fettsäuren, den Produkten der OmElo3-Reaktion. Dies bedeutet, dass das Gen OmElo3 funktional exprimiert werden konnte.

Figur 2 zeigt, dass die OmElo3 eine Substratspezifität aufweist, die mit hoher Spezifität zur Verlängerung von $\Delta$5- und A6-Fettsäuren mit einer $\omega$3-Doppelbindung führt. Es konnte in geringerer Spezifität des weiteren auch w6-Fettsäuren (C18 und C20) elongiert werden. Stearidonsäure (C18:4 $\omega$3) und Eicosapentaensäure (C20:5 $\omega$3) stellen die besten Substrate für die OmElo3 dar (bis zu 66 % Elongation).

Beispiel 8: Rekonstitution der Synthese von DHA in Hefe

Die Rekonstitution der Biosynthese von DHA (22:6 ω3) wurde ausgehend von EPA (20:5 ω3) bzw. Stearidonsäure (18:4 ω3) durch die Coexpression der OmElo3 mit der Δ-4-Desaturase aus *Euglena gracilis* bzw. der Δ-5-Desaturase aus *Phaeodactylum tricornutum* und der Δ-4-Desaturase aus *Euglena gracilis* durchgeführt. Dazu wurden weiterhin die Expressionsvektoren pYes2-EgD4 und pESCLeu-PtD5 konstruiert. Der o.g. Hefestamm, der bereits mit dem pYes3-OmElo3 (SEQ ID NO: 55) transformiert ist, wurde weiter mit dem pYes2-EgD4 bzw. gleichzeitig mit pYes2-EgD4 und pESCLeu-PtD5 transformiert. Die Selektion der transformierten Hefen erfolgte auf Komplett-Minimalmedium-Agarplatten mit 2% Glucose, aber ohne Tryptophan und Uracil im Falle des pYes3-OmELO/pYes2-EgD4-Stammes und ohne Tryptophan, Uracil und Leucin im Falle des pYes3-OmELO/pYes2-EgD4+pESCLeu-PtD5-Stammes. Die Expression wurde wie oben angegeben durch die Zugabe von 2% (w/v) Galactose induziert. Die Kulturen wurden für weitere 120 h bei 15°C inkubiert.

Figur 3 zeigt die Fettsäureprofile von transgenen Hefen, die mit 20:5 ω3 gefüttert wurden. In der Kontroll-Hefe (A), die mit dem pYes3-OmElo3-Vektor und dem leeren Vektor pYes2 transformiert wurden, wurde 20:5 ω3 sehr effizient zu 22:5 ω3 elongiert (65% Elongation). Die zusätzliche Einführung der EgΔ-4-Desaturase führte zu der Umsetzung von 22:5 ω3 zu 22:6 ω3 (DHA). Die Fettsäure-Zusammensetzung der transgenen Hefen ist in Figure 5 wiedergegeben. Nach der Co-Expression von OmElo3 und EgD4 konnte bis zu 3% DHA in Hefen nachgewiesen werden.

In einem weiteren Co-Expressionsexperiment wurden OmElo3, EgD4 und eine Δ5-Desaturase aus *P. tricornutum* (PtD5) zusammen exprimiert. Die transgenen Hefen wurden mit Stearidonsäure (18:4 ω3) gefüttert und analysiert (Figur 4). Die Fettsäure-Zusammensetzung dieser Hefen ist in Figur 5 aufgeführt. Durch OmElo3 wurde die gefütterte Fettsäure 18:4 ω3 zu 20:4 ω3 elongiert (60% Elongation). Letztere wurde durch die PtD5 zu 20:5 ω3 desaturiert. Die Aktivität der PtD5 betrug 15%. 20:5 ω3 konnte weiterhin durch die OmElo3 zu 22:5 ω3 elongiert werden. Im Anschluß wurde die neu synthetisierte 22:5 ω3 zu 22:6 ω3 (DHA) desaturiert. In diesen Experimenten konnte bis zu 0,7% DHA erzielt werden.

Aus diesen Experimenten geht hervor, dass die in dieser Erfindung verwendeten Sequenzen OmElo3, EgD4 und PtD5 für die Produktion von DHA in eukaryotischen Zellen geeignet sind.

Beispiel 9: Erzeugung von transgenen Pflanzen

a) Erzeugung transgener Rapspflanzen (verändert nach Moloney et al., 1992, Plant Cell Reports, 8:238-242)

Zur Erzeugung transgener Rapspflanzen können binäre Vektoren in Agrobacterium tumefaciens C58C1:pGV2260 oder Escherichia coli genutzt (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland), wird eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 $cm^2$) werden in einer Petrischale mit einer 1:50 Agrobakterienverdünnung für 5-10 Minuten inkubiert. Es folgt eine 3-tägige Colnkubation in Dunkelheit bei 25°C auf 3MS-Medium mit 0,8 % Bacto-Agar. Die Kultivierung wird nach 3 Tagen mit 16 Stunden Licht / 8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse werden auf MS-Medium mit 2 % Saccharose, 250 mg/l Claforan und 0,8 % Bacto-Agar überführt. Bilden sich nach drei Wochen keine Wurzeln, so wurde als Wachstumshormon 2-Indolbuttersäure zum Bewurzeln zum Medium gegeben.

Regenerierte Sprosse werden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Elongase-Expression wie Δ-5-Elongase- oder Δ-6-Elongaseaktivität oder ω-3-Desaturaseaktivität mittels Lipidanalysen untersucht. Linien mit erhöhten Gehalten an C20- und C22 mehrfachungesättigten Fettsäuren können so identifiziert werden.

b) Herstellung von transgenen Leinpflanzen

Die Herstellung von transgenen Leinpflanzen können zum Beispiel nach der Methode von Bell et al., 1999, In Vitro Cell. Dev. Biol.-Plant. 35(6):456-465 mittels particle bombartment erzeugt werden. In der Regel wurde eine Agrobakterien-vermittelte Transformation zum Beispiel nach Mlynarova et al. (1994), Plant Cell Report 13: 282-285 zur Leintransformation verwendet.

Beispiel 10: Klonierung von Δ5-Elongase-Genen aus Thraustochytrium aureum ATCC34304 und Thraustochytrium ssp.

Durch Vergleiche der verschiedenen in dieser Anmeldung gefundenen Elongase-Proteinsequenzen konnten konservierte Nukleinsäurebereiche definiert werden (Histidin-Box: His-Val-X-His-His, Tyrosin-Box: Met-Tyr-X-Tyr-Tyr). Mit Hilfe dieser Sequenzen wurde eine EST-Datenbank von T. aureum ATCC34304 und Thraustochytrium ssp. nach weiteren Δ-5-Elongasen durchsucht. Folgende neue Sequenzen konnten gefunden werden:

| Gen-Name | Nukleotide | Aminosäuren |
|---|---|---|
| BioTaurELO1 | 828 bp | 275 |
| TL16y2 | 831 | 276 |

Gesamt-RNA von T. aureum ATCC34304 und Thraustochytrium ssp. wurde mit Hilfe des RNAeasy Kits der Firma Qiagen (Valencia, CA, US) isoliert. Aus der Gesamt-RNA wurde mit Hilfe des PolyATract Isolierungssystems (Promega) mRNA isoliert. Die mRNA wurde mit dem Marathon cDNA Amplification-Kit (BD Biosciences) reverse transkribiert und entsprechend der Herstellerangaben Adaptoren ligiert. Die cDNA-Bank wurde dann für die PCR zur Klonierung von Expressionsplasmiden mittels 5'- und 3'-RACE (rapid amplification of cDNA ends) verwendet.

Beispiel 11: Klonierung von Expressionsplasmiden zur heterologen Expression in Hefen

Für die Klonierung der Sequenz zur heterologen Expression in Hefen wurden folgende Oligonukleotide für die PCR-Reaktion verwendet:

| Primer | Nukleotidsequenz |
|---|---|
| 5' f* BioTaurELO1 | 5' gacataatgacgagcaacatgag (SEQ ID NO: 170) |
| 3' r* BioTaurELO1 | 5' cggcttaggccgacttggccttggg (SEQ ID NO: 171) |
| 5'f*TL16y2 | 5' agacataatggacgtcgtcgagcagcaatg (SEQ ID NO: 172) |
| 3'r*TL16y2 | 5' ttagatggtcttctgcttcttgggcgcc (SEQ ID NO: 173) |
| * f: forward, r: reverse | |

Zusammensetzung des PCR-Ansatzes (50 µL):

5,00 µL Template cDNA

5,00 µL 10x Puffer (Advantage-Polymerase)+ 25mM MgCl2

5,00 µL 2mM dNTP

1,25 µL je Primer (10 pmol/µL)

0,50 µL pfu-Polymerase

Die Advantage-Polymerase von Clontech wurde eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte BioTaurELO1 (siehe SEQ ID NO: 65) und TL16y2 (siehe SEQ ID NO: 83) wurde für 30 min bei 21 °C mit dem Hefe-Expressionsvektor pYES2.1-TOPO (Invitrogen) inkubiert gemäss Herstellerangaben. Das PCR-Produkt wird dabei durch einen T-Überhang und Aktivität einer Topoisomerase (Invitrogen) in den Vektor ligiert. Nach der Inkubation erfolgte dann die Transformation von E. coli DH5α Zellen. Entsprechende Klone wurden durch PCR identifiziert, die Plasmid-DNA mittels Qiagen DNAeasy-Kit isoliert und durch Sequenzierung verifiziert. Die korrekte

Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-BioTaurELO1 und pYES2.1-TL16y2. Nach der Selektion wurden je zwei Transformaten zur weiteren funktionellen Expression ausgewählt.

Beispiel 12: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende des kodierenden Sequenz eingefügt:

PSUN-BioTaurELO1
Forward: 5-GCGGCCGCATAATGACGAGCAACATGAGC (SEQ ID NO: 166)
Reverse: 3'-GCGGCCGCTTAGGCCGACTTGGCCTTGGG (SEQ ID NO: 167)

PSUN-TL 16y2
Forward: 5'-GCGGCCGCACCATGGACGTCGTCGAGCAGCAATG (SEQ ID NO: 168)
Reverse: 5'-GCGGCCGCTTAGATGGTCTTCTGCTTCTTGGGCGCC (SEQ ID NO: 169)

Zusammensetzung des PCR-Ansatzes (50 μL):

5,00 μL Template cDNA
5,00 μL 10x Puffer (Advantage-Polymerase)+ 25mM MgCl2
5,00 μL 2mM dNTP
1,25 μL je Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37°C mit dem Restriktionsenzym NotI inkubiert. Der Pflanzen-Expressionsvektor pSUN300-USP wurde in gleicherweise inkubiert Anschliessend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel Purification Kit gemäss Herstellerangaben. Anschließend wurden Vektor und PCR-Produkte ligiert. Dazu wurde das Rapid Ligation Kit von Roche verwendet. Die entstandenen Plasmide pSUN-BioTaurELO1 und pSUN-TL16y2 wurden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standard-primer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 165). Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Die Lipidextraktion aus Hefen und Samen erfolgte identisch zu Beispiel 6.

Beispiel 13: Funktionelle Charakterisierung von BioTaurELO1 und TL16y2:

Die Substratspezifität der BioTaurELO1 konnte nach Expression und Fütterung verschiedener Fettsäuren ermittelt werden (Figur 6). Figur 6 zeigt die Fütterungsexperimente zur Bestimmung der Funktionalität und Substratspezifität mit Hefestämmen, die entweder den Vektor pYes2.1 (Kontrolle = Control) oder den Vektor pYes2.1-BioTaurELO1 (= BioTaur) mit der Δ-5-Elongase enthalten. In beiden Ansätzen wurde 200 uM γ-Linolensäure und Eicosapentaensäure dem Hefeinkubationsmedium zugesetzt und 24 h inkubiert. Nach Extraktion der Fettsäuren aus den Hefen wurden diese transmethyliert und gaschromatographisch aufgetrennt. Die aus den beiden gefütterten Fettsäuren entstandenen Elongationsprodukte sind durch Pfeile markiert.

Die gefütterten Substrate sind in großen Mengen in allen transgenen Hefen nachzuweisen. Alle transgene Hefen zeigen die Synthese neuer Fettsäuren, den Produkten der BioTaurELO1-Reaktion. Dies bedeutet, dass das Gen BioTaurELO1 funktional exprimiert-werden konnte.

Figur 6 zeigt, dass die BioTaurELO1 eine Substratspezifität aufweist, die mit hoher Spezifität zur Verlängerung von Δ5-und Δ6-Fettsäuren mit einer ω3-Doppelbindung führt. Des weiteren konnten auch ω6-Fettsäuren (C18 und C20) elongiert werden. Es werden γ-Linolensäure (C18:3 ω6) mit 65,28 %, Stearidonsäure (C18:4 ω3) mit 65.66 % und Eicosapentaensäure (C20:5 ω3) mit 22,01 % Konversion umgesetzt. Die Substratspezifitäten der verschiedenen Fütterungsexperimente sind in Tabelle 3 dargestellt (siehe am Ende der Beschreibung).

Die Konversionsrate von GLA bei Fütterung von GLA und EPA betrug 65,28 %. Die Konversionsrate von EPA bei gleicher Fütterung von GLA und EPA betrug 9,99 %. Wurde nur EPA gefüttert, so betrug die Konversionsrate von EPA 22,01 %. Auch Arachidonsäure (= ARA) wurde bei Fütterung umgesetzt. Die Konversionsrate betrug 14,47 %. Auch Stearidonsäure (= SDA) wurde umgesetzt. In diesem Fall betrug die Konversionsrate 65,66 %.

Die Funktionalität und Substratspezifität von TL16y2 konnte nach Expression und Fütterung verschiedener Fettsäuren ermittelt werden. Tabelle 4 zeigt die Fütterungsexperimente. Die Fütterungsversuche wurden in gleicherweise durchgeführt wie für BioTaurELO1 beschrieben. Die gefütterten Substrate sind in großen Mengen in allen transgenen Hefen nachzuweisen. Die transgenen Hefen zeigten die Synthese neuer Fettsäuren, den Produkten der TL16y2-Reaktion (Fig. 11). Dies bedeutet, dass das Gen TL16y2 funktional exprimiert werden konnte.

Tabelle 4: Expression von TL16y2 in Hefe.

| Flächen der gaschromatographischen Analyse in % | | | | | | | | | |
|---|---|---|---|---|---|---|---|---|---|
| Plasmid | Fettsäure | C18:3 (n-6) | C18:4 (n-3) | C20:3 (n-6) | C20:4 (n-6) | C20:4 (n-3) | C20:5 (n-3) | C22:4 (n-6) | C22:5 (n-3) |
| pYES | 250 uM EPA | | | | | | 13,79 | | |
| TL16y2 | 250 uM EPA | | | | | | 25,81 | | 2,25 |
| pYES | 50 uM EPA | | | | | | 5,07 | | |
| TL16y2 | 50 uM EPA | | | | | | 2,48 | | 1,73 |
| pYES | 250 uMGLA | 8,31 | | | | | | | |
| TL16y2 | 250 uM GLA | 3,59 | | 10,71 | | | | | |
| pYES | 250 uM ARA | | | | 16,03 | | | | |
| TL16y2 | 250 uM ARA | | | | 15,2 | | 3,87 | | |
| pYES | 250 uM SDA | | 26,79 | | | 0,35 | | | |
| TL16y2 | 250 uM SDA | | 7,74 | | | 29,17 | | | |

Die in Tabelle 4 wiedergegebenen Ergebnisse zeigen mit TL16y2 gegenüber der Kontrolle folgende prozentuale Umsätze: a) % Umsatz EPA (250 uM): 8 %, b) % Umsatz EPA (50 uM): 41 %, c) % Umsatz ARA: 20,3 %, d) % Umsatz SDA: 79, 4% und e) % Umsatz GLA: 74,9 %.

TL16y2 zeigt damit Δ5-, Δ6- und Δ8-Elongaseaktivität. Dabei ist die Aktivität für C18-Fettsäuren mit Δ6-Doppelbindung am höchsten. Abhängig von der Konzentration an gefütterten Fettsäuren werden dann C20-Fettsäuren mit einer Δ5- bzw. Δ8-Doppelbindung verlängert.

Beispiel 14: Klonierung von Genen aus Ostreococcus tauri

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten zwei Sequenzen mit entsprechenden Motiven in einer Ostreococcus tauri Sequenzdatenbank (genomische Sequenzen) identifiziert werden.

Es handelt sich dabei um die folgenden Sequenzen:

| Gen-Name | SEQ ID | Aminosäuren |
|---|---|---|
| OtELO1, (Δ-5-Elongase) | SEQ ID NO: 67 | 300 |
| OtELO2, (Δ-6-Elongase) | SEQ ID NO: 69 | 292 |

OtElo1 weist die höchste Ähnlichkeit zu einer Elongase aus Danio rerio auf (GenBank AAN77156; ca. 26 % Identität), während OtElo2 die größte Ähnlichkeit zur Physcomitrella Elo (PSE) [ca. 36 % Identität] aufweist (Alignments wurden mit dem tBLASTn-Aalgorithmus (Altschul et äl., J. Mol. Biol. 1990, 215: 403 - 410) durchgeführt.

Die Klonierung wurde wie folgt durchgeführt:

40 ml einer *Ostreococcus tauri* Kultur in der stationären Phase wurden abzentrifugiert und in 100 µl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtElo-DNAs wurde jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel 15: Klonierung von Expressionsplasmiden zur heterologen Expression in Hefen:

Zur Charakterisierung der Funktion der Elongasen aus *Ostreococcus tauri* wurden die offenen Leserahmen der jeweiligen DNAs stromabwärts des Galactose-induzierbaren GAL1-Promotors von pYES2.1N5-His-TOPO (Invitrogen) kloniert, wobei pOTE1 und pOTE2 erhalten wurden.

Der *Saccharomyces cerevisiae-Stamm* 334 wurde durch Elektroporation (1500 V) mit dem Vektor pOTE1 bzw. pOTE2 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der Ot-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.

5 ml CMdum-Flüssigmedium (ohne Uracil) mit 2% Raffinose und 300 µM verschiedener Fettsäuren wurden dann mit den Vorkulturen auf eine $OD_{600}$ von 0,05 angeimpft.

Die Expression wurde durch die Zugabe von 2% (w/v) Galactose induziert. Die Kulturen wurden für weitere 96 h bei 20°C inkubiert.

Beispiel 16: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurden mittels PCR NotI-Schnittstellen am 5' und 3'-Ende der kodierenden Sequenzen eingefügt. Die entsprechenden Primersequenzen wurden von den 5'- und 3-Bereich von OtElo1 und OtElo2 abgeleitet.

Zusammensetzung des PCR-Ansatzes (50 µL):

5,00 µL Template cDNA

5,00 µL 10x Puffer (Advantage-Polymerase)+ 25mM $MgCl_2$
5,00 µL 2mM dNTP
1,25 µL je Primer (10 pmol/µL)
0,50 µL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37°C mit dem Restriktionsenzym NotI inkubiert. Der Pflanzen-Expressionsvektor pSUN300-USP wurde in gleicherweise inkubiert. Anschliessend wurde die PCR Produkte sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäss Herstellerangaben. Anschliessend wurden Vektor und PCR-Produkte ligiert. Dazu wurde das Rapid Ligation Kit von Roche verwendet. Die entstandenen Plasmide pSUN-OtELO1 und pSUN-OtELO2 wurde durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 164).
Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel 17: Expression von OtELO1 und OtELO2 in Hefen

Hefen, die wie unter Beispiel 15 mit den Plasmiden pYES3, pYES3-OtELO1 und pYES3-OtELO2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfemen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 18: Funktionelle Charakterisierung von OtELO1 und OtELO2:

Die Substratspezifität der OtELO1 konnte nach Expression und Fütterung verschiedener Fettsäuren ermittelt werden (Tab.5). Die gefütterten Substrate sind in großen Mengen in allen transgenen Hefen nachzuweisen. Die transgenen Hefen zeigten die Synthese neuer Fettsäuren, den Produkten der OtElo1-Reaktion. Dies bedeutet, dass das Gen

OtElo1 funktional exprimiert werden konnte.

Tabelle 4 zeigt, dass die OtElo1 eine enge Substratspezifität aufweist. Die OtElo1 konnte nur die C20-Fettsäuren Eicosapentaensäure (Figur 7) und Arachidonsäure (Figur 8) elongieren, bevorzugte aber die ω-3-desaturierte Eicosapentaensäure.

Tabelle 5:

| Fettsäuresubstrat | Umsatz (in %) |
|---|---|
| 16:0 | - |
| $16:1^{\Delta 9}$ | - |
| 18:0 | - |
| $18:1^{\Delta 9}$ | - |
| $18:1^{\Delta 11}$ | - |
| $18:2^{\Delta 9,12}$ | - |
| $18:3^{\Delta 6,9,12}$ | - |
| $18:3^{\Delta 5,9,12}$ | - |
| $20:3^{\Delta 8,11,14}$ | - |
| $20:4^{\Delta 5,8,11,14}$ | **10,8 ± 0,6** |
| $20:5^{\Delta 5,8,11.14,17}$ | **46,8 ± 3,6** |
| $22:4^{\Delta 7,10.13,16}$ | - |
| $22:6^{\Delta 4,7,10,13,16,19}$ | - |

Tabelle 5 zeigt die Substratspezifität der Elongase OtElo1 für C20 polyungesättigte Fettsäuren mit einer Doppelbindung in Δ5 Position gegenüber verschiedenen Fettsäuren.

Die Hefen, die mit dem Vektor pOTE1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) ± Standardabweichung wieder.

Die Substratspezifität der OtElo2 (SEQ ID NO: 81) konnte nach Expression und Fütterung verschiedener Fettsäuren ermittelt werden (Tab. 6). Die gefütterten Substrate sind in großen Mengen in allen transgenen Hefen nachzuweisen. Die transgenen Hefen zeigten die Synthese neuer Fettsäuren, den Produkten der OtElo2-Reaktion. Dies bedeutet, dass das Gen OtElo2 funktional exprimiert werden konnte.

Tabelle 6:

| Fettsäuresubstrat | Umsatz (in %) |
|---|---|
| 16:0 | - |
| $16:1^{\Delta 9}$ | - |
| $16:3^{\Delta 7,10,13}$ | - |
| 18:0 | - |
| $18:1^{\Delta 6}$ | - |
| $18:1^{\Delta 9}$ | - |
| $18:1^{\Delta 11}$ | - |
| $18:2^{\Delta 9,12}$ | - |
| $18:3^{\Delta 6,9,12}$ | **15,3±** |
| $18:3^{\Delta 5,9,12}$ | - |
| $18:4^{6\Delta,9,12,15}$ | **21,1±** |

(fortgesetzt)

| Fettsäuresubstrat | Umsatz (in %) |
|---|---|
| $20:2^{\Delta 11,14}$ | - |
| $20:3^{\Delta 8.11,14}$ | - |
| $20:4^{\Delta 5,8,11,14}$ | - |
| $20:5^{\Delta 5,8,11,14.17}$ | - |
| $22:4^{\Delta 7,10,13,16}$ | - |
| $22:5^{\Delta 7,10,13,16,19}$ | - |
| $22:6^{\Delta 4,7,10,13,16,19}$ | - |

Tabelle 6 zeigt die Substratspezifität der Elongase OtElo2 gegenüber verschiedenen Fettsäuren.
Die Hefen, die mit dem Vektor pOTE2 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) ± Standardabweichung wieder.
Die enzymatische Aktivität, die in Tabelle 6 wiedergegeben wird, zeigt klar, dass OTELO2 eine Δ-6-Elongase ist.

Beispiel 19: Klonierung von Genen aus Thalassiosira pseudonana

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten zwei Sequenzen mit entsprechenden Motiven in einer Thalassiosira pseudonana Sequenzdatenbank (genomische Sequenzen) identifiziert werden. Es handelt sich dabei um die folgenden Sequenzen:

| Gen-Name | SEQ ID | Aminosäuren |
|---|---|---|
| TpELO1 (Δ5-Elongase) | 43 | 358 |
| TpELO2 (Δ5-Elongase) | 59 | 358 |
| TpELO3 (Δ6-Elongase) | 45 | 272 |

Eine 2 L Kultur von T. pseudonana wurde in f/2 Medium (Guillard, R.R.L. 1975. Culture of phytoplankton for feeding marine invertebrates. In Culture of Marine Invertebrate Animals (Eds. Smith, W.L. and Chanley, M.H.), Plenum Press, New York, pp 29-60.) für 14 d (= Tage) bei einer Lichtstärke von 80 $E/cm^2$ angezogen. Nach Zentrifugation der Zellen wurde RNA mit Hilfe des RNAeasy Kits der Firma Quiagen (Valencia, CA, US) nach Herstellerangaben isoliert. Die mRNA wurde mit dem Marathon cDNA Amplification-Kit (BD Biosciences) reverse transkribiert und entsprechend den Herstellerangaben Adaptoren ligiert. Die cDNA-Bank wurde dann für die PCR zur Klonierung von Expressionsplasmiden mittels 5'- und 3'-RACE (rapid amplification of cDNA ends) verwendet.

Beispiel 20: Klonierung von Expressionsplasmiden zur heterologen Expression in - Hefen

Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der TpElo-DNAs wurde jeweils mit 1 μL cDNA, 200 μM dNTPs, 2,5 U *Advantage*-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.
Für die Klonierung der Sequenz zur heterologen Expression in Hefen wurden folgende Oligonukleotide für die PCR-Reaktion verwendet:

| Gen-Name und SEQ ID NO: | Primersequenz |
|---|---|
| TpELO1 (Δ5-Elongase), SEQ ID NO: 59 | F:5'-accatgtgctcaccaccgccgtc (SEQ ID NO: 158) |

(fortgesetzt)

| Gen-Name und SEQ ID NO: | Primersequenz |
|---|---|
| | R:5'- ctacatggcaccagtaac (SEQ ID NO: 159) |
| TpELO2 (Δ5-Elongase), SEQ ID NO: 85 | F:5'-accatgtgctcatcaccgccgtc (SEQ ID NO: 160)<br>R:5'-ctacatggcaccagtaac (SEQ ID NO: 161) |
| TpELO3 (Δ6-Elongase), SEQ ID NO:45 | F:5'-accatggacgcctacaacgctgc (SEQ ID NO: 162)<br>R:5'- ctaagcactcttcttcttt (SEQ ID NO: 163) |
| *F=forward primer, R=reverse primer | |

Die PCR Produkte wurde für 30 min bei 21 °C mit dem Hefe-Expressionsvektor - pYES2.1-TOPO (Invitrogen) gemäß Herstellerangaben inkubiert. Das PCR-Produkt wird dabei durch einen T-Überhang und Aktivität einer Topoisomerase (Invitrogen) in den Vektor ligiert. Nach der Inkubation erfolgte dann die Transformation von E. coli DH5α Zellen. Entsprechende Klone wurden durch PCR identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-TpELO1, pYES2.1-TpELO2 und pYES2.1-TpELO3. Nach der Selektion wurden je zwei Transformaten zur weiteren funktionellen Expression ausgewählt.

Beispiel 21: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu wird mit folgendem Primerpaar NotI-Schnittstellen am 5' und 3'-Ende der kodierenden Sequenz eingefügt:.

PSUN-TPELO1
Forward: 5'-GCGGCCGCACCATGTGCTCACCACCGCCGTC (SEQ ID NO: 152)
Reverse: 3'-GCGGCCGCCTACATGGCACCAGTAAC (SEQ ID NO: 153)

PSUN-TPELO2
Forward: 5'-GCGGCCGCACCATGTGCTCATCACCGCCGTC (SEQ ID NO: 154)
Reverse: 3'-GCGGCCGCCTAGATGGCACCAGTAAC (SEQ ID NO: 155)

PSUN-TPELO3
Forward: 5'-GCGGCCGCaccatggacgcctacaacgctgc (SEQ ID NO: 156)
Reverse: 3'-GC-GGCCGCCTAAGCACTCTTCTTCTTT (SEQ ID NO: 157)

Zusammensetzung des PCR-Ansatzes (50μL):

5,00 μL Template cDNA
5,00 μL 10x Puffer (Advantage-Polymerase)+ 25mM. $MgCl_2$
5,00 μL 2mM dNTP
1,25 μL je Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37°C mit dem Restriktionsenzym NotI inkubiert. Der Pflanzen-Expressionsvektor pSUN300-USP wird in gleicherweise inkubiert. Anschliessend werden die PCR Produkte sowie der 7624

bp große Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschließend werden Vektor und PCR-Produkte ligiert. Dazu wird das Rapid Ligation Kit von Roche verwendet. Die entstandenen Plasmide pSUN-TPELO1, pSUN-TPELO2 und pSUN-TPELO3 werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence -and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz: 5'-GTCGACCCGCGGACTAGTGGGCCCTCTAGACCCGGGGGATCC GGATCTGCTGGCTATGAA-3'; SEQ ID NO: 151).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Die Lipidextraktion aus Hefen und Samen erfolgte identisch zu Beispiel 6.

Beispiel 22: Expression von TpELO1, TpELO2 und TpELO3 in Hefen

Hefen, die wie unter Beispiel 4 mit den Plasmiden pYES2, pYES2-TpELO1, pYES2-TpELO2 und pYES2-TpELO3 transformiert wurden, wurden folgendermaßen analysiert:

Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 $\mu$l PEaufgenommen. Die Proben wurden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766;. Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 23: Funktionelle Charakterisierung von TpELO1 und TpEL03:

Die Substratspezifität der TpElo1 konnte nach Expression und Fütterung verschiedener Fettsäuren ermittelt werden (Fig. 9). Die gefütterten Substrate sind in großen Mengen in allen transgenen Hefen nachzuweisen. Die transgenen Hefen zeigten die Synthese neuer Fettsäuren, den Produkten der TpElo1-Reaktion. Dies bedeutet, dass das Gen TpElo1 funktional exprimiert werden konnte.

Tabelle 7 zeigt, dass die TpElo1 eine enge Substratspezifität aufweist. Die TpElo1 konnte nur die C20-Fettsäuren Eicosapentaensäure und Arachidonsäure elongieren, bevorzugte aber die $\omega$-3-desaturierte Eicosapentaensäure.

Die Hefen, die mit dem Vektor pYES2-TpEL01 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 7: Expression von TpELO1 in Hefe. In den Spalten 1 und 3 sind die Kontrolreaktionen für die Spalten 2 (gefüttert 250 $\mu$M 20:4 $\Delta$5,8,11,14) und 4 (gefüttert 250 $\mu$M 20:5 $\Delta$5,8,11,14,17) wiedergegeben.

| | Expression | Expression | Expression | Expression |
|---|---|---|---|---|
| Fettsäuren | 1 | 2 | 3 | 4 |
| 16:0 | 18.8 | 17.8 | 25.4 | 25.2 |
| $16:1^{\Delta 9}$ | 28.0 | 29.8 | 36.6 | 36.6 |
| 18:0 | 5.2 | 5.0 | 6.8 | 6.9 |
| 18:1 | 25.5 | 23.6 | 24.6 | 23.9 |
| $20:4^{\Delta 5,8,11,14}$ | **22.5** | **23.4** | - | - |
| $22:4^{\Delta 7,10,13,16}$ | - | **0.4** | **-** | - |
| $20:5^{\Delta 5,11,14,17}$ | - | - | **6.6** | **6.5** |
| $22:5^{\Delta 7,10,13,16,19}$ | - | - | - | **0.9** |
| **% Umsatz** | **0** | **1.7** | **0** | **12.2** |

Die Substratspezifität der TpElo3 konnte nach Expression und Fütterung verschiedener Fettsäuren ermittelt werden (Fig. 10). Die gefütterten Substrate sind in großen Mengen in allen transgenen Hefen nachzuweisen. Die transgenen Hefen zeigten die Synthese neuer Fettsäuren, den Produkten der TpElo3-Reaktion. Dies bedeutet, dass das Gen TpElo3 funktional exprimiert werden konnte.

Tabelle 8 zeigt, dass die TpElo3 eine enge Substratspezifität aufweist. Die TpElo3 konnte nur die C18-Fettsäuren $\gamma$-Linolensäure und Stearidonsäure elongieren, bevorzugte aber die $\omega$-3-desaturierte Stearidonsäure.

Die Hefen, die mit dem Vektor pYES2-TpELO3 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 8: Expression von TpELO3 in Hefe. Spalte 1 zeigt das Fettsäureprofil von Hefe ohne Fütterung. Spalte 2 zeigt die Kontrollreaktion. In den Spalten 3 bis 6 wurden $\gamma$-Linolensäure, Stearidonsäure, Arachidonsäure und Eicosapentaensäure gefüttert (250 $\mu$M jeder Fettsäure).

| Fettsäuren | 1 | 2 | 3 | 4 | 5 | 6 |
|---|---|---|---|---|---|---|
| 16:0 | 17.9 | 20.6 | 17.8 | 16.7 | 18.8 | 18.8 |
| $16:1^{\Delta 9}$ | 41.7 | 18.7 | 27.0 | 33.2 | 24.0 | 31.3 |
| 18:0 | 7.0 | 7.7 | 6.4 | 6.6 | 5.2 | 6,0 |
| $18:1^{\Delta 9}$ | 33.3 | 16.8 | 24.2 | 31.8 | 25.5 | 26.4 |
| $18:2^{\Delta 9,12}$ | - | **36.1** | **-** | - | - | - |
| $18:3^{\Delta 6,9,12}$ | - | - | **6.1** | - | - | |
| $18:4^{\Delta 6,9,12,15}$ | - | - | - | **1.7** | - | |
| $20:2^{\Delta 11,14}$ | - | **0** | - | - | - | |
| $20:3^{\Delta 8,11,14}$ | - | - | **18.5** | - | - | |
| $20:4^{\Delta 8,11,14,17}$ | - | - | - | **10.0** | - | |
| $20:4^{\Delta 5,8,11,14}$ | - | - | - | - | **22.5** | |
| $22:4^{\Delta 7,11,13,16}$ | - | - | - | - | **0** | |
| $20:5^{\Delta 5,8,11,14,17}$ | - | - | - | - | - | **17.4** |
| $22:5^{\Delta 7,10,13,16,19}$ | - | - | - | - | - | **0** |
| **% Umsatz** | **0** | **0** | **75** | **85** | **0** | **0** |

Beispiel 24: Klonierung eines Expressionsplasmides zur heterologen Expression der Pi-omega3Des in Hefen

Der Pi-omega3Des Klon wurde für die heterologe Expression in Hefen über PCR mit entsprechenden Pi-omega3Des spezifischen Primern in den Hefe-Expressionsvektor pYES3 kloniert. Dabei wurde ausschließlich der für das Pi-omega3Des Protein kodierende offene Leseraster des Gens amplifiziert und mit zwei Schnittstellen für die Klonierung in den pYES3 Expressionsvektor versehen:

Forward Primer 5'-TAAGCTTACATGGCGACGAAGGAGG (SEQ ID NO: 149)
Reverse Primer 5'-TGGATCCACTTACGTGGACTTGGT (SEQ ID NO: 150)

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL des 5'-ATG sowie des 3'-Stopp Primers)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR Produkt wurde für 2 h bei 37°C mit den Restriktionsenzymen HindIII und BamHI inkubiert. Der Hefe-Expressionsvektor pYES3 (Invitrogen) wurde in gleicherweise inkubiert. Anschließend wurde das 1104 bp große PCR Produkt sowie der Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschließend wurden Vektor und Desaturase-cDNA ligiert. Dazu wurde das Rapid Ligation Kit von Roche verwendet. Das entstandene Plasmid pYES3-Pi-omega3Des wurde durch Sequenzierung überprüftt und in den Sacchäromyces Stamm INVSc1 (Invitrogen) durch Elektroporation (1500 V) transformiert. Zur Kontrolle wurde pYES3 parallel transformiert. Anschliessend wurden die Hefen auf Komplett-Minimalmedium ohne Tryptophan mit 2 % Glucose ausplattiert. Zellen, die auf ohne Tryptophan im Medium wachstumsfähig waren, enthalten damit die entsprechenden Plasmide pYES3, pYES3-Pi-omega3Des. Nach der Selektion wurden je zwei Transformaten zur weiteren funktionellen Expression ausgewählt.

Beispiel 25: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende der kodierenden Sequenz eingefügt:.
PSUN-Pi-omega3Des
Reverse: 3'-GCGGCCGCTTACGTGGACTTGGTC (SEQ ID NO: 147)
Forward: 5'-GCGGCCGCatGGCGACGAAGGAGG (SEQ ID NO: 148)
Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C

Denaturierungstemperatur: 1 min 94°C
Elongationstemperatur: 2 min 72°C
Anzahl der Zyklen: 35

Die PCR Produkte wurden für 4 h bei 37°C mit dem Restriktionsenzym NotI inkubiert. Der Pflanzen-Expressionsvektor pSUN300-USP wurde in gleicherweise inkubiert. Anschließend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und PCR-Produkte ligiert. Dazu wurde das Rapid Ligation Kit von Roche verwendet. Das entstandene Plasmid pSUN-Piomega3Des wurde durch Sequenzierung verifiziert.

Beispiel 26: Expression von Pi-omega3Des in Hefen

Hefen, die wie unter Beispiel 24 mit dem Plasmid pYES3 oder pYES3- Pi-omega3Des transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfemen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 $\mu$l PE aufgenommen. Die Proben wurden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.
Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 27: Funktionelle Charakterisierung von Pi-omega3Des:

Die Substratspezifität der Pi-omega3Des konnte nach Expression und Fütterung verschiedener Fettsäuren ermittelt werden (Figur 12 bis 18). Die gefütterten Substrate liegen in großen Mengen in allen transgenen Hefen vor, wodurch die Aufnahme dieser Fettsäuren in die Hefen bewiesen ist. Die transgenen Hefen zeigen die Synthese neuer Fettsäuren, den Produkten der Pi-omega3Des-Reaktion. Dies bedeutet, dass das Gen Pi-omega3Des funktional exprimiert werden konnte.
Figur 12 gibt die Desaturierung von Linolsäure (18:2 $\omega$-6-Fettsäure) zu $\alpha$-Linolensäure (18:3 $\omega$-3-Fettsäure) durch die Pi-omega3Des wieder. Die Synthese der Fettsäuremethylester erfolgte durch saure Methanolyse intakter Zellen, die mit dem Leervektor pYES2 (Figur 12 A) oder dem Vektor pYes3-Pi-omega3Des (Figur 12 B) transformiert worden waren. Die Hefen wurden in Minimalmedium in Gegenwart von C18:$2^{\Delta 9,12}$-Fettsäure (300 $\mu$M) kultiviert. Anschließend wurden die FAMEs über GLC analysiert.
In Figur 13 ist die Desaturierung von $\gamma$-Linolensäure (18:3 $\omega$-6-Fettsäure) zu Stearidonsäure (18:4 $\omega$-3-Fettsäure) durch Pi-omega3Des wiedergegeben. Die Synthese der Fettsäuremethylester erfolgte durch saure Methanolyse intakter Zellen, die mit dem Leervektor pYES2 (Figur 13 A) oder dem Vektor pYes3-Pi-omega3Des (Figur 13 B) transformiert worden waren. Die Hefen wurden in Minimalmedium in Gegenwart von $\gamma$-C18:$3^{\Delta 6,9,12}$-Fettsäure (300 $\mu$M) kultiviert. Anschließend wurden die FAMEs über GLC analysiert.
Figur 14 gibt die Desaturierung von C20:2-w-6-Fettsäure zu C20:3-$\omega$-3-Fettsäure durch Pi-omega3Des wieder. Die Synthese der Fettsäuremethylester erfolgte durch saure Methanolyse intakter Zellen, die mit dem Leervektor pYES2 (Figur 14A) oder dem Vektor pYes3-Pi-omega3Des (Figur 14B) transformiert worden waren. Die Hefen wurden in Minimalmedium in Gegenwart von C20:$2^{\Delta 11,14}$-Fettsäure (300 $\mu$M) kultiviert. Anschließend wurden die FAMEs über GLC analysiert.
Figur 15 gibt die Desaturierung von C20:3-$\omega$-6-Fettsäure zu C20:4-$\omega$-3-Fettsäure durch Pi-omega3Des wieder. Die Synthese der Fettsäuremethylester erfolgte durch saure Methanolyse intakter Zellen, die mit dem Leervektor pYES2 (Figur 15 A) oder dem Vektor pYes3-Pi-omega3Des (Figur 15 B) transformiert worden waren. Die Hefen wurden in Minimalmedium in Gegenwart von C20:$3^{\Delta 8,11,14}$-Fettsäure (300 $\mu$M) kultiviert. Anschließend wurden die FAMEs über GLC analysiert.
In Figur 16 wird die Desaturierung von Arachidonsäure (C20:4-$\omega$-6-Fettsäure) zu Eicosapentaensäure (C20:5-ω-3-Fettsäure) durch die Pi-omega3Des gezeigt.

Die Synthese der Fettsäuremethylester erfolgte durch saure Methanolyse intakter Zellen, die mit dem Leervektor pYES2 (Figur 16 A) oder dem Vektor pYes3-Pi-omega3Des (Figur 16 B) transformiert worden waren. Die Hefen wurden in Minimalmedium in Gegenwart von C20:4$^{\Delta 5,8,11,14}$-Fettsäure (300 μM) kultiviert. Anschließend wurden die FAMEs über GLC analysiert.

Figur 17 gibt die Desaturierung von Docosatetraensäure (C22:4-ω-6-Fettsäure) zu Docosapentaensäure (C22:5-ω-3-Fettsäure) durch Pi-omega3Des wieder. Die Synthese der Fettsäuremethylester erfolgte durch saure Methanolyse intakter Zellen, die mit dem Leervektor pYES2 (Figur 17 A) oder dem Vektor pYes3-Pi-omega3Des (Figur 17 B) transformiert worden waren. Die Hefen wurden in Minimalmedium in Gegenwart von C22:4$^{\Delta 7,10,13,16}$-Fettsäure (300 μM) kultiviert. Anschließend wurden die FAMEs über GLC analysiert.

Die Substratspezifität der Pi-omega3Des gegenüber verschiedenen Fettsäuren ist Figur 18 zu entnehmen. Die Hefen, die mit dem Vektor pYes3-Pi-omega3Des transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt einen Mittelwert aus drei Messungen wieder. Die Umsetzungsraten (% Desaturation) wurden mit der Formel:

$$[Produkt]/[Produkt]+[Substrat]*100$$

errechnet.

Wie unter Beispiel 9 beschrieben kann auch die Pi-omega3Des zur Erzeugung transgener Pflanzen verwendet werden. Aus den Samen dieser Pflanzen kann dann die Lipidextraktion wie unter Beispiel 6 beschrieben erfolgen.

Beispiel 28: Klonierung von Desaturasegenen aus Ostreococcus tauri

Durch Suche nach konservierten Bereichen in den Proteinsequenzen mit Hilfe von konservierten Motiven (His-Boxen, Domergue et al. 2002, Eur. J. Biochem. 269,4105-4113) konnten fünf Sequenzen mit entsprechenden Motiven in einer Ostreococcus tauri Sequenzdatenbank (genomische Sequenzen) identifiziert werden. Es handelt sich dabei um die folgenden Sequenzen:

| Gen-Name | SEQ ID | Aminosäuren | Homologie |
|---|---|---|---|
| OtD4 | SEQ ID NO: 95 | 536 | Δ-4-Desaturase |
| OtD5.1 | SEQ ID NO: 91 | 201 | Δ-5-Desaturase |
| OtD5.2 | SEQ ID NO: 93 | 237 | Δ-5-Desaturase |
| OtD6.1 | SEQ ID NO: 89 | 456 | Δ-6-Desaturase |
| OtFad2 | SEQ ID NO: 107 | 361 | Δ-12-Desaturase |

Die Alignments zur Auffindung von Homologien der einzelnen Gene wurden mit dem tBLASTn-Aalgorithmus (Altschul et al., J. Mol. Biol. 1990, 215: 403 - 410) durchgeführt.

Die Klonierung erfolgte wie folgt:

40 ml einer *Ostreococcus tauri* Kultur in der stationären Phase wurden abzentrifugiert und in 100 μl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtDes-DNAs wurde jeweils mit 1 μl aufgetauten Zellen, 200 μM dNTPs, 2,5 U *Taq*-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Folgende Primer wurden für die PCR eingesetzt:

OtDes6.1 Forward: 5'ggtaccacataatgtgcgtggagacggaaaataacg3' (SEQ ID NO: 145)
OtDes6.1 Reverse: 5'ctcgagttacgccgtctttccggagtgttggcc3' (SEQ ID NO: 146)

Beispiel: 29 Klonierung von Expressionsplasmiden zur heterologen Expression in Hefen:

Zur Charakterisierung der Funktion der Desaturase OtDes6.1 (= Δ-6-Desaturase) aus *Ostreococcus tauri* wurde der offenen Leserahmen der DNA stromabwärts des Galactose-induzierbaren GAL1-Promotors von pYES2.1/V5-His-TOPO (Invitrogen) kloniert, wobei der entsprechenden pYES2.1-OtDes6.1 Klon erhalten wurde. In entsprechender Art und Weise können weitere Desaturase-Gene aus Ostreococcus kloniert werden.

Der *Saccharomyces cerevisiae-Stamm* 334 wurde durch Elektroporation (1500 V) mit dem Vektor pYES2.1-OtDes6.1 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der OtDes6.1 Desaturase wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert. 5 ml CMdum-Flüssigmedium (ohne Uracil) mit 2% Raffinose und 300 μM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel: 30 Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mittels PCR NotI-Schnittstellen am 5' und 3'-Ende der kodierenden Sequenzen eingefügt. Die entsprechenden Primersequenzen werden von den 5'- und 3-Bereich der Desaturasen abgeleitet.

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37°C mit dem Restriktionsenzym NotI inkubiert. Der Pflanzen-Expressionsvektor pSUN300-USP wird in gleicherweise inkubiert. Anschliessend werden die PCR Produkte sowie der Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschliessend werden Vektor und PCR-Produkte ligiert. Dazu wurde das Rapid Ligation Kit von Roche verwendet. Die entstandenen Plasmide werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 144).
Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel: 31 Expression von OtDes6.1 in Hefen

Hefen, die wie unter Beispiel 4 mit den Plasmiden pYES2 und pYES2-OtDes6.2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel: 32 Funktionelle Charakterisierung von Desaturasen aus Ostreococcus:

Die Substratspezifität von Desaturasen kann nach Expression in Hefe (siehe Beispiele Klonierung von Desaturase-Genen, Hefeexpression) durch die Fütterung mittels verschiedener Hefen ermittelt werden. Beschreibungen für die Bestimmung der einzelnen Aktivitäten finden sich in WO 93/11245 für Δ 15-Desaturasen, WO 94/11516 für Δ12-Desaturasen, WO 93/06712, US 5,614,393, US5614393, WO 96/21022, WO0021557 und WO 99/27111 für Δ6-Desaturasen, Qiu et al. 2001, J. Biol. Chem. 276, 31561-31566 für Δ4-Desaturasen, Hong et al. 2002, Lipids 37,863-868 für Δ5-Desaturasen.

Tabelle 9 gibt die Substratspezifität der Desaturase OtDes6.1 gegenüber verschiedenen Fettsäuren wieder. Die Substratspezifität der OtDes6.1 konnte nach Expression und Fütterung verschiedener Fettsäuren ermittelt werden. Die gefütterten Substrate sind in großen Mengen in allen transgenen Hefen nachzuweisen. Die transgenen Hefen zeigten die Synthese neuer Fettsäuren, den Produkten der OtDes6.2-Reaktion (Fig. 20). Dies bedeutet, dass das Gen OtDes6.1 funktional exprimiert werden konnte.

Die Hefen, die mit dem Vektor pYES2-OtDes6.1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) ± Standardabweichung wieder. Die Aktivität entspricht der Konversionsrate errechnet nach [Substrat/(Substrat+Produkt)*100].

Tabelle 9 zeigt, dass die OtDes6.1 eine Substratspezifität für Linol- und Linolensäure (18:2 und 18:3) aufweist, da mit diesen Fettsäuren die höchsten Aktivitäten erreicht werden. Die Aktivität für Ölsäure (18:1) und Palmitoleinsäure (16:1) ist dagegen deutlich geringer. Die bevorzugte Umsetzung von Linol- und Linolensäure zeigt die Eignung dieser Desaturase für die Herstellung von polyungesättigten Fettsäuren.

| Substrate | Aktivität in % |
|---|---|
| $16:1^{\Delta 9}$ | 5,6 |
| $18:1^{\Delta 9}$ | 13,1 |
| $18:2^{\Delta 9,12}$ | 68,7 |
| $18:3^{\Delta 9,12,15}$ | 64,6 |

Figur 20 zeigt die Umsetzung von Linolsäure durch OtDes6.1. Die Analyse der FAMEs erfolgte über Gaschromatographie. Das gefütterte Substrat (C18:2) wird zu γ-C18:3 umgesetzt. Sowohl Edukt als auch das entstandene Produkt sind durch Pfeile markiert.

In Figur 21 wird die Umsetzung von Linolsäure (= LA) und α-Linolensäure (= ALA) in Gegenwart von OtDes6.1 zu γ-Linolensäure (= GLA) bzw. Stearidonsäure (= STA) wiedergegeben (Figur 21A und C). Weiterhin zeigt Figur 21 die Umsetzung von Linolsäure (= LA) und α-Linolensäure (= ALA) in Gegenwart der Δ-6-Desaturase OtDes6.1 zusammen mit der Δ-6-Elongase PSE1 aus Physcomitrella patens (Zank et al. 2002, Plant J. 31:255-268) und der Δ-5-Desaturase

PtD5 aus Phaeodactylum tricornutum (Domergue et al. 2002, Eur. J. Biochem. 269, 4105-4113) zu Dihomo-γ-linolensäure (= DHGLA) und Arachidonsäure (= ARA, Figur 21 B) bzw. zu Dihomostearidonsäure (= DHSTA) bzw. Eicosapentaensäure (= EPA, Figur 21 D). Figur 21 zeigt deutlich, dass die Reaktionsprodukte GLA und STA der Δ-6-Desaturase OtDes6.1 in Gegenwart der Δ-6-Elongase PSE1 fast quantitativ zu DHGLA bzw. DHSTA elongiert wird. Die nachfolgende Desaturierung durch die Δ-5-Desaturase PtD5 erfolgt ebenfalls reibungslos zu ARA bzw. EPA. Es werden ca. 25 - 30% des Elongaseprodukts desaturiert (Figur 21B und D).

Die folgenden Tabelle 10 gibt eine Übersiche über die klonierten Ostreococcus Desaturasen wieder:

| **Ostreococcus tauri Desaturasen** | | | | | | | |
|---|---|---|---|---|---|---|---|
| | | | | | | | |
| **Name** | **bp** | **aa** | **Homologie** | **Cyt. B5** | **His-Box1** | **His-Box2** | **His-Box3** |
| | | | | | | | |
| OtD4 | 1611 | 536 | Δ-4- Desaturase | HPGG | HCANH | WRYHHQVSHH | QVEHHLFP |
| OtD5.1 | 606 | 201 | Δ-5-Desaturase | - | - | - | QWHHLFP |
| OtD5.2 | 714 | 237 | Δ-5-Desaturase | - | - | WRYHHMVSHH | QIEHHLPF |
| OtD6.1 | 1443 | 480 | Δ-6-Desaturase | HPGG | HEGGH | WNSMHNKHH | QVIHHLFP |
| OtFAD2 | 1086 | 361 | Δ-12-Desaturase | - | HECGH | WQRSHAVHH | HVAHH |

Beispiel: 33 Klonierung von Desaturasegenen aus Thalassiosira pseudonana

Durch Suche nach konservierten Bereichen in den Proteinsequenzen mit Hilfe von konservierten Motiven (His-Boxen, siehe Motive) konnten sechs Sequenzen mit entsprechenden Motiven in einer Thalassiosira pseudonana Sequenzdatenbank (genomische Sequenzen) identifiziert werden. Es handelt sich dabei um die folgenden Sequenzen:

| Gen-Name | SEQ ID | Aminosäuren | Homologie |
|---|---|---|---|
| TpD4 | SEQ ID NO: 103 | 503 | Δ-4-Desaturase |
| TpD5-1 | SEQ ID NO: 99 | 476 | Δ-5-Desaturase |
| TpD5-2 | SEQ ID NO: 101 | 482 | Δ-5-Desaturase |
| TpD6 | SEQ ID NO: 97 | 484 | Δ-6-Desaturase |
| TpFAD2 | SEQ ID NO: 109 | 434 | Δ-12-Desaturase |
| TpO3 | SEQ ID NO: 105 | 418 | ω-3-Desaturase |

Die Klonierung erfolgte wie folgt:
40 ml einer *Thalassiosira pseudonana* Kultur in der stationären Phase wurden abzentrifugiert und in 100 μl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der TpDes-DNAs wurde jeweils mit 1 μl aufgetauten Zellen, 200 μM dNTPs, 2,5 U *Taq*-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel: 34 Klonierung von Expressionsplasmiden zur heterologen Expression in Hefen:

Zur Charakterisierung der Funktion der Desaturasen aus *Thalassiosira pseudonana* wird der offenen Leserahmen der jeweiligen DNA stromabwärts des Galactose-induzierbaren GAL1-Promotors von pYES2.1/V5-His-TOPO (Invitrogen) kloniert, wobei der entsprechenden pYES2.1-Klone erhalten werden.

Der *Saccharomyces cerevisiae*-Stamm 334 wird durch Elektroporation (1500 V) mit den Vektoren pYES2.1-TpDesaturasen transformiert. Als Kontrolle wird eine Hefe verwendet, die mit dem leeren Vektor pYES2 transformiert wird. Die Selektion der transformierten Hefen erfolgt auf Komplett-Minimalmedium (CMdum)-Agarplatten mit 2% Glucose,

aber ohne Uracil. Nach der Selektion werden je drei Transformanten zur weiteren funktionellen Expression ausgewählt.

Für die Expresssion der Tp-Desaturasen werden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.

5 ml CMdum-Flüssigmedium (ohne Uracil) mit 2% Raffinose und 300 µM verschiedener Fettsäuren werden dann mit den Vorkulturen auf eine $OD_{600}$ von 0,05 angeimpft. Die Expression wird durch die Zugabe von 2% (w/v) Galactose induziert. Die Kulturen werden für weitere 96 h bei 20°C inkubiert.

Beispiel: 35 Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mittels PCR Notl-Schnittstellen am 5' und 3'-Ende der kodierenden Sequenzen eingefügt. Die entsprechenden Primersequenzen werden von den 5'- und 3-Bereich der Desaturasen abgeleitet.

Zusammensetzung des PCR-Ansatzes (50 µL):

5,00 µL Template cDNA
5,00 µL 10x Puffer (Advantage-Polymerase)+ 25mM $MgCl_2$
5,00 µL 2mM dNTP
1,25 µL je Primer (10 pmol/µL)
0,50 µL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37°C mit dem Restriktionsenzym Notl inkubiert. Der Pflanzen-Expressionsvektor pSUN300-USP wird in gleicherweise inkubiert. Anschliessend werden die PCR Produkte sowie der Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschliessend werden Vektor und PCR-Produkte ligiert. Dazu wurde das Rapid Ligation Kit von Roche verwendet. Die entstandenen Plasmide werden durch Sequenzierung verifiziert.

pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des OCS-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3'; SEQ ID NO: 143)

Das PCR-Fragment wurde mit EcoRI/Sall nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel: 36 Expression von Tp-Desaturasen in Hefen

Hefen, die wie unter Beispiel 4 mit den Plasmiden pYES2 und pYES2-TpDesaturasen transformiert werden, werden folgendermaßen analysiert:

Die Hefezellen aus den Hauptkulturen werden durch Zentrifugation (100 x g, 5 min, 20°C) geerntet und mit 100 mM $NaHCO_3$, pH 8,0 gewaschen, um restliches Medium und Fettsäuren zu entfemen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES

erfolgte durch zweimalige Extraktion mit Petrolether (PE). Zur Entfernung nicht derivatisierter Fettsäuren werden die organischen Phasen je einmal mit 2 ml 100 mM $NaHCO_3$, pH 8,0 und 2 ml Aqua dest. gewaschen. Anschließend werden die PE-Phasen mit $Na_2SO_4$ getrocknet, unter Argon eingedampft und in 100 µl PE aufgenommen. Die Proben werden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse sind wie folgt: Die Ofentemperatur wird von 50°C bis 250°C mit einer Rate von 5°C/min und schließlich 10 min bei 250°C(halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel: 37 Funktionelle Charakterisierung von Desaturasen aus Thalassiosira pseudonana:

Die Substratspezifität von Desaturasen kann nach Expression in Hefe (siehe Beispiele Klonierung von Desaturase-Genen, Hefeexpression) durch die Fütterung mittels verschiedener Hefen ermittelt werden. Beschreibungen für die Bestimmung der einzelnen Aktivitäten finden sich in WO 93/11245 für Δ 15-Desaturasen. WO 94/11516 für Δ 12-Desaturasen, WO 93/06712, US 5,614,393, US5614393, WO 96/21022, WO0021557 und WO 99/27111 für Δ6-Desaturasen, Qiu et al. 2001, J. Biol. Chem. 276, 31561-31566 für Δ4-Desaturasen, Hong et al. 2002, Lipids 37,863-868 für Δ5-Desaturasen.

Die Aktivität der einzelnen Desaturasen wird aus der Konversionsrate errechnet nach der Formel [Substrat/(Substrat+Produkt)*100].

Die folgenden Tabellen 11 und 12 geben eine Übersicht über die clonierten Thalassiosira pseudonana Desaturasen wieder.

Tabelle 11: Länge und charakteristische Merkmale der clonierten Thalassiosira Desaturasen.

| Desaturase | cDNA (bp) | Protein (aa) | Cyt. B5 | His-Box1 | His-Box2 | His-Box3 |
|---|---|---|---|---|---|---|
| | | | | | | |
| TpD4 | 1512 | 503 | HPGG | HDGNH | WELQHMLGHH | QIEHHLFP |
| TpD5-1 | 1431 | 476 | HPGG | HDANH | WMAQHWTHH | QVEHHLFP |
| TpD5-2 | 1443 | 482 | HPGG | HDANH | WLAQHWTHH | QVEHHLFP |
| TpD6 | 1449 | 484 | HPGG | HDFLH | WKNKHNGHH | QVDHHLFP |
| TpFAD2 (d12) | 1305 | 434 | - | HECGH | HAKHH | HVAHHLFH |
| TpO3 | 1257 | 419 | - | HDAGH | WLFMVTYLQH H | HWHHLF |

Tabelle 12: Länge, Exons, Homolgie und Identitäten der clonierten Desaturasen.

| Des. | GDN A (bp) | Exon 1 | Exon 2 | First Blast Hit | Hom./Iden. |
|---|---|---|---|---|---|
| | | | | | |
| TpD4 | 2633 | 496-1314 | 1571-2260 | Thrautochitrium D4-des | 56% / 43% |
| TpD5-1 | 2630 | 490-800 | 900-2019 | Phaeodactylum D5-des | 74% / 62% |
| TpD5-2 | 2643 | 532-765 | 854-2068 | Phaeodactylum D5-des | 72% / 61% |
| TpD6 | 2371 | 379-480 | 630-1982 | Phaeodactylum D6-des | 83% / 69% |
| TpFAD2 | 2667 | 728-2032 | - | Phaeodactylum FAD2 | 76% / 61% |
| Tp03 | 2402 | 403-988 | 1073-1743 | Chaenorhabdidis Fad2 | 49% / 28% |

Analog zu den vorgenannten Beispielen lassen sich auch die Δ-12-Desaturasegene aus Ostreococcus und Thalassiosira clonieren.

Beispiel 38 Klonierung von Elongase Genen aus Xenopus laevis und Ciona intestinalis

Durch Suche nach konservierten Bereichen (siehe Konsensus-Sequenzen, SEQ ID NO: 115 und SEQ ID NO: 116) in den Proteinsequenzen in Gendatenbanken (Genbank) mit Hilfe der in der Anmeldung aufgeführten Elongase-Gene mit Δ-5-Elongaseaktivität oder Δ-6-Elongaseaktivität konnten weitere Elongasesequenzen aus anderen Organismen identifiziert und isoliert werden. Aus X. laevis bzw. aus C. intestinalis konnten mit entsprechenden Motiven jeweils weitere Sequenzen identifiziert werden. Es handelt sich dabei um die folgenden Sequenzen:

| Gen-Name | Organismus | Genbank-Nr. | SEQ ID NO: | Aminosäuren |
|---|---|---|---|---|
| ELO(XI) | Xenopus laevis | BC044967 | 117 | 303 |
| ELO(Ci) | Ciona intestinalis | AK112719 | 119 | 290 |

Der cDNA Klon von X. laevis wurde vom NIH (National Institut of Health) bezogen [Genetic and genomic tools for Xenopus research: The NIH Xenopus initiative, Dev. Dyn. 225 (4), 384-391 (2002)].

Der cDNA Klon von C. inetstinalis wurde von der Universität von Kyto bezogen [Satou,Y., Yamada,L., Mochizuki,Y., Takatori,N., Kawashima,T., Sasaki,A., Hamagu-chi,M., Awazu,S., Yagi,K., Sasakura,Y., Nakayama,A., Ishikawa,H., Inaba,K. and Satoh,N. "A cDNA resource from the basal chordate Ciona intestinalis" JOURNAL Genesis 33 (4), 153-154 (2002)].

Beispiel 39: Klonierung von Expressionsplasmiden zur heterologen Expression in - Hefen

Die Amplifizierung der Elongase-DNAs wurde jeweils mit 1 µL cDNA, 200 µM dNTPs, 2,5 U Advantage-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide für die PCR-Reaktion verwendet:

| Gen-Name und SEQ ID NO: | | Primersequenz |
|---|---|---|
| ELO(XI) | SEQ ID NO: 121<br>SEQ ID NO: 122 | F:5'- AGGATCCATGGCCTTCAAGGAGCTCACATC<br>R:5'- CCTCGAGTCAATGGTTTTTGCTTTTCAATG-CACCG |
| ELO(Ci), | SEQ ID NO: 123<br>SEQ ID NO: 124 | F:5'-TAAGCTTATGGACGTACTTCATCGT<br>R:5'-TCAGATCTTTAATCGGTTTTACCATT |
| *F=forward primer, R=reverse primer | | |

Die PCR Produkte wurde für 30 min bei 21 °C mit dem Hefe-Expressionsvektor - pYES2.1-TOPO (Invitrogen) gemäß Herstellerangaben inkubiert. Das PCR-Produkt wird dabei durch einen T-Überhang und Aktivität einer Topoisomerase (Invitrogen) nach Herstellerangaben in den Vektor ligiert. Nach der Inkubation erfolgte dann die Transformation von E. coli DH5α Zellen. Entsprechende Klone wurden durch PCR - identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-ELO(XI) und pYES2.1-ELO(Ci). Nach der Selektion wurden je zwei Transformaten zur weiteren funktionellen Expression ausgewählt.

Beispiel 40: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mit folgendem Primerpaar NotI-Schnittstellen am 5' und 3'-Ende der kodierenden Sequenz eingefügt:.

pSUN-ELO(XI)

Forward: 5'-GCGGCCGCACCATGGCCTTCAAGGAGCTCACATC (SEQ ID NO: 125)
Reverse: 3'-GCGGCCGCCTTCAATGGTTTTTGCTTTTCAATGCACCG (SEQ ID NO: 126)

pSUN-ELO(Ci)
Forward: 5'-GCGGCCGCACCATGGACGTACTTCATCGT (SEQ ID NO: 127)
Reverse: 3'-GCGGCCGCTTTAATCGGTTTTACCATT (SEQ ID NO: 128)

Zusammensetzung des PCR-Ansatzes (50 µL):

5,00 µL Template cDNA
5,00 µL 10x Puffer (Advantage-Polymerase)+ 25mM MgCl2
5,00 µL 2mM dNTP
1,25 µL je Primer (10 pmol/µL)
0,50 µL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkte ligiert. Dazu wurde das Rapid Ligation Kit von Roche verwendet. Die entstandenen Plasmide pSUN-ELO(XI) und pSUN-ELO(Ci) wurden durch Sequenzierung verifiziert.
pSUN300 ist ein Derivat des Plasmides pPZP [Hajdukiewicz,P, Svab, Z, Maliga, P., (1994) The small versatile pPZP family of Agrobacterium binary vectors forplant transformation. Plant Mol Biol 25:989-994]. pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.
Primersequenz: 5'-GTCGACCCGCGGACTAGTGGGCCCTCTAGACCCGGGGGATCC GGATCTGCTGGCTATGAA-3' (SEQ ID NO: 129).
Das PCR-Fragment wurde mit EcoRI/SalI nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.
Die Lipidextraktion aus Hefen und Samen erfolgte identisch zu Beispiel 6.

Beispiel 41: Expression von ELO(XI) und ELO(Ci) in Hefen

Hefen, die wie unter Beispiel 4 mit den Plasmiden pYES2, pYES2-ELO(XI) und pYES2-ELO(Ci) transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen

mit Flammenionisationsdetektor getrennt. Die Bedingungen für die GLC-Analysewaren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8): 761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 42: Funktionelle Charakterisierung von ELO(XI) und ELO(Ci):

Die Substratspezifität der ELO(XI) konnte nach Expression und Fütterung verschiedener Fettsäuren ermittelt werden (Fig. 22). Die gefütterten Substrate sind in großen Mengen in allen transgenen Hefen nachzuweisen. Die transgenen Hefen zeigten die Synthese neuer Fettsäuren, den Produkten der ELO(XI)-Reaktion. Dies bedeutet, dass das Gen ELO(XI) funktional exprimiert werden konnte.

Tabelle 13 zeigt, dass die ELO(XI) eine breite Substratspezifität aufweist. Es werden sowohl C18 als auch C20 Fettsäuren verlängert, wobei ein Bevorzugung von Δ5- und Δ6-desaturierten Fettsäuren zu beobachten ist.

Die Hefen, die mit dem Vektor pYES2-ELO(XI) transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 13: Expression von ELO(XI) in Hefe. Beschrieben ist die Umsetzungsrate (Konversionsrate) verschiedener Edukte (gefüttert jeweils 250 μM).

| Edukte | Konversion der Edukte durch ELO(XI) in % |
|---|---|
| 16:0 | 3 |
| $16:1^{\Delta 9}$ | 0 |
| 18:0 | 2 |
| $18:1^{\Delta 9}$ | 0 |
| $18:2^{\Delta 9,12}$ | 3 |
| $18;3^{\Delta 6,9,12}$ | 12 |
| $18:3^{\Delta 5,9,12}$ | 13 |
| $18:3^{\Delta 9,12,15}$ | 3 |
| $18:4^{\Delta 6,9,12,15}$ | 20 |
| $20:3^{\Delta 8,11,14}$ | 5 |
| $20:3^{\Delta 11,14,17}$ | 13 |
| $20:4^{\Delta 5,8,11,14}$ | 15 |
| $20:5^{\Delta 5,8,11,14,17}$ | 10 |
| $22:4^{\Delta 7,10,13,16}$ | 0 |
| $22:6^{\Delta 4,7,10,13,16.19}$ | 0 |

Die Substratspezifität der ELO(Ci) konnte nach Expression und Fütterung verschiedener Fettsäuren ermittelt werden (Fig. 23). Die gefütterten Substrate sind in großen Mengen in allen transgenen Hefen nachzuweisen. Die transgenen Hefen zeigten die Synthese neuer Fettsäuren, den Produkten der ELO(Ci)-Reaktion. Dies bedeutet, dass das Gen ELO(Ci) funktional exprimiert werden konnte.

Tabelle 14: Expression von ELO(Ci) in Hefe. Beschrieben ist die Umsetzungsrate (Konversionsrate) verschiedener Edukte (gefüttert jeweils 250 μM).

| Edukte | Konversion der Edukte durch ELO(Ci) in % |
|---|---|
| 16:0 | 0 |
| $16:1^{\Delta 9}$ | 0 |

(fortgesetzt)

| Edukte | Konversion der Edukte durch ELO(Ci) in % |
|---|---|
| 18:0 | 0 |
| 18:1$^{\Delta 9}$ | 0 |
| 18;$^{2A9,12}$ | 23 |
| 18:3$^{\Delta 6,9,12}$ | 10 |
| 18:3$^{\Delta 5,9,12}$ | 38 |
| 18:3$^{\Delta 9,12,15}$ | 25 |
| 18:4$^{\Delta 6,9,12,15}$ | 3 |
| 20:3$^{\Delta 8,11,14}$ | 10 |
| ·20:3$^{\Delta 11,14,17}$ | 8 |
| 20:4Δ5,8,11,14 | 10 |
| 20:5Δ5,8,11,14,17 | 15 |
| 22:4Δ7,10,13,16 | 0 |
| 22:6Δ4,7,10,13,16,19 | 0 |

Tabelle 14 zeigt, dass die ELO(Ci) eine breite Substratspezifität aufweist. Es werden sowohl C18 als auch C20 Fettsäuren verlängert, wobei ein Bevorzugung von Δ5- und Δ6-desaturierten Fettsäuren zu beobachten ist.

Die Hefen, die mit dem Vektor pYES2-ELO(Ci) transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Beispiel 43: Klonierung von Genen aus Ostreococcus tauri

Durch Suche nach konservierten Bereichen in den Proteinsequenzen mit Hilfe der hierin beschriebenen Elongase-Gene mit Δ-5-Elongaseaktivität oder Δ-6-Elongaseaktivität konnten je zwei Sequenzen mit entsprechenden Motiven in einer Ostreococcus tauri Sequenzdatenbank (genomische Sequenzen) identifiziert werden. Es handelt sich dabei um die folgenden Sequenzen:

| Gen-Name | SEQ ID | Aminosäuren |
|---|---|---|
| OtELO1, (Δ-5-Elongase) | SEQ ID NO: 67 | 300 |
| OtELO1.2, (Δ-5-Elongase) | SEQ ID NO: 113 | 300 |
| OtELO2, (Δ-6-Elongase) | SEQ ID NO: 69 | 292 |
| OtELO2.1, (Δ-6-Elongase) | SEQ ID NO: 111 | 292 |

OtElo1 und OtElo1.2 weisen die höchste Ähnlichkeit zu einer Elongase aus Danio rerio auf (GenBank AAN77156; ca. 26 % Identität), während OtElo2 und OtElo2.1 die größte Ähnlichkeit zur Physcomitrella Elo (PSE) [ca. 36 % Identität] aufweisen (Alignments wurden mit dem tBLASTn-Aalgorithmus (Altschul et al., J. Mol. Biol. 1990, 215: 403 - 410) durchgeführt.

Die Klonierung der Elongasen wurde wie folgt durchgeführt:

40 ml einer *Ostreococcus tauri* Kultur in der stationären Phase wurden abzentrifugiert und in 100 μl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtElo-DNAs wurde jeweils mit 1 μl aufgetauten Zellen, 200 μM dNTPs, 2,5 U *Taq-Polymerase* und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel 44: Klonierung von Expressionsplasmiden zur heterologen Expression in Hefen:

Zur Charakterisierung der Funktion der Elongasen aus *Ostreococcus tauri* wurden die offenen Leserahmen der jeweiligen DNAs stromabwärts des Galactose-induzierbaren GAL1-Promotors von pYES2.1/V5-His-TOPO (Invitrogen) kloniert, wobei pOTE1, pOTE1.2, pOTE2 und pOTE2.1 erhalten wurden.

Der *Saccharomyces cerevisiae-Stamm* 334 wurde durch Elektroporation (1500 V) mit dem Vektor pOTE1, pOTE1.2, pOTE2 bzw. pOTE2.1 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der Ot-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.

5 ml CMdum-Flüssigmedium (ohne Uracil) mit 2% Raffinose und 300 μM verschiedener Fettsäuren wurden dann mit den Vorkulturen auf eine $OD_{6o0}$ von 0,05 angeimpft. Die Expression wurde durch die Zugabe von 2% (w/v) Galactose induziert. Die Kulturen wurden für weitere 96 h bei 20°C inkubiert.

Beispiel 45: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurden mittels PCR NotI-Schnittstellen am 5' und 3'-Ende der kodierenden Sequenzen eingefügt. Die entsprechenden Primersequenzen wurden von den 5'- und 3-Bereich von OtElo1, OtElo1.2, OtElo2 und OtElo2.1 abgeleitet.

Zusammensetzung des PCR-Ansatzes (50 μL):

5,00 μL Template cDNA
5,00 μL 10x Puffer (Advantage-Polymerase)+ 25mM MgCl2
5,00 μL 2mM dNTP
1,25 μL je Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschließend wurden die PCR Produkte sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschliessend wurden Vektor und PCR-Produkte ligiert. Dazu wurde das Rapid Ligation Kit von Roche verwendet. Die entstandenen Plasmide pSUN-OtELO1, pSUN-OtELO1.2, pSUN-OtELO2 und pSUN-OtELO2.2 wurden durch Sequenzierung verifiziert.

pSUN300 ist ein Derivat des Plasmides pPZP [Hajdukiewicz,P, Svab, Z, Maliga, P., (1994) The small versatile pPZP family of Agrobacterium binary vectors for plant transformation. Plant Mol Biol 25:989-994]. pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

Primersequenz:

5'-GTCGACCCGCGGACTAGTGGGCCCTCTAGACCCGGGGGATCC GGATCTGCTGGCTATGAA-3'). (SEQ ID NO: 130)

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.

Beispiel 46: Expression von OtElo1, OtElo1.2, OtElo2 und OtELO2.2 in Hefen

Hefen, die wie unter Beispiel 15 mit den Plasmiden pYES3, pYES3-OtEL01, pYES3-OtELO1.2, pYES3-OtELO2 und pYES3-OtELO2.2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$. pH 8,0 gewaschen, um restliches Medium und Fettsäuren zu entfemen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 $\mu$l PE aufgenommen. Die Proben wurden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C (halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 47: Funktionelle Charakterisierung von OtElo1, Otelo1.2, OtElo2 und OtElo2.1 :

Die Substratspezifität der OtElo1 konnte nach Expression und Fütterung verschiedener Fettsäuren ermittelt werden (Tab. 15). Die gefütterten Substrate sind in großen Mengen in allen transgenen Hefen nachzuweisen. Die transgenen Hefen zeigten die Synthese neuer Fettsäuren, den Produkten der OtElo1-Reaktion. Dies bedeutet, dass das Gen OtElo1 funktional exprimiert werden konnte.

Tabelle 15 zeigt, dass OtElo1 bzw. OtElo1.2 eine enge Substratspezifität aufweist. OtElo1 bzw. OtElo1.2 konnte nur die C20-Fettsäuren Eicosapentaensäure (Figur 24A, 24B) und Arachidonsäure (Figur 25A, 25B) elongieren, bevorzugte aber die $\omega$-3-desaturierte Eicosapentaensäure.

Tabelle 15 zeigt die Substratspezifität der Elongase OtElo1 und OtElo1.2 für C20 polyungesättigte Fettsäuren mit einer Doppelbindung in $\Delta$5 Position gegenüber verschiedenen Fettsäuren.

Die Hefen, die mit dem Vektor pOTE1 bzw. pOTE1.2 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Die Substratspezifität der OtElo2 (SEQ ID NO: 81) OtElo2.1 (SEQ ID NO: 111) konnte nach Expression und Fütterung verschiedener Fettsäuren ermittelt werden (Tab. 16). Die gefütterten Substrate sind in großen Mengen in allen transgenen Hefen nachzuweisen. Die transgenen Hefen zeigten die Synthese neuer Fettsäuren, den Produkten der OtElo2-Reaktion. Dies bedeutet, dass die Gene OtElo2 und OtElo2.1 funktional exprimiert werden konnte.

Tabelle 15:

| Fettsäuresubstrat | Umsatz (in %) OtElo1 | Umsatz (in %) OtElo1.2 |
|---|---|---|
| 16:0 | - | - |
| $16:1^{\Delta 9}$ | - | - |
| 18:0 | - | - |
| $18:1^{\Delta 9}$ | - | - |
| $18:1^{\Delta 11}$ | - | - |
| $18:2^{\Delta 9,12}$ | - | - |
| $18:3^{\Delta 6,9,12}$ | - | - |
| $18:3^{\Delta 5,9,12}$ | - | - |
| $20:3^{\Delta 8,11,14}$ | - | - |

(fortgesetzt)

| Fettsäuresubstrat | Umsatz (in %) OtElo1 | Umsatz (in %) OtElo1.2 |
|---|---|---|
| 20:4 $^{\Delta 5,8,11,14}$ | **10,8 ± 0,6** | **38,0** |
| 20:5 $^{\Delta 5.8,11,14,17}$ | **46,8 ± 3,6** | **68,6** |
| 22:4 $^{\Delta 7,10,13,16}$ | - | - |
| 22:6$^{\Delta 4,7,10,13,16,19}$ | - | - |

Tabelle 16 zeigt die Substratspezifität der Elongase OtElo2 und OtElo2.1 gegenüber verschiedenen Fettsäuren. OtElo2.1 zeigt eine deutlich höhere Aktivität.

Die Hefen, die mit dem Vektor pOTE2 bzw. pOTE2.1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Die enzymatische Aktivität, die in Tabelle 16 wiedergegeben wird, zeigt klar, dass OtElo2 bzw. OtElo2.1 eine Δ-6-Elongase ist.

Tabelle 16:

| Fettsäuresubstrat | Umsatz (in %) OtElo2 | Umsatz (in %)OtELO2.2 |
|---|---|---|
| 16:0 | - | - |
| 16:1$^{\Delta 9}$ | - | - |
| 16:3$^{\Delta 7,10,13}$ | - | - |
| 18:0 | - | - |
| 18:1$^{\Delta 6}$ | - | - |
| 18:1$^{\Delta 9}$ | - | - |
| 18:1$^{\Delta 11}$ | - | - |
| 18:2$^{\Delta 9,12}$ | - | - |
| 18:3$^{\Delta 6,9,12}$ | **15,3** | **55,7** |
| 18:3$^{\Delta 5,9,12}$ | **-** | **-** |
| 18:4 $^{\Delta 6,9,12,15}$ | **21,1** | **70,4** |
| 20:2$^{\Delta 11,14}$ | **-** | **-** |
| 20:3 $^{\Delta 8,11,14}$ | **-** | **-** |
| 20:4 $^{\Delta 5,8,11,14}$ | **-** | **-** |
| 20:5 $^{\Delta 5,6,11,14,17}$ | **-** | **-** |
| 22:4 $^{\Delta 7,10,13,16}$ | **-** | **-** |
| 22:5 $^{\Delta 7,10,13,16,19}$ | **-** | **-** |
| 22:6$^{\Delta 4,7,10,13,16,19}$ | **-** | **-** |

Figur 24 A - D zeigt die Elongation von Eicosapentaensäure durch OtElo1 (B) bzw. OtElo1.2 (D). Die Kontrollen (A, C) zeigen nicht das Produkt der Elongation (22:5ω3).

Figur 25 A - D zeigt die Elongation von Arachidonsäure durch OtElo1 (B) bzw. OtElo1.2 (D). Die Kontrollen (A, C) zeigen nicht das Produkt der Elongation (22:4ω6).

Beispiel 48: Klonierung von Elongase-Genen aus Euglena gracilis und Arabidopsis thaliana

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten Sequenzen aus Arabidopsis thaliana bzw. Euglena gracilis mit entsprechenden Motiven in Sequenzdatenbanken (Genbank, Euglena EST Bank) identifiziert werden. Es handelt sich dabei um die folgenden Sequenzen:

| Gen-Name | SEQ ID | Aminosäuren |
|---|---|---|
| EGY1019 (E. gracilis) | SEQ ID NO: 131 | 262 |
| EGY2019 (E. gracilis) | SEQ ID NO: 133 | 262 |
| At3g06460 (A. thaliana) | SEQ ID NO: 135 | 298 |
| At3g06470 (A. thaliana) | SEQ ID NO: 137 | 278 |

Die Klonierung der Elongasen aus Euglena gracilis wurden wie folgt durchgeführt:
Der Euglena gracilis Stamm 1224-5/25 wurde erhalten von der Sammlung für Algenkulturen Göttingen (SAG). Zur Isolierung wurde der Stamm in Medium II (Calvayrac R and Douce R, FEBS Letters 7:259-262, 1970) für 4 Tage bei 23 °C unter einem Licht-/ Dunkelintervall von 8 h / 16 h (35 mol s-1 m-2 Lichtstärke) angezogen.

Gesamt-RNA von einer viertägigen Euglena Kultur wurde mit Hilfe des RNAeasy Kits der Firma Qiagen (Valencia, CA, US) isoliert. Aus der Gesamt-RNA wurde mit Hilfe von oligo-dT-Cellulose poly-A+ RNA (mRNA) isoliert (Sambrook et al., 1989). Die RNA wurde mit dem Reverse Transcription System Kit von Promega revers transcribiert und die synthetisierte cDNA in den lambda ZAP Vektor (lambda ZAP Gold, Stratagene) kloniert. Entsprechend der Herstellerangaben wurde die cDNA zur Plasmid-DNA entpackt und Klone wurden zur Zufallssequenzierung ansequenziert. Aus der Gesamt-RNA wurde mit Hilfe des PolyATract Isolierungssystems (Promega) mRNA isoliert. Die mRNA wurde mit dem Marathon cDNA Amplification-Kit (BD Biosciences) reverse transkribiert und entsprechend der Herstellerangaben wurden die Adaptoren ligiert. Die cDNA-Bank wurde dann für die PCR zur Klonierung von Expressionsplasmiden mittels 5'- und 3'-RACE (rapid amplification of cDNA ends) verwendet.

Die Klonierung der Elongasen aus Arabidopsis thaliana wurde wie folgt durchgeführt:
Ausgehend von der genomischen DNA wurden für die beiden Gene Primer entsprechend am 5'- und 3'-Ende des offenen Leserahmens abgeleitet.

Zur Isolierung von Gesamt-RNA aus *A. thaliana* wurde nach Chrigwin *et al.,* (1979) verfahren. Blätter von 21 Tage alten Pflanzen wurden in flüssigem Stickstoff zermörsert, mit Aufschlusspuffer versetzt und für 15 min bei 37 °C inkubiert. Nach Zentrifugation (10 min, 4 oC, 12000xg) wurde die RNA im Überstand mit 0,02 Volumen 3 M Natriumacetat pH 5,0 und 0,75 Volumen Ethanol bei -20 oC für 5 h präzipitiert. Die RNA wurde dann nach einem weiteren Zentrifugationsschritt in 1 mL TES pro g Ausgangsmaterial aufgenommen, einmal mit einem Volumen Phenol-Chloroform und einmal mit einem Volumen Chloroform extrahiert und die RNA mit 2,5 M LiCl gefällt. Nach anschliessendem Zentrifugieren und Waschen mit 80 %igem Ethanol wurde die RNA in Wasser resuspendiert. Entsprechend Sambrook et al. 1989 wurde die cDNA synthetisiert und RT-PCR mit den abgeleiteten Primer durchgeführt. Die PCR-Produkte wurden nach Herstellerangaben in den Vektor pYES2.1-TOPO (Invitrogen) kloniert.

Beispiel 49: Klonierung von Expressionsplasmiden zur heterologen Expression in Hefen:

Zur Charakterisierung der Funktion der Elongasen aus *A. thalina* wurden die offenen Leserahmen der jeweiligen DNAs stromabwärts des Galactose-induzierbaren GAL1-Promotors von pYES2.1/V5-His-TOPO (Invitrogen) kloniert, wobei pAt60 und pAt70 erhalten wurden.

Der *Saccharomyces cerevisiae*-Stamm 334 wurde durch Elektroporation (1500 V) mit dem Vektor pAt60 bzw. pAt70 transformiert. Als Kontrolle wurde eine Hefe verwendet, die mit dem leeren Vektor pYES2.1 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der At-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.
5 ml CMdum-Flüssigmedium (ohne Uracil) mit 2% Raffinose und 300 $\mu$M verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel 50: Expression von pAt60 und pAt70 in Hefen

Hefen, die wie unter Beispiel 5 mit den Plasmiden pYES2.1, pAt60 bzw. pAt70 transformiert wurden, wurden folgendermaßen analysiert:
Die Hefezellen aus den Hauptkulturen wurden durch Zentrifugation (100 x g, 5 min, 20°C) geerntet und mit 100 mM

$NaHCO_3$, pH 8,0 gewaschen, um restliches Medium und Fettsäuren zu entfemen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 $\mu$l PE aufgenommen. Die Proben wurden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 51: Funktionelle Charakterisierung von pAt60 und pAt70

Die Substratspezifität der Elongasen At3g06460 bzw. At3g06470 konnte nach Expression und Fütterung verschiedener Fettsäuren ermittelt werden (Tab. 17, Fig. 26). Die gefütterten Substrate sind in allen transgenen Hefen nachzuweisen. Die transgenen Hefen zeigten die Synthese neuer Fettsäuren, den Produkten der Gene At3g06460 bzw. At3g06470. Dies bedeutet, dass diese Gene funktional exprimiert werden konnte.

Tabelle 17: Elongation von EPA durch die Elongasen At3g06460 bzw. At3g06470. Messung der Hefeextrakte nach Fütterung mit 250 uM EPA.

| Gen | Gefütterte Fettsäure | Gehalt anC20:5n-3 | Gehalt an C22: 5n-3 |
|---|---|---|---|
| At3g06460 | EPA (C20:5n-3) | 20.8 | 0,6 |
| At3g06460 | EPA (C20:5n-3) | 25,4 | 1,1 |
| Konversionsrate von EPA | At3g06460: 3,0 % | At3g06470: 4,1 % | |

Figur 26 gibt die Elongation von 20:5n-3 durch die Elongasen At3g06470 wieder.

Beispiel 52: Klonierung einer Elongase aus Phaeodactylum tricornutum

Ausgehend von konservierten Bereichen in den Proteinsequenzen mit Hilfe der in der Anmeldung aufgeführten Elongase-Gene mit Δ-6-Elongaseaktivität wurden degenerierte Primer hergestellt und mit diesen eine *Phaeodactylum* cDNA Bank mittels PCR durchsucht. Folgende Primer-Sequenzen wurden eingesetzt:

| Primer-Name | Sequenz 5'-3' Orientierung | Korrespondierende Aminosäuren |
|---|---|---|
| Phaelo forward1 | AA(C/T)CTUCTUTGGCTUTT(C/T)TA (SEQ ID NO. 185) | NLLWLFY |
| Phaelo reverse1 | GA(C/T)TGUAC(A/G)AA(A/G)AA(C/T)TGUG C(A/G)AA (SEQ ID NO. 186) | FAQFFVQS |

Nukleotidbasen in Klammem bedeuten, dass eine Mischung von Oligonukleotiden mit jeweils der einen oder anderen Nukleotidbase vorliegen.

Herstellung der *Phaeodactylum* cDNA Bank:

Eine 2 L Kultur von P. tricomutum UTEX 646 wurde in f/2 Medium (Guillard, R.R.L. 1975. Culture of phytoplankton for feeding marine invertebrates. In Culture of Marine Invertebrate Animals (Eds. Smith, W.L. and Chanley, M.H.), Plenum Press, New York, pp 29-60.) für 14 d (= Tage) bei einer Lichtstärke von 35 E/cm$^2$ angezogen. Gefrorene Zellen wurden nach Zentrifugation in der Gegenwart von flüssigem Stickstoff zu einem feinen Pulver gemahlen und mit 2 mL Homogenisierungspuffer (0,33 M Sorbitol, 0,3 M NaCl, 10 mM EDTA, 10 mM EGTA, 2% SDS, 2% Mercaptoethanol in 0,2 M Tris-Cl ph 8,5) resuspendiert. Nach Zugabe von 4 mL Phenol und 2 mL Chloroform wurde 15 min kräftig bei 40-50 °C

geschüttelt. Anschliessend wurde zentrifugiert (10 min x 10000g) und die wässerige Phase schrittweise mit Chloroform extrahiert. Nukleinsäuren wurden dann durch Zugabe von 1/20 Volumen 4 M Natriumhydrogencarboriatlösung gefällt und zentrifugiert. Das Pellet wurde in 80 mM Tris-borat pH 7,0 und 1 mM EDTA aufgenommen und die RNA mit 8 M Lithiumclorid gefällt. Nach Zentrifugation und Waschen mit 70%igem Ethanol wurde das RNA-Pellet mit Rnase-freiem Wasser aufgenommen. Poly(A)-RNA wurde mit Dynabeads (Dynal, Oslo, Norwegen) nach Herstellerangaben isoliert und die Erst-Strang-cDNA-Synthese mit MLV-Rtase von Roche (Mannheim) durchgeführt. Die Zweit-Strang-Synthese erfolgte dann mittels DNA Polymerase I und Klenow Fragment, gefolgt von einem RnaseH Verdau. Die cDNA wurde mit T4 DNA Polymerase behandelt und anschliessend EcoRI/XhoI Adaptoren (Pharmacia, Freiburg) mittels T4 Ligase angehängt. Nach XhoI Verdau, Phosphorylierung und Geltrennung wurden Fragmente grösser als 300 bp entsprechend der Herstellerangaben in den lambda ZAP Express Phagen ligiert (Stratagene, Amsterdam, Niederlande). Nach Massenexcision der cDNA-Bank und Plasmid-Rückgewinnung wurde die Plasmid-Bank in E. coli DH10B Zellen transformiert und zur PCR-Sichtung eingesetzt.

Mittels den oben genannten degenerierten Primem konnte das PCR-Fragment mit der Sequenznummer SEQ ID NO: 187 generiert werden.

Dieses Fragment wurde mit Digoxigenin markiert (Roche, Mannheim) und als Sonde für die Sichtung der Phagen-Bank verwendet.

Mit Hilfe der Sequenz SEQ ID NO: 187 konnte die Gensequenz SEQ ID NO: 183 erhalten werden, die das Volllängen-RNA-Molekül der Δ6-Elongase von Phaeodactylum darstellt:

Beispiel 53: Klonierung von Expressionsplasmiden zur heterologen Expression in - Hefen

Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der PtELO6-DNA wurde jeweils mit 1 μL cDNA, 200 μM dNTPs, 2,5 U Advantage-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide für die PCR-Reaktion verwendet:

| Gen-Name und SEQ ID NO: | Primersequenz |
|---|---|
| PtELO6 (SEQ ID NO: 183) | F:5'-GCGGCCGCACATAATGATGGTACCTTCAAG (SEQ ID NO: 188)<br>R:3'- GAAGACAGCTT AAT AGACT AGT (SEQ ID NO: 189) |
| *F=forward primer, R=reverse primer | |

Die PCR Produkte wurden für 30 min bei 21 °C mit dem Hefe-Expressionsvektor - pYES2.1-TOPO (Invitrogen) gemäß Herstellerangaben inkubiert. Das PCR-Produkt (siehe SEQ ID NO: 192) wurde dabei durch einen T-Überhang und Aktivität einer Topoisomerase (Invitrogen) in den Vektor ligiert. Nach der Inkubation erfolgte dann die Transformation von E. coli DH5α Zellen. Entsprechende Klone wurden durch PCR - identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1 und pYES2.1-PtELO6. Nach der Selektion wurden je zwei Transformaten zur weiteren funktionellen Expression ausgewählt.

Beispiel 54: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu wird mit folgendem Primerpaar NotI-Schnittstellen am 5' und 3'-Ende der kodierenden Sequenz eingefügt:.
PSUN-PtEL06
Forward: 5'-GCGGCCGCACCATGATGGTACCTTCAAGTTA (SEQ ID NO: 190)
Reverse: 3'-GAAGACAGCTTAATAGGCGGCCGC (SEQ ID NO: 191)

Zusammensetzung des PCR-Ansatzes (50 μL):

5,00 μL Template cDNA
5,00μL 10x Puffer (Advantage-Polymerase)+ 25mM MgCl2

5,00 µL 2mM dNTP
1,25 µL je Primer (10 pmol/µL)
0,50 µL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschließend werden Vektor und PCR-Produkte ligiert. Dazu wird das Rapid Ligation Kit von Roche verwendet. Das entstandene Plasmide pSUN-PtELO wird durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.
(Primersequenz: 5'-GTCGACCCGCGGACTAGTGGGCCCTCTAGACCCGGGGGATCC GGATCTGCTGGCTATGAA-3'; SEQ ID NO: 151).
Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.
Die Lipidextraktion aus Hefen und Samen erfolgte identisch zu Beispiel 6.

Beispiel 55: Expression von PtElo in Hefen

Hefen, die wie unter Beispiel 4 mit den Plasmiden pYES2 und pYES2-PtELO6 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 µl PE aufgenommen. Die Proben wurden auf einer DB-23-Kapillarsäule (30 m, 0,25 mm, 0,25 µm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.
Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 56: Funktionelle Charakterisierung von PtELO6:

In Figur 29 ist die Umsetzung von $C18{:}3^{\Delta 6,9,12}$ und $C18{:}4^{\Delta 6,9,12,15}$ wiedergegeben. Die Substrate werden um je zwei Kohlenstoffatome elongiert es entstehen jeweils die Fettsäuren $C20{:}3^{\Delta 8,11,14}$ bzw. $C20{:}4^{\Delta 8,11,14,17}$. Die Substratspezifität von PtELO6 konnte nach Expression und Fütterung verschiedener Fettsäuren ermittelt werden (Fig. 30). Die gefütterten Substrate sind in großen Mengen in allen transgenen Hefen nachzuweisen. Die transgenen Hefen zeigten die Synthese neuer Fettsäuren, den Produkten der PtElo6-Reaktion. Dies bedeutet, dass das Gen PtEL06 funktional exprimiert werden konnte.

Tabelle 18 zeigt, dass die PtElo6 eine enge Substratspezifität aufweist. PtELO6 konnte nur die C18-Fettsäuren Linolsäure, Linolensäure, γ-Linotensäure und Stearidonsäure elongieren, bevorzugte aber die ω-3-desaturierte Stearidonsäure (siehe auch Figur 30). Fütterungsexperiment: Fettsäuren (fett) wurden jeweils mit 250 μM zugegeben. Die unterstrichenen Fettsäuren wurden neu gebildet.

Tabelle 18: Substratspezifität der PtElo6

| **gefütterte Fettsäure:** | | **+ 18:2** | **+ 18:3** | **+ 18:3** | **+ 18:4** |
|---|---|---|---|---|---|
| 16:0 | 16,2 | 18,2 | 15,2 | 20 | 04:48 |
| 16:1 | 50,6 | 20,5 | 22,8 | 33,5 | 34,2 |
| 18:0 | 5,4 | 6,3 | 6,2 | 5,2 | 12,4 |
| 18:1 | 27,7 | 14,6 | 19,6 | 19,3 | 16,7 |
| 18:2 | | 40 | | | |
| 18:3 | | | 32,9 | | |
| 18:3 | | | | 12,3 | |
| 18:4 | | | | | 4,5 |
| 20:2 | | 0,4 | | | |
| 20:3 | | | 3,4 | | |
| 20:3 | | | | 9,7 | |
| 20:4 | | | | | 14,5 |
| **% Elongation** | **0,0** | **0,99** | **9,37** | **44,09** | **76,32** |

Folgende Fettsäuren wurden gefüttert, aber nicht umgesetzt:

- $18{:}1^{\Delta 6}$, $18{:}1^{\Delta 9}$, $18{:}1^{\Delta 11}$
- $20{:}2^{\Delta 11,14}$, $20{:}3^{\Delta 11,14,17}$, $20{:}3^{\Delta 68,11,14}$, $20{:}4^{\Delta 5,8,11,14}$, $20{:}5^{\Delta 58,11,14,17}$
- $22{:}4^{\Delta 7,10,13,16}$

Die Hefen, die mit dem Vektor pYES2-PtELO6 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. So wurden die Ergebnisse, die in den Figuren 29 und 30 sowie in der Tabelle 16 dargestellt wurden, ermittelt.

Äquivalente:

Der Fachmann erkennt oder kann viele Äquivalente der hier beschriebenen erfindungsgemäßen spezifischen Ausführungsformen feststellen, indem er lediglich Routineexperimente verwendet. Diese Äquivalente sollen von den Patentansprüchen umfasst sein.

Tabelle 3: Umsetzungsraten der gefütterten Fettsäuren. Die Konversionsraten wurden berechnet nach der Formel: [Konversionsrate]=[Produkt]/[[Substrat]+[Produkt]*100.

| BioTaur-Klone Fläche in % der GC-Analyse | | | | | | | | | | | | | | |
|---|---|---|---|---|---|---|---|---|---|---|---|---|---|---|
| Clone | Fettsäure | C16:0 | C16:1 (n-7) | C18:0 | C18:1 (n-9) | C18:3 (n-6) | C18:4 (n-3) | C20:3 (n-6) | C20:4 (n-6) | C20:4 (n-3) | C20:5 (n-3) | C22:4 (n-6) | C22:4 (n-3) | C22:5 (n-3) |
| Vector | keine | 21.261 | 41.576 | 4.670 | 25.330 | | | | | | | | | |
| BioTaur | Keine | 20.831 | 37.374 | 4.215 | 26.475 | | | | | | | | | |
| Vector | GLA + EPA | 22.053 | 23.632 | 5.487 | 17.289 | **11.574** | | | | | **13.792** | | | |
| BioTaur | GLA + EPA | 20.439 | 25.554 | 6.129 | 19.587 | **3.521** | | **6.620** | | | **10.149** | | | **1.127** |
| Vector | EPA | 20.669 | 28.985 | 6.292 | 21.712 | | | | | | **16.225** | | | |
| BioTaur | EPA | 20.472 | 26.913 | 6.570 | 23.131 | | | | | | **11.519** | | | **3.251** |
| Vector | ARA | 23.169 | 23.332 | 6.587 | 12.735 | | | | **27.069** | | | | | |
| BioTaur | ARA | 20.969 | 31.281 | 5.367 | 21.351 | | | | **9.648** | | | **1.632** | | |
| Vector | SDA | 18.519 | 12.626 | 6.642 | 6.344 | | **47.911** | | | | | | | |
| BioTaur | SDA | 19.683 | 15.878 | 7.246 | 8.403 | | **13.569** | | | **25.946** | | | **0.876** | |

Weitere Ausführungsformen

1. Verfahren zur Herstellung von Verbindungen der allgemeinen Formel I

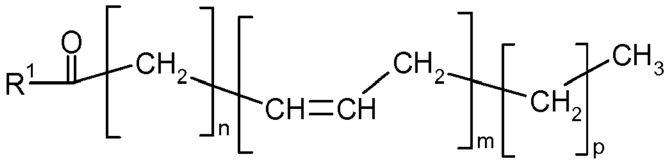

(I)

in transgenen Organismen mit einem Gehalt von mindestens 1 Gew.-% dieser Verbindungen bezogen auf den Gesamtlipidgehalt des transgenen Organismus, dadurch gekennzeichnet, dass es folgende Verfahrensschritte umfasst:

a) Einbringen mindestens einer Nukleinsäuresequenz in den Organismus, welche für eine Δ-9-Elongase- oder eine Δ-6-Desaturase-Aktivität codiert, und

b) Einbringen mindestens einer Nukleinsäuresequenz in den Organismus, welche für eine Δ-8-Desaturase- oder eine Δ-6-Elongase-Aktivität codiert, und

c) Einbringen mindestens einer Nukleinsäuresequenz in den Organismus, welche für eine Δ-5-Desaturase-Aktivität codiert, und

d) Einbringen mindestens einer Nukleinsäuresequenz in den Organismus, welche für eine Δ-5-Elongase-Aktivität codiert, und

e) Einbringen mindestens einer Nukleinsäuresequenz in den Organismus, welche für eine Δ-4-Desaturase-Aktivität codiert, und

Wobei die Variablen und Substituenten in der Formel I die folgende Bedeutung haben:

$R^1$ = Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

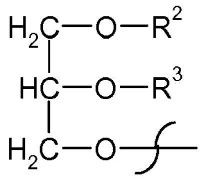

(II)

$R^2$ = Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes $C_2$-$C_{24}$-Alkylcarbonyl-,

$R^3$ = Wasserstoff-, gesättigtes oder ungesättigtes $C_2$-$C_{24}$-Alkylcarbonyl-, - oder $R^2$ oder $R^3$ unabhängig voneinander einen Rest der allgemeinen Formel Ia:

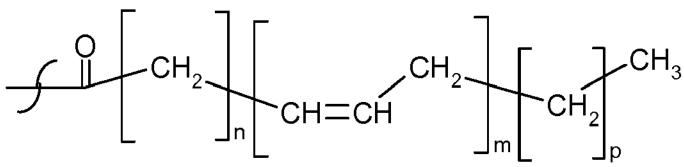

(Ia)

n = 2, 3, 4, 5, 6 oder 9, m = 2, 3, 4, 5 oder 6 und p = 0 oder 3.

2. Verfahren nach Ausführungsform 1, dadurch gekennzeichnet, dass die Nukleinsäuresequenzen, die für Polypeptide mit Δ-9-Elongase-, Δ-6-Desaturase-, Δ-8-Desaturase-, Δ-6-Elongase-, Δ-5-Desaturase-, Δ-5-Elongase- oder Δ-4-Desaturaseaktivität codieren, ausgewählt sind aus der Gruppe bestehend aus:

a) einer Nukleinsäuresequenz mit der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51 oder SEQ ID NO: 53 dargestellten Sequenz, oder

b) Nukleinsäuresequenzen, die sich als Ergebnis des degenerierten genetischen Codes von den in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52 oder SEQ ID NO: 54 dargestellten Aminosäuresequenzen ableiten lassen, oder

c) Derivate der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51 oder SEQ ID NO: 53 dargestellten Nukleinsäuresequenz, die für Polypeptide mit mindestens 40 % Identität auf Aminosäureebene mit SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52 oder SEQ ID NO: 54 codieren und eine Δ-9-Elongase-, Δ-6-Desaturase-, Δ-8-Desaturase-, Δ-6-Elongase-, Δ-5-Desaturase-, Δ-5-Elongase- oder Δ-4-Desaturaseaktivität aufweisen.

3. Verfahren nach Ausführungsform 1 oder 2, dadurch gekennzeichnet, dass die Substituenten $R^2$ oder $R^3$ unabhängig voneinander gesättigtes oder ungesättigtes $C_{18}$-$C_{22}$-Alkylcarbonyl-bedeuten.

4. Verfahren nach den Ausführungsformen 1 bis 3, dadurch gekennzeichnet, dass die Substituenten $R^2$ oder $R^3$ unabhängig voneinander ungesättigtes $C_{18}$-, $C_{20}$- oder $C_{22}$-Alkylcarbonyl- mit mindestens zwei Doppelbindungen bedeuten.

5. Verfahren nach den Ausführungsformen 1 bis 4, dadurch gekennzeichnet, dass der transgene Organismus eine transgene Pflanze ist.

6. Verfahren nach den Ausführungsformen 1 bis 5, dadurch gekennzeichnet, dass der transgene Organismus eine Öl-produzierende Pflanze ist.

7. Verfahren nach den Ausführungsformen 1 bis 6, dadurch gekennzeichnet, dass die transgene Organismus eine transgene Pflanze ausgewählt aus der Gruppe: Raps, Canola, Mohn, Senf, Hanf, Rizinus, Sesam, Olive, Calendula, Punica, Haselnuss, Mandel, Macadamia, Avocado, Kürbis, Walnuss, Lorbeer, Pistazie, Nachtkerze, Erdnuss, Lein, Soja, Safflower, Sonnenblume oder Borretsch ist.

8. Verfahren nach den Ausführungsformen 1 bis 7, dadurch gekennzeichnet, dass die Verbindungen der allgemeinen Formel I aus dem Organismus in Form ihrer Öle, Lipide oder freien Fettsäuren isoliert werden.

9. Verfahren nach den Ausführungsformen 1 bis 8, dadurch gekennzeichnet, dass die Verbindungen der allgemeinen Formel I in einer Konzentration von mindestens 5 Gew.-% bezogenen auf den gesamten Lipidgehalt des transgenen Organismus isoliert werden.

10. Öl, Lipide oder Fettsäuren oder eine Fraktion davon, hergestellt durch das Verfahren nach einer der Ausführungsformen 1 bis 9.

11. Öl-, Lipid- oder Fettsäurezusammensetzung, die PUFAs hergestellt nach einem Verfahren nach einer der Ausführungsformen 1 bis 9 umfasst und von transgenen Pflanzen stammt.

12. Verwendung von Öl, Lipide oder Fettsäuren gemäß Ausführungsform 10 oder Öl-, Lipid- oder Fettsäurezusammensetzung gemäß Ausführungsform 11 in Futter, Nahrungsmitteln, Kosmetika oder Pharmazeutika.

13. Isolierte Nukleinsäuresequenzen, die für Polypeptide mit Δ-5-Elongaseaktivität codieren, ausgewählt aus der Gruppe:

a) einer Nukleinsäuresequenz mit der in SEQ ID NO: 43, SEQ ID NO: 45, SEQ ID NO: 47 oder SEQ ID NO: 49 dargestellten Sequenz,

b) Nukleinsäuresequenzen, die sich als Ergebnis des degenerierten genetischen Codes von der in SEQ ID NO: 44, SEQ ID NO: 46, SEQ ID NO: 48 oder SEQ ID NO: 50 dargestellten Aminosäuresequenz ableiten lassen, oder

c) Derivate der in SEQ ID NO: 43, SEQ ID NO: 45, SEQ ID NO: 47 oder SEQ ID NO: 49 dargestellten Nukleinsäuresequenz, die für Polypeptide mit mindestens 40 % Homologie auf Aminosäureebene mit SEQ ID NO: 44, SEQ ID NO: 46, SEQ ID NO: 48 oder SEQ ID NO: 50 aufweisen und eine A-5-Elongaseaktivität aufweisen.

14. Isolierte Nukleinsäuresequenz nach Ausführungsform 13, wobei die Sequenz von einer Pflanze oder einem Tier stammt.

15. Isolierte Nukleinsäuresequenz nach Ausführungsform 13 oder 14, wobei die Sequenz aus der Ordnung Salmoniformes, oder von Diatomenn wie den Gattungen Thallasiosira oder Crythecodinium stammt.

16. Aminosäuresequenz, die von einer isolierten Nukleinsäuresequenz nach einer der Ausführungsformen 13 bis 15 codiert wird.

17. Genkonstrukt, enthaltend eine isolierte Nukleinsäure nach einer der Ausführungsformen 13 bis 15, wobei die Nukleinsäure funktionsfähig mit einem oder mehreren Regulationssignalen verbunden ist.

18. Genkonstrukt nach Ausführungsform 17, dadurch gekennzeichnet, dass das Nukleinsäurekonstrukt zusätzliche Biosynthesegene des Fettsäure- oder Lipidstoffwechsels enthält ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lyso-phospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n).

19. Genkonstrukt nach Ausführungsform 17 oder 18, dadurch gekennzeichnet, dass das Nukleinsäurekonstrukt zusätzliche Biosynthesegene des Fettsäure- oder Lipidstoffwechsels enthält ausgewählt aus der Gruppe der A-4-Desaturase-, Δ-5-Desaturase-, Δ-6-Desaturase-, Δ-8-Desatuase-, Δ-9-Desaturase-, Δ-12-Desaturase-, Δ-6-Elongase- oder Δ-9-Elongase.

20. Vektor, enthaltend eine Nukleinsäure nach den Ausführungsformen 13 bis 15 oder ein Genkonstrukt nach Ausführungsform 17.

21. Transgener nicht-humaner Organismus, enthaltend mindestens eine Nukleinsäure nach den Ausführungsformen 13 bis 15, ein Genkonstrukt nach Ausführungsform 17 oder einen Vektor nach Ausführungsform 20.

22. Transgener nicht-humaner Organismus nach Ausführungsform 21, wobei der Organismus ein Mikroorganismus, ein nicht-humanes Tier oder eine Pflanze ist.

23. Transgener nicht-humaner Organismus nach Ausführungsform 21 oder 22, wobei der Organismus eine Pflanze ist.

**Patentansprüche**

**1.** Verfahren zur Herstellung von Ölen, Lipiden oder Fettsäuren enthaltend C18-, C20- oder C22-Fettsäuremoleküle mit mindestens zwei Doppelbindungen im Fettsäuremolekül in einem nicht-human Organismus, wobei der Organismus

i. eine delta-9-Elongase- oder eine delta-6-Desaturase-Aktivität exprimiert und
ii. eine delta-8-Desaturase- oder eine delta-6-Elongase-Aktivität exprimiert und
iii. eine delta-5-Desaturase-Aktivität exprimiert und
iv. eine delta-5-Elongase-Aktivität exprimiert und

v. eine delta-4-Desaturase-Aktivität exprimiert,

umfassend die Isolierung von Öl, Lipiden oder Fettsäuren aus dem Organismus, wobei die Nukleinsäure mit einer Sequenz, die für das Polypeptid mit Δ6-Desaturaseaktivität kodiert, ausgewählt ist aus der Gruppe bestehend aus:

a) einer Nukleinsäure mit der in SEQ ID NO: 89 dargestellten Sequenz, oder
b) Nukleinsäuren, die sich als Ergebnis des degenerierten genetischen Codes von der in SEQ ID NO: 90 dargestellten Aminosäuresequenz ableiten lassen, oder
c) Derivate der in SEQ ID NO: 89 dargestellten Nukleinsäuresequenz, die für Polypeptide mit mindestens 60% Identität auf Aminosäureebene mit SEQ ID NO: 90 kodieren und eine Δ6-Desaturaseaktivität aufweisen.

**2.** Verfahren zur Herstellung von Futtermitteln, Nahrungsmitteln, Kosmetika oder Pharmazeutika Öl, Lipiden oder Fettsäuren enthaltend C18-, C20- und/oder C22-Fettsäuremolekülen, wobei Öl, Lipide oder Fettsäuren aus einem Organismus, der

vi. eine delta-9-Elongase- oder eine delta-6-Desaturase-Aktivität exprimiert und
vii. eine delta-8-Desaturase- oder eine delta-6-Elongase-Aktivität exprimiert und
viii. eine delta-5-Desaturase-Aktivität exprimiert und
ix. eine delta-5-Elongase-Aktivität exprimiert und
x. eine delta-4-Desaturase-Aktivität exprimiert,

wobei die Nukleinsäure mit einer Sequenz, die für das Polypeptid mit Δ6-Desaturaseaktivität kodiert, ausgewählt ist aus der Gruppe bestehend aus:

a) einer Nukleinsäure mit der in SEQ ID NO: 89 dargestellten Sequenz, oder
b) Nukleinsäuren, die sich als Ergebnis des degenerierten genetischen Codes von der in SEQ ID NO: 90 dargestellten Aminosäuresequenz ableiten lassen, oder
c) Derivate der in SEQ ID NO: 89 dargestellten Nukleinsäuresequenz, die für Polypeptide mit mindestens 60% Identität auf Aminosäureebene mit SEQ ID NO: 90 kodieren und eine Δ6-Desaturaseaktivität aufweisen

und wobei das Öl, Lipiden oder Fettsäuren mindestens 0,5% EPA und/oder DHA enthält und in den Futtermitteln, Nahrungsmitteln, Kosmetika oder Pharmazeutika verwendet wird.

**3.** Verfahren nach Anspruch 1, wobei das Öls, Lipid oder die Fettsäure in Futtermitteln, Nahrungsmitteln, Kosmetika oder Pharmazeutika verwendet werden.

**4.** Verfahren nach einem der Ansprüche 1 bis 3, wobei ein Fettsäureester mit mehrfach ungesättigten C18-, C20- und/oder C22-Fettsäuremolekülen mit mindestens drei, vier, fünf oder sechs Doppelbindungen im Fettsäureester hergestellt wird.

**5.** Verfahren nach einem der vorausgegangenen Ansprüche, wobei mindestens 1% EPA und/oder DHA bezogen auf den Gesamtfettsäuregehalt des Organismus in dem Öl, Lipid oder den freien Fettsäuren enthalten sind.

**6.** Verfahren nach einem der vorausgehenden Ansprüche, wobei ein die isolierten Öle, Lipide, Fettsäuren oder Fettsäuregemische mit Öle, Lipide, Fettsäuren oder Fettsäuregemische tierischen Ursprungs gemischt werden.

**7.** Verfahren nach einem der vorausgehenden Ansprüche, wobei die delta-6-Elongase und/oder die delta-5-Elongase eine monofunktionale Elongase ist.

**8.** Verfahren nach einem der vorausgehenden Ansprüche, wobei die Pflanze auch eine omega-3-Desaturase umfasst.

**9.** Verfahren oder Pflanze nach einem der vorausgehenden Ansprüche, wobei die Pflanze eine Ölfruchtpflanze ist.

**10.** Verfahren oder Pflanze nach einem der vorausgehenden Ansprüche, wobei die Nukleinsäuresequenz, die für die Polypeptide mit Δ6-Elongaseaktivität kodiert, ausgewählt ist aus der Gruppe bestehend aus:

a) Nukleinsäuren mit den in SEQ ID NO: 69 oder SEQ ID NO: 111 dargestellten Sequenzen, oder
b) Nukleinsäurenn, die sich als Ergebnis des degenerierten genetischen Codes von den in SEQ ID NO: 70 oder

SEQ ID NO: 112 dargestellten Aminosäuresequenzen ableiten lassen, oder
c) Derivate der in SEQ ID NO: 69 oder SEQ ID NO: 111 dargestellten Nukleinsäuresequenzen, die für Polypeptide mit mindestens 40% Identität auf Aminosäureebe-ne mit SEQ ID NO: 70 oder SEQ ID NO: 112 kodieren und eine Δ6-Elongaseaktivität aufweisen.

**11.** Verfahren oder Pflanze nach einem der vorausgehenden Ansprüche, wobei die Nukleinsäuresequenz, die für das Polypeptid mit Δ5-Elongaseaktivität kodiert, ausgewählt ist aus der Gruppe bestehend aus

a) der Sequenz SEQ ID NO. 118 oder
b) wobei das Polypeptid mindestens 90% identisch ist zu der in SEQ ID NO. 118 dargestellten Aminosäuresequenz und eine Δ5-Elongaseaktivität aufweist.

**12.** Verfahren oder Pflanze nach einem der vorausgehenden Ansprüche, wobei zumindest eine der delta-4-Desaturase, delta-5-Desaturase oder delta-6-Desaturase CoA-Fettsäureester als Substrat verwenden kann.

**13.** Pflanzen-Öl enthaltend mindestens 0,5% EPA and/or DHA und weniger als 30% Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopentendodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11 - diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure).

**14.** Öl nach Anspruch 13, wobei das Öle, Lipide, Fettsäuren oder Fettsäuregemische tierischen Ursprungs gemischt ist.

Figur 1: Verschiedene Synthese-Wege zur Biosynthese von DHA (Docosahexaensäure)

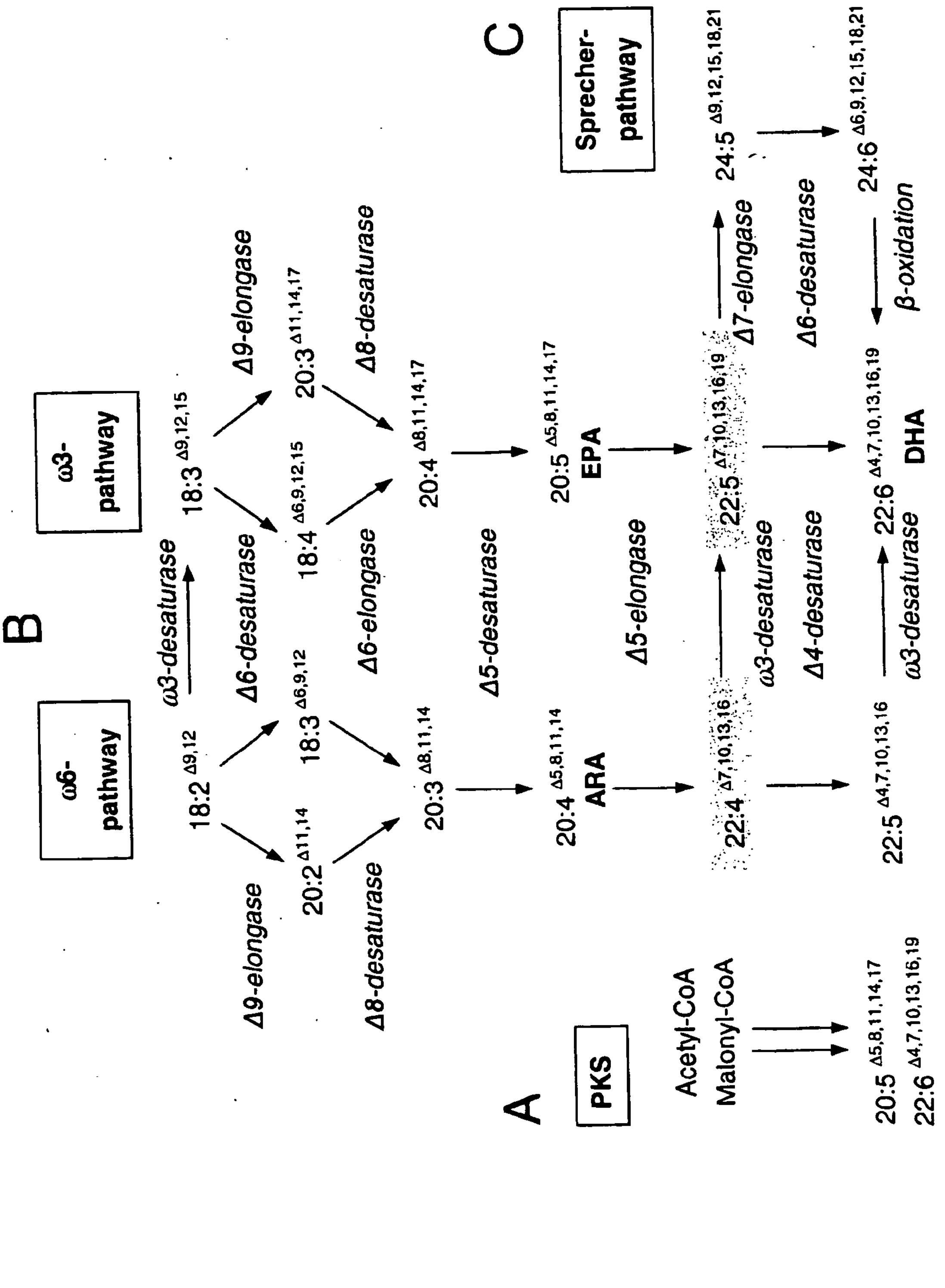

Figur 2: Substratspezifität der Δ-5-Elongase (SEQ ID NO: 53) gegenüber verschiedenen Fettsäuren

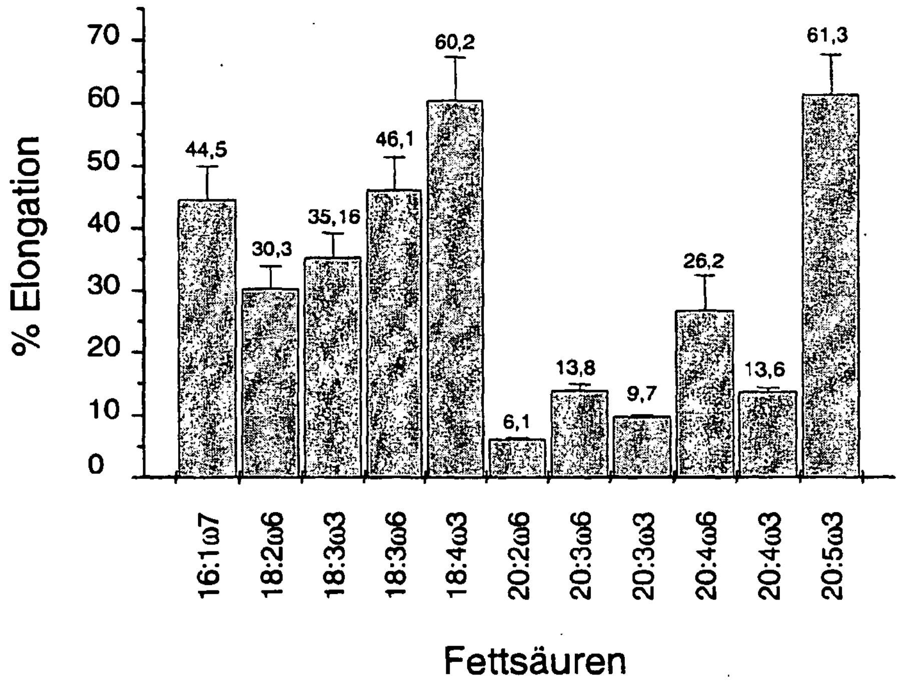

Figur 3: Rekonstitution der DHA-Biosynthese in Hefe ausgehend von 20:5ω3.

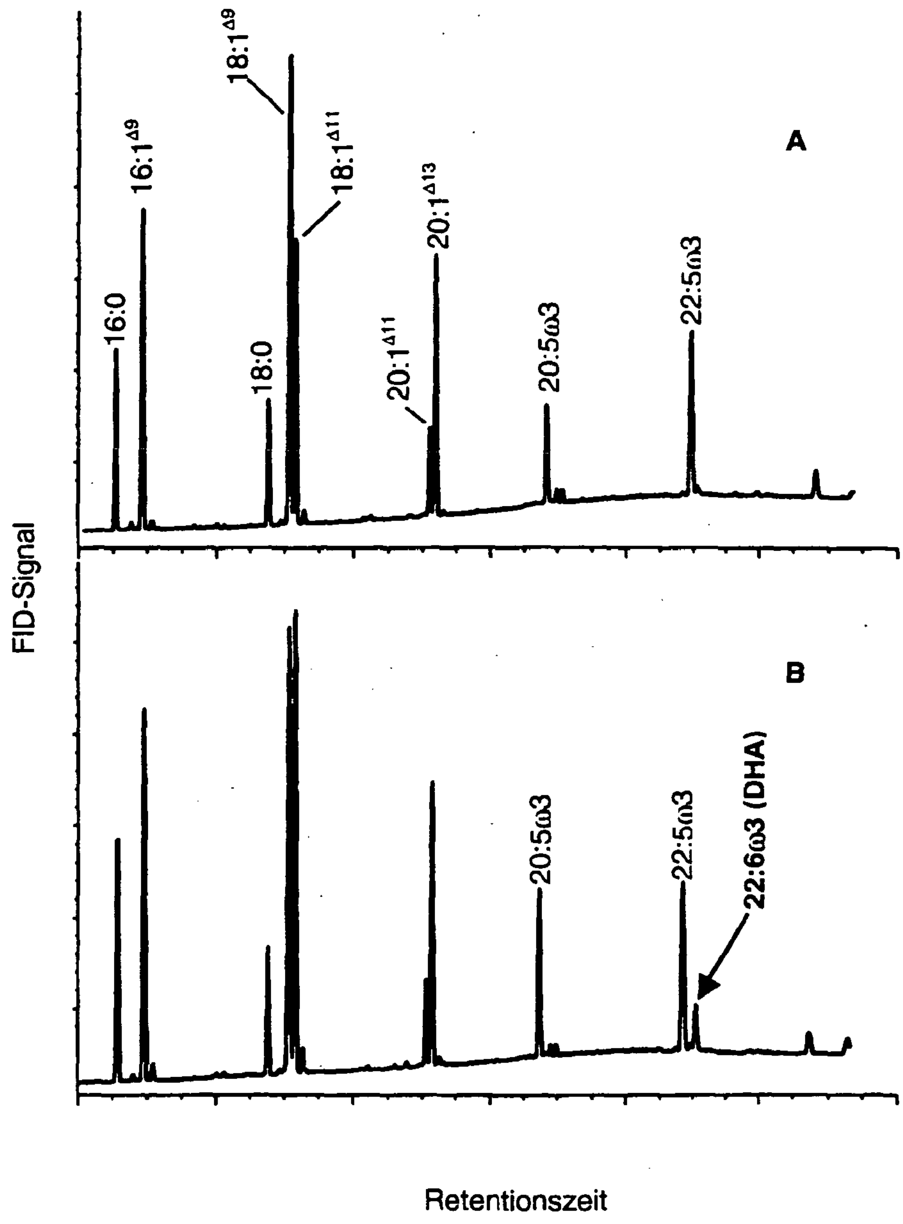

Figur 4: Rekonstitution der DHA-Biosynthese in Hefe ausgehend von 18:4ω3.

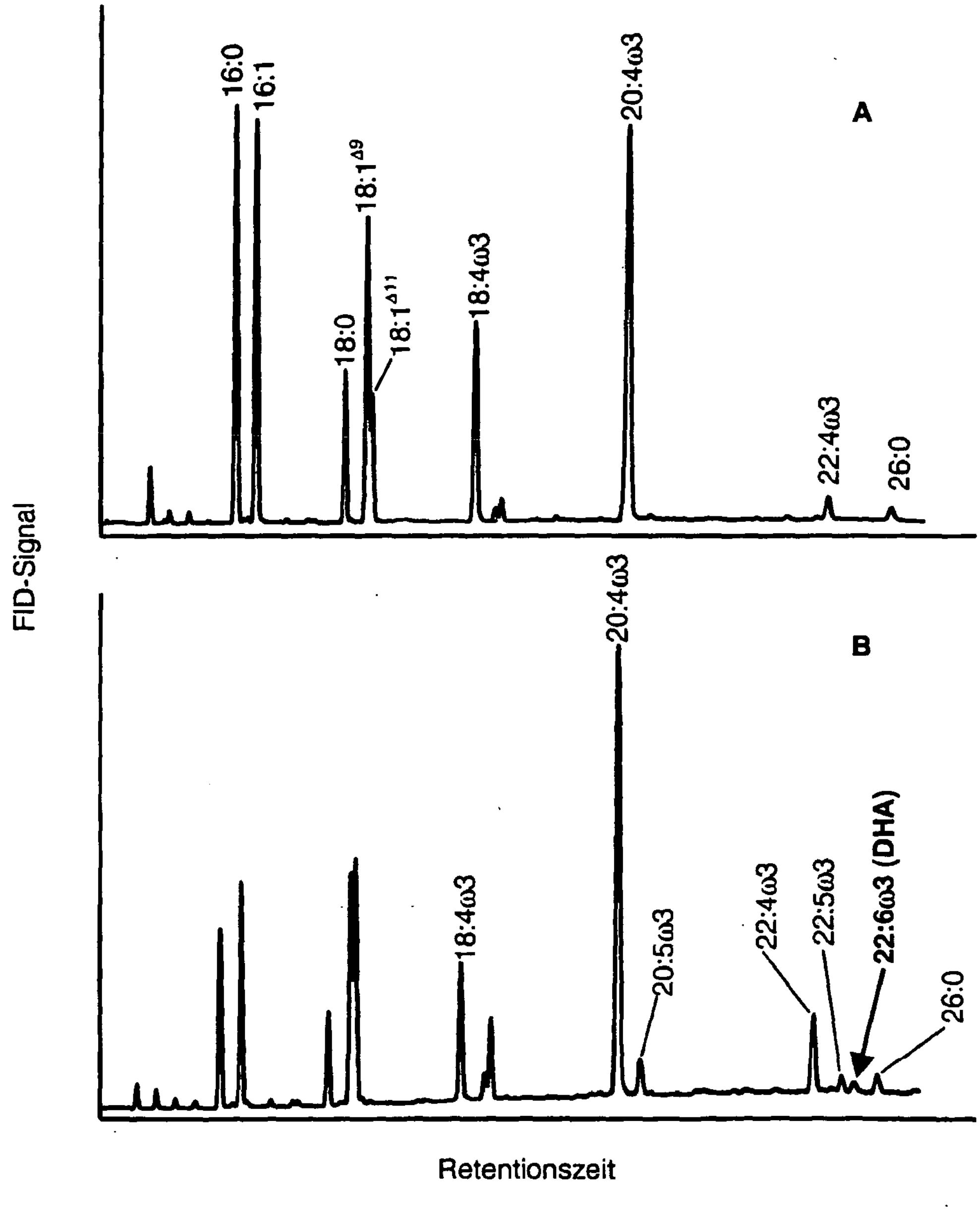

Figur 5: Fettsäure-Zusammensetzung (in Mol %) transgener Hefen, die mit den Vektoren pYes3-OmELO3/pYes2-EgD4 oder pYes3-OmELO3/pYes2-EgD4+pESCLeu-PtD5 transformiert worden waren. Die Hefezellen wurden in Minimalmedium ohne Tryptophan und Uracil / und Leucin in Gegenwart von 250 µM 20:5$^{\Delta5,8,11,14,17}$ bzw. 18:4$^{\Delta6,9,12,15}$ kultiviert. Die Fettsäuremethylester wurden durch saure Methanolyse aus Zellsedimenten gewonnen und über GLC analysiert. Jeder Wert gibt den Mittelwert (n=4) ± Standardabweichung wieder.

| | pYes3-OmELO/pYes2-EgD4 | pYes3-OmELO/pYes2-EgD4 EgD4 + pESCLeu-PtD5 |
|---|---|---|
| Fettsäuren | Fütterung mit 20:5$^{\Delta5,8,11,14,17}$ | Fütterung mit 18:4$^{\Delta6,9,12,15}$ |
| 16:0 | 9,35 ± 1,61 | 7,35 ± 1,37 |
| 16:1 $^{\Delta9}$ | 14,70 ± 2,72 | 10,02 ± 1,81 |
| 18:0 | 5,11 ± 1,09 | 4,27 ± 1,21 |
| 18:1 $^{\Delta9}$ | 19,49 ± 3,01 | 10,81 ± 1,95 |
| **18:1 $^{\Delta11}$** | **18,93 ± 2,71** | **11,61 ± 1,48** |
| **18:4 $^{\Delta6,9,12,15}$** | **-** | **7,79 ± 1,29** |
| **20:1 $^{\Delta11}$** | **3,24 ± 0,41** | **1,56 ± 0,23** |
| **20:1 $^{\Delta13}$** | **11,13± 2,07** | **4,40 ± 0,78** |
| **20:4$^{\Delta8,11,14,17}$** | **-** | **30,05 ± 3,16** |
| **20:5$^{\Delta5,8,11,14,17}$** | **6,91± 1,10** | **3,72 ± 0,59** |
| **22:4$^{\Delta10,13,16,17}$** | **-** | **5,71 ± 1,30** |
| **22:5 $^{\Delta7,10,13,16,19}$** | **8,77 ± 1,32** | **1,10 ± 0,27** |
| **22:6 $^{\Delta4,7,10,13,16,19}$** | **2,73 ± 0,39** | **0,58 ± 0,10** |

Figur 6: Fütterungsexperiment zur Bestimmung der Funktionalität und Substratspezifität mit Hefestämmen

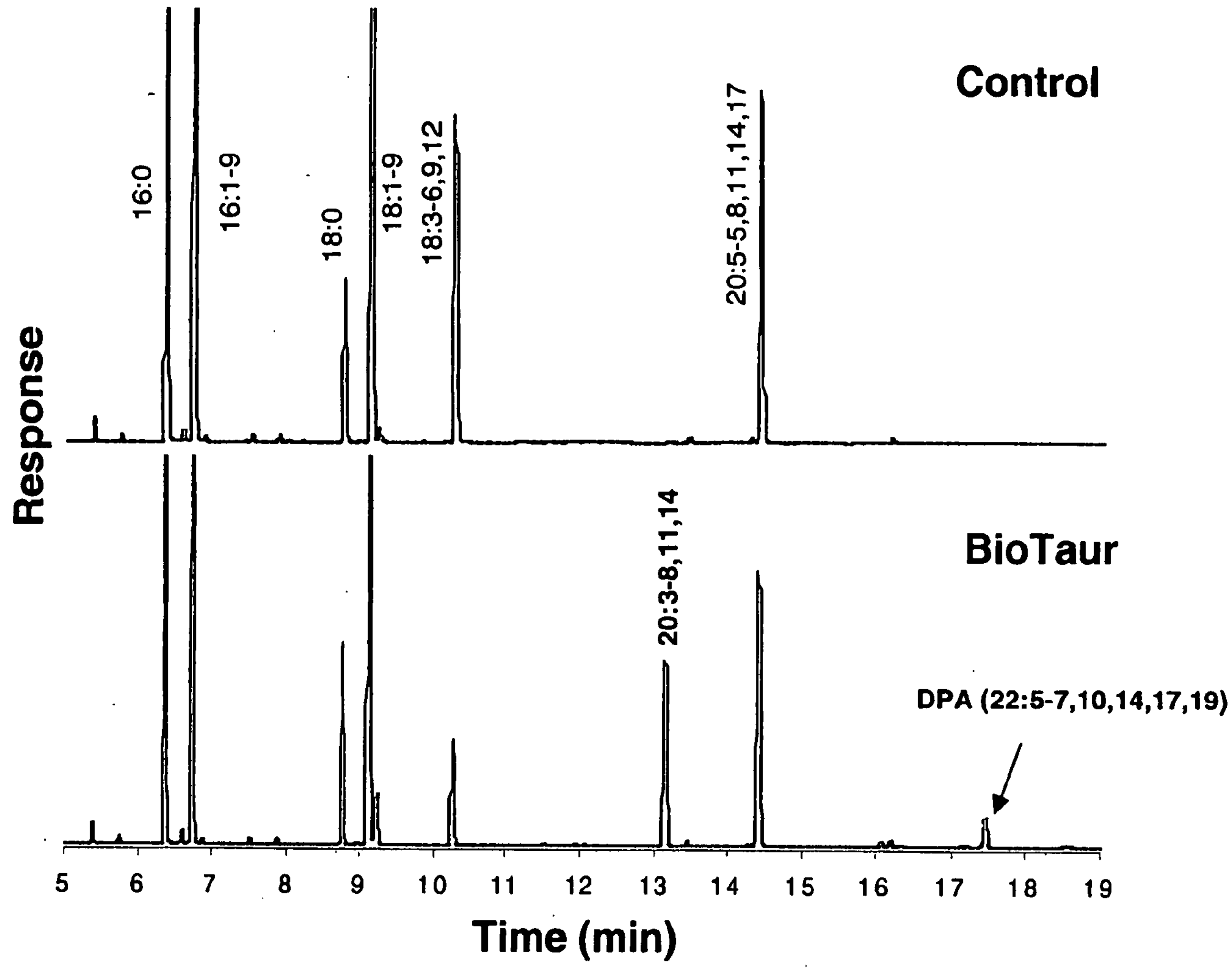

Figur 7: Elongation von Eicosapentaensäure durch OtElo1

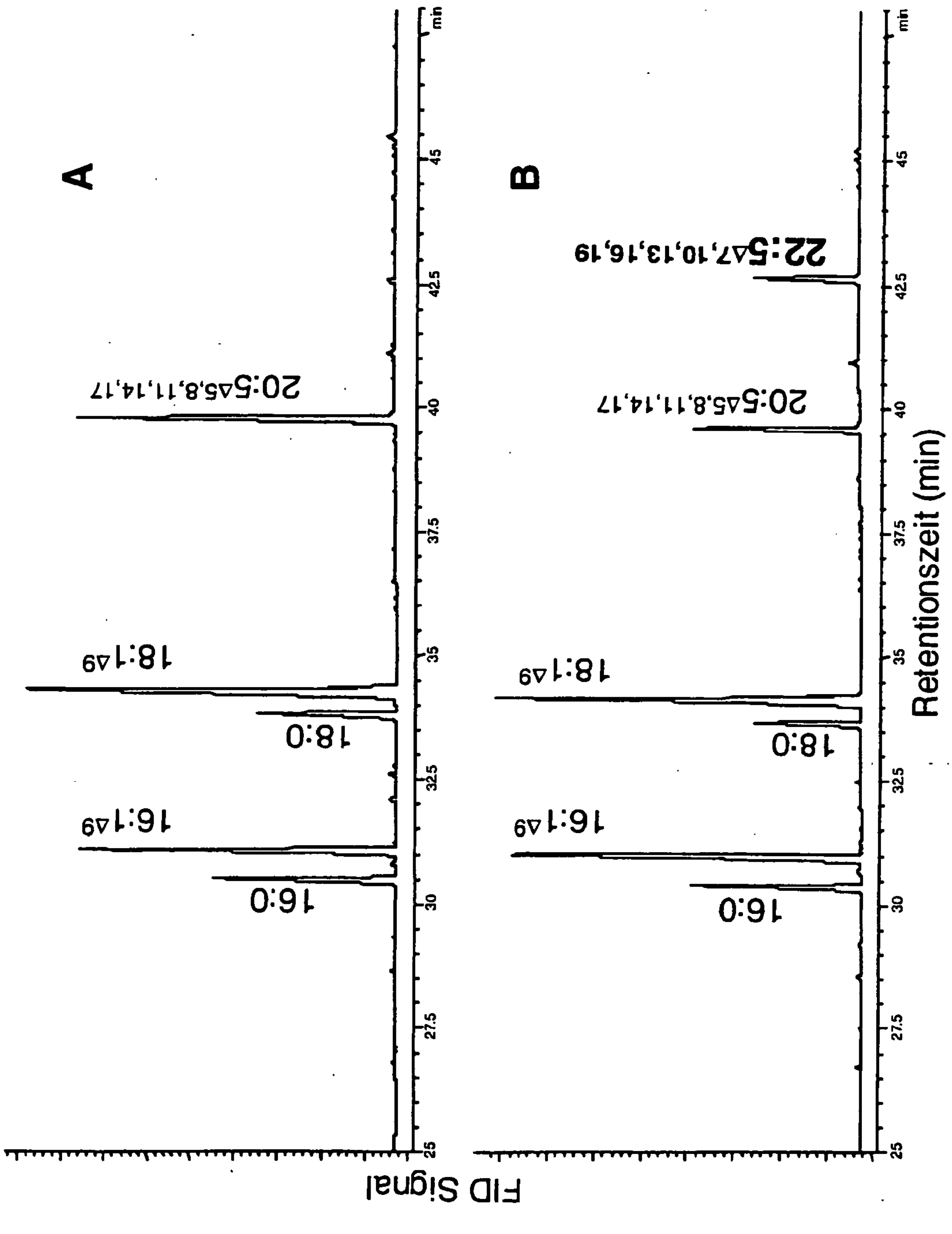

Figur 8: Elongation von Arachidonsäure durch OtElo1

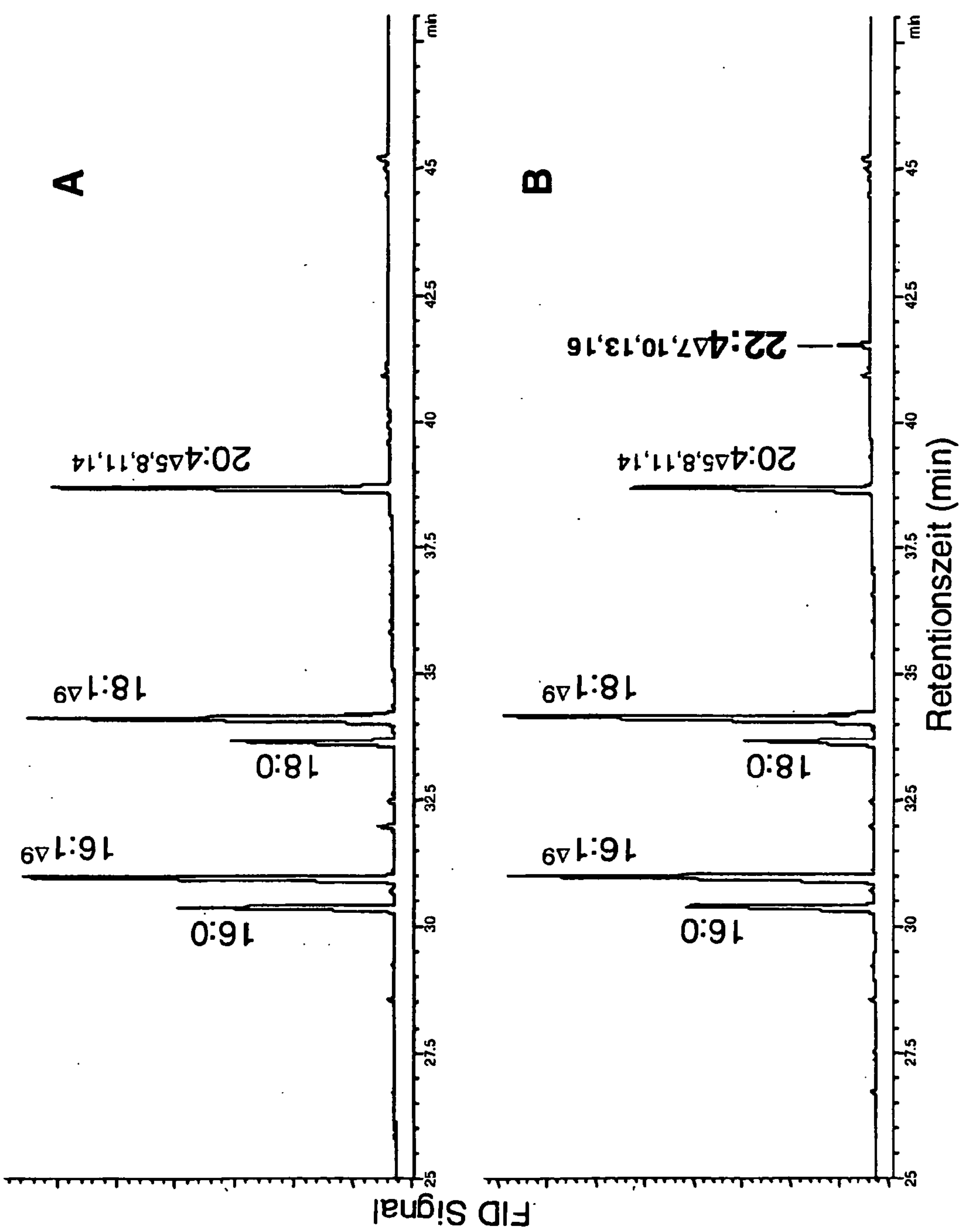

Figur 9: Expression von TpELO1 in Hefe

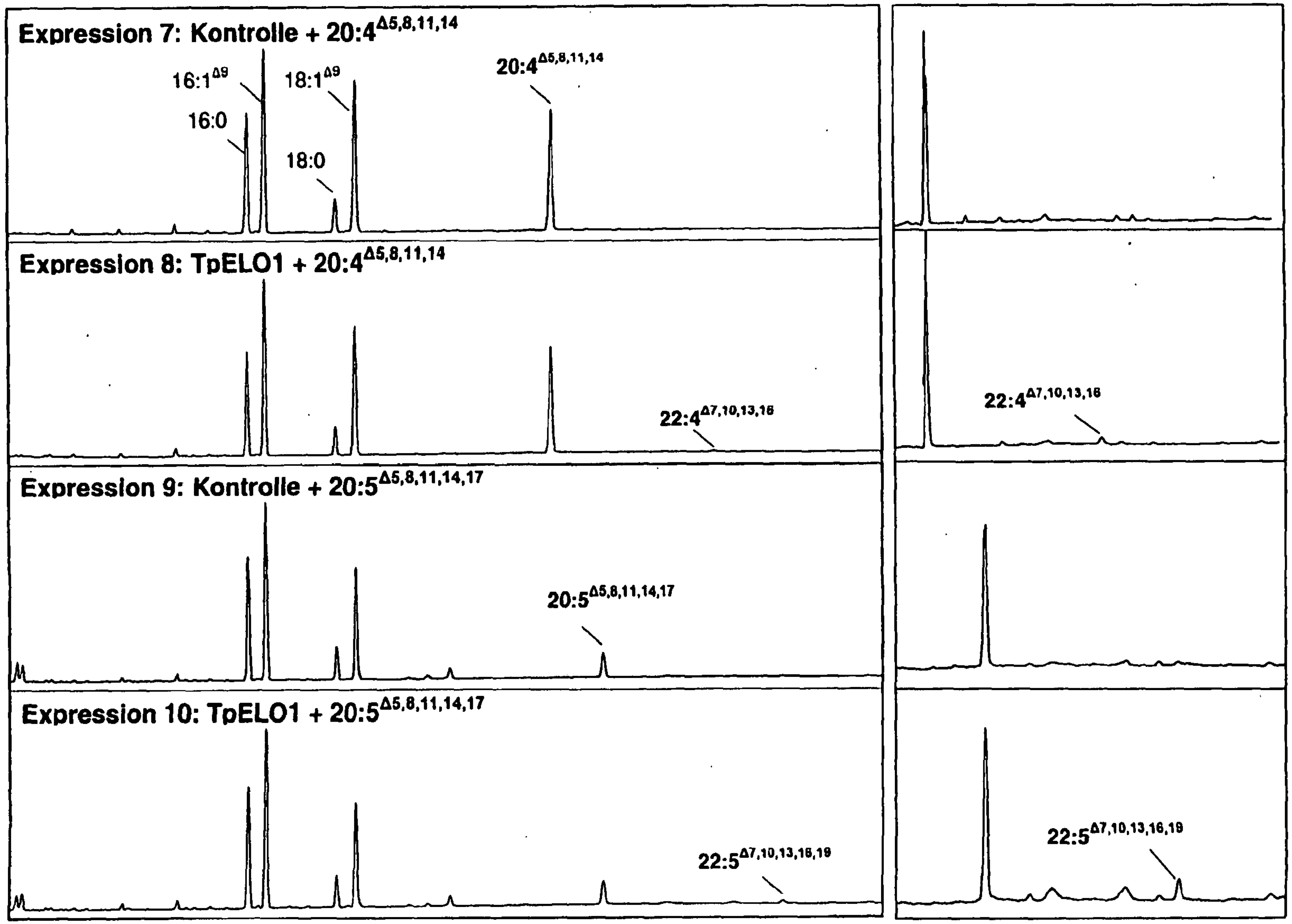

Figur 10: Expression von TpELO3 in Hefe.

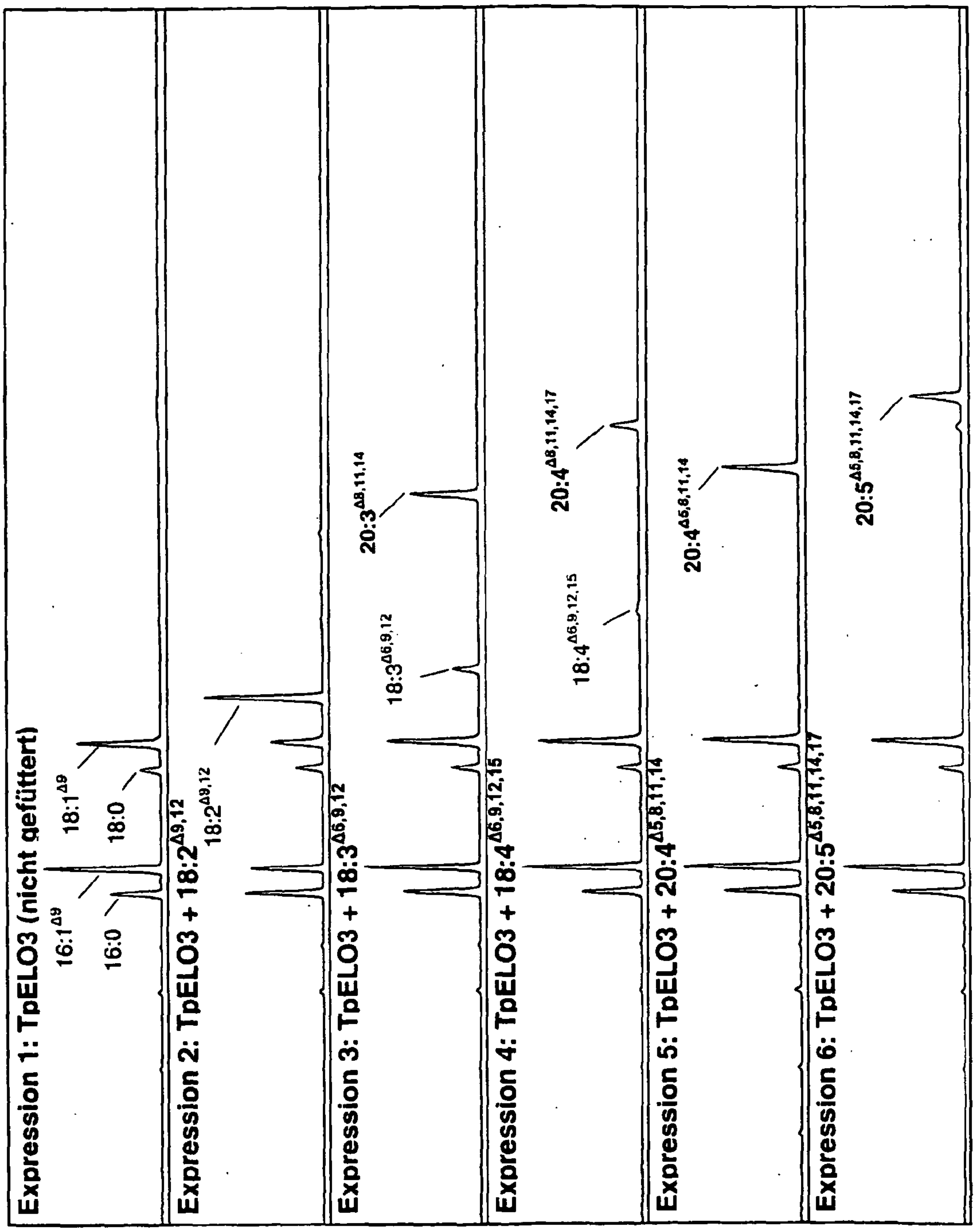

Figur 11: Expression von Thraustochytrium Δ5-Elongase TL16/pYES2.1 in Hefe.

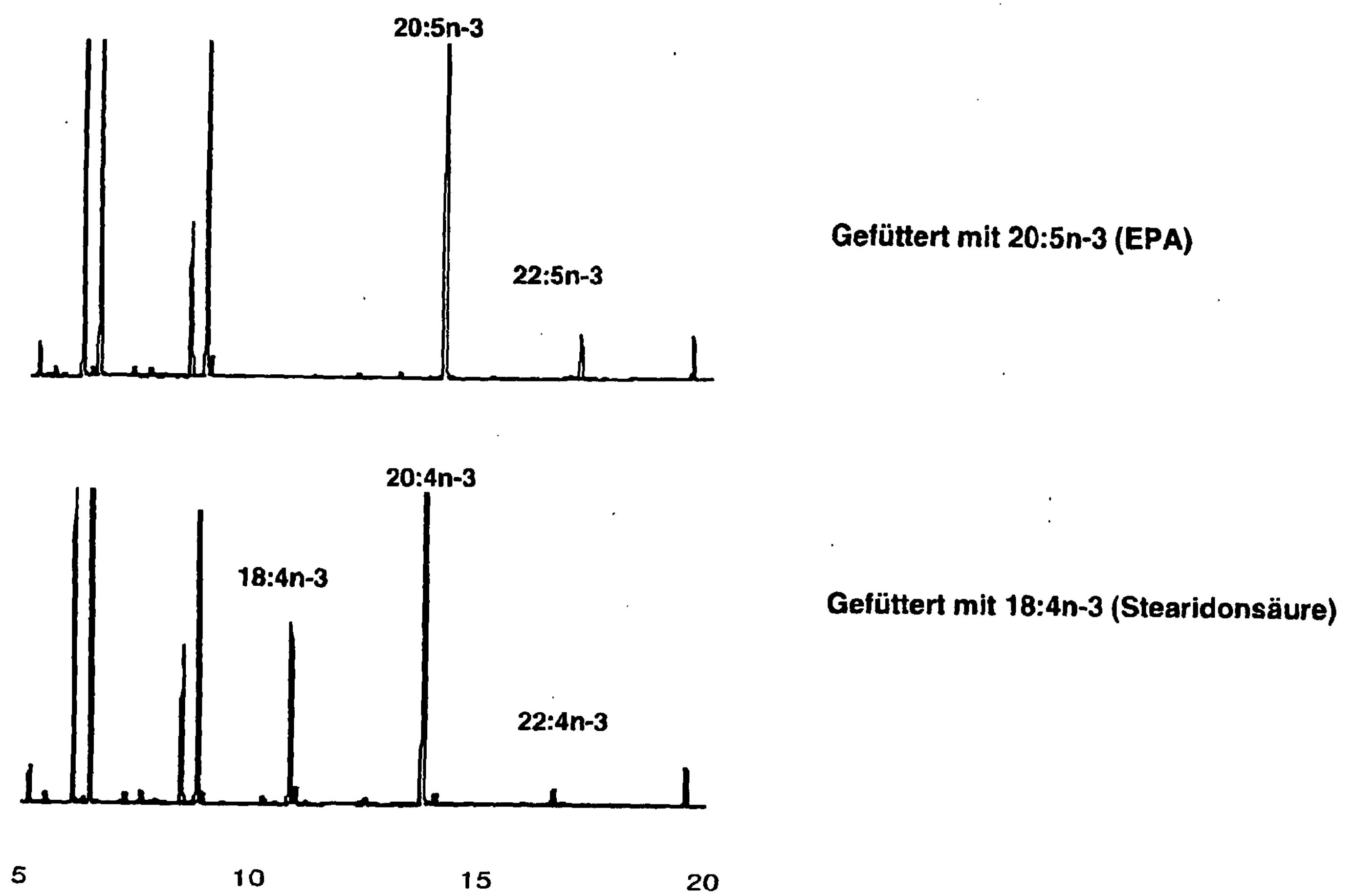

Figur 12: Desaturierung von Linolsäure (18:2 ω-6-Fettsäure) zu α-Linolensäure (18:3 ω-3-Fettsäure) durch Pi-omega3Des.

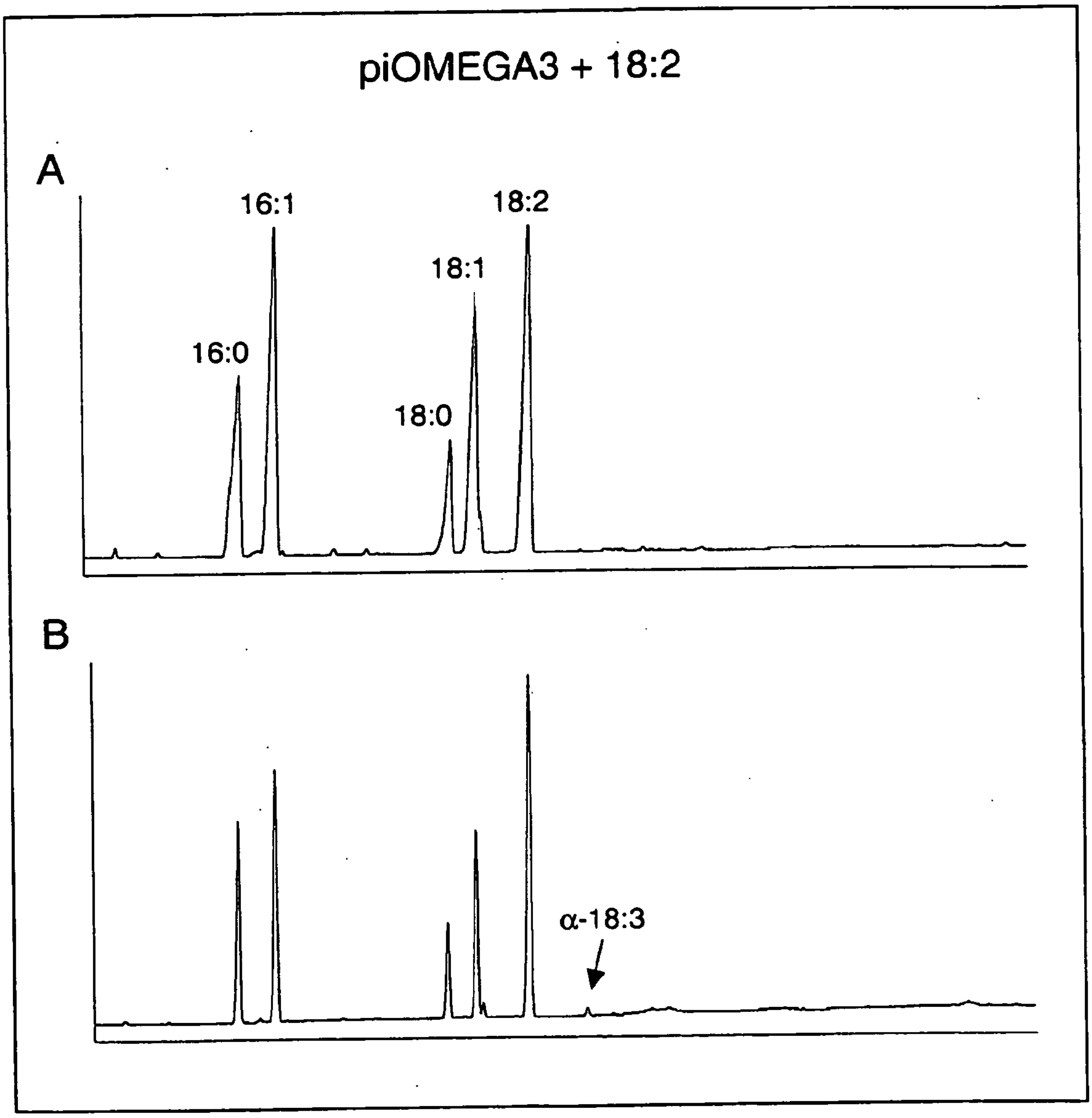

Figur 13: Desaturierung von γ-Linolensäure (18:3 ω-6-Fettsäure) zu Stearidonsäure (18:4 ω-3-Fettsäure) durch Pi-omega3Des.

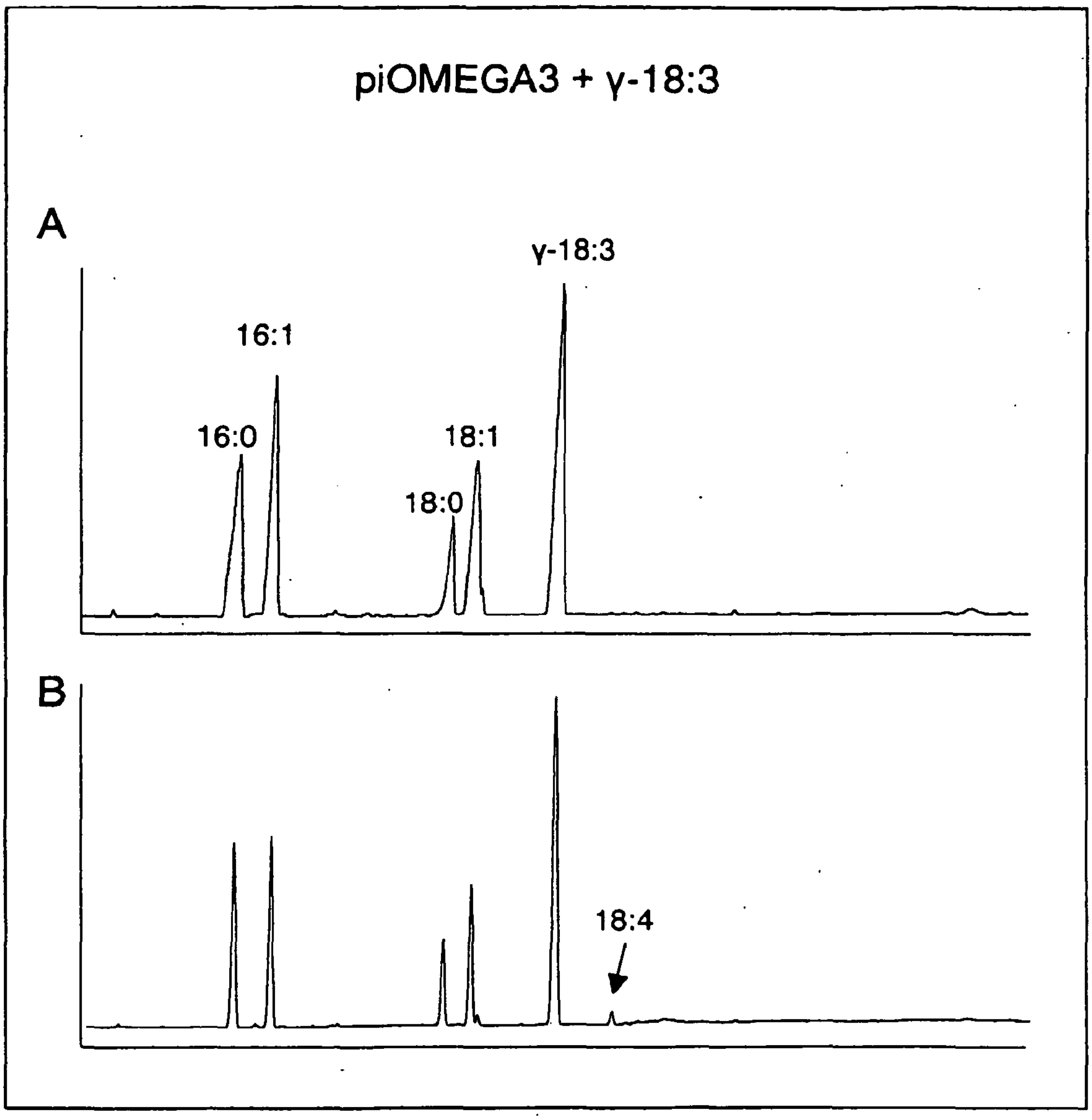

Figur 14: Desaturierung von C20:2 ω-6-Fettsäure zu C20:3 ω-3-Fettsäure durch Pi-omega3Des.

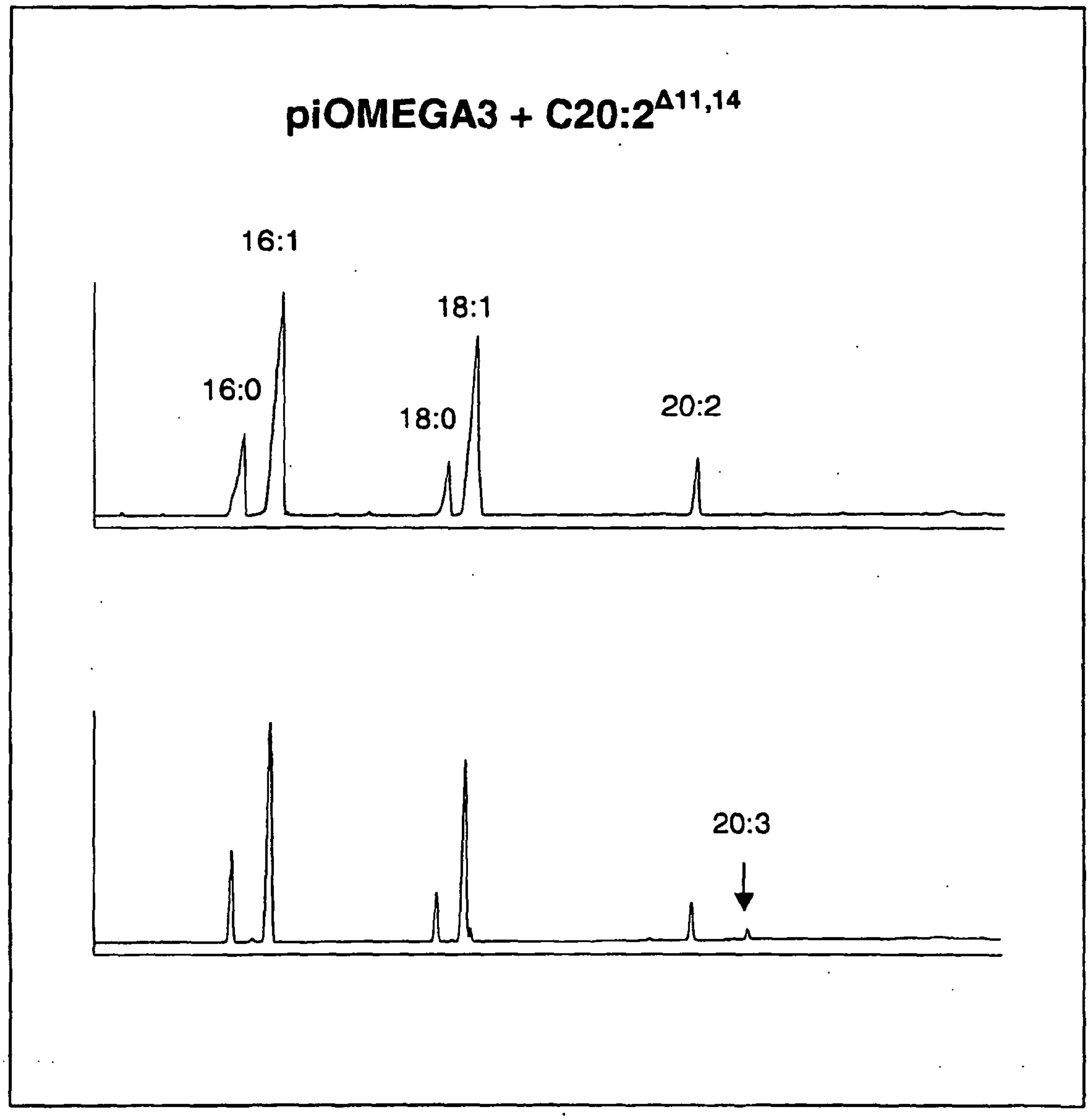

Figur 15: Desaturierung von C20:3-ω-6-Fettsäure zu C20:4-ω-3-Fettsäure durch Pi-omega3Des.

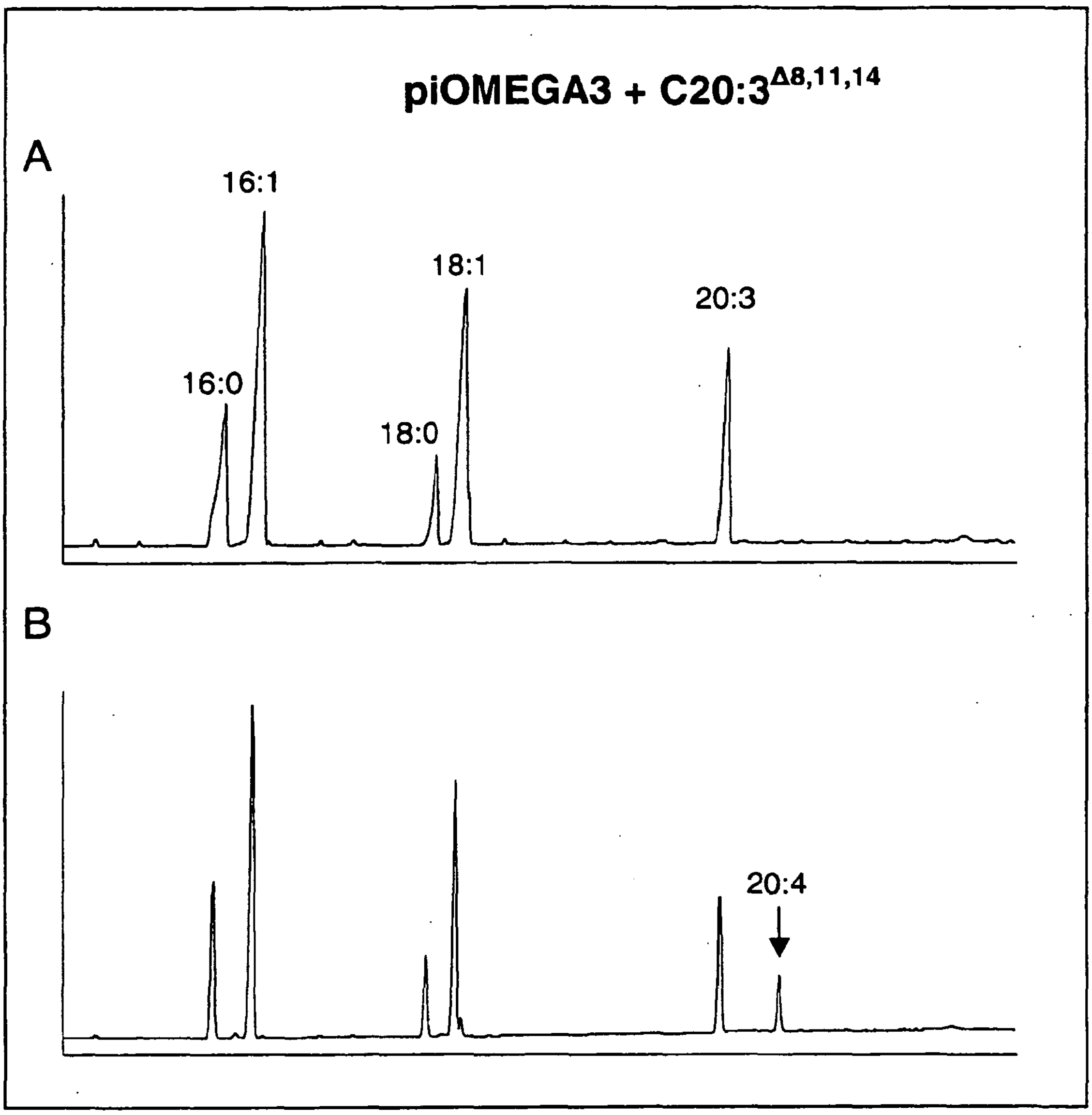

Figur 16: Desaturierung von Arachidonsäure (C20:4-ω-6-Fettsäure) zu Eicosapentaensäure (C20:5-ω-3-Fettsäure) durch die Pi-omega3Des.

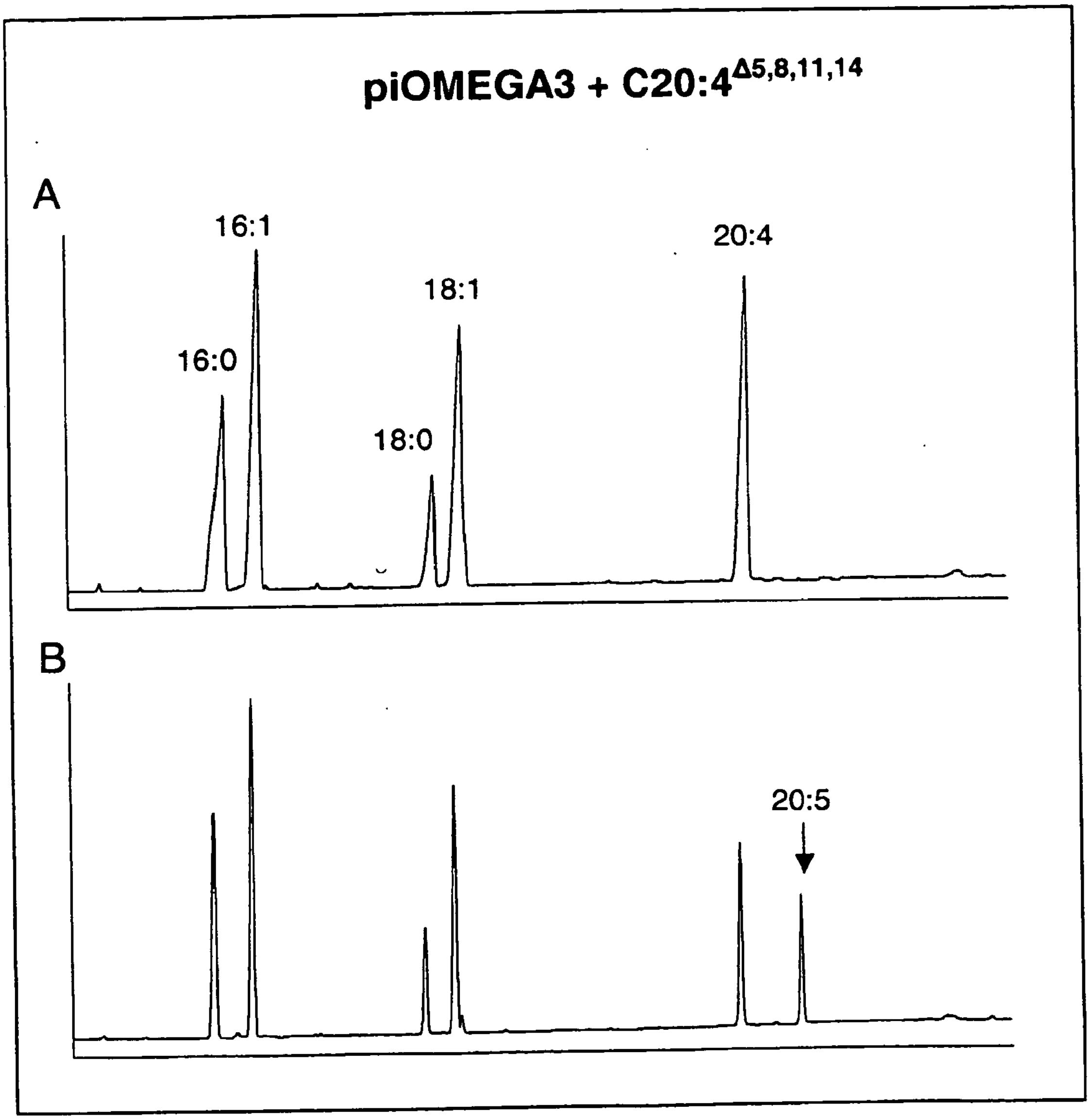

Figur 17: Desaturierung von Docosatetraensäure (C22:4-ω-6-Fettsäure) zu Docosapentaensäure (C22:5-ω-3-Fettsäure) durch Pi-omega3Des.

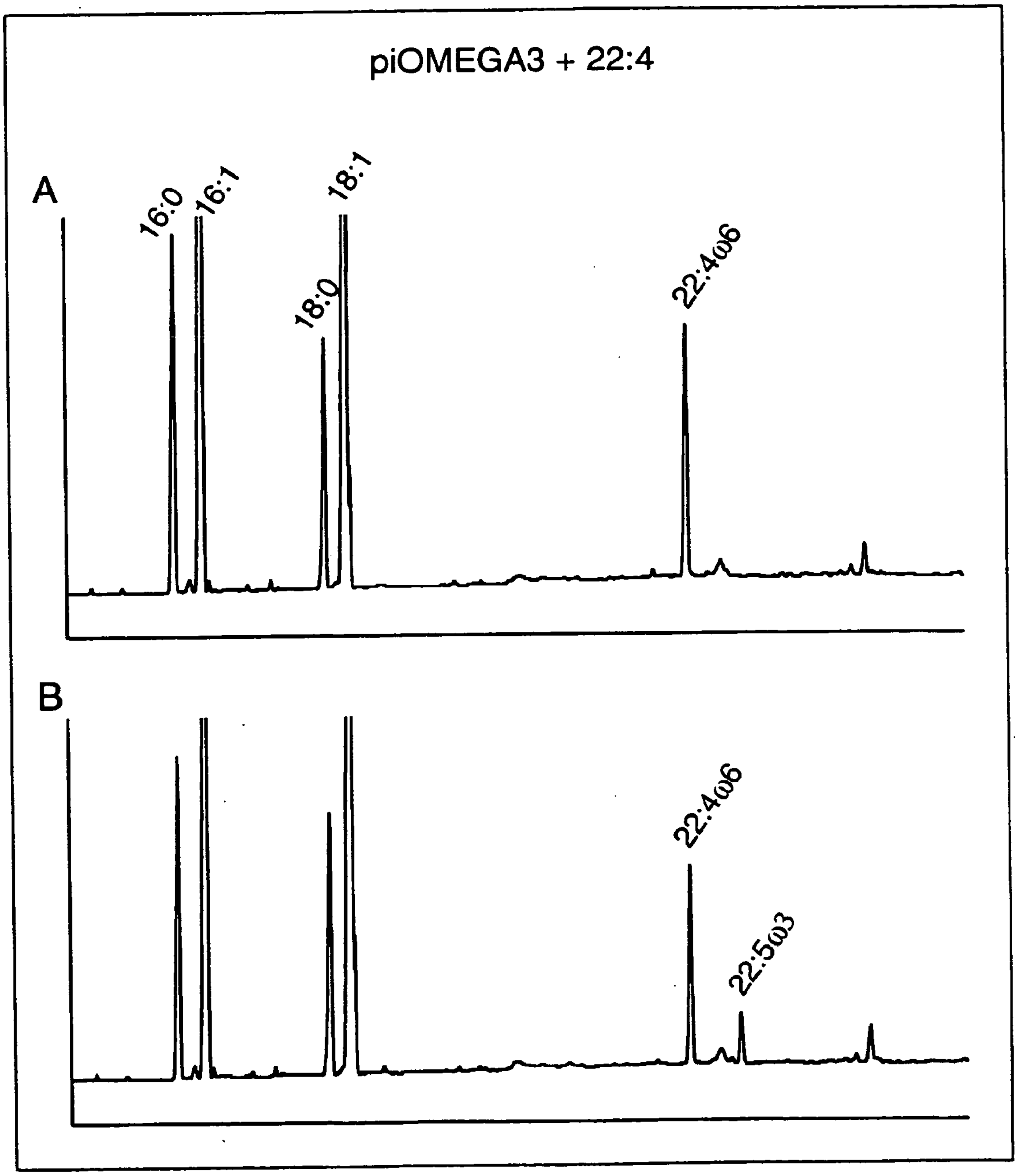

Figur 18: Substratspezifität der Pi-omega3Des gegenüber verschiedenen Fettsäuren

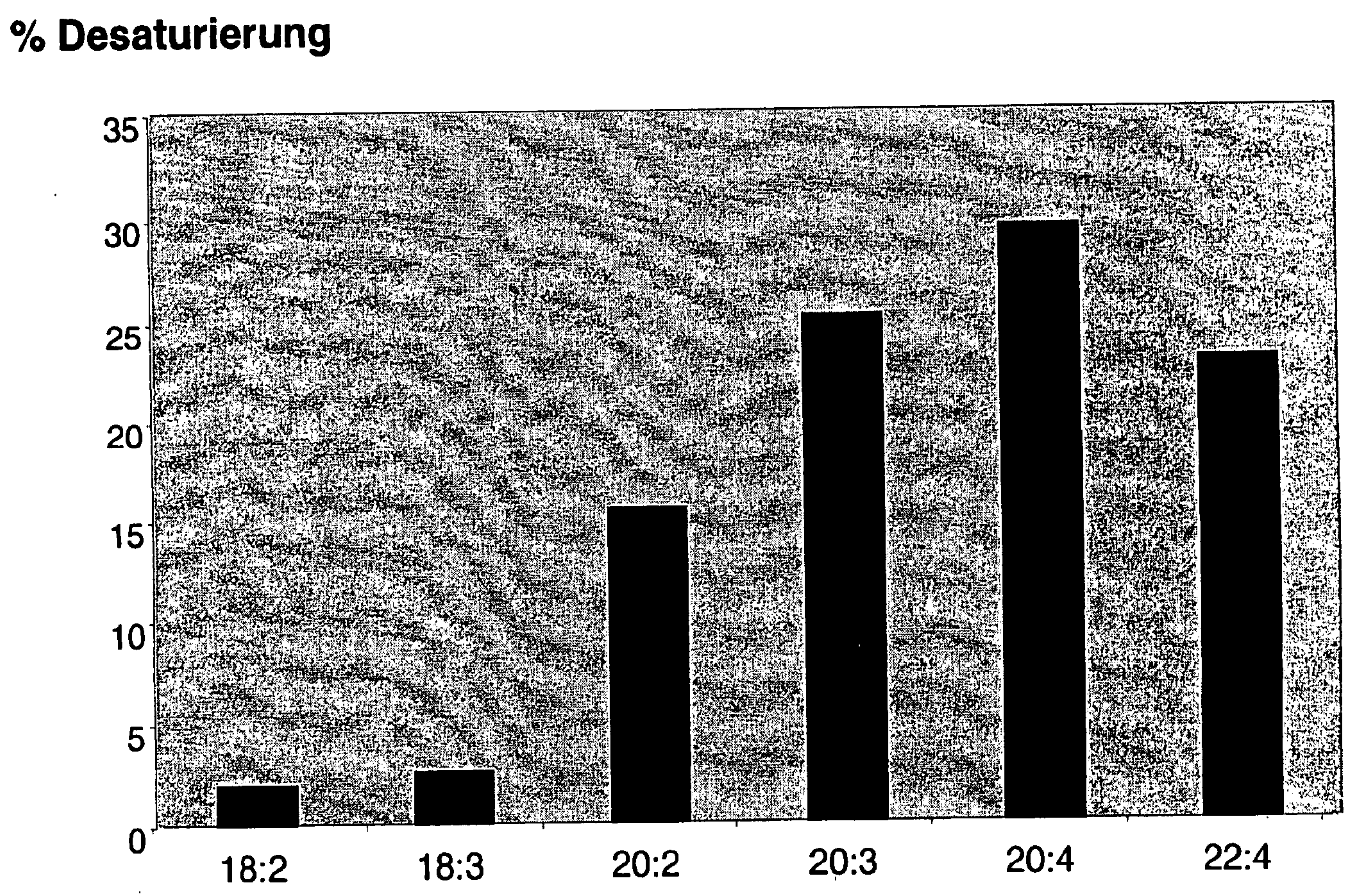

Figur 19: Desaturierung von Phospholipid gebundener Arachidonsäure zu EPA durch die Pi-Omega3Des

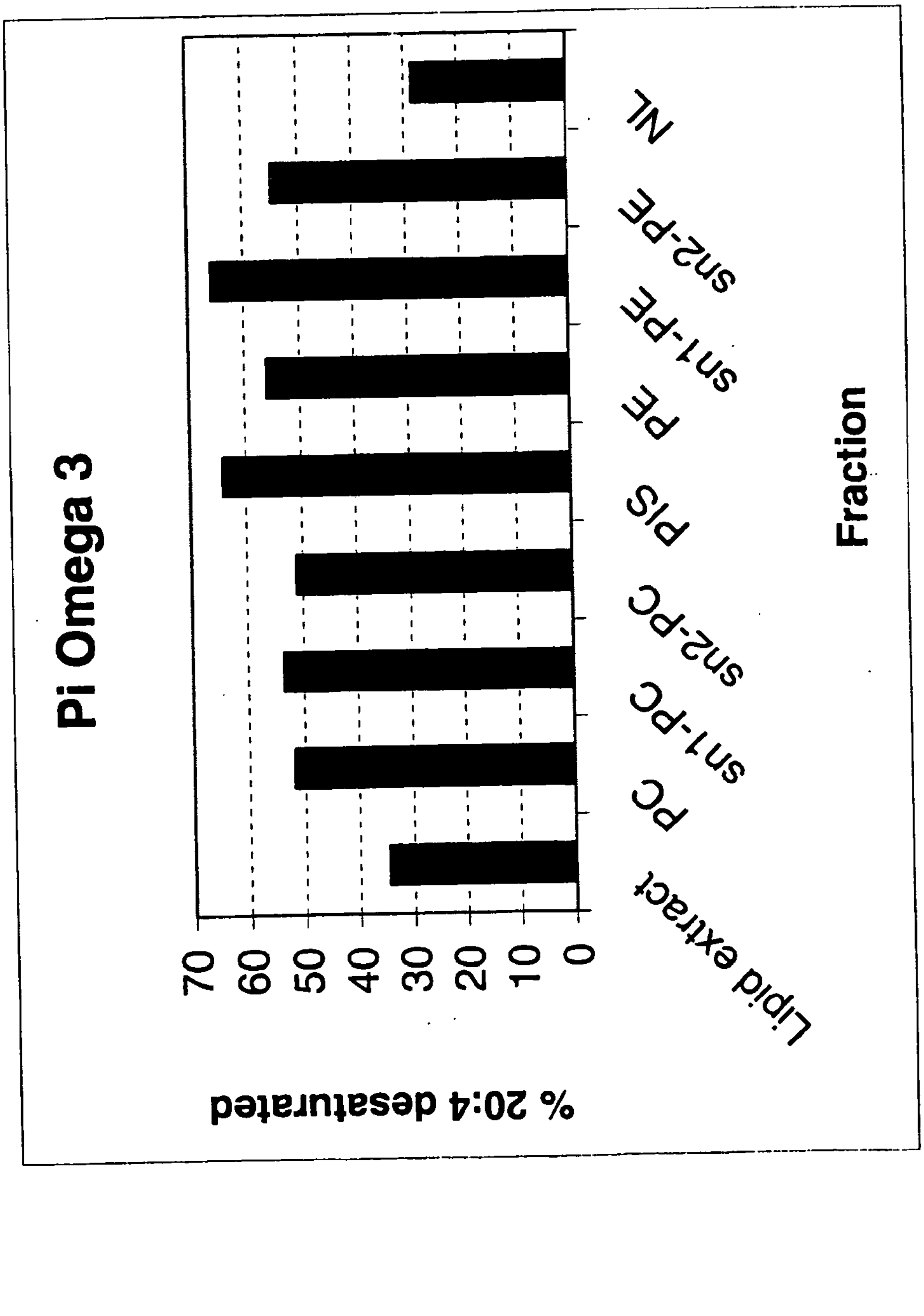

Figur 20: Umsetzung von Linolsäure (Pfeil) zu γ-Linolensäure (γ-18:3) durch Ot-Des6.1.

Absorption mAU

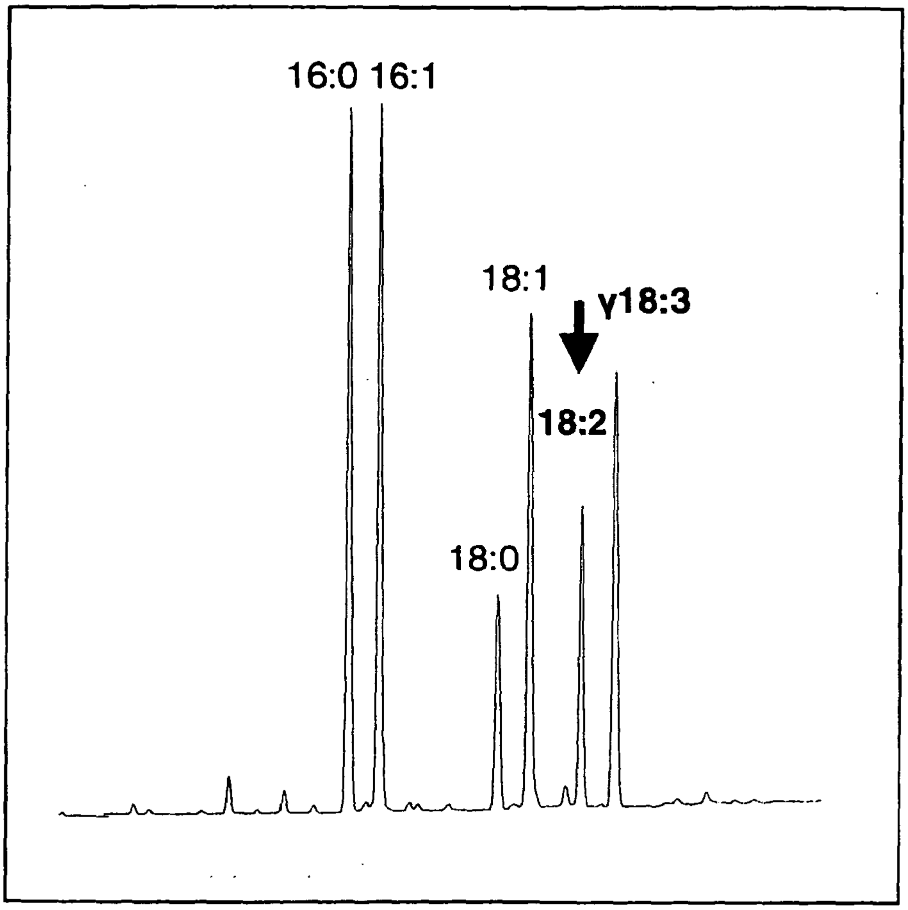


Retentionszeit

Figur 21: Umsetzung von Linolsäure und α-Linolensäure (A und C), sowie Rekonstitution des ARA- bzw. EPA-Syntheseweges in Hefe (B und D) in Gegenwart von OtD6.1.

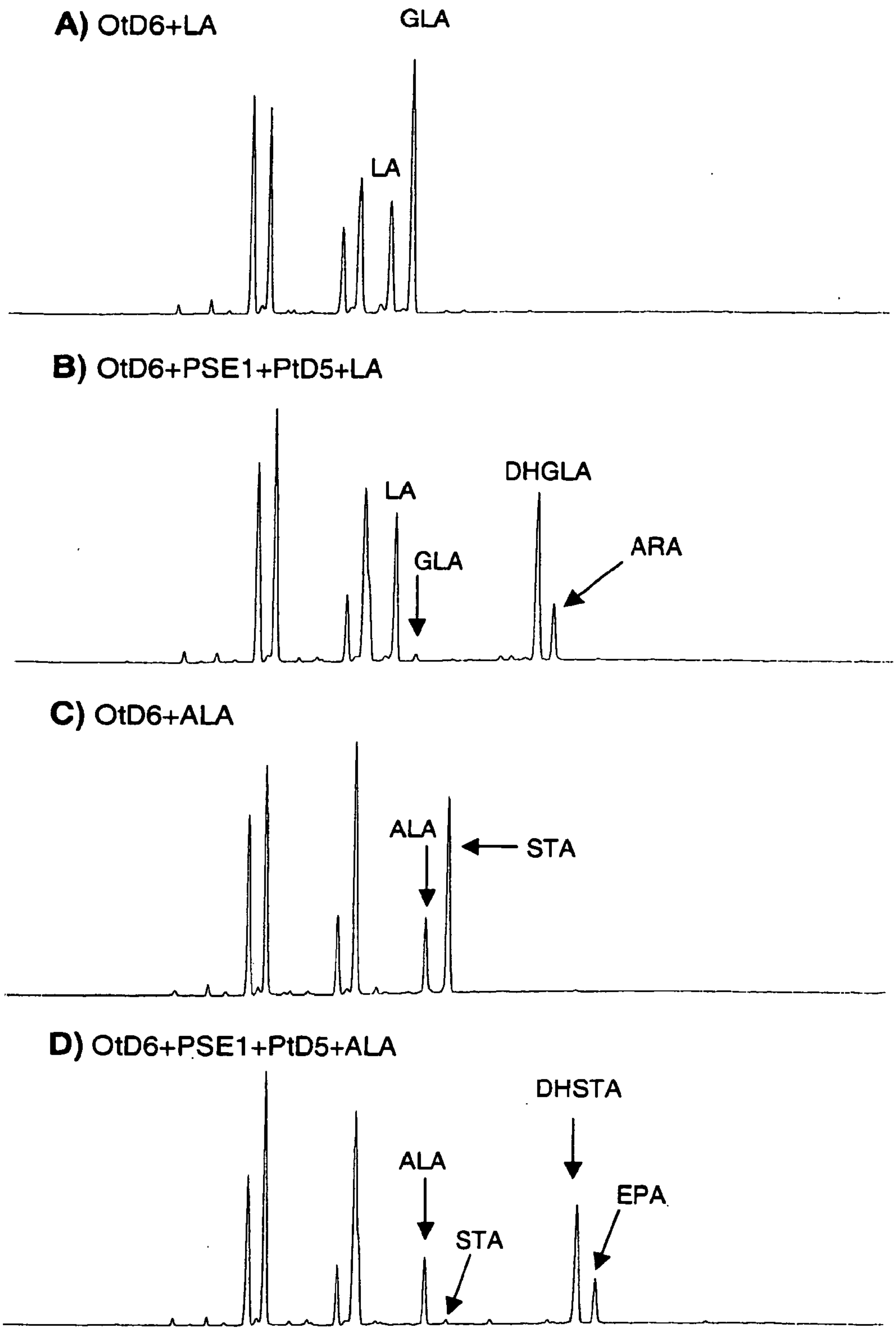

Figur 22: Expression von ELO(XI) in Hefe.

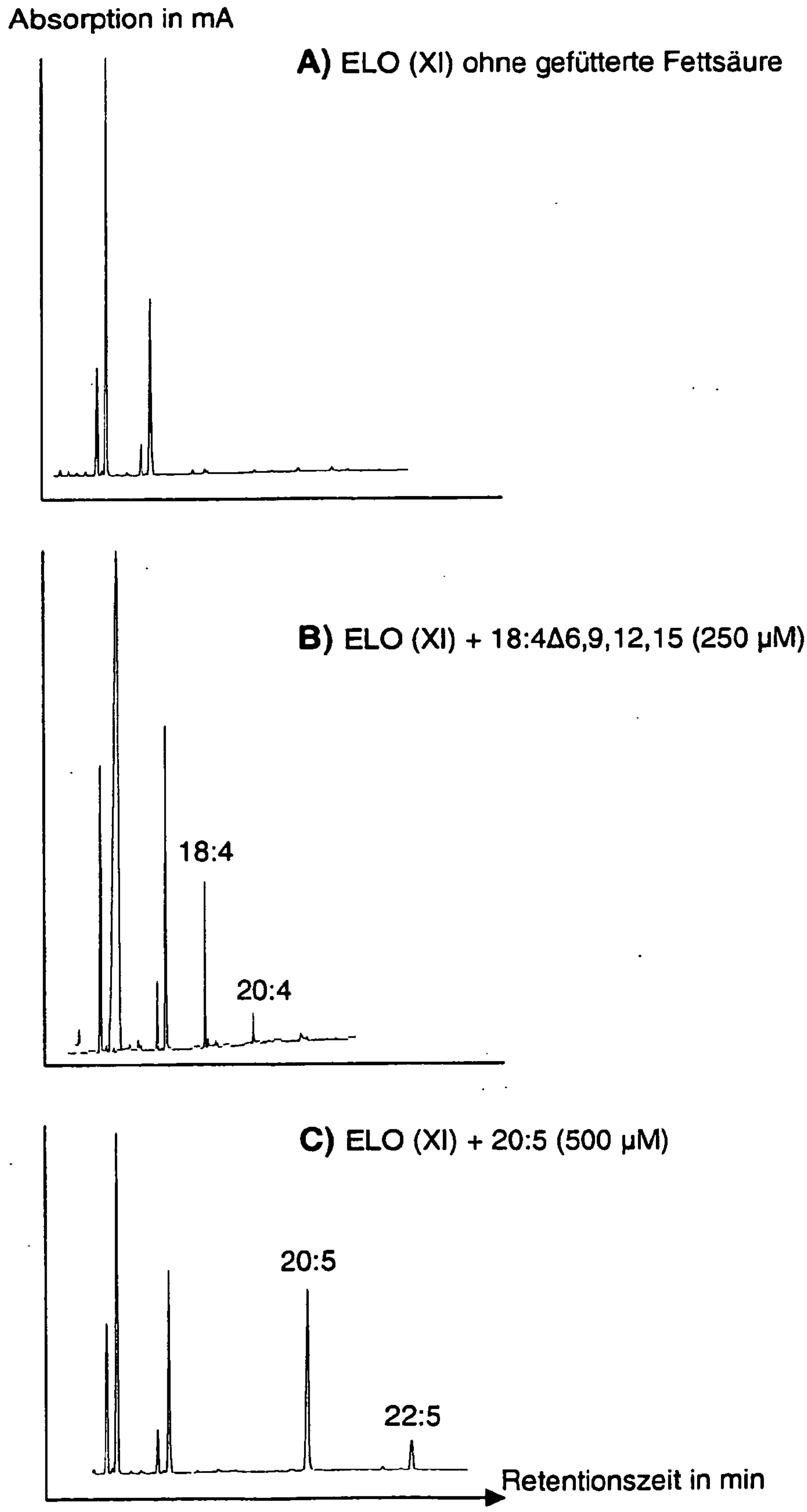

Figur 23:

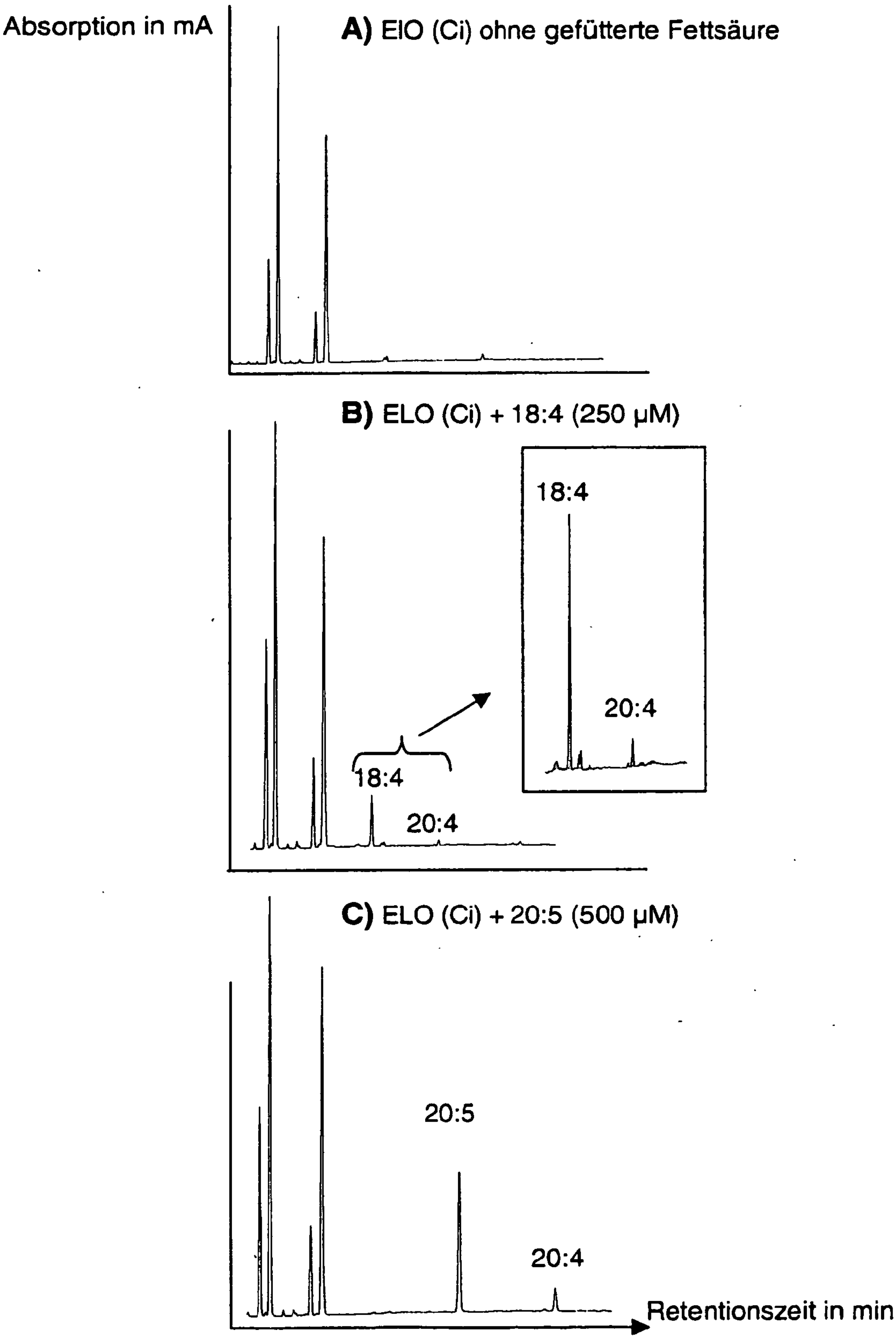
Absorption in mA
A) ElO (Ci) ohne gefütterte Fettsäure
B) ELO (Ci) + 18:4 (250 µM)
18:4
20:4
18:4
20:4
C) ELO (Ci) + 20:5 (500 µM)
20:5
20:4
Retentionszeit in min

Figur 24: Elongation von Eicosapentaensäure durch OtElo1 (B) bzw. OtElo1.2 (D). Die Kontrollen (A, C) zeigen nicht das Produkt der Elongation (22:5ω3).

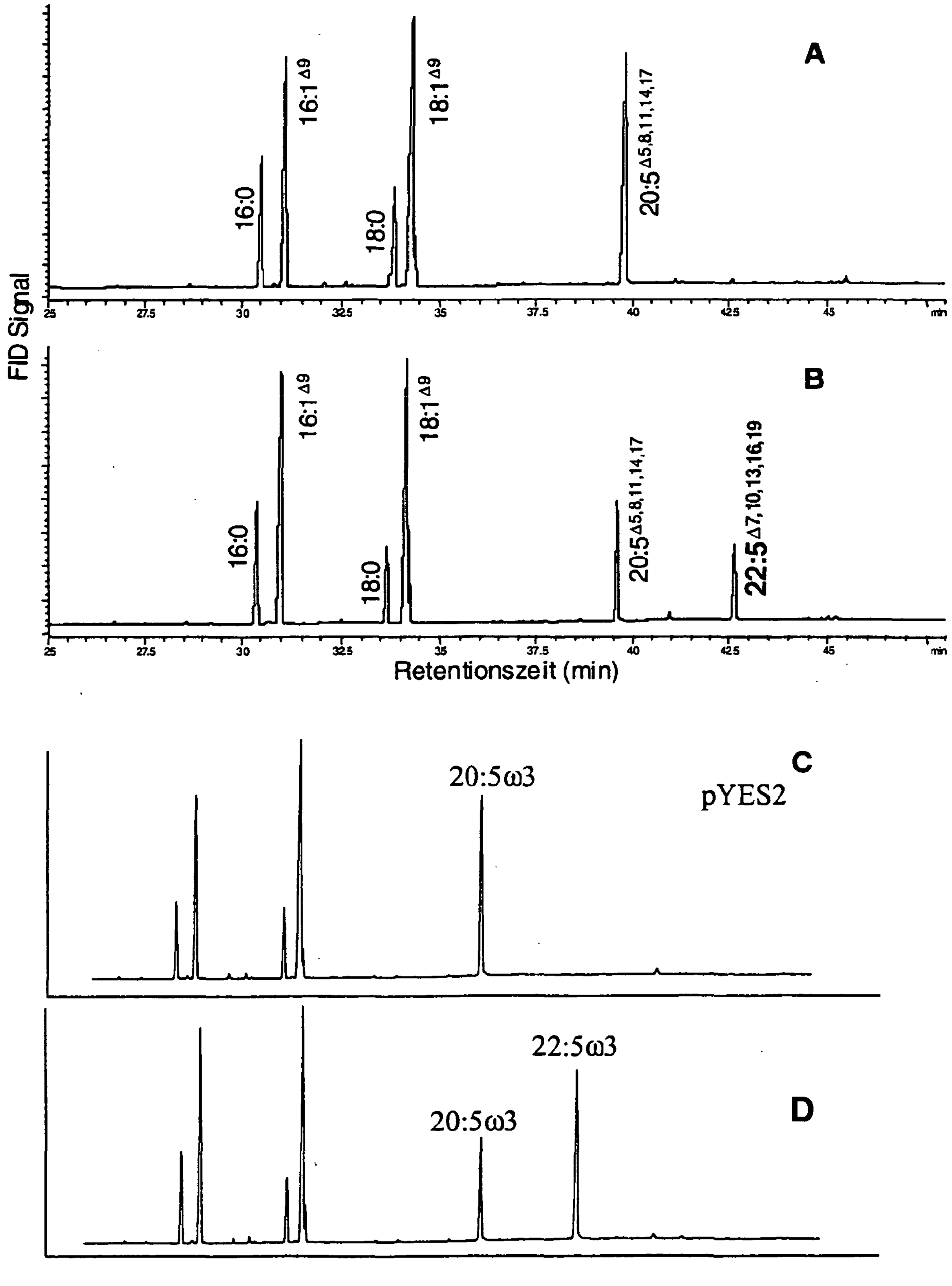

Figur 25: Elongation von Arachidonsäure durch OtElo1 (B) bzw. OtElo1.2 (D). Die Kontrollen (A, C) zeigen nicht das Produkt der Elongation (22:4ω6).

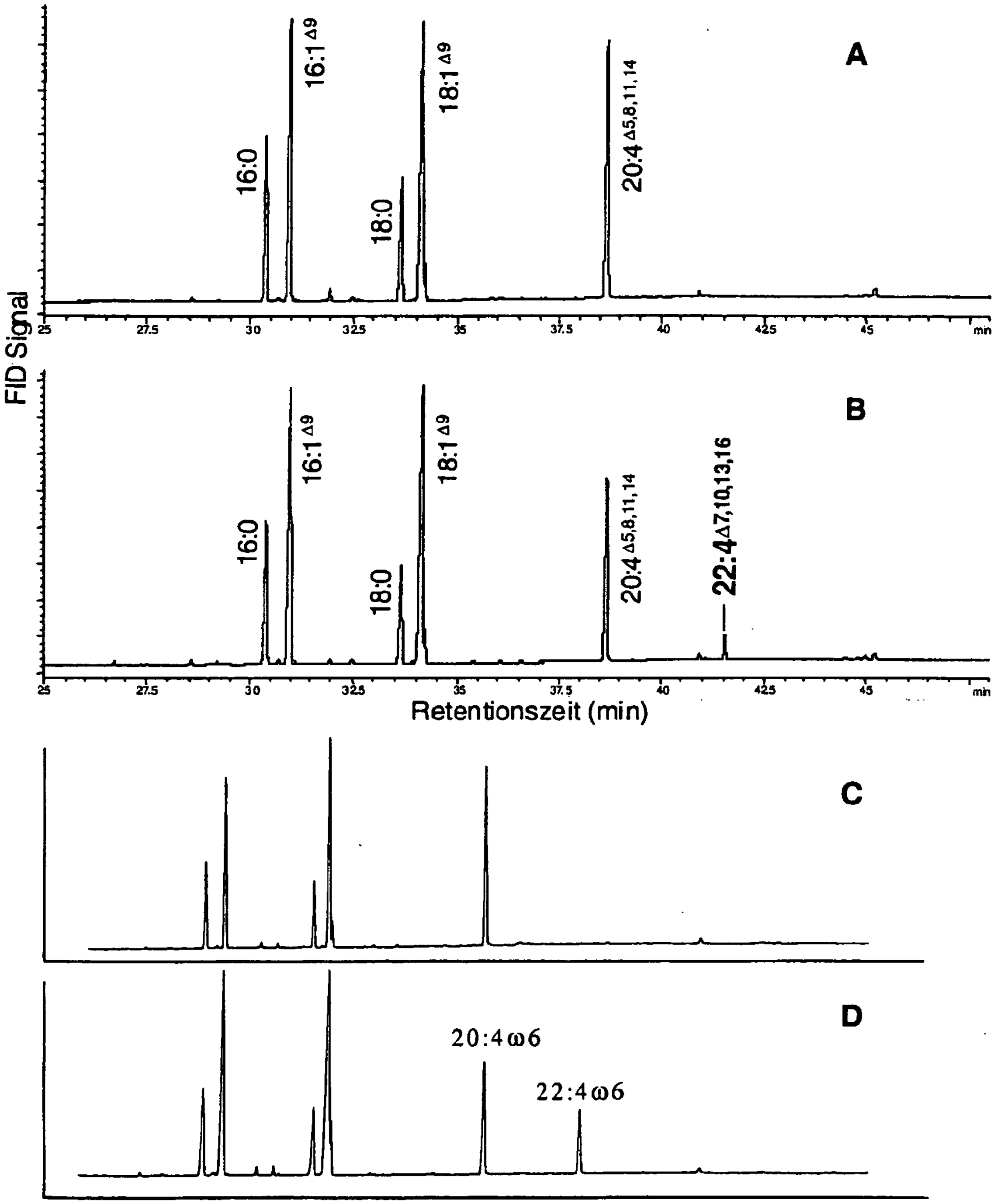

Figur 26: Elongation von 20:5n-3 durch die Elongasen At3g06470.

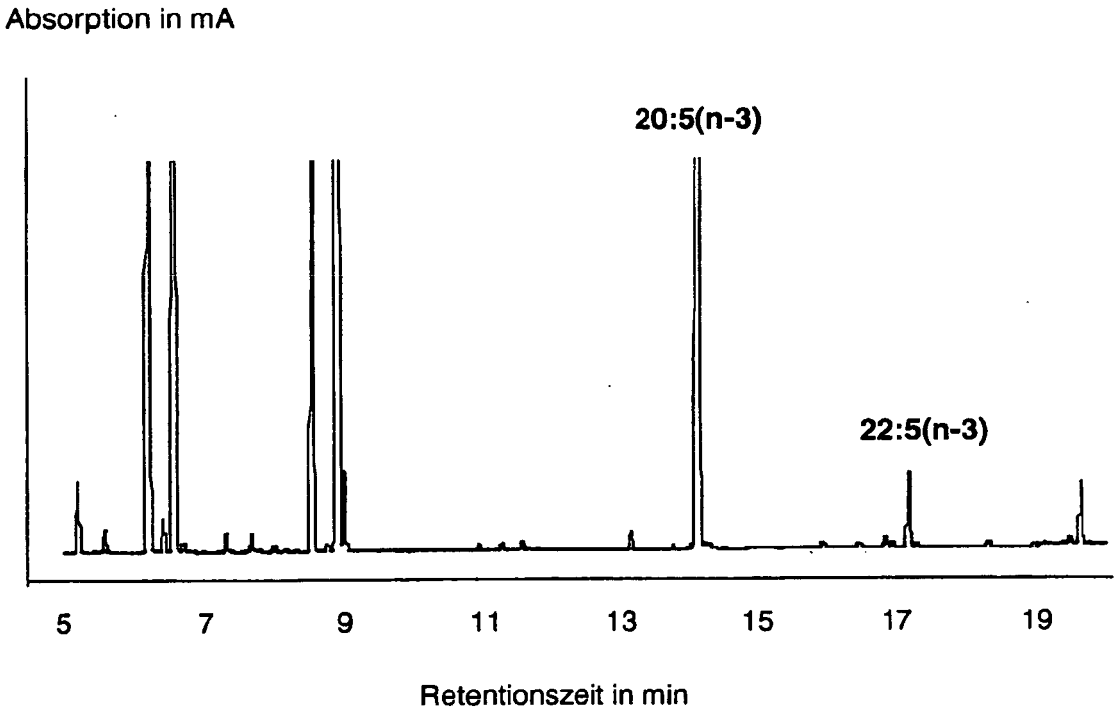

Figur 27: Substratspezifität der Xenopus Elongase (A), Ciona Elongase (B) und Oncorhynchus Elongase (C)

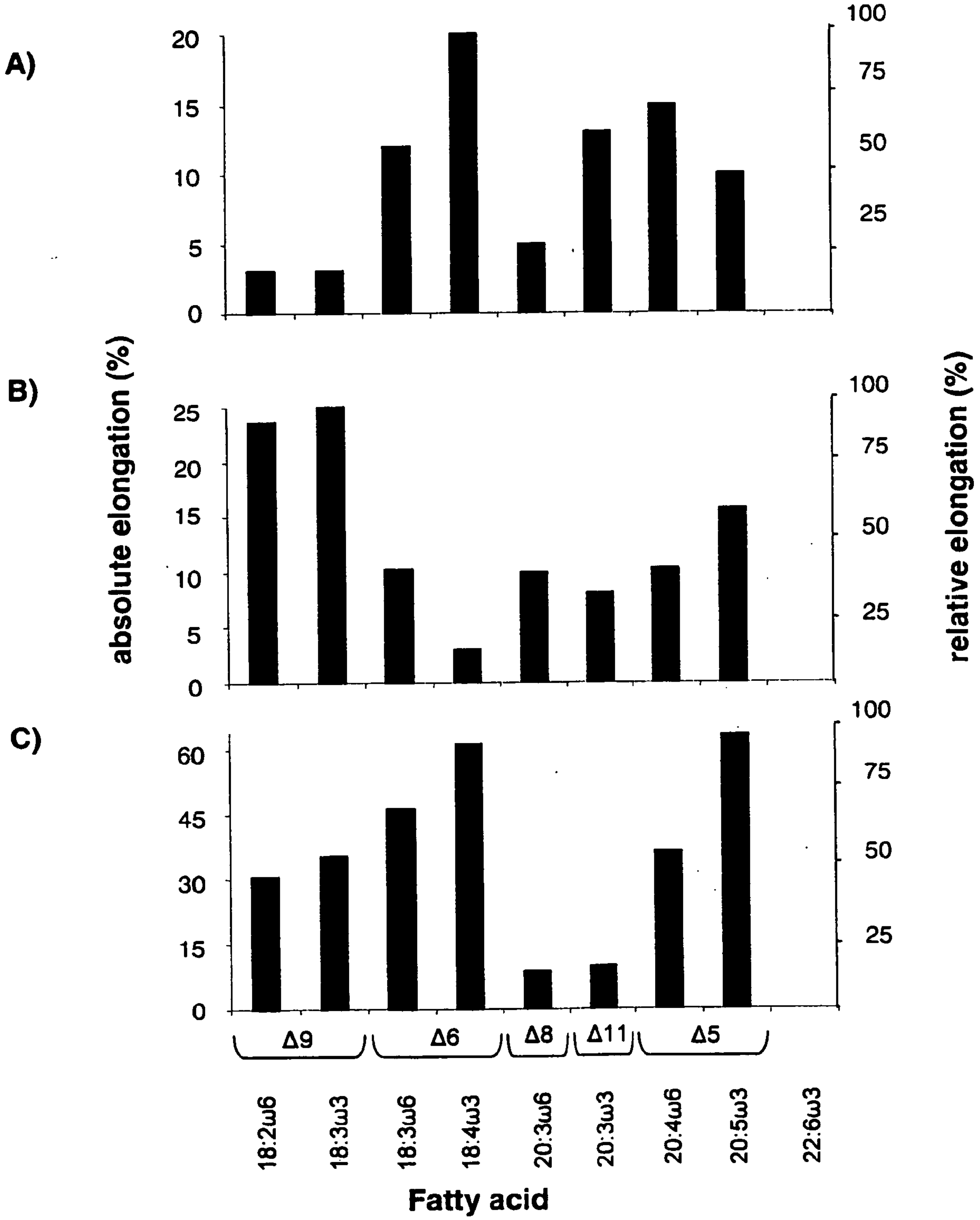

Figur 28: Substratspezifität der Ostreococcus Δ-5-Elongase (A), der Ostreococcus Δ-6-Elongase (B), der Thalassiosira Δ-5-Elongase (C) und Thalassiosira Ostreococcus Δ-6-Elongase (D)

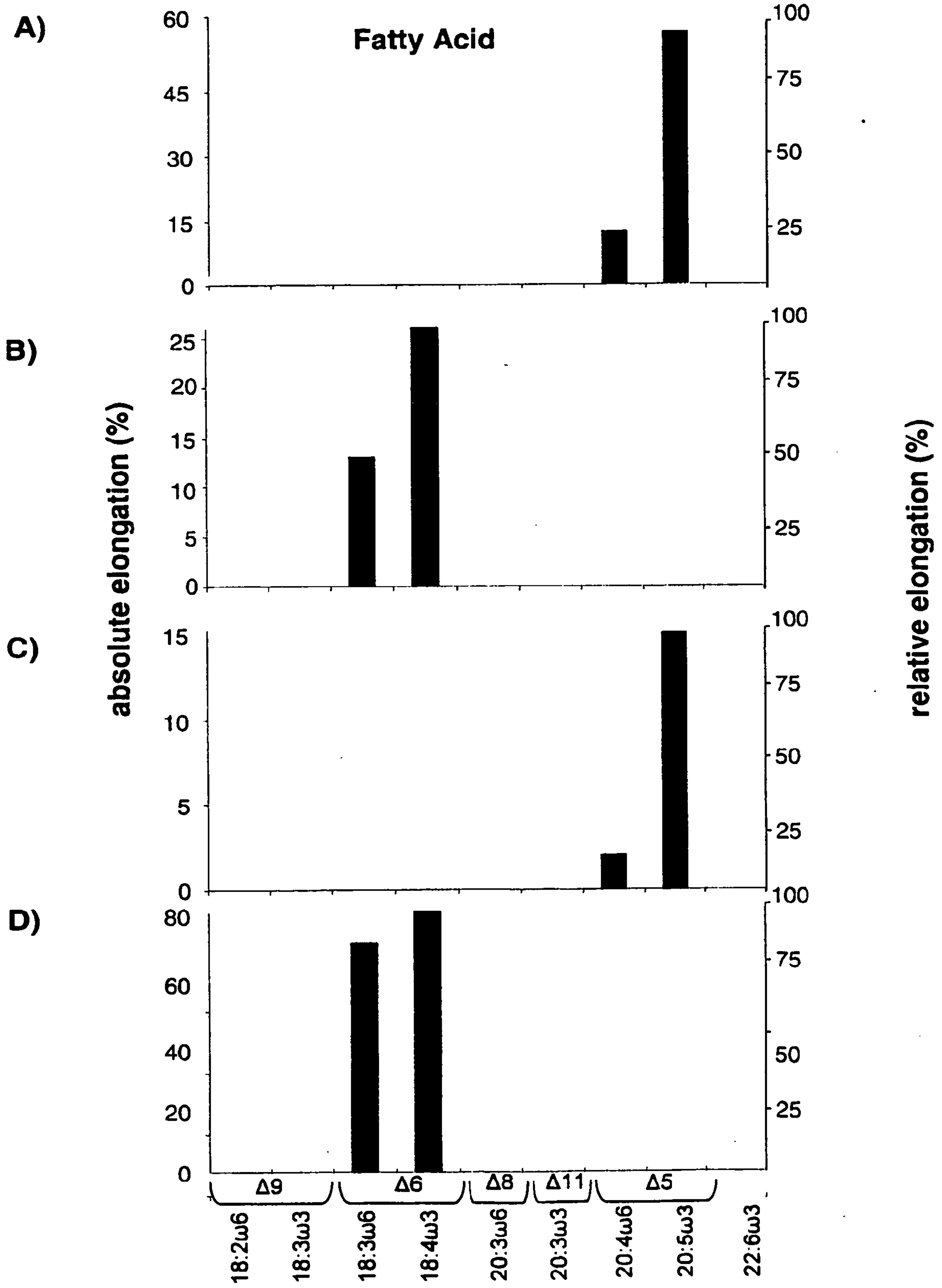

Figur 29: Expression der Phaeodactylum tricornutum Δ-6-Elongase (PtELO6) in Hefe. A) zeigt die Elongation der $C18:3^{\Delta6,9,12}$ Fettsäure und B) die Elongation der $C18:4^{\Delta6,9,12,15}$ Fettsäure

A)

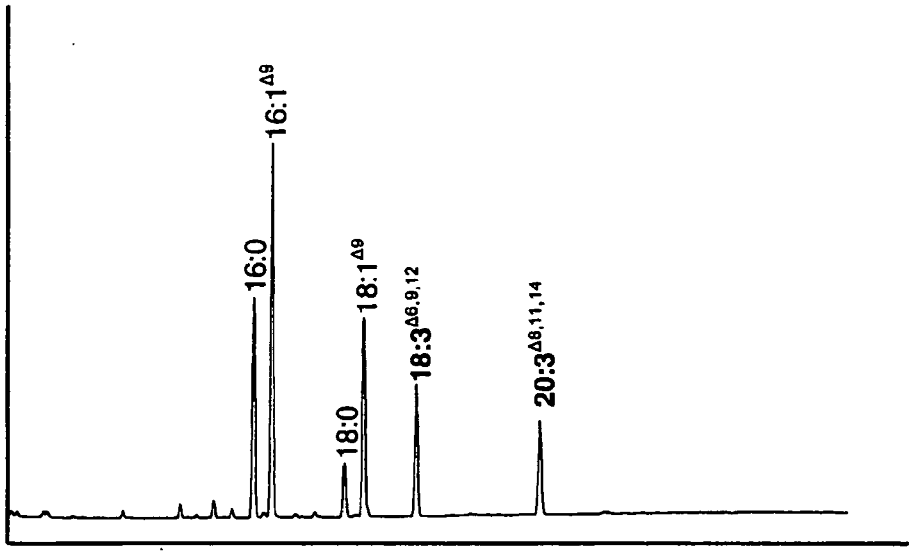


B)

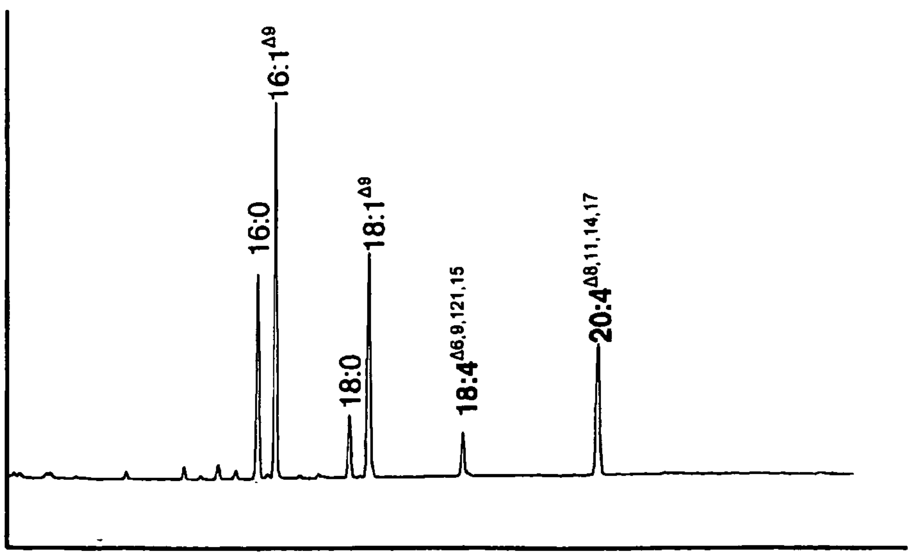

Figur 30: Figur 30 zeigt die Substratspezifität von PtELO6 in Bezug auf die gefütterten Substrate.

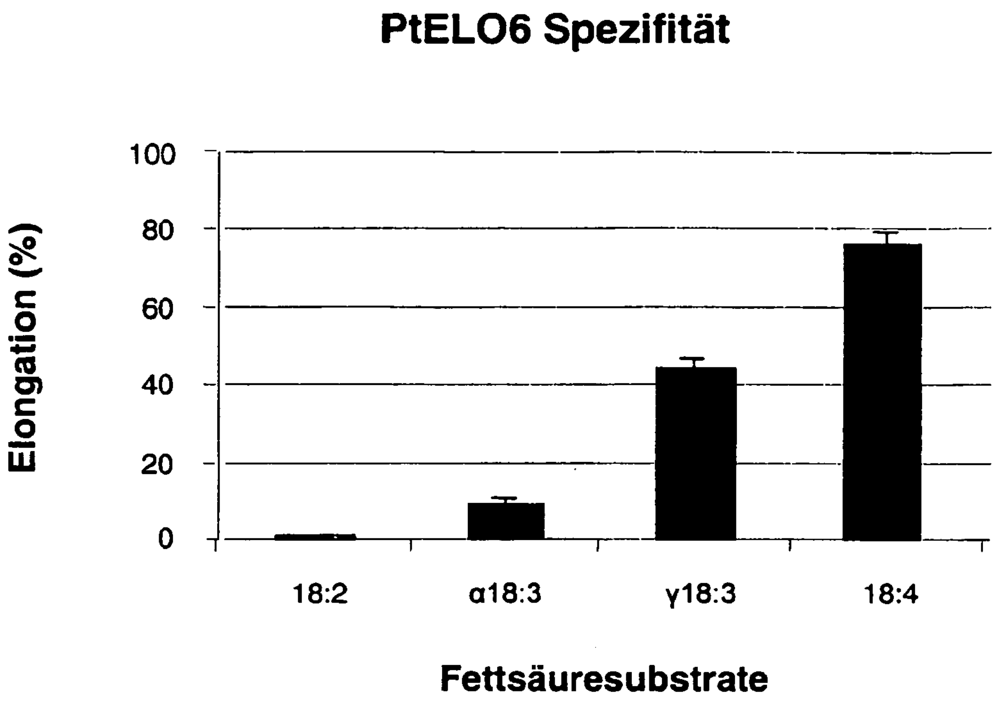

Europäisches Patentamt
European Patent Office
Office européen des brevets

# EUROPÄISCHER RECHERCHENBERICHT

Nummer der Anmeldung
EP 18 15 5821

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| A | WO 02/081668 A2 (ABBOTT LAB [US]) 17. Oktober 2002 (2002-10-17) * Seite 26, Zeile 29 - Seite 29, Zeile 3; Ansprüche 1-13; Sequenzen 3,4,33,34 * ----- | 1-14 | INV. C12N9/02 |
| A | SPERLING P ET AL: "The evolution of desaturases.", PROSTAGLANDINS LEUKOTRIENES AND ESSENTIAL FATTY ACIDS, Bd. 68, Nr. 2, Februar 2003 (2003-02), Seiten 73-95, XP002566213, ISSN: 0952-3278 * das ganze Dokument * ----- | 1-14 | |
| A,P | NAKAMURA MANABU T ET AL: "Structure, function, and dietary regulation of Delta 6, Delta 5, and Delta 9 desaturases", ANNUAL REVIEW OF NUTRITION, Bd. 24, 2004, Seiten 345-376, XP002566314, ISSN: 0199-9885 * Zusammenfassung * * Seite 349, Absatz 2 - Seite 358, Absatz 2; Abbildung 1 * ----- | 1-14 | RECHERCHIERTE SACHGEBIETE (IPC) C12N |
| T | WU G ET AL: "STEPWISE ENGINEERING TO PRODUCE HIGH YIELDS OF VERY LONG-CHAIN POLYUNSATURATED FATTY ACIDS IN PLANTS", NATURE BIOTECHNOLOGY, NATURE PUBLISHING GROUP, NEW YORK, NY, US, Bd. 23, Nr. 8, 1. August 2005 (2005-08-01), Seiten 1013-1017, XP007900180, ISSN: 1087-0156 * das ganze Dokument * ----- | 1-14 | |

-/--

Der vorliegende Recherchenbericht wurde für alle Patentansprüche erstellt

2

| Recherchenort | Abschlußdatum der Recherche | Prüfer |
|---|---|---|
| München | 17. September 2018 | Strobel, Andreas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Seite 1 von 2

Europäisches Patentamt
European Patent Office
Office européen des brevets

# EUROPÄISCHER RECHERCHENBERICHT

Nummer der Anmeldung
EP 18 15 5821

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | GUNSTONE F D: "Movements towards tailor-made fats", PROGRESS IN LIPID RESEARCH, Bd. 37, Nr. 5, November 1998 (1998-11), Seiten 277-305, XP002784802, ISSN: 0163-7827 * das ganze Dokument * ----- | 13,14 | |
| Y | THELEN JAY J ET AL: "Metabolic engineering of fatty acid biosynthesis in plants", METABOLIC ENGINEERING, ACADEMIC PRESS, US, Bd. 4, Nr. 1, 1. Januar 2002 (2002-01-01), Seiten 12-21, XP002257832, ISSN: 1096-7176, DOI: 10.1006/MBEN.2001.0204 * das ganze Dokument * ----- | 13,14 | |
| | | | RECHERCHIERTE SACHGEBIETE (IPC) |

Der vorliegende Recherchenbericht wurde für alle Patentansprüche erstellt

2

| Recherchenort | Abschlußdatum der Recherche | Prüfer |
|---|---|---|
| München | 17. September 2018 | Strobel, Andreas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................................
& : Mitglied der gleichen Patentfamilie, übereinstimmendes Dokument

EPO FORM 1503 03.82 (P04C03)

Seite 2 von 2

## ANHANG ZUM EUROPÄISCHEN RECHERCHENBERICHT ÜBER DIE EUROPÄISCHE PATENTANMELDUNG NR. EP 18 15 5821

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-09-2018

| Im Recherchenbericht angeführtes Patentdokument | Datum der Veröffentlichung | Mitglied(er) der Patentfamilie | Datum der Veröffentlichung |
|---|---|---|---|
| WO 02081668 A2 | 17-10-2002 | AU 2002309482 A2 | 21-10-2002 |
| | | BR 0205508 A | 03-05-2005 |
| | | CA 2435685 A1 | 17-10-2002 |
| | | EP 1392823 A2 | 03-03-2004 |
| | | JP 4347572 B2 | 21-10-2009 |
| | | JP 2005503770 A | 10-02-2005 |
| | | MX PA03006663 A | 31-05-2004 |
| | | WO 02081668 A2 | 17-10-2002 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9113972 A
- WO 9311245 A
- WO 9411516 A
- EP 0550162 A
- WO 9418337 A
- WO 9730582 A
- WO 9721340 A
- WO 9518222 A
- EP 0794250 A
- WO 9306712 A
- US 5614393 A
- WO 9621022 A
- WO 0021557 A
- WO 9927111 A
- WO 9846763 A
- WO 9846764 A
- WO 9846765 A
- WO 9964616 A
- WO 9846776 A
- WO 0159128 A
- WO 0012720 A
- WO 02077213 A
- WO 0208401 A
- WO 0244320 A
- DE 10219203
- US 5565350 A
- WO 0015815 A
- EP 0388186 A
- EP 0335528 A
- WO 9321334 A
- EP 0249676 A
- US 5608152 A
- WO 9845461 A
- US 5504200 A
- WO 9113980 A
- WO 9515389 A
- WO 9523230 A
- WO 9916890 A
- US 5315001 A
- WO 9218634 A
- WO 9320216 A
- US 5677474 A
- US 5530149 A
- EP 571741 A
- JP 6062870 A
- WO 9808962 A
- US 5689040 A
- EP 781849 A
- WO 9519443 A
- DE 10102337
- DE 10102338
- WO 9801572 A
- US 5352605 A
- WO 8402913 A
- US 4962028 A
- US 5187267 A
- WO 9612814 A
- EP 0375091 A
- WO 9516783 A
- WO 9706250 A
- WO 9946394 A


### In der Beschreibung aufgeführte Nicht-Patentliteratur


- **POULOS, A.** *Lipids,* 1995, vol. 30, 1-14
- **HORROCKS, LA ; YEO YK.** *Pharmacol Res,* 1999, vol. 40, 211-225
- **STUKEY et al.** *J. Biol. Chem.,* 1990, vol. 265, 20144-20149
- **WADA et al.** *Nature,* 1990, vol. 347, 200-203
- **HUANG et al.** *Lipids,* 1999, vol. 34, 649-659
- **MCKEON et al.** *Methods in Enzymol.,* 1981, vol. 71, 12141-12147
- **WANG et al.** *Plant Physiol. Biochem.,* 1988, vol. 26, 777-792
- **R. VAZHAPPILLY ; F. CHEN.** *Botanica Marina,* 1998, vol. 41, 553-558
- **K. TOTANI ; K. OBA.** *Lipids,* 1987, vol. 22, 1060-1062
- **M. AKIMOTO et al.** *Appl. Biochemistry and Biotechnology,* 1998, vol. 73, 269-278
- **YU, R. et al.** *Lipids,* 2000, vol. 35, 1061-1064
- **TAKEYAMA, H. et al.** *Microbiology,* 1997, vol. 143, 2725-2731
- **ZANK, T.K. et al.** *Plant Journal,* 2002, vol. 31, 255-268
- **SAKURADANI, E. et al.** *Gene,* 1999, vol. 238, 445-453
- **SPRECHER.** *Biochim. Biophys. Acta,* 2000, vol. 1486, 219-231

- **TOCHER et al.** *Prog. Lipid Res.,* 1998, vol. 37, 73-117
- **DOMERGUE et al.** *Eur. J. Biochem.,* 2002, vol. 269, 4105-4113
- **SHIMIKAWA.** *World Rev. Nutr. Diet.,* 2001, vol. 88, 100-108
- **CALDER.** *Proc. Nutr. Soc.,* 2002, vol. 61, 345-358
- **CLELAND ; JAMES.** *J. Rheumatol.,* 2000, vol. 27, 2305-2307
- **PEREIRA et al.** *Biochem. J.,* 2004, vol. 378, 665-71
- **MILLAR ; KUNST.** *Plant Journal,* 1997, vol. 12, 121-131
- **MILLAR et al.** *Plant Cell,* 1999, vol. 11, 825-838
- **TVRDIK et al.** *JCB,* 2000, vol. 149, 707-717
- Technique & Documentation. **E. UCCIANI ; LAVOISIER.** Nouveau Dictionnaire des Huiles Végétales. 1995
- **MIKOKLAJCZAK et al.** *Journal of the American Oil Chemical Society,* 1961, vol. 38, 678-681
- **GOEDDEL.** Gene Expression Technology: Methods in Enzymology. Academic Press, 1990, vol. 185
- **GOTTESMAN, S.** Gene Expression Technology: Methods in Enzymology. Academic Press, 1990, vol. 185, 119-128
- Bioprozeßtechnik 1. Einführung in die Bioverfahrenstechnik. **GUSTAV FISCHER.** Lehrbuch von Chmiel. Verlag, 1991
- Bioreaktoren und periphere Einrichtungen. Lehrbuch von Storhas. Vieweg Verlag, 1994
- Manual of Methods für General Bacteriology. Handbuch. merican Society für Bacteriology, 1981
- Applied Microbiol. Physiology, A Practical Approach. Lehrbuch. IRL Press, 1997, 53-73
- **HELLENS et al.** *Trends in Plant Science,* 2000, vol. 5, 446-451
- Plant Molecular Biology and Biotechnology. CRC Press, 1993, 71-119
- Vectors for Gene Transfer in Higher Plants; in: Transgenic Plants. **F.F. WHITE.** Engineering and Utilization. Academic Press, 1993, vol. 1, 15-38
- Techniques for Gene Transfer, in: Transgenic Plants. **B. JENES et al.** Engineering and Utilization. Academic Press, 1993, vol. 1, 128-143
- **POTRYKUS.** *Annu. Rev. Plant Physiol. Plant Molec. Biol.,* 1991, vol. 42, 205-225
- *J. Mol. Evolution.,* 1987, vol. 25, 351-360
- **HIGGINS et al.** *CABIOS,* 1989, vol. 5, 151-153
- **NEEDLEMAN ; WUNSCH.** *J. Mol. Biol.,* 1970, vol. 48, 443-453
- **SMITH ; WATERMAN.** *Adv. Appl. Math.,* 1981, vol. 2, 482-489
- **FRANCK et al.** *Cell,* 1980, vol. 21, 285-294
- **WARD et al.** *Plant. Mol. Biol.,* 1993, vol. 22
- *Plant J.,* 1992, vol. 2, 397-404
- **STOCKHAUS et al.** *EMBO J.,* 1989, vol. 8, 2445
- **BAEUMLEIN et al.** *Plant J.,* 1992, vol. 2 (2), 233-239
- **BÄUM-LEIN et al.** *Mol. Gen Genet.,* 1991, vol. 225 (3
- **BÄUMLEIN et al.** *Plant J.,* 1992, vol. 2, 2
- Übersicht in Gatz. *Annu. Rev. Plant Physiol. Plant Mol. Biol.,* 1997, vol. 48, 89-108
- **GATZ et al.** *Plant J.,* 1992, vol. 2, 397-404
- **GRUBER ; CROSBY.** Methods in Plant Molecular Biology and Biotechnolgy. CRC Press, 89-108
- **ROMANOS, M.A. et al.** Foreign gene expression in yeast: a review. *Yeast,* 1992, vol. 8, 423-488
- Heterologous gene expression in filamentous fungi. **VAN DEN HONDEL ; C.A.M.J.J. et al.** More Gene Manipulations in Fungi. Academic Press, 1991, 396-428
- Gene transfer systems and vector development for filamentous fungi. **VAN DEN HONDEL ; C.A.M.J.J. ; PUNT, P.J. et al.** Applied Molecular Genetics of Fungi. Cambridge University Press, 1991, 1-28
- **FALCIATORE et al.** *Marine Biotechnology,* 1999, vol. 1, 3, 239-251
- **SCHMIDT, R. ; WILLMITZER, L.** High efficiency Agrobacterium tumefaciens-mediated transformation of Arabidopsis thaliana leaf and cotyledon explants. *Plant Cell Rep.,* 1988, 583-586
- Plant Molecular Biology and Biotechnology. C Press, 1993, 71-119
- Techniques for Gene Transfer, in: Transgenic Plants. **F.F. WHITE ; B. JENES et al.** Engineering and Utilization. Academic Press, 1993, vol. 1, 128-43
- **SMITH, D.B. ; JOHNSON, K.S.** Gene. Pharmacia Biotech Inc, 1988, vol. 67, 31-40
- **AMANN et al.** *Gene,* 1988, vol. 69, 301-315
- **STUDIER et al.** Gene Expression Technology: Methods in Enzymology. Academic Press, 1990, vol. 185, 60-89
- **BALDARI et al.** *Embo J.,* 1987, vol. 6, 229-234
- **KURJAN ; HERSKOWITZ.** *Cell,* 1982, vol. 30, 933-943
- **SCHULTZ et al.** *Gene,* 1987, vol. 54, 113-123
- Gene transfer systems and vector development for filamentous fungi. **VAN DEN HONDEL ; C.A.M.J.J. ; PUNT, P.J. et al.** Applied Molecular Genetics of fungi. Cambridge University Press, 1991, 1-28
- More Gene Manipulations in Fungi. Academic Press, 396-428
- **SMITH et al.** *Mol. Cell Biol.,* 1983, vol. 3, 2156-2165

- **LUCKLOW ; SUMMERS.** *Virology,* 1989, vol. 170, 31-39
- Cloning Vectors. Elsevier, 1985
- **SAMBROOK, J. ; FRITSCH, E.F. ; MANIATIS, T.** Molecular Cloning: A Laboratory Manual. Cold Spring Harbor Laboratory Press, 1989
- **FALCIATORE et al.** *Marine Biotechnology,* 1999, vol. 1 (3), 239-251
- **BECKER, D. ; KEMPER, E. ; SCHELL, J. ; MASTERSON, R.** New plant binary vectors with selectable markers located proximal to the left border. *Plant Mol. Biol.,* 1992, vol. 20, 1195-1197
- **BEVAN, M.W.** Binary Agrobacterium vectors for plant transformation. *Nucl. Acids Res.,* 1984, vol. 12, 8711-8721
- Vectors for Gene Transfer in Higher Plants; in: Transgenic Plants. Engineering and Utilization. Academic Press, 1993, vol. 1, 15-38
- **GIELEN et al.** *EMBO J.,* 1984, vol. 3, 835ff
- **GALLIE et al.** *Nucl. Acids Research,* 1987, vol. 15, 8693-8711
- **BENFEY et al.** *EMBO J.,* 1989, vol. 8, 2195-2202
- **KERMODE.** *Crit. Rev. Plant Sci.,* 1996, vol. 15 (4), 285-423
- **WARD et al.** *Plant. Mol. Biol.,* 1993, vol. 22, 361-366
- **BAEUMLEIN et al.** *Mol Gen Genet,* 1991, vol. 225 (3), 459-67
- **BAEUMLEIN et al.** *Plant Journal,* 1992, vol. 2 (2), 233-9
- **SAMBROOK et al.** Molecular Cloning: A Laboratory Manual. Cold Spring Harbor Laboratory Press, 1989
- Agrobacterium protocols. Labor-Handbüchern, wie Methods in Molecular Biology. Humana Press, 1995, vol. 44
- **SAMBROOK et al.** Molecular Cloning: A Laboratory Manual. Cold Spring Harbor Laboratory Press, 1989
- **CHIRGWIN et al.** *Biochemistry,* 1979, vol. 18, 5294-5299
- **F.C. NEIDHARDT et al.** E. coli und Salmonella. ASM Press, 1996, 612-636
- Biology of Procaryotes. Thieme, 1999
- **MAGNUSON, K. et al.** Microbiological Reviews. 1993, vol. 57, 522-542
- **FRENTZEN.** *Lipid,* 1998, vol. 100 (4-5), 161-166
- **KINNEY.** Genetic Engeneering. 1997, vol. 19, 149-166
- **OHLROGGE ; BROWSE.** *Plant Cell,* 1995, vol. 7, 957-970
- **SHANKLIN ; CAHOON.** *Annu. Rev. Plant Physiol. Plant Mol. Biol.,* 1998, vol. 49, 611-641
- **VOELKER.** Genetic Engeneering. 1996, vol. 18, 111-13
- **GERHARDT.** *Prog. Lipid R.,* 1992, vol. 31, 397-417
- **GÜHNEMANN-SCHÄFER ; KINDL.** *Biochim. Biophys Acta,* 1995, vol. 1256, 181-186
- **KUNAU et al.** *Prog. Lipid Res.,* 1995, vol. 34, 267-342
- **STYMNE et al.** Biochemistry and Molecular Biology of Membrane and Storage Lipids of Plants. American Society of Plant Physiologists, 1993, 150-158
- **MURPHY ; ROSS.** *Plant Journal,* 1998, vol. 13 (1), 1-16
- Current Protocols in Molecular Biology. John Wiley & Sons, 1989, 6.3.1-6.3.6
- Molecular Cloning. **SAMBROOK et al.** Lehrbüchern. Cold Spring Harbor Laboratory, 1989
- Nucleic Acids Hybridization: A Practical Approach. IRL Press at Oxford University Press, 1985
- Essential Molecular Biology: A Practical Approach. IRL Press at Oxford University Press, 1991
- **SANGER et al.** *Proc. Natl. Acad. Sci. USA,* 1977, vol. 74, 5463-5467
- **HAJDUKIEWICZ,P ; SVAB, Z ; MALIGA, P.** The small versatile pPZP family of Agrobacterium binary vectors forplant transformation. *Plant Mol Biol,* 1994, vol. 25, 989-994
- **DE GREVE,H. ; DHAESE,P. ; SEURINCK,J. ; LEMMERS,M. ; VAN MONTAGU,M. ; SCHELL,J.** Nucleotide sequence and transcript map of the Agrobacterium tumefaciens Ti plasmid-encoded octopine synthase gene. *J. Mol. Appl. Genet.,* 1982, vol. 1 (6), 499-511
- **ULLMAN.** Encyclopedia of Industrial Chemistry. VCH, 1985, vol. A2, 89-90, 443-613
- **FALLON, A. et al.** Applications of HPLC in Biochemistry. *Laboratory Techniques in Biochemistry and Molecular Biology,* 1987, vol. 17
- Product recovery and purification. **REHM et al.** Biotechnology. VCH, 1993, vol. 3, 469-714
- **BELTER, P.A. et al.** Bioseparations: downstream processing for Biotechnology. John Wiley and Sons, 1988
- **KENNEDY, J.F. ; CABRAL, J.M.S.** Recovery processes for biological Materials. John Wiley and Sons, 1992
- Biochemical Separations. **SHAEIWITZ, J.A. ; HENRY, J.D.** Ullmann's Encyclopedia of Industrial Chemistry. VCH, 1988, vol. B3, 1-27
- **DECHOW, F.J.** Separation and purification techniques in biotechnology. Noyes Publications, 1989
- **CAHOON et al.** *Proc. Natl. Acad. Sci. USA,* 1999, vol. 96 (22), 12935-12940
- **BROWSE et al.** *Analytic Biochemistry,* 1986, vol. 152, 141-145
- **CHRISTIE, WILLIAM W.** Advances in Lipid Methodology. Oily Press (Oily Press Lipid Library

- Gas Chromatography and Lipids. **CHRISTIE, WILLIAM W.** A Practical Guide. Oily Press, 1989, vol. IX, 307
- Progress in Lipid Research. Pergamon Press, 1952, vol. 1, 16
- Applied Microbial Physiology. A Practical Approach. IRL Press, 103-129, 131-163, 165-192
- Advances on Lipid Methodology. Oily Press, 1997, 119-169
- Gaschromatographie-Massenspektrometrie-Verfahren. *Lipide,* 1998, vol. 33, 343-353
- **NAPIER ; MICHAELSON.** *Lipids,* 2001, vol. 36 (8), 761-766
- **SAYANOVA et al.** *Journal of Experimental Botany,* 2001, vol. 52 (360), 1581-1585
- **SPERLING et al.** *Arch. Biochem. Biophys.,* 2001, vol. 388 (2), 293-298
- **MICHAELSON et al.** *FEBS Letters,* 1998, vol. 439 (3), 215-218
- **DEBLAERE et al.** *Nucl. Acids. Res.,* 1984, vol. 13, 4777-4788
- **MURASHIGE ; SKOOG.** *Physiol. Plant.,* 1962, vol. 15, 473
- **BELL et al.** *In Vitro Cell. Dev. Biol.-Plant.,* 1999, vol. 35 (6), 456-465
- **MLYNAROVA et al.** *Plant Cell Report,* 1994, vol. 13, 282-285
- **ALTSCHUL.** *J. Mol. Biol.,* 1990, vol. 215, 403-410
- **KOZAK.** *Cell,* 1986, vol. 44, 283-292
- **HAJDUKIEWICZ,P ; SVAB, Z ; MALIGA, P.** The small versatile pPZP family of Agrobacterium binary vectors for plant transformation. *Plant Mol Biol,* 1994, vol. 25, 989-994
- Culture of phytoplankton for feeding marine invertebrates. **GUILLARD, R.R.L.** In Culture of Marine Invertebrate Animals. Plenum Press, 1975, 29-60
- **DE GREVE,H. ; DHAESE,P. ; SEURINCK,J. ; LEMMERS,M. ; VAN MONTAGU,M. ; SCHELL,J.** Nucleotide sequence -and transcript map of the Agrobacterium tumefaciens Ti plasmid-encoded octopine synthase gene. *J. Mol. Appl. Genet.,* 1982, vol. 1 (6), 499-511
- **HIS-BOXEN ; DOMERGUE et al.** *Eur. J. Biochem.,* 2002, vol. 269, 4105-4113
- **ALTSCHUL et al.** *J. Mol. Biol.,* 1990, vol. 215, 403-410
- **DE GREVE,H. ; DHAESE,P. ; SEURINCK,J. ; LEMMERS,M. ; VAN MONTAGU,M. ; SCHELL,J.** Nucleotide sequence and transcript map of the Agrobacterium tumefaciens Ti plasmid-encoded octopine synthase gene. *J. Mol. Appl. Genet.,* 1982, vol. 1 (6), 499-511
- **QIU et al.** *J. Biol. Chem.,* 2001, vol. 276, 31561-31566
- **HONG et al.** *Lipids,* 2002, vol. 37, 863-868
- **ZANK et al.** *Plant J.,* 2002, vol. 31, 255-268
- Genetic and genomic tools for Xenopus research: The NIH Xenopus initiative. *Dev. Dyn.,* 2002, vol. 225 (4), 384-391
- **SATOU,Y. ; YAMADA,L. ; MOCHIZUKI,Y. ; TAKATORI,N. ; KAWASHIMA,T. ; SASAKI,A. ; HAMAGU-CHI,M. ; AWAZU,S. ; YAGI,K. ; SASAKURA,Y.** A cDNA resource from the basal chordate Ciona intestinalis. *JOURNAL Genesis,* 2002, vol. 33 (4), 153-154
- **MICHAELSON et al.** *FEBS Letters.,* 1998, vol. 439 (3), 215-218
- **CALVAYRAC R ; DOUCE R.** *FEBS Letters,* 1970, vol. 7, 259-262

SEQUENCE LISTING

<110>  BASF Plant Science GmbH

<120>  Verfahren zur Herstellung mehrfach ungesättigter Fettsäuren in transgenen Organismen

<130>  54756EP12

<160>  58

<170>  PatentIn version 3.1

<210>  1
<211>  1266
<212>  DNA
<213>  Euglena gracilis

<220>
<221>  CDS
<222>  (1)..(1266)
<223>  Delta-8-Desaturase

<400>  1
```
atg aag tca aag cgc caa gcg ctt ccc ctt aca att gat gga aca aca      48
Met Lys Ser Lys Arg Gln Ala Leu Pro Leu Thr Ile Asp Gly Thr Thr
1               5                   10                  15

tat gat gtg tct gcc tgg gtc aat ttc cac cct ggt ggt gcg gaa att      96
Tyr Asp Val Ser Ala Trp Val Asn Phe His Pro Gly Gly Ala Glu Ile
            20                  25                  30

ata gag aat tac caa gga agg gat gcc act gat gcc ttc atg gtt atg     144
Ile Glu Asn Tyr Gln Gly Arg Asp Ala Thr Asp Ala Phe Met Val Met
        35                  40                  45

cac tct caa gaa gcc ttc gac aag ctc aag cgc atg ccc aaa atc aat     192
His Ser Gln Glu Ala Phe Asp Lys Leu Lys Arg Met Pro Lys Ile Asn
    50                  55                  60

ccc agt tct gag ttg cca ccc cag gct gca gtg aat gaa gct caa gag     240
Pro Ser Ser Glu Leu Pro Pro Gln Ala Ala Val Asn Glu Ala Gln Glu
65                  70                  75                  80

gat ttc cgg aag ctc cga gaa gag ttg atc gca act ggc atg ttt gat     288
Asp Phe Arg Lys Leu Arg Glu Glu Leu Ile Ala Thr Gly Met Phe Asp
                85                  90                  95

gcc tcc ccc ctc tgg tac tca tac aaa atc agc acc aca ctg ggc ctt     336
Ala Ser Pro Leu Trp Tyr Ser Tyr Lys Ile Ser Thr Thr Leu Gly Leu
            100                 105                 110

gga gtg ctg ggt tat ttc ctg atg gtt cag tat cag atg tat ttc att     384
Gly Val Leu Gly Tyr Phe Leu Met Val Gln Tyr Gln Met Tyr Phe Ile
        115                 120                 125

ggg gca gtg ttg ctt ggg atg cac tat caa cag atg ggc tgg ctt tct     432
Gly Ala Val Leu Leu Gly Met His Tyr Gln Gln Met Gly Trp Leu Ser
    130                 135                 140

cat gac att tgc cac cac cag act ttc aag aac cgg aac tgg aac aac     480
His Asp Ile Cys His His Gln Thr Phe Lys Asn Arg Asn Trp Asn Asn
```

```
145                 150                 155                 160

ctc gtg gga ctg gta ttt ggc aat ggt ctg caa ggt ttt tcc gtg aca      528
Leu Val Gly Leu Val Phe Gly Asn Gly Leu Gln Gly Phe Ser Val Thr
                165                 170                 175

tgc tgg aag gac aga cac aat gca cat cat tcg gca acc aat gtt caa      576
Cys Trp Lys Asp Arg His Asn Ala His His Ser Ala Thr Asn Val Gln
            180                 185                 190

ggg cac gac cct gat att gac aac ctc ccc ctc tta gcc tgg tct gag      624
Gly His Asp Pro Asp Ile Asp Asn Leu Pro Leu Leu Ala Trp Ser Glu
        195                 200                 205

gat gac gtc aca cgg gcg tca ccg att tcc cgc aag ctc att cag ttc      672
Asp Asp Val Thr Arg Ala Ser Pro Ile Ser Arg Lys Leu Ile Gln Phe
    210                 215                 220

cag cag tat tat ttc ttg gtc atc tgt atc ttg ttg cgg ttc att tgg      720
Gln Gln Tyr Tyr Phe Leu Val Ile Cys Ile Leu Leu Arg Phe Ile Trp
225                 230                 235                 240

tgt ttc cag agc gtg ttg acc gtg cgc agt ctg aag gac aga gat aac      768
Cys Phe Gln Ser Val Leu Thr Val Arg Ser Leu Lys Asp Arg Asp Asn
                245                 250                 255

caa ttc tat cgc tct cag tat aag aag gag gcc att ggc ctc gcc ctg      816
Gln Phe Tyr Arg Ser Gln Tyr Lys Lys Glu Ala Ile Gly Leu Ala Leu
            260                 265                 270

cat tgg aca ttg aag gcc ctg ttc cac tta ttc ttt atg ccc agc atc      864
His Trp Thr Leu Lys Ala Leu Phe His Leu Phe Phe Met Pro Ser Ile
        275                 280                 285

ctc aca tcg ctg ttg gta ttt ttc gtt tcg gag ctg gtt ggc ggc ttc      912
Leu Thr Ser Leu Leu Val Phe Phe Val Ser Glu Leu Val Gly Gly Phe
    290                 295                 300

ggc att gcg atc gtg gtg ttc atg aac cac tac cca ctg gag aag atc      960
Gly Ile Ala Ile Val Val Phe Met Asn His Tyr Pro Leu Glu Lys Ile
305                 310                 315                 320

ggg gac tcg gtc tgg gat ggc cat gga ttc tcg gtt ggc cag atc cat     1008
Gly Asp Ser Val Trp Asp Gly His Gly Phe Ser Val Gly Gln Ile His
                325                 330                 335

gag acc atg aac att cgg cga ggg att atc aca gat tgg ttt ttc gga     1056
Glu Thr Met Asn Ile Arg Arg Gly Ile Ile Thr Asp Trp Phe Phe Gly
            340                 345                 350

ggc ttg aac tac cag atc gag cac cat ttg tgg ccg acc ctc cct cgc     1104
Gly Leu Asn Tyr Gln Ile Glu His His Leu Trp Pro Thr Leu Pro Arg
        355                 360                 365

cac aac ctg aca gcg gtt agc tac cag gtg gaa cag ctg tgc cag aag     1152
His Asn Leu Thr Ala Val Ser Tyr Gln Val Glu Gln Leu Cys Gln Lys
    370                 375                 380

cac aac ctg ccg tat cgg aac ccg ctg ccc cat gaa ggg ttg gtc atc     1200
His Asn Leu Pro Tyr Arg Asn Pro Leu Pro His Glu Gly Leu Val Ile
385                 390                 395                 400

ctg ctg cgc tat ctg gcg gtg ttc gcc cgg atg gcg gag aag caa ccc     1248
```

```
Leu Leu Arg Tyr Leu Ala Val Phe Ala Arg Met Ala Glu Lys Gln Pro
                405                 410                 415

gcg ggg aag gct cta taa                                             1266
Ala Gly Lys Ala Leu
            420


<210>  2
<211>  421
<212>  PRT
<213>  Euglena gracilis

<400>  2

Met Lys Ser Lys Arg Gln Ala Leu Pro Leu Thr Ile Asp Gly Thr Thr
1               5                   10                  15


Tyr Asp Val Ser Ala Trp Val Asn Phe His Pro Gly Gly Ala Glu Ile
            20                  25                  30


Ile Glu Asn Tyr Gln Gly Arg Asp Ala Thr Asp Ala Phe Met Val Met
        35                  40                  45


His Ser Gln Glu Ala Phe Asp Lys Leu Lys Arg Met Pro Lys Ile Asn
    50                  55                  60


Pro Ser Ser Glu Leu Pro Pro Gln Ala Ala Val Asn Glu Ala Gln Glu
65                  70                  75                  80


Asp Phe Arg Lys Leu Arg Glu Glu Leu Ile Ala Thr Gly Met Phe Asp
                85                  90                  95


Ala Ser Pro Leu Trp Tyr Ser Tyr Lys Ile Ser Thr Thr Leu Gly Leu
            100                 105                 110


Gly Val Leu Gly Tyr Phe Leu Met Val Gln Tyr Gln Met Tyr Phe Ile
        115                 120                 125


Gly Ala Val Leu Leu Gly Met His Tyr Gln Gln Met Gly Trp Leu Ser
    130                 135                 140


His Asp Ile Cys His His Gln Thr Phe Lys Asn Arg Asn Trp Asn Asn
145                 150                 155                 160


Leu Val Gly Leu Val Phe Gly Asn Gly Leu Gln Gly Phe Ser Val Thr
                165                 170                 175


Cys Trp Lys Asp Arg His Asn Ala His His Ser Ala Thr Asn Val Gln
            180                 185                 190
```

```
Gly His Asp Pro Asp Ile Asp Asn Leu Pro Leu Leu Ala Trp Ser Glu
        195                 200                 205

Asp Asp Val Thr Arg Ala Ser Pro Ile Ser Arg Lys Leu Ile Gln Phe
    210                 215                 220

Gln Gln Tyr Tyr Phe Leu Val Ile Cys Ile Leu Leu Arg Phe Ile Trp
225                 230                 235                 240

Cys Phe Gln Ser Val Leu Thr Val Arg Ser Leu Lys Asp Arg Asp Asn
                245                 250                 255

Gln Phe Tyr Arg Ser Gln Tyr Lys Lys Glu Ala Ile Gly Leu Ala Leu
            260                 265                 270

His Trp Thr Leu Lys Ala Leu Phe His Leu Phe Phe Met Pro Ser Ile
        275                 280                 285

Leu Thr Ser Leu Leu Val Phe Phe Val Ser Glu Leu Val Gly Gly Phe
    290                 295                 300

Gly Ile Ala Ile Val Val Phe Met Asn His Tyr Pro Leu Glu Lys Ile
305                 310                 315                 320

Gly Asp Ser Val Trp Asp Gly His Gly Phe Ser Val Gly Gln Ile His
                325                 330                 335

Glu Thr Met Asn Ile Arg Arg Gly Ile Ile Thr Asp Trp Phe Phe Gly
            340                 345                 350

Gly Leu Asn Tyr Gln Ile Glu His His Leu Trp Pro Thr Leu Pro Arg
        355                 360                 365

His Asn Leu Thr Ala Val Ser Tyr Gln Val Glu Gln Leu Cys Gln Lys
    370                 375                 380

His Asn Leu Pro Tyr Arg Asn Pro Leu Pro His Glu Gly Leu Val Ile
385                 390                 395                 400

Leu Leu Arg Tyr Leu Ala Val Phe Ala Arg Met Ala Glu Lys Gln Pro
                405                 410                 415

Ala Gly Lys Ala Leu
            420
```

<210> 3
<211> 777
<212> DNA

<213> Isochrysis galbana

<220>
<221> CDS
<222> (1)..(777)
<223> Delta-9-Elongase

<400> 3
atg gcc ctc gca aac gac gcg gga gag cgc atc tgg gcg gct gtg acc 48
Met Ala Leu Ala Asn Asp Ala Gly Glu Arg Ile Trp Ala Ala Val Thr
1 5 10 15

gac ccg gaa atc ctc att ggc acc ttc tcg tac ttg cta ctc aaa ccg 96
Asp Pro Glu Ile Leu Ile Gly Thr Phe Ser Tyr Leu Leu Leu Lys Pro
20 25 30

ctg ctc cgc aat tcc ggg ctg gtg gat gag aag aag ggc gca tac agg 144
Leu Leu Arg Asn Ser Gly Leu Val Asp Glu Lys Lys Gly Ala Tyr Arg
35 40 45

acg tcc atg atc tgg tac aac gtt ctg ctg gcg ctc ttc tct gcg ctg 192
Thr Ser Met Ile Trp Tyr Asn Val Leu Leu Ala Leu Phe Ser Ala Leu
50 55 60

agc ttc tac gtg acg gcg acc gcc ctc ggc tgg gac tat ggt acg ggc 240
Ser Phe Tyr Val Thr Ala Thr Ala Leu Gly Trp Asp Tyr Gly Thr Gly
65 70 75 80

gcg tgg ctg cgc agg caa acc ggc gac aca ccg cag ccg ctc ttc cag 288
Ala Trp Leu Arg Arg Gln Thr Gly Asp Thr Pro Gln Pro Leu Phe Gln
85 90 95

tgc ccg tcc ccg gtt tgg gac tcg aag ctc ttc aca tgg acc gcc aag 336
Cys Pro Ser Pro Val Trp Asp Ser Lys Leu Phe Thr Trp Thr Ala Lys
100 105 110

gca ttc tat tac tcc aag tac gtg gag tac ctc gac acg gcc tgg ctg 384
Ala Phe Tyr Tyr Ser Lys Tyr Val Glu Tyr Leu Asp Thr Ala Trp Leu
115 120 125

agg gtc tcc ttt ctc cag gcc ttc cac cac ttt ggc gcg ccg tgg gat 432
Arg Val Ser Phe Leu Gln Ala Phe His His Phe Gly Ala Pro Trp Asp
130 135 140

gtg tac ctc ggc att cgg ctg cac aac gag ggc gta tgg atc ttc atg 480
Val Tyr Leu Gly Ile Arg Leu His Asn Glu Gly Val Trp Ile Phe Met
145 150 155 160

ttt ttc aac tcg ttc att cac acc atc atg tac acc tac tac ggc ctc 528
Phe Phe Asn Ser Phe Ile His Thr Ile Met Tyr Thr Tyr Tyr Gly Leu
165 170 175

acc gcc gcc ggg tat aag ttc aag gcc aag ccg ctc atc acc gcg atg 576
Thr Ala Ala Gly Tyr Lys Phe Lys Ala Lys Pro Leu Ile Thr Ala Met
180 185 190

cag atc tgc cag ttc gtg ggc ggc ttc ctg ttg gtc tgg gac tac atc 624
Gln Ile Cys Gln Phe Val Gly Gly Phe Leu Leu Val Trp Asp Tyr Ile
195 200 205

aac gtc ccc tgc ttc aac tcg gac aaa ggg aag ttg ttc agc tgg gct 672
Asn Val Pro Cys Phe Asn Ser Asp Lys Gly Lys Leu Phe Ser Trp Ala

```
    210                 215                 220

ttc aac tat gca tac gtc ggc tcg gtc ttc ttg ctc ttc tgc cac ttt       720
Phe Asn Tyr Ala Tyr Val Gly Ser Val Phe Leu Leu Phe Cys His Phe
225                 230                 235                 240

ttc tac cag gac aac ttg gca acg aag aaa tcg gcc aag gcg ggc aag       768
Phe Tyr Gln Asp Asn Leu Ala Thr Lys Lys Ser Ala Lys Ala Gly Lys
                245                 250                 255

cag ctc tag                                                           777
Gln Leu



<210>  4
<211>  258
<212>  PRT
<213>  Isochrysis galbana

<400>  4

Met Ala Leu Ala Asn Asp Ala Gly Glu Arg Ile Trp Ala Ala Val Thr
1               5                   10                  15


Asp Pro Glu Ile Leu Ile Gly Thr Phe Ser Tyr Leu Leu Leu Lys Pro
            20                  25                  30


Leu Leu Arg Asn Ser Gly Leu Val Asp Glu Lys Lys Gly Ala Tyr Arg
        35                  40                  45


Thr Ser Met Ile Trp Tyr Asn Val Leu Leu Ala Leu Phe Ser Ala Leu
    50                  55                  60


Ser Phe Tyr Val Thr Ala Thr Ala Leu Gly Trp Asp Tyr Gly Thr Gly
65                  70                  75                  80


Ala Trp Leu Arg Arg Gln Thr Gly Asp Thr Pro Gln Pro Leu Phe Gln
                85                  90                  95


Cys Pro Ser Pro Val Trp Asp Ser Lys Leu Phe Thr Trp Thr Ala Lys
            100                 105                 110


Ala Phe Tyr Tyr Ser Lys Tyr Val Glu Tyr Leu Asp Thr Ala Trp Leu
        115                 120                 125


Arg Val Ser Phe Leu Gln Ala Phe His His Phe Gly Ala Pro Trp Asp
    130                 135                 140


Val Tyr Leu Gly Ile Arg Leu His Asn Glu Gly Val Trp Ile Phe Met
145                 150                 155                 160


Phe Phe Asn Ser Phe Ile His Thr Ile Met Tyr Thr Tyr Tyr Gly Leu
```

```
                165                 170                 175

Thr Ala Ala Gly Tyr Lys Phe Lys Ala Lys Pro Leu Ile Thr Ala Met
            180                 185                 190

Gln Ile Cys Gln Phe Val Gly Gly Phe Leu Leu Val Trp Asp Tyr Ile
        195                 200                 205

Asn Val Pro Cys Phe Asn Ser Asp Lys Gly Lys Leu Phe Ser Trp Ala
    210                 215                 220

Phe Asn Tyr Ala Tyr Val Gly Ser Val Phe Leu Leu Phe Cys His Phe
225                 230                 235                 240

Phe Tyr Gln Asp Asn Leu Ala Thr Lys Lys Ser Ala Lys Ala Gly Lys
                245                 250                 255

Gln Leu



<210>  5
<211>  1410
<212>  DNA
<213>  Phaeodactylum tricornutum

<220>
<221>  CDS
<222>  (1)..(1410)
<223>  Delta-5-Desaturase


<400>  5
atg gct ccg gat gcg gat aag ctt cga caa cgc cag acg act gcg gta        48
Met Ala Pro Asp Ala Asp Lys Leu Arg Gln Arg Gln Thr Thr Ala Val
1               5                   10                  15

gcg aag cac aat gct gct acc ata tcg acg cag gaa cgc ctt tgc agt        96
Ala Lys His Asn Ala Ala Thr Ile Ser Thr Gln Glu Arg Leu Cys Ser
            20                  25                  30

ctg tct tcg ctc aaa ggc gaa gaa gtc tgc atc gac gga atc atc tat       144
Leu Ser Ser Leu Lys Gly Glu Glu Val Cys Ile Asp Gly Ile Ile Tyr
        35                  40                  45

gac ctc caa tca ttc gat cat ccc ggg ggt gaa acg atc aaa atg ttt       192
Asp Leu Gln Ser Phe Asp His Pro Gly Gly Glu Thr Ile Lys Met Phe
    50                  55                  60

ggt ggc aac gat gtc act gta cag tac aag atg att cac ccg tac cat       240
Gly Gly Asn Asp Val Thr Val Gln Tyr Lys Met Ile His Pro Tyr His
65                  70                  75                  80

acc gag aag cat ttg gaa aag atg aag cgt gtc ggc aag gtg acg gat       288
Thr Glu Lys His Leu Glu Lys Met Lys Arg Val Gly Lys Val Thr Asp
                85                  90                  95
```

```
ttc gtc tgc gag tac aag ttc gat acc gaa ttt gaa cgc gaa atc aaa      336
Phe Val Cys Glu Tyr Lys Phe Asp Thr Glu Phe Glu Arg Glu Ile Lys
            100                 105                 110

cga gaa gtc ttc aag att gtg cga cga ggc aag gat ttc ggt act ttg      384
Arg Glu Val Phe Lys Ile Val Arg Arg Gly Lys Asp Phe Gly Thr Leu
        115                 120                 125

gga tgg ttc ttc cgt gcg ttt tgc tac att gcc att ttc ttc tac ctg      432
Gly Trp Phe Phe Arg Ala Phe Cys Tyr Ile Ala Ile Phe Phe Tyr Leu
    130                 135                 140

cag tac cat tgg gtc acc acg gga acc tct tgg ctg ctg gcc gtg gcc      480
Gln Tyr His Trp Val Thr Thr Gly Thr Ser Trp Leu Leu Ala Val Ala
145                 150                 155                 160

tac gga atc tcc caa gcg atg att ggc atg aat gtc cag cac gat gcc      528
Tyr Gly Ile Ser Gln Ala Met Ile Gly Met Asn Val Gln His Asp Ala
                165                 170                 175

aac cac ggg gcc acc tcc aag cgt ccc tgg gtc aac gac atg cta ggc      576
Asn His Gly Ala Thr Ser Lys Arg Pro Trp Val Asn Asp Met Leu Gly
            180                 185                 190

ctc ggt gcg gat ttt att ggt ggt tcc aag tgg ctc tgg cag gaa caa      624
Leu Gly Ala Asp Phe Ile Gly Gly Ser Lys Trp Leu Trp Gln Glu Gln
        195                 200                 205

cac tgg acc cac cac gct tac acc aat cac gcc gag atg gat ccc gat      672
His Trp Thr His His Ala Tyr Thr Asn His Ala Glu Met Asp Pro Asp
    210                 215                 220

agc ttt ggt gcc gaa cca atg ctc cta ttc aac gac tat ccc ttg gat      720
Ser Phe Gly Ala Glu Pro Met Leu Leu Phe Asn Asp Tyr Pro Leu Asp
225                 230                 235                 240

cat ccc gct cgt acc tgg cta cat cgc ttt caa gca ttc ttt tac atg      768
His Pro Ala Arg Thr Trp Leu His Arg Phe Gln Ala Phe Phe Tyr Met
                245                 250                 255

ccc gtc ttg gct gga tac tgg ttg tcc gct gtc ttc aat cca caa att      816
Pro Val Leu Ala Gly Tyr Trp Leu Ser Ala Val Phe Asn Pro Gln Ile
            260                 265                 270

ctt gac ctc cag caa cgc ggc gca ctt tcc gtc ggt atc cgt ctc gac      864
Leu Asp Leu Gln Gln Arg Gly Ala Leu Ser Val Gly Ile Arg Leu Asp
        275                 280                 285

aac gct ttc att cac tcg cga cgc aag tat gcg gtt ttc tgg cgg gct      912
Asn Ala Phe Ile His Ser Arg Arg Lys Tyr Ala Val Phe Trp Arg Ala
    290                 295                 300

gtg tac att gcg gtg aac gtg att gct ccg ttt tac aca aac tcc ggc      960
Val Tyr Ile Ala Val Asn Val Ile Ala Pro Phe Tyr Thr Asn Ser Gly
305                 310                 315                 320

ctc gaa tgg tcc tgg cgt gtc ttt gga aac atc atg ctc atg ggt gtg     1008
Leu Glu Trp Ser Trp Arg Val Phe Gly Asn Ile Met Leu Met Gly Val
                325                 330                 335

gcg gaa tcg ctc gcg ctg gcg gtc ctg ttt tcg ttg tcg cac aat ttc     1056
Ala Glu Ser Leu Ala Leu Ala Val Leu Phe Ser Leu Ser His Asn Phe
            340                 345                 350
```

gaa tcc gcg gat cgc gat ccg acc gcc cca ctg aaa aag acg gga gaa 1104
Glu Ser Ala Asp Arg Asp Pro Thr Ala Pro Leu Lys Lys Thr Gly Glu
355 360 365

cca gtc gac tgg ttc aag aca cag gtc gaa act tcc tgc act tac ggt 1152
Pro Val Asp Trp Phe Lys Thr Gln Val Glu Thr Ser Cys Thr Tyr Gly
370 375 380

gga ttc ctt tcc ggt tgc ttc acg gga ggt ctc aac ttt cag gtt gaa 1200
Gly Phe Leu Ser Gly Cys Phe Thr Gly Gly Leu Asn Phe Gln Val Glu
385 390 395 400

cac cac ttg ttc cca cgc atg agc agc gct tgg tat ccc tac att gcc 1248
His His Leu Phe Pro Arg Met Ser Ser Ala Trp Tyr Pro Tyr Ile Ala
405 410 415

ccc aag gtc cgc gaa att tgc gcc aaa cac ggc gtc cac tac gcc tac 1296
Pro Lys Val Arg Glu Ile Cys Ala Lys His Gly Val His Tyr Ala Tyr
420 425 430

tac ccg tgg atc cac caa aac ttt ctc tcc acc gtc cgc tac atg cac 1344
Tyr Pro Trp Ile His Gln Asn Phe Leu Ser Thr Val Arg Tyr Met His
435 440 445

gcg gcc ggg acc ggt gcc aac tgg cgc cag atg gcc aga gaa aat ccc 1392
Ala Ala Gly Thr Gly Ala Asn Trp Arg Gln Met Ala Arg Glu Asn Pro
450 455 460

ttg acc gga cgg gcg taa 1410
Leu Thr Gly Arg Ala
465

<210> 6
<211> 469
<212> PRT
<213> Phaeodactylum tricornutum

<400> 6

Met Ala Pro Asp Ala Asp Lys Leu Arg Gln Arg Gln Thr Thr Ala Val
1 5 10 15

Ala Lys His Asn Ala Ala Thr Ile Ser Thr Gln Glu Arg Leu Cys Ser
20 25 30

Leu Ser Ser Leu Lys Gly Glu Glu Val Cys Ile Asp Gly Ile Ile Tyr
35 40 45

Asp Leu Gln Ser Phe Asp His Pro Gly Gly Glu Thr Ile Lys Met Phe
50 55 60

Gly Gly Asn Asp Val Thr Val Gln Tyr Lys Met Ile His Pro Tyr His
65 70 75 80

Thr Glu Lys His Leu Glu Lys Met Lys Arg Val Gly Lys Val Thr Asp
85 90 95

Phe Val Cys Glu Tyr Lys Phe Asp Thr Glu Phe Glu Arg Glu Ile Lys
100 105 110

Arg Glu Val Phe Lys Ile Val Arg Arg Gly Lys Asp Phe Gly Thr Leu
115 120 125

Gly Trp Phe Phe Arg Ala Phe Cys Tyr Ile Ala Ile Phe Phe Tyr Leu
130 135 140

Gln Tyr His Trp Val Thr Thr Gly Thr Ser Trp Leu Leu Ala Val Ala
145 150 155 160

Tyr Gly Ile Ser Gln Ala Met Ile Gly Met Asn Val Gln His Asp Ala
165 170 175

Asn His Gly Ala Thr Ser Lys Arg Pro Trp Val Asn Asp Met Leu Gly
180 185 190

Leu Gly Ala Asp Phe Ile Gly Gly Ser Lys Trp Leu Trp Gln Glu Gln
195 200 205

His Trp Thr His His Ala Tyr Thr Asn His Ala Glu Met Asp Pro Asp
210 215 220

Ser Phe Gly Ala Glu Pro Met Leu Leu Phe Asn Asp Tyr Pro Leu Asp
225 230 235 240

His Pro Ala Arg Thr Trp Leu His Arg Phe Gln Ala Phe Phe Tyr Met
245 250 255

Pro Val Leu Ala Gly Tyr Trp Leu Ser Ala Val Phe Asn Pro Gln Ile
260 265 270

Leu Asp Leu Gln Gln Arg Gly Ala Leu Ser Val Gly Ile Arg Leu Asp
275 280 285

Asn Ala Phe Ile His Ser Arg Arg Lys Tyr Ala Val Phe Trp Arg Ala
290 295 300

Val Tyr Ile Ala Val Asn Val Ile Ala Pro Phe Tyr Thr Asn Ser Gly
305 310 315 320

Leu Glu Trp Ser Trp Arg Val Phe Gly Asn Ile Met Leu Met Gly Val
325 330 335

Ala Glu Ser Leu Ala Leu Ala Val Leu Phe Ser Leu Ser His Asn Phe

340 345 350

Glu Ser Ala Asp Arg Asp Pro Thr Ala Pro Leu Lys Lys Thr Gly Glu
355 360 365

Pro Val Asp Trp Phe Lys Thr Gln Val Glu Thr Ser Cys Thr Tyr Gly
370 375 380

Gly Phe Leu Ser Gly Cys Phe Thr Gly Gly Leu Asn Phe Gln Val Glu
385 390 395 400

His His Leu Phe Pro Arg Met Ser Ser Ala Trp Tyr Pro Tyr Ile Ala
405 410 415

Pro Lys Val Arg Glu Ile Cys Ala Lys His Gly Val His Tyr Ala Tyr
420 425 430

Tyr Pro Trp Ile His Gln Asn Phe Leu Ser Thr Val Arg Tyr Met His
435 440 445

Ala Ala Gly Thr Gly Ala Asn Trp Arg Gln Met Ala Arg Glu Asn Pro
450 455 460

Leu Thr Gly Arg Ala
465

<210> 7
<211> 1344
<212> DNA
<213> Ceratodon purpureus

<220>
<221> CDS
<222> (1)..(1344)
<223> Delta-5-Desaturase

<400> 7
atg gta tta cga gag caa gag cat gag cca ttc ttc att aaa att gat 48
Met Val Leu Arg Glu Gln Glu His Glu Pro Phe Phe Ile Lys Ile Asp
1 5 10 15

gga aaa tgg tgt caa att gac gat gct gtc ctg aga tca cat cca ggt 96
Gly Lys Trp Cys Gln Ile Asp Asp Ala Val Leu Arg Ser His Pro Gly
20 25 30

ggt agt gca att act acc tat aaa aat atg gat gcc act acc gta ttc 144
Gly Ser Ala Ile Thr Thr Tyr Lys Asn Met Asp Ala Thr Thr Val Phe
35 40 45

cac aca ttc cat act ggt tct aaa gaa gcg tat caa tgg ctg aca gaa 192
His Thr Phe His Thr Gly Ser Lys Glu Ala Tyr Gln Trp Leu Thr Glu
50 55 60

```
ttg aaa aaa gag tgc cct aca caa gaa cca gag atc cca gat att aag      240
Leu Lys Lys Glu Cys Pro Thr Gln Glu Pro Glu Ile Pro Asp Ile Lys
65                  70                  75                  80

gat gac cca atc aaa gga att gat gat gtg aac atg gga act ttc aat      288
Asp Asp Pro Ile Lys Gly Ile Asp Asp Val Asn Met Gly Thr Phe Asn
                85                  90                  95

att tct gag aaa cga tct gcc caa ata aat aaa agt ttc act gat cta      336
Ile Ser Glu Lys Arg Ser Ala Gln Ile Asn Lys Ser Phe Thr Asp Leu
            100                 105                 110

cgt atg cga gtt cgt gca gaa gga ctt atg gat gga tct cct ttg ttc      384
Arg Met Arg Val Arg Ala Glu Gly Leu Met Asp Gly Ser Pro Leu Phe
        115                 120                 125

tac att aga aaa att ctt gaa aca atc ttc aca att ctt ttt gca ttc      432
Tyr Ile Arg Lys Ile Leu Glu Thr Ile Phe Thr Ile Leu Phe Ala Phe
    130                 135                 140

tac ctt caa tac cac aca tat tat ctt cca tca gct att cta atg gga      480
Tyr Leu Gln Tyr His Thr Tyr Tyr Leu Pro Ser Ala Ile Leu Met Gly
145                 150                 155                 160

gtt gcg tgg caa caa ttg gga tgg tta atc cat gaa ttc gca cat cat      528
Val Ala Trp Gln Gln Leu Gly Trp Leu Ile His Glu Phe Ala His His
                165                 170                 175

cag ttg ttc aaa aac aga tac tac aat gat ttg gcc agc tat ttc gtt      576
Gln Leu Phe Lys Asn Arg Tyr Tyr Asn Asp Leu Ala Ser Tyr Phe Val
            180                 185                 190

gga aac ttt tta caa gga ttc tca tct ggt ggt tgg aaa gag cag cac      624
Gly Asn Phe Leu Gln Gly Phe Ser Ser Gly Gly Trp Lys Glu Gln His
        195                 200                 205

aat gtg cat cac gca gcc aca aat gtt gtt gga cga gac gga gat ctt      672
Asn Val His His Ala Ala Thr Asn Val Val Gly Arg Asp Gly Asp Leu
    210                 215                 220

gat tta gtc cca ttc tat gct aca gtg gca gaa cat ctc aac aat tat      720
Asp Leu Val Pro Phe Tyr Ala Thr Val Ala Glu His Leu Asn Asn Tyr
225                 230                 235                 240

tct cag gat tca tgg gtt atg act cta ttc aga tgg caa cat gtt cat      768
Ser Gln Asp Ser Trp Val Met Thr Leu Phe Arg Trp Gln His Val His
                245                 250                 255

tgg aca ttc atg tta cca ttc ctc cgt ctc tcg tgg ctt ctt cag tca      816
Trp Thr Phe Met Leu Pro Phe Leu Arg Leu Ser Trp Leu Leu Gln Ser
            260                 265                 270

atc att ttt gtt agt cag atg cca act cat tat tat gac tat tac aga      864
Ile Ile Phe Val Ser Gln Met Pro Thr His Tyr Tyr Asp Tyr Tyr Arg
        275                 280                 285

aat act gcg att tat gaa cag gtt ggt ctc tct ttg cac tgg gct tgg      912
Asn Thr Ala Ile Tyr Glu Gln Val Gly Leu Ser Leu His Trp Ala Trp
    290                 295                 300

tca ttg ggt caa ttg tat ttc cta ccc gat tgg tca act aga ata atg      960
Ser Leu Gly Gln Leu Tyr Phe Leu Pro Asp Trp Ser Thr Arg Ile Met
305                 310                 315                 320
```

```
ttc ttc ctt gtt tct cat ctt gtt gga ggt ttc ctg ctc tct cat gta      1008
Phe Phe Leu Val Ser His Leu Val Gly Gly Phe Leu Leu Ser His Val
                325                 330                 335

gtt act ttc aat cat tat tca gtg gag aag ttt gca ttg agc tcg aac      1056
Val Thr Phe Asn His Tyr Ser Val Glu Lys Phe Ala Leu Ser Ser Asn
            340                 345                 350

atc atg tca aat tac gct tgt ctt caa atc atg acc aca aga aat atg      1104
Ile Met Ser Asn Tyr Ala Cys Leu Gln Ile Met Thr Thr Arg Asn Met
        355                 360                 365

aga cct gga aga ttc att gac tgg ctt tgg gga ggt ctt aac tat cag      1152
Arg Pro Gly Arg Phe Ile Asp Trp Leu Trp Gly Gly Leu Asn Tyr Gln
    370                 375                 380

att gag cac cat ctt ttc cca acg atg cca cga cac aac ttg aac act      1200
Ile Glu His His Leu Phe Pro Thr Met Pro Arg His Asn Leu Asn Thr
385                 390                 395                 400

gtt atg cca ctt gtt aag gag ttt gca gca gca aat ggt tta cca tac      1248
Val Met Pro Leu Val Lys Glu Phe Ala Ala Ala Asn Gly Leu Pro Tyr
                405                 410                 415

atg gtc gac gat tat ttc aca gga ttc tgg ctt gaa att gag caa ttc      1296
Met Val Asp Asp Tyr Phe Thr Gly Phe Trp Leu Glu Ile Glu Gln Phe
            420                 425                 430

cga aat att gca aat gtt gct gct aaa ttg act aaa aag att gcc tag      1344
Arg Asn Ile Ala Asn Val Ala Ala Lys Leu Thr Lys Lys Ile Ala
        435                 440                 445


<210>  8
<211>  447
<212>  PRT
<213>  Ceratodon purpureus

<400>  8

Met Val Leu Arg Glu Gln Glu His Glu Pro Phe Phe Ile Lys Ile Asp
1               5                   10                  15


Gly Lys Trp Cys Gln Ile Asp Asp Ala Val Leu Arg Ser His Pro Gly
            20                  25                  30


Gly Ser Ala Ile Thr Thr Tyr Lys Asn Met Asp Ala Thr Thr Val Phe
        35                  40                  45


His Thr Phe His Thr Gly Ser Lys Glu Ala Tyr Gln Trp Leu Thr Glu
    50                  55                  60


Leu Lys Lys Glu Cys Pro Thr Gln Glu Pro Glu Ile Pro Asp Ile Lys
65                  70                  75                  80


Asp Asp Pro Ile Lys Gly Ile Asp Asp Val Asn Met Gly Thr Phe Asn
                85                  90                  95
```

Ile Ser Glu Lys Arg Ser Ala Gln Ile Asn Lys Ser Phe Thr Asp Leu
100 105 110

Arg Met Arg Val Arg Ala Glu Gly Leu Met Asp Gly Ser Pro Leu Phe
115 120 125

Tyr Ile Arg Lys Ile Leu Glu Thr Ile Phe Thr Ile Leu Phe Ala Phe
130 135 140

Tyr Leu Gln Tyr His Thr Tyr Tyr Leu Pro Ser Ala Ile Leu Met Gly
145 150 155 160

Val Ala Trp Gln Gln Leu Gly Trp Leu Ile His Glu Phe Ala His His
165 170 175

Gln Leu Phe Lys Asn Arg Tyr Tyr Asn Asp Leu Ala Ser Tyr Phe Val
180 185 190

Gly Asn Phe Leu Gln Gly Phe Ser Ser Gly Gly Trp Lys Glu Gln His
195 200 205

Asn Val His His Ala Ala Thr Asn Val Val Gly Arg Asp Gly Asp Leu
210 215 220

Asp Leu Val Pro Phe Tyr Ala Thr Val Ala Glu His Leu Asn Asn Tyr
225 230 235 240

Ser Gln Asp Ser Trp Val Met Thr Leu Phe Arg Trp Gln His Val His
245 250 255

Trp Thr Phe Met Leu Pro Phe Leu Arg Leu Ser Trp Leu Leu Gln Ser
260 265 270

Ile Ile Phe Val Ser Gln Met Pro Thr His Tyr Tyr Asp Tyr Tyr Arg
275 280 285

Asn Thr Ala Ile Tyr Glu Gln Val Gly Leu Ser Leu His Trp Ala Trp
290 295 300

Ser Leu Gly Gln Leu Tyr Phe Leu Pro Asp Trp Ser Thr Arg Ile Met
305 310 315 320

Phe Phe Leu Val Ser His Leu Val Gly Gly Phe Leu Leu Ser His Val
325 330 335

Val Thr Phe Asn His Tyr Ser Val Glu Lys Phe Ala Leu Ser Ser Asn

340 345 350

Ile Met Ser Asn Tyr Ala Cys Leu Gln Ile Met Thr Thr Arg Asn Met
355 360 365

Arg Pro Gly Arg Phe Ile Asp Trp Leu Trp Gly Gly Leu Asn Tyr Gln
370 375 380

Ile Glu His His Leu Phe Pro Thr Met Pro Arg His Asn Leu Asn Thr
385 390 395 400

Val Met Pro Leu Val Lys Glu Phe Ala Ala Ala Asn Gly Leu Pro Tyr
405 410 415

Met Val Asp Asp Tyr Phe Thr Gly Phe Trp Leu Glu Ile Glu Gln Phe
420 425 430

Arg Asn Ile Ala Asn Val Ala Ala Lys Leu Thr Lys Lys Ile Ala
435 440 445

<210> 9
<211> 1443
<212> DNA
<213> Physcomitrella patens

<220>
<221> CDS
<222> (1)..(1443)
<223> Delta-5-Desaturase

<400> 9
atg gcg ccc cac tct gcg gat act gct ggg ctc gtg cct tct gac gaa 48
Met Ala Pro His Ser Ala Asp Thr Ala Gly Leu Val Pro Ser Asp Glu
1 5 10 15

ttg agg cta cga acg tcg aat tca aag ggt ccc gaa caa gag caa act 96
Leu Arg Leu Arg Thr Ser Asn Ser Lys Gly Pro Glu Gln Glu Gln Thr
20 25 30

ttg aag aag tac acc ctt gaa gat gtc agc cgc cac aac acc cca gca 144
Leu Lys Lys Tyr Thr Leu Glu Asp Val Ser Arg His Asn Thr Pro Ala
35 40 45

gat tgt tgg ttg gtg ata tgg ggc aaa gtc tac gat gtc aca agc tgg 192
Asp Cys Trp Leu Val Ile Trp Gly Lys Val Tyr Asp Val Thr Ser Trp
50 55 60

att ccc aat cat ccg ggg ggc agt ctc atc cac gta aaa gca ggg cag 240
Ile Pro Asn His Pro Gly Gly Ser Leu Ile His Val Lys Ala Gly Gln
65 70 75 80

gat tcc act cag ctt ttc gat tcc tat cac ccc ctt tat gtc agg aaa 288
Asp Ser Thr Gln Leu Phe Asp Ser Tyr His Pro Leu Tyr Val Arg Lys
85 90 95

```
atg ctc gcg aag tac tgt att ggg gaa cta gta ccg tct gct ggt gat        336
Met Leu Ala Lys Tyr Cys Ile Gly Glu Leu Val Pro Ser Ala Gly Asp
            100                 105                 110

gac aag ttt aag aaa gca act ctg aag tat gca gat gcc gaa aat gaa        384
Asp Lys Phe Lys Lys Ala Thr Leu Lys Tyr Ala Asp Ala Glu Asn Glu
        115                 120                 125

gat ttc tat ttg gtt gtg aag caa cga gtt gaa tct tat ttc aag agt        432
Asp Phe Tyr Leu Val Val Lys Gln Arg Val Glu Ser Tyr Phe Lys Ser
    130                 135                 140

aac aag ata aac ccc caa att cat cca cat atg atc ctg aag tca ttg        480
Asn Lys Ile Asn Pro Gln Ile His Pro His Met Ile Leu Lys Ser Leu
145                 150                 155                 160

ttc att ctt ggg gga tat ttc gcc agt tac tat tta gcg ttc ttc tgg        528
Phe Ile Leu Gly Gly Tyr Phe Ala Ser Tyr Tyr Leu Ala Phe Phe Trp
                165                 170                 175

tct tca agt gtc ctt gtt tct ttg ttt ttc gca ttg tgg atg ggg ttc        576
Ser Ser Ser Val Leu Val Ser Leu Phe Phe Ala Leu Trp Met Gly Phe
            180                 185                 190

ttc gca gcg gaa gtc ggc gtg tcg att caa cat gat gga aat cat ggt        624
Phe Ala Ala Glu Val Gly Val Ser Ile Gln His Asp Gly Asn His Gly
        195                 200                 205

tca tac act aaa tgg cgt ggc ttt gga tat atc atg gga gcc tcc cta        672
Ser Tyr Thr Lys Trp Arg Gly Phe Gly Tyr Ile Met Gly Ala Ser Leu
    210                 215                 220

gat cta gtc gga gcc agt agc ttc atg tgg aga cag caa cac gtt gtg        720
Asp Leu Val Gly Ala Ser Ser Phe Met Trp Arg Gln Gln His Val Val
225                 230                 235                 240

gga cat cac tcg ttt aca aat gtg gac aac tac gat cct gat att cgt        768
Gly His His Ser Phe Thr Asn Val Asp Asn Tyr Asp Pro Asp Ile Arg
                245                 250                 255

gtg aaa gat cca gat gtc agg agg gtt gcg acc aca caa cca aga caa        816
Val Lys Asp Pro Asp Val Arg Arg Val Ala Thr Thr Gln Pro Arg Gln
            260                 265                 270

tgg tat cat gcg tat cag cat atc tac ctg gca gta tta tat gga act        864
Trp Tyr His Ala Tyr Gln His Ile Tyr Leu Ala Val Leu Tyr Gly Thr
        275                 280                 285

cta gct ctt aag agt att ttt cta gat gat ttc ctt gcg tac ttc aca        912
Leu Ala Leu Lys Ser Ile Phe Leu Asp Asp Phe Leu Ala Tyr Phe Thr
    290                 295                 300

gga tca att ggc cct gtc aag gtg gcg aaa atg acc ccc ctg gag ttc        960
Gly Ser Ile Gly Pro Val Lys Val Ala Lys Met Thr Pro Leu Glu Phe
305                 310                 315                 320

aac atc ttc ttt cag gga aag ctg cta tat gcg ttc tac atg ttc gtg       1008
Asn Ile Phe Phe Gln Gly Lys Leu Leu Tyr Ala Phe Tyr Met Phe Val
                325                 330                 335

ttg cca tct gtg tac ggt gtt cac tcc gga gga act ttc ttg gca cta       1056
Leu Pro Ser Val Tyr Gly Val His Ser Gly Gly Thr Phe Leu Ala Leu
            340                 345                 350
```

```
tat gtg gct tct cag ctc att aca ggt tgg atg tta gct ttt ctt ttt      1104
Tyr Val Ala Ser Gln Leu Ile Thr Gly Trp Met Leu Ala Phe Leu Phe
        355                 360                 365

caa gta gca cat gtc gtg gat gat gtt gca ttt cct aca cca gaa ggt      1152
Gln Val Ala His Val Val Asp Asp Val Ala Phe Pro Thr Pro Glu Gly
    370                 375                 380

ggg aag gtg aag gga gga tgg gct gca atg cag gtt gca aca act acg      1200
Gly Lys Val Lys Gly Gly Trp Ala Ala Met Gln Val Ala Thr Thr Thr
385                 390                 395                 400

gat ttc agt cca cgc tca tgg ttc tgg ggt cat gtc tct gga gga tta      1248
Asp Phe Ser Pro Arg Ser Trp Phe Trp Gly His Val Ser Gly Gly Leu
                405                 410                 415

aac aac caa att gag cat cat ctg ttt cca gga gtg tgc cat gtt cat      1296
Asn Asn Gln Ile Glu His His Leu Phe Pro Gly Val Cys His Val His
            420                 425                 430

tat cca gcc att cag cct att gtc gag aag acg tgc aag gaa ttc gat      1344
Tyr Pro Ala Ile Gln Pro Ile Val Glu Lys Thr Cys Lys Glu Phe Asp
        435                 440                 445

gtg cct tat gta gcc tac cca act ttt tgg act gcg ttg aga gcc cac      1392
Val Pro Tyr Val Ala Tyr Pro Thr Phe Trp Thr Ala Leu Arg Ala His
    450                 455                 460

ttt gcg cat ttg aaa aag gtt gga ttg aca gag ttt cgg ctc gat ggc      1440
Phe Ala His Leu Lys Lys Val Gly Leu Thr Glu Phe Arg Leu Asp Gly
465                 470                 475                 480

tga                                                                  1443


<210>  10
<211>  480
<212>  PRT
<213>  Physcomitrella patens

<400>  10

Met Ala Pro His Ser Ala Asp Thr Ala Gly Leu Val Pro Ser Asp Glu
1               5                   10                  15


Leu Arg Leu Arg Thr Ser Asn Ser Lys Gly Pro Glu Gln Glu Gln Thr
            20                  25                  30


Leu Lys Lys Tyr Thr Leu Glu Asp Val Ser Arg His Asn Thr Pro Ala
        35                  40                  45


Asp Cys Trp Leu Val Ile Trp Gly Lys Val Tyr Asp Val Thr Ser Trp
    50                  55                  60


Ile Pro Asn His Pro Gly Gly Ser Leu Ile His Val Lys Ala Gly Gln
65                  70                  75                  80
```

Asp Ser Thr Gln Leu Phe Asp Ser Tyr His Pro Leu Tyr Val Arg Lys
85 90 95

Met Leu Ala Lys Tyr Cys Ile Gly Glu Leu Val Pro Ser Ala Gly Asp
100 105 110

Asp Lys Phe Lys Lys Ala Thr Leu Lys Tyr Ala Asp Ala Glu Asn Glu
115 120 125

Asp Phe Tyr Leu Val Val Lys Gln Arg Val Glu Ser Tyr Phe Lys Ser
130 135 140

Asn Lys Ile Asn Pro Gln Ile His Pro His Met Ile Leu Lys Ser Leu
145 150 155 160

Phe Ile Leu Gly Gly Tyr Phe Ala Ser Tyr Tyr Leu Ala Phe Phe Trp
165 170 175

Ser Ser Ser Val Leu Val Ser Leu Phe Phe Ala Leu Trp Met Gly Phe
180 185 190

Phe Ala Ala Glu Val Gly Val Ser Ile Gln His Asp Gly Asn His Gly
195 200 205

Ser Tyr Thr Lys Trp Arg Gly Phe Gly Tyr Ile Met Gly Ala Ser Leu
210 215 220

Asp Leu Val Gly Ala Ser Ser Phe Met Trp Arg Gln Gln His Val Val
225 230 235 240

Gly His His Ser Phe Thr Asn Val Asp Asn Tyr Asp Pro Asp Ile Arg
245 250 255

Val Lys Asp Pro Asp Val Arg Arg Val Ala Thr Thr Gln Pro Arg Gln
260 265 270

Trp Tyr His Ala Tyr Gln His Ile Tyr Leu Ala Val Leu Tyr Gly Thr
275 280 285

Leu Ala Leu Lys Ser Ile Phe Leu Asp Asp Phe Leu Ala Tyr Phe Thr
290 295 300

Gly Ser Ile Gly Pro Val Lys Val Ala Lys Met Thr Pro Leu Glu Phe
305 310 315 320

Asn Ile Phe Phe Gln Gly Lys Leu Leu Tyr Ala Phe Tyr Met Phe Val
325 330 335

```
Leu Pro Ser Val Tyr Gly Val His Ser Gly Gly Thr Phe Leu Ala Leu
            340                 345                 350

Tyr Val Ala Ser Gln Leu Ile Thr Gly Trp Met Leu Ala Phe Leu Phe
        355                 360                 365

Gln Val Ala His Val Val Asp Asp Val Ala Phe Pro Thr Pro Glu Gly
    370                 375                 380

Gly Lys Val Lys Gly Gly Trp Ala Ala Met Gln Val Ala Thr Thr Thr
385                 390                 395                 400

Asp Phe Ser Pro Arg Ser Trp Phe Trp Gly His Val Ser Gly Gly Leu
                405                 410                 415

Asn Asn Gln Ile Glu His His Leu Phe Pro Gly Val Cys His Val His
            420                 425                 430

Tyr Pro Ala Ile Gln Pro Ile Val Glu Lys Thr Cys Lys Glu Phe Asp
        435                 440                 445

Val Pro Tyr Val Ala Tyr Pro Thr Phe Trp Thr Ala Leu Arg Ala His
    450                 455                 460

Phe Ala His Leu Lys Lys Val Gly Leu Thr Glu Phe Arg Leu Asp Gly
465                 470                 475                 480


<210>  11
<211>  1320
<212>  DNA
<213>  Thraustrochytrium

<220>
<221>  CDS
<222>  (1)..(1320)
<223>  Delta-5-Desaturase


<400>  11
atg ggc aag ggc agc gag ggc cgc agc gcg gcg cgc gag atg acg gcc       48
Met Gly Lys Gly Ser Glu Gly Arg Ser Ala Ala Arg Glu Met Thr Ala
1               5                   10                  15

gag gcg aac ggc gac aag cgg aaa acg att ctg atc gag ggc gtc ctg       96
Glu Ala Asn Gly Asp Lys Arg Lys Thr Ile Leu Ile Glu Gly Val Leu
            20                  25                  30

tac gac gcg acg aac ttt aag cac ccg ggc ggt tcg atc atc aac ttc      144
Tyr Asp Ala Thr Asn Phe Lys His Pro Gly Gly Ser Ile Ile Asn Phe
        35                  40                  45

ttg acc gag ggc gag gcc ggc gtg gac gcg acg cag gcg tac cgc gag      192
Leu Thr Glu Gly Glu Ala Gly Val Asp Ala Thr Gln Ala Tyr Arg Glu
```

```
    50                  55                  60

ttt cat cag cgg tcc ggc aag gcc gac aag tac ctc aag tcg ctg ccg       240
Phe His Gln Arg Ser Gly Lys Ala Asp Lys Tyr Leu Lys Ser Leu Pro
65                  70                  75                  80

aag ctg gat gcg tcc aag gtg gag tcg cgg ttc tcg gcc aaa gag cag       288
Lys Leu Asp Ala Ser Lys Val Glu Ser Arg Phe Ser Ala Lys Glu Gln
                85                  90                  95

gcg cgg cgc gac gcc atg acg cgc gac tac gcg gcc ttt cgc gag gag       336
Ala Arg Arg Asp Ala Met Thr Arg Asp Tyr Ala Ala Phe Arg Glu Glu
            100                 105                 110

ctc gtc gcc gag ggg tac ttt gac ccg tcg atc ccg cac atg att tac       384
Leu Val Ala Glu Gly Tyr Phe Asp Pro Ser Ile Pro His Met Ile Tyr
        115                 120                 125

cgc gtc gtg gag atc gtg gcg ctc ttc gcg ctc tcg ttc tgg ctc atg       432
Arg Val Val Glu Ile Val Ala Leu Phe Ala Leu Ser Phe Trp Leu Met
    130                 135                 140

tcc aag gcc tcg ccc acc tcg ctc gtg ctg ggc gtg gtg atg aac ggc       480
Ser Lys Ala Ser Pro Thr Ser Leu Val Leu Gly Val Val Met Asn Gly
145                 150                 155                 160

att gcg cag ggc cgc tgc ggc tgg gtc atg cac gag atg ggc cac ggg       528
Ile Ala Gln Gly Arg Cys Gly Trp Val Met His Glu Met Gly His Gly
                165                 170                 175

tcg ttc acg ggc gtc atc tgg ctc gac gac cgg atg tgc gag ttc ttc       576
Ser Phe Thr Gly Val Ile Trp Leu Asp Asp Arg Met Cys Glu Phe Phe
            180                 185                 190

tac ggc gtc ggc tgc ggc atg agc ggg cac tac tgg aag aac cag cac       624
Tyr Gly Val Gly Cys Gly Met Ser Gly His Tyr Trp Lys Asn Gln His
        195                 200                 205

agc aag cac cac gcc gcg ccc aac cgc ctc gag cac gat gtc gat ctc       672
Ser Lys His His Ala Ala Pro Asn Arg Leu Glu His Asp Val Asp Leu
    210                 215                 220

aac acg ctg ccc ctg gtc gcc ttt aac gag cgc gtc gtg cgc aag gtc       720
Asn Thr Leu Pro Leu Val Ala Phe Asn Glu Arg Val Val Arg Lys Val
225                 230                 235                 240

aag ccg gga tcg ctg ctg gcg ctc tgg ctg cgc gtg cag gcg tac ctc       768
Lys Pro Gly Ser Leu Leu Ala Leu Trp Leu Arg Val Gln Ala Tyr Leu
                245                 250                 255

ttt gcg ccc gtc tcg tgc ctg ctc atc ggc ctt ggc tgg acg ctc tac       816
Phe Ala Pro Val Ser Cys Leu Leu Ile Gly Leu Gly Trp Thr Leu Tyr
            260                 265                 270

ctg cac ccg cgc tac atg ctg cgc acc aag cgg cac atg gag ttc gtc       864
Leu His Pro Arg Tyr Met Leu Arg Thr Lys Arg His Met Glu Phe Val
        275                 280                 285

tgg atc ttc gcg cgc tac att ggc tgg ttc tcg ctc atg ggc gct ctc       912
Trp Ile Phe Ala Arg Tyr Ile Gly Trp Phe Ser Leu Met Gly Ala Leu
    290                 295                 300

ggc tac tcg ccg ggc acc tcg gtc ggg atg tac ctg tgc tcg ttc ggc       960
```

```
Gly Tyr Ser Pro Gly Thr Ser Val Gly Met Tyr Leu Cys Ser Phe Gly
305                 310                 315                 320

ctc ggc tgc att tac att ttc ctg cag ttc gcc gtc agc cac acg cac      1008
Leu Gly Cys Ile Tyr Ile Phe Leu Gln Phe Ala Val Ser His Thr His
                325                 330                 335

ctg ccg gtg acc aac ccg gag gac cag ctg cac tgg ctc gag tac gcg      1056
Leu Pro Val Thr Asn Pro Glu Asp Gln Leu His Trp Leu Glu Tyr Ala
            340                 345                 350

gcc gac cac acg gtg aac att agc acc aag tcc tgg ctc gtc acg tgg      1104
Ala Asp His Thr Val Asn Ile Ser Thr Lys Ser Trp Leu Val Thr Trp
        355                 360                 365

tgg atg tcg aac ctg aac ttt cag atc gag cac cac ctc ttc ccc acg      1152
Trp Met Ser Asn Leu Asn Phe Gln Ile Glu His His Leu Phe Pro Thr
    370                 375                 380

gcg ccg cag ttc cgc ttc aag gaa atc agt cct cgc gtc gag gcc ctc      1200
Ala Pro Gln Phe Arg Phe Lys Glu Ile Ser Pro Arg Val Glu Ala Leu
385                 390                 395                 400

ttc aag cgc cac aac ctc ccg tac tac gac ctg ccc tac acg agc gcg      1248
Phe Lys Arg His Asn Leu Pro Tyr Tyr Asp Leu Pro Tyr Thr Ser Ala
                405                 410                 415

gtc tcg acc acc ttt gcc aat ctt tat tcc gtc ggc cac tcg gtc ggc      1296
Val Ser Thr Thr Phe Ala Asn Leu Tyr Ser Val Gly His Ser Val Gly
            420                 425                 430

gcc gac acc aag aag cag gac tga                                      1320
Ala Asp Thr Lys Lys Gln Asp
        435


<210>  12
<211>  439
<212>  PRT
<213>  Thraustrochytrium

<400>  12

Met Gly Lys Gly Ser Glu Gly Arg Ser Ala Ala Arg Glu Met Thr Ala
1               5                   10                  15


Glu Ala Asn Gly Asp Lys Arg Lys Thr Ile Leu Ile Glu Gly Val Leu
            20                  25                  30


Tyr Asp Ala Thr Asn Phe Lys His Pro Gly Gly Ser Ile Ile Asn Phe
        35                  40                  45


Leu Thr Glu Gly Glu Ala Gly Val Asp Ala Thr Gln Ala Tyr Arg Glu
    50                  55                  60


Phe His Gln Arg Ser Gly Lys Ala Asp Lys Tyr Leu Lys Ser Leu Pro
65                  70                  75                  80
```

```
Lys Leu Asp Ala Ser Lys Val Glu Ser Arg Phe Ser Ala Lys Glu Gln
                85                  90                  95

Ala Arg Arg Asp Ala Met Thr Arg Asp Tyr Ala Ala Phe Arg Glu Glu
            100                 105                 110

Leu Val Ala Glu Gly Tyr Phe Asp Pro Ser Ile Pro His Met Ile Tyr
        115                 120                 125

Arg Val Val Glu Ile Val Ala Leu Phe Ala Leu Ser Phe Trp Leu Met
    130                 135                 140

Ser Lys Ala Ser Pro Thr Ser Leu Val Leu Gly Val Val Met Asn Gly
145                 150                 155                 160

Ile Ala Gln Gly Arg Cys Gly Trp Val Met His Glu Met Gly His Gly
                165                 170                 175

Ser Phe Thr Gly Val Ile Trp Leu Asp Asp Arg Met Cys Glu Phe Phe
            180                 185                 190

Tyr Gly Val Gly Cys Gly Met Ser Gly His Tyr Trp Lys Asn Gln His
        195                 200                 205

Ser Lys His His Ala Ala Pro Asn Arg Leu Glu His Asp Val Asp Leu
    210                 215                 220

Asn Thr Leu Pro Leu Val Ala Phe Asn Glu Arg Val Val Arg Lys Val
225                 230                 235                 240

Lys Pro Gly Ser Leu Leu Ala Leu Trp Leu Arg Val Gln Ala Tyr Leu
                245                 250                 255

Phe Ala Pro Val Ser Cys Leu Leu Ile Gly Leu Gly Trp Thr Leu Tyr
            260                 265                 270

Leu His Pro Arg Tyr Met Leu Arg Thr Lys Arg His Met Glu Phe Val
        275                 280                 285

Trp Ile Phe Ala Arg Tyr Ile Gly Trp Phe Ser Leu Met Gly Ala Leu
    290                 295                 300

Gly Tyr Ser Pro Gly Thr Ser Val Gly Met Tyr Leu Cys Ser Phe Gly
305                 310                 315                 320

Leu Gly Cys Ile Tyr Ile Phe Leu Gln Phe Ala Val Ser His Thr His
                325                 330                 335
```

Leu Pro Val Thr Asn Pro Glu Asp Gln Leu His Trp Leu Glu Tyr Ala
340 345 350

Ala Asp His Thr Val Asn Ile Ser Thr Lys Ser Trp Leu Val Thr Trp
355 360 365

Trp Met Ser Asn Leu Asn Phe Gln Ile Glu His His Leu Phe Pro Thr
370 375 380

Ala Pro Gln Phe Arg Phe Lys Glu Ile Ser Pro Arg Val Glu Ala Leu
385 390 395 400

Phe Lys Arg His Asn Leu Pro Tyr Tyr Asp Leu Pro Tyr Thr Ser Ala
405 410 415

Val Ser Thr Thr Phe Ala Asn Leu Tyr Ser Val Gly His Ser Val Gly
420 425 430

Ala Asp Thr Lys Lys Gln Asp
435

<210> 13
<211> 1341
<212> DNA
<213> Mortierella alpina

<220>
<221> CDS
<222> (1)..(1341)
<223> Delta-5-Desaturase

<400> 13
atg gga acg gac caa gga aaa acc ttc acc tgg gaa gag ctg gcg gcc 48
Met Gly Thr Asp Gln Gly Lys Thr Phe Thr Trp Glu Glu Leu Ala Ala
1 5 10 15

cat aac acc aag gac gac cta ctc ttg gcc atc cgc ggc agg gtg tac 96
His Asn Thr Lys Asp Asp Leu Leu Leu Ala Ile Arg Gly Arg Val Tyr
20 25 30

gat gtc aca aag ttc ttg agc cgc cat cct ggt gga gtg gac act ctc 144
Asp Val Thr Lys Phe Leu Ser Arg His Pro Gly Gly Val Asp Thr Leu
35 40 45

ctg ctc gga gct ggc cga gat gtt act ccg gtc ttt gag atg tat cac 192
Leu Leu Gly Ala Gly Arg Asp Val Thr Pro Val Phe Glu Met Tyr His
50 55 60

gcg ttt ggg gct gca gat gcc att atg aag aag tac tat gtc ggt aca 240
Ala Phe Gly Ala Ala Asp Ala Ile Met Lys Lys Tyr Tyr Val Gly Thr
65 70 75 80

ctg gtc tcg aat gag ctg ccc atc ttc ccg gag cca acg gtg ttc cac 288
Leu Val Ser Asn Glu Leu Pro Ile Phe Pro Glu Pro Thr Val Phe His

```
            85                  90                  95

aaa acc atc aag acg aga gtc gag ggc tac ttt acg gat cgg aac att        336
Lys Thr Ile Lys Thr Arg Val Glu Gly Tyr Phe Thr Asp Arg Asn Ile
            100                 105                 110

gat ccc aag aat aga cca gag atc tgg gga cga tac gct ctt atc ttt        384
Asp Pro Lys Asn Arg Pro Glu Ile Trp Gly Arg Tyr Ala Leu Ile Phe
        115                 120                 125

gga tcc ttg atc gct tcc tac tac gcg cag ctc ttt gtg cct ttc gtt        432
Gly Ser Leu Ile Ala Ser Tyr Tyr Ala Gln Leu Phe Val Pro Phe Val
    130                 135                 140

gtc gaa cgc aca tgg ctt cag gtg gtg ttt gca atc atc atg gga ttt        480
Val Glu Arg Thr Trp Leu Gln Val Val Phe Ala Ile Ile Met Gly Phe
145                 150                 155                 160

gcg tgc gca caa gtc gga ctc aac cct ctt cat gat gcg tct cac ttt        528
Ala Cys Ala Gln Val Gly Leu Asn Pro Leu His Asp Ala Ser His Phe
                165                 170                 175

tca gtg acc cac aac ccc act gtc tgg aag att ctg gga gcc acg cac        576
Ser Val Thr His Asn Pro Thr Val Trp Lys Ile Leu Gly Ala Thr His
            180                 185                 190

gac ttt ttc aac gga gca tcg tac ctg gtg tgg atg tac caa cat atg        624
Asp Phe Phe Asn Gly Ala Ser Tyr Leu Val Trp Met Tyr Gln His Met
        195                 200                 205

ctc ggc cat cac ccc tac acc aac att gct gga gca gat ccc gac gtg        672
Leu Gly His His Pro Tyr Thr Asn Ile Ala Gly Ala Asp Pro Asp Val
    210                 215                 220

tcg acg tct gag ccc gat gtt cgt cgt atc aag ccc aac caa aag tgg        720
Ser Thr Ser Glu Pro Asp Val Arg Arg Ile Lys Pro Asn Gln Lys Trp
225                 230                 235                 240

ttt gtc aac cac atc aac cag cac atg ttt gtt cct ttc ctg tac gga        768
Phe Val Asn His Ile Asn Gln His Met Phe Val Pro Phe Leu Tyr Gly
                245                 250                 255

ctg ctg gcg ttc aag gtg cgc att cag gac atc aac att ttg tac ttt        816
Leu Leu Ala Phe Lys Val Arg Ile Gln Asp Ile Asn Ile Leu Tyr Phe
            260                 265                 270

gtc aag acc aat gac gct att cgt gtc aat ccc atc tcg aca tgg cac        864
Val Lys Thr Asn Asp Ala Ile Arg Val Asn Pro Ile Ser Thr Trp His
        275                 280                 285

act gtg atg ttc tgg ggc ggc aag gct ttc ttt gtc tgg tat cgc ctg        912
Thr Val Met Phe Trp Gly Gly Lys Ala Phe Phe Val Trp Tyr Arg Leu
    290                 295                 300

att gtt ccc ctg cag tat ctg ccc ctg ggc aag gtg ctg ctc ttg ttc        960
Ile Val Pro Leu Gln Tyr Leu Pro Leu Gly Lys Val Leu Leu Leu Phe
305                 310                 315                 320

acg gtc gcg gac atg gtg tcg tct tac tgg ctg gcg ctg acc ttc cag       1008
Thr Val Ala Asp Met Val Ser Ser Tyr Trp Leu Ala Leu Thr Phe Gln
                325                 330                 335

gcg aac cac gtt gtt gag gaa gtt cag tgg ccg ttg cct gac gag aac       1056
```

```
Ala Asn His Val Val Glu Glu Val Gln Trp Pro Leu Pro Asp Glu Asn
            340                 345                 350

ggg atc atc caa aag gac tgg gca gct atg cag gtc gag act acg cag      1104
Gly Ile Ile Gln Lys Asp Trp Ala Ala Met Gln Val Glu Thr Thr Gln
        355                 360                 365

gat tac gca cac gat tcg cac ctc tgg acc agc atc act ggc agc ttg      1152
Asp Tyr Ala His Asp Ser His Leu Trp Thr Ser Ile Thr Gly Ser Leu
    370                 375                 380

aac tac cag gct gtg cac cat ctg ttc ccc aac gtg tcg cag cac cat      1200
Asn Tyr Gln Ala Val His His Leu Phe Pro Asn Val Ser Gln His His
385                 390                 395                 400

tat ccc gat att ctg gcc atc atc aag aac acc tgc agc gag tac aag      1248
Tyr Pro Asp Ile Leu Ala Ile Ile Lys Asn Thr Cys Ser Glu Tyr Lys
                405                 410                 415

gtt cca tac ctt gtc aag gat acg ttt tgg caa gca ttt gct tca cat      1296
Val Pro Tyr Leu Val Lys Asp Thr Phe Trp Gln Ala Phe Ala Ser His
            420                 425                 430

ttg gag cac ttg cgt gtt ctt gga ctc cgt ccc aag gaa gag tag          1341
Leu Glu His Leu Arg Val Leu Gly Leu Arg Pro Lys Glu Glu
        435                 440                 445


<210>  14
<211>  446
<212>  PRT
<213>  Mortierella alpina

<400>  14

Met Gly Thr Asp Gln Gly Lys Thr Phe Thr Trp Glu Glu Leu Ala Ala
1               5                   10                  15


His Asn Thr Lys Asp Asp Leu Leu Leu Ala Ile Arg Gly Arg Val Tyr
            20                  25                  30


Asp Val Thr Lys Phe Leu Ser Arg His Pro Gly Gly Val Asp Thr Leu
        35                  40                  45


Leu Leu Gly Ala Gly Arg Asp Val Thr Pro Val Phe Glu Met Tyr His
    50                  55                  60


Ala Phe Gly Ala Ala Asp Ala Ile Met Lys Lys Tyr Tyr Val Gly Thr
65                  70                  75                  80


Leu Val Ser Asn Glu Leu Pro Ile Phe Pro Glu Pro Thr Val Phe His
                85                  90                  95


Lys Thr Ile Lys Thr Arg Val Glu Gly Tyr Phe Thr Asp Arg Asn Ile
            100                 105                 110
```

Asp Pro Lys Asn Arg Pro Glu Ile Trp Gly Arg Tyr Ala Leu Ile Phe
        115                 120                 125

Gly Ser Leu Ile Ala Ser Tyr Tyr Ala Gln Leu Phe Val Pro Phe Val
    130                 135                 140

Val Glu Arg Thr Trp Leu Gln Val Val Phe Ala Ile Ile Met Gly Phe
145                 150                 155                 160

Ala Cys Ala Gln Val Gly Leu Asn Pro Leu His Asp Ala Ser His Phe
                165                 170                 175

Ser Val Thr His Asn Pro Thr Val Trp Lys Ile Leu Gly Ala Thr His
            180                 185                 190

Asp Phe Phe Asn Gly Ala Ser Tyr Leu Val Trp Met Tyr Gln His Met
        195                 200                 205

Leu Gly His His Pro Tyr Thr Asn Ile Ala Gly Ala Asp Pro Asp Val
    210                 215                 220

Ser Thr Ser Glu Pro Asp Val Arg Arg Ile Lys Pro Asn Gln Lys Trp
225                 230                 235                 240

Phe Val Asn His Ile Asn Gln His Met Phe Val Pro Phe Leu Tyr Gly
                245                 250                 255

Leu Leu Ala Phe Lys Val Arg Ile Gln Asp Ile Asn Ile Leu Tyr Phe
            260                 265                 270

Val Lys Thr Asn Asp Ala Ile Arg Val Asn Pro Ile Ser Thr Trp His
        275                 280                 285

Thr Val Met Phe Trp Gly Gly Lys Ala Phe Phe Val Trp Tyr Arg Leu
    290                 295                 300

Ile Val Pro Leu Gln Tyr Leu Pro Leu Gly Lys Val Leu Leu Leu Phe
305                 310                 315                 320

Thr Val Ala Asp Met Val Ser Ser Tyr Trp Leu Ala Leu Thr Phe Gln
                325                 330                 335

Ala Asn His Val Val Glu Glu Val Gln Trp Pro Leu Pro Asp Glu Asn
            340                 345                 350

Gly Ile Ile Gln Lys Asp Trp Ala Ala Met Gln Val Glu Thr Thr Gln
        355                 360                 365

```
Asp Tyr Ala His Asp Ser His Leu Trp Thr Ser Ile Thr Gly Ser Leu
    370                 375                 380

Asn Tyr Gln Ala Val His His Leu Phe Pro Asn Val Ser Gln His His
385                 390                 395                 400

Tyr Pro Asp Ile Leu Ala Ile Ile Lys Asn Thr Cys Ser Glu Tyr Lys
                405                 410                 415

Val Pro Tyr Leu Val Lys Asp Thr Phe Trp Gln Ala Phe Ala Ser His
            420                 425                 430

Leu Glu His Leu Arg Val Leu Gly Leu Arg Pro Lys Glu Glu
        435                 440                 445


<210>  15
<211>  1344
<212>  DNA
<213>  Caenorhabditis elegans

<220>
<221>  CDS
<222>  (1)..(1344)
<223>  Delta-5-Desaturase


<400>  15
atg gta tta cga gag caa gag cat gag cca ttc ttc att aaa att gat       48
Met Val Leu Arg Glu Gln Glu His Glu Pro Phe Phe Ile Lys Ile Asp
1               5                   10                  15

gga aaa tgg tgt caa att gac gat gct gtc ctg aga tca cat cca ggt       96
Gly Lys Trp Cys Gln Ile Asp Asp Ala Val Leu Arg Ser His Pro Gly
            20                  25                  30

ggt agt gca att act acc tat aaa aat atg gat gcc act acc gta ttc      144
Gly Ser Ala Ile Thr Thr Tyr Lys Asn Met Asp Ala Thr Thr Val Phe
        35                  40                  45

cac aca ttc cat act ggt tct aaa gaa gcg tat caa tgg ctg aca gaa      192
His Thr Phe His Thr Gly Ser Lys Glu Ala Tyr Gln Trp Leu Thr Glu
    50                  55                  60

ttg aaa aaa gag tgc cct aca caa gaa cca gag atc cca gat att aag      240
Leu Lys Lys Glu Cys Pro Thr Gln Glu Pro Glu Ile Pro Asp Ile Lys
65                  70                  75                  80

gat gac cca atc aaa gga att gat gat gtg aac atg gga act ttc aat      288
Asp Asp Pro Ile Lys Gly Ile Asp Asp Val Asn Met Gly Thr Phe Asn
                85                  90                  95

att tct gag aaa cga tct gcc caa ata aat aaa agt ttc act gat cta      336
Ile Ser Glu Lys Arg Ser Ala Gln Ile Asn Lys Ser Phe Thr Asp Leu
            100                 105                 110

cgt atg cga gtt cgt gca gaa gga ctt atg gat gga tct cct ttg ttc      384
Arg Met Arg Val Arg Ala Glu Gly Leu Met Asp Gly Ser Pro Leu Phe
```

```
        115                 120                 125

tac att aga aaa att ctt gaa aca atc ttc aca att ctt ttt gca ttc      432
Tyr Ile Arg Lys Ile Leu Glu Thr Ile Phe Thr Ile Leu Phe Ala Phe
    130                 135                 140

tac ctt caa tac cac aca tat tat ctt cca tca gct att cta atg gga      480
Tyr Leu Gln Tyr His Thr Tyr Tyr Leu Pro Ser Ala Ile Leu Met Gly
145                 150                 155                 160

gtt gcg tgg caa caa ttg gga tgg tta atc cat gaa ttc gca cat cat      528
Val Ala Trp Gln Gln Leu Gly Trp Leu Ile His Glu Phe Ala His His
                165                 170                 175

cag ttg ttc aaa aac aga tac tac aat gat ttg gcc agc tat ttc gtt      576
Gln Leu Phe Lys Asn Arg Tyr Tyr Asn Asp Leu Ala Ser Tyr Phe Val
            180                 185                 190

gga aac ttt tta caa gga ttc tca tct ggt ggt tgg aaa gag cag cac      624
Gly Asn Phe Leu Gln Gly Phe Ser Ser Gly Gly Trp Lys Glu Gln His
        195                 200                 205

aat gtg cat cac gca gcc aca aat gtt gtt gga cga gac gga gat ctt      672
Asn Val His His Ala Ala Thr Asn Val Val Gly Arg Asp Gly Asp Leu
    210                 215                 220

gat tta gtc cca ttc tat gct aca gtg gca gaa cat ctc aac aat tat      720
Asp Leu Val Pro Phe Tyr Ala Thr Val Ala Glu His Leu Asn Asn Tyr
225                 230                 235                 240

tct cag gat tca tgg gtt atg act cta ttc aga tgg caa cat gtt cat      768
Ser Gln Asp Ser Trp Val Met Thr Leu Phe Arg Trp Gln His Val His
                245                 250                 255

tgg aca ttc atg tta cca ttc ctc cgt ctc tcg tgg ctt ctt cag tca      816
Trp Thr Phe Met Leu Pro Phe Leu Arg Leu Ser Trp Leu Leu Gln Ser
            260                 265                 270

atc att ttt gtt agt cag atg cca act cat tat tat gac tat tac aga      864
Ile Ile Phe Val Ser Gln Met Pro Thr His Tyr Tyr Asp Tyr Tyr Arg
        275                 280                 285

aat act gcg att tat gaa cag gtt ggt ctc tct ttg cac tgg gct tgg      912
Asn Thr Ala Ile Tyr Glu Gln Val Gly Leu Ser Leu His Trp Ala Trp
    290                 295                 300

tca ttg ggt caa ttg tat ttc cta ccc gat tgg tca act aga ata atg      960
Ser Leu Gly Gln Leu Tyr Phe Leu Pro Asp Trp Ser Thr Arg Ile Met
305                 310                 315                 320

ttc ttc ctt gtt tct cat ctt gtt gga ggt ttc ctg ctc tct cat gta     1008
Phe Phe Leu Val Ser His Leu Val Gly Gly Phe Leu Leu Ser His Val
                325                 330                 335

gtt act ttc aat cat tat tca gtg gag aag ttt gca ttg agc tcg aac     1056
Val Thr Phe Asn His Tyr Ser Val Glu Lys Phe Ala Leu Ser Ser Asn
            340                 345                 350

atc atg tca aat tac gct tgt ctt caa atc atg acc aca aga aat atg     1104
Ile Met Ser Asn Tyr Ala Cys Leu Gln Ile Met Thr Thr Arg Asn Met
        355                 360                 365

aga cct gga aga ttc att gac tgg ctt tgg gga ggt ctt aac tat cag     1152
```

Arg Pro Gly Arg Phe Ile Asp Trp Leu Trp Gly Gly Leu Asn Tyr Gln
    370                 375                 380

att gag cac cat ctt ttc cca acg atg cca cga cac aac ttg aac act      1200
Ile Glu His His Leu Phe Pro Thr Met Pro Arg His Asn Leu Asn Thr
385                 390                 395                 400

gtt atg cca ctt gtt aag gag ttt gca gca gca aat ggt tta cca tac      1248
Val Met Pro Leu Val Lys Glu Phe Ala Ala Ala Asn Gly Leu Pro Tyr
                405                 410                 415

atg gtc gac gat tat ttc aca gga ttc tgg ctt gaa att gag caa ttc      1296
Met Val Asp Asp Tyr Phe Thr Gly Phe Trp Leu Glu Ile Glu Gln Phe
            420                 425                 430

cga aat att gca aat gtt gct gct aaa ttg act aaa aag att gcc tag      1344
Arg Asn Ile Ala Asn Val Ala Ala Lys Leu Thr Lys Lys Ile Ala
        435                 440                 445

<210>  16
<211>  447
<212>  PRT
<213>  Caenorhabditis elegans

<400>  16

Met Val Leu Arg Glu Gln Glu His Glu Pro Phe Phe Ile Lys Ile Asp
1               5                   10                  15

Gly Lys Trp Cys Gln Ile Asp Asp Ala Val Leu Arg Ser His Pro Gly
            20                  25                  30

Gly Ser Ala Ile Thr Thr Tyr Lys Asn Met Asp Ala Thr Thr Val Phe
        35                  40                  45

His Thr Phe His Thr Gly Ser Lys Glu Ala Tyr Gln Trp Leu Thr Glu
    50                  55                  60

Leu Lys Lys Glu Cys Pro Thr Gln Glu Pro Glu Ile Pro Asp Ile Lys
65                  70                  75                  80

Asp Asp Pro Ile Lys Gly Ile Asp Asp Val Asn Met Gly Thr Phe Asn
                85                  90                  95

Ile Ser Glu Lys Arg Ser Ala Gln Ile Asn Lys Ser Phe Thr Asp Leu
            100                 105                 110

Arg Met Arg Val Arg Ala Glu Gly Leu Met Asp Gly Ser Pro Leu Phe
        115                 120                 125

Tyr Ile Arg Lys Ile Leu Glu Thr Ile Phe Thr Ile Leu Phe Ala Phe
    130                 135                 140

Tyr Leu Gln Tyr His Thr Tyr Tyr Leu Pro Ser Ala Ile Leu Met Gly
145 150 155 160

Val Ala Trp Gln Gln Leu Gly Trp Leu Ile His Glu Phe Ala His His
165 170 175

Gln Leu Phe Lys Asn Arg Tyr Tyr Asn Asp Leu Ala Ser Tyr Phe Val
180 185 190

Gly Asn Phe Leu Gln Gly Phe Ser Ser Gly Gly Trp Lys Glu Gln His
195 200 205

Asn Val His His Ala Ala Thr Asn Val Val Gly Arg Asp Gly Asp Leu
210 215 220

Asp Leu Val Pro Phe Tyr Ala Thr Val Ala Glu His Leu Asn Asn Tyr
225 230 235 240

Ser Gln Asp Ser Trp Val Met Thr Leu Phe Arg Trp Gln His Val His
245 250 255

Trp Thr Phe Met Leu Pro Phe Leu Arg Leu Ser Trp Leu Leu Gln Ser
260 265 270

Ile Ile Phe Val Ser Gln Met Pro Thr His Tyr Tyr Asp Tyr Tyr Arg
275 280 285

Asn Thr Ala Ile Tyr Glu Gln Val Gly Leu Ser Leu His Trp Ala Trp
290 295 300

Ser Leu Gly Gln Leu Tyr Phe Leu Pro Asp Trp Ser Thr Arg Ile Met
305 310 315 320

Phe Phe Leu Val Ser His Leu Val Gly Gly Phe Leu Leu Ser His Val
325 330 335

Val Thr Phe Asn His Tyr Ser Val Glu Lys Phe Ala Leu Ser Ser Asn
340 345 350

Ile Met Ser Asn Tyr Ala Cys Leu Gln Ile Met Thr Thr Arg Asn Met
355 360 365

Arg Pro Gly Arg Phe Ile Asp Trp Leu Trp Gly Gly Leu Asn Tyr Gln
370 375 380

Ile Glu His His Leu Phe Pro Thr Met Pro Arg His Asn Leu Asn Thr
385 390 395 400

```
Val Met Pro Leu Val Lys Glu Phe Ala Ala Ala Asn Gly Leu Pro Tyr
                405                 410                 415

Met Val Asp Asp Tyr Phe Thr Gly Phe Trp Leu Glu Ile Glu Gln Phe
            420                 425                 430

Arg Asn Ile Ala Asn Val Ala Ala Lys Leu Thr Lys Lys Ile Ala
        435                 440                 445


<210>  17
<211>  1687
<212>  DNA
<213>  Borago officinalis

<220>
<221>  CDS
<222>  (42)..(1388)
<223>  Delta-6-Desaturase


<400>  17
tatctgccta ccctcccaaa gagagtagtc atttttcatc a atg gct gct caa atc      56
                                              Met Ala Ala Gln Ile
                                              1               5

aag aaa tac att acc tca gat gaa ctc aag aac cac gat aaa ccc gga       104
Lys Lys Tyr Ile Thr Ser Asp Glu Leu Lys Asn His Asp Lys Pro Gly
                10                  15                  20

gat cta tgg atc tcg att caa ggg aaa gcc tat gat gtt tcg gat tgg       152
Asp Leu Trp Ile Ser Ile Gln Gly Lys Ala Tyr Asp Val Ser Asp Trp
            25                  30                  35

gtg aaa gac cat cca ggt ggc agc ttt ccc ttg aag agt ctt gct ggt       200
Val Lys Asp His Pro Gly Gly Ser Phe Pro Leu Lys Ser Leu Ala Gly
        40                  45                  50

caa gag gta act gat gca ttt gtt gca ttc cat cct gcc tct aca tgg       248
Gln Glu Val Thr Asp Ala Phe Val Ala Phe His Pro Ala Ser Thr Trp
    55                  60                  65

aag aat ctt gat aag ttt ttc act ggg tat tat ctt aaa gat tac tct       296
Lys Asn Leu Asp Lys Phe Phe Thr Gly Tyr Tyr Leu Lys Asp Tyr Ser
70                  75                  80                  85

gtt tct gag gtt tct aaa gat tat agg aag ctt gtg ttt gag ttt tct       344
Val Ser Glu Val Ser Lys Asp Tyr Arg Lys Leu Val Phe Glu Phe Ser
                90                  95                  100

aaa atg ggt ttg tat gac aaa aaa ggt cat att atg ttt gca act ttg       392
Lys Met Gly Leu Tyr Asp Lys Lys Gly His Ile Met Phe Ala Thr Leu
            105                 110                 115

tgc ttt ata gca atg ctg ttt gct atg agt gtt tat ggg gtt ttg ttt       440
Cys Phe Ile Ala Met Leu Phe Ala Met Ser Val Tyr Gly Val Leu Phe
        120                 125                 130

tgt gag ggt gtt ttg gta cat ttg ttt tct ggg tgt ttg atg ggg ttt       488
Cys Glu Gly Val Leu Val His Leu Phe Ser Gly Cys Leu Met Gly Phe
```

```
        135                 140                 145

ctt tgg att cag agt ggt tgg att gga cat gat gct ggg cat tat atg      536
Leu Trp Ile Gln Ser Gly Trp Ile Gly His Asp Ala Gly His Tyr Met
150                 155                 160                 165

gta gtg tct gat tca agg ctt aat aag ttt atg ggt att ttt gct gca      584
Val Val Ser Asp Ser Arg Leu Asn Lys Phe Met Gly Ile Phe Ala Ala
                170                 175                 180

aat tgt ctt tca gga ata agt att ggt tgg tgg aaa tgg aac cat aat      632
Asn Cys Leu Ser Gly Ile Ser Ile Gly Trp Trp Lys Trp Asn His Asn
            185                 190                 195

gca cat cac att gcc tgt aat agc ctt gaa tat gac cct gat tta caa      680
Ala His His Ile Ala Cys Asn Ser Leu Glu Tyr Asp Pro Asp Leu Gln
        200                 205                 210

tat ata cca ttc ctt gtt gtg tct tcc aag ttt ttt ggt tca ctc acc      728
Tyr Ile Pro Phe Leu Val Val Ser Ser Lys Phe Phe Gly Ser Leu Thr
    215                 220                 225

tct cat ttc tat gag aaa agg ttg act ttt gac tct tta tca aga ttc      776
Ser His Phe Tyr Glu Lys Arg Leu Thr Phe Asp Ser Leu Ser Arg Phe
230                 235                 240                 245

ttt gta agt tat caa cat tgg aca ttt tac cct att atg tgt gct gct      824
Phe Val Ser Tyr Gln His Trp Thr Phe Tyr Pro Ile Met Cys Ala Ala
                250                 255                 260

agg ctc aat atg tat gta caa tct ctc ata atg ttg ttg acc aag aga      872
Arg Leu Asn Met Tyr Val Gln Ser Leu Ile Met Leu Leu Thr Lys Arg
            265                 270                 275

aat gtg tcc tat cga gct cat gaa ctc ttg gga tgc cta gtg ttc tcg      920
Asn Val Ser Tyr Arg Ala His Glu Leu Leu Gly Cys Leu Val Phe Ser
        280                 285                 290

att tgg tac ccg ttg ctt gtt tct tgt ttg cct aat tgg ggt gaa aga      968
Ile Trp Tyr Pro Leu Leu Val Ser Cys Leu Pro Asn Trp Gly Glu Arg
    295                 300                 305

att atg ttt gtt att gca agt ttg tca gtg act gga atg caa caa gtt     1016
Ile Met Phe Val Ile Ala Ser Leu Ser Val Thr Gly Met Gln Gln Val
310                 315                 320                 325

cag ttc tcc ttg aac cac ttc tct tca agt gtt tat gtt gga aag cct     1064
Gln Phe Ser Leu Asn His Phe Ser Ser Ser Val Tyr Val Gly Lys Pro
                330                 335                 340

aaa ggg aat aat tgg ttt gag aaa caa acg gat ggg aca ctt gac att     1112
Lys Gly Asn Asn Trp Phe Glu Lys Gln Thr Asp Gly Thr Leu Asp Ile
            345                 350                 355

tct tgt cct cct tgg atg gat tgg ttt cat ggt gga ttg caa ttc caa     1160
Ser Cys Pro Pro Trp Met Asp Trp Phe His Gly Gly Leu Gln Phe Gln
        360                 365                 370

att gag cat cat ttg ttt ccc aag atg cct aga tgc aac ctt agg aaa     1208
Ile Glu His His Leu Phe Pro Lys Met Pro Arg Cys Asn Leu Arg Lys
    375                 380                 385

atc tcg ccc tac gtg atc gag tta tgc aag aaa cat aat ttg cct tac     1256
```

```
Ile Ser Pro Tyr Val Ile Glu Leu Cys Lys Lys His Asn Leu Pro Tyr
390                 395                 400                 405

aat tat gca tct ttc tcc aag gcc aat gaa atg aca ctc aga aca ttg      1304
Asn Tyr Ala Ser Phe Ser Lys Ala Asn Glu Met Thr Leu Arg Thr Leu
                410                 415                 420

agg aac aca gca ttg cag gct agg gat ata acc aag ccg ctc ccg aag      1352
Arg Asn Thr Ala Leu Gln Ala Arg Asp Ile Thr Lys Pro Leu Pro Lys
            425                 430                 435

aat ttg gta tgg gaa gct ctt cac act cat ggt taa aattaccctt          1398
Asn Leu Val Trp Glu Ala Leu His Thr His Gly
        440                 445

agttcatgta ataatttgag attatgtatc tcctatgttt gtgtcttgtc ttggttctac   1458

ttgttggagt cattgcaact tgtcttttat ggtttattag atgtttttta atatatttta   1518

gaggttttgc tttcatctcc attattgatg aataaggagt tgcatattgt caattgttgt   1578

gctcaatatc tgatattttg gaatgtactt tgtaccactg tgttttcagt tgaagctcat   1638

gtgtacttct atagactttg tttaaatggt tatgaaaaaa aaaaaaaaa               1687


<210>  18
<211>  448
<212>  PRT
<213>  Borago officinalis

<400>  18

Met Ala Ala Gln Ile Lys Lys Tyr Ile Thr Ser Asp Glu Leu Lys Asn
1               5                   10                  15


His Asp Lys Pro Gly Asp Leu Trp Ile Ser Ile Gln Gly Lys Ala Tyr
            20                  25                  30


Asp Val Ser Asp Trp Val Lys Asp His Pro Gly Gly Ser Phe Pro Leu
        35                  40                  45


Lys Ser Leu Ala Gly Gln Glu Val Thr Asp Ala Phe Val Ala Phe His
    50                  55                  60


Pro Ala Ser Thr Trp Lys Asn Leu Asp Lys Phe Phe Thr Gly Tyr Tyr
65                  70                  75                  80


Leu Lys Asp Tyr Ser Val Ser Glu Val Ser Lys Asp Tyr Arg Lys Leu
                85                  90                  95


Val Phe Glu Phe Ser Lys Met Gly Leu Tyr Asp Lys Lys Gly His Ile
            100                 105                 110


Met Phe Ala Thr Leu Cys Phe Ile Ala Met Leu Phe Ala Met Ser Val
        115                 120                 125
```

Tyr Gly Val Leu Phe Cys Glu Gly Val Leu Val His Leu Phe Ser Gly
130 135 140

Cys Leu Met Gly Phe Leu Trp Ile Gln Ser Gly Trp Ile Gly His Asp
145 150 155 160

Ala Gly His Tyr Met Val Val Ser Asp Ser Arg Leu Asn Lys Phe Met
165 170 175

Gly Ile Phe Ala Ala Asn Cys Leu Ser Gly Ile Ser Ile Gly Trp Trp
180 185 190

Lys Trp Asn His Asn Ala His His Ile Ala Cys Asn Ser Leu Glu Tyr
195 200 205

Asp Pro Asp Leu Gln Tyr Ile Pro Phe Leu Val Val Ser Ser Lys Phe
210 215 220

Phe Gly Ser Leu Thr Ser His Phe Tyr Glu Lys Arg Leu Thr Phe Asp
225 230 235 240

Ser Leu Ser Arg Phe Phe Val Ser Tyr Gln His Trp Thr Phe Tyr Pro
245 250 255

Ile Met Cys Ala Ala Arg Leu Asn Met Tyr Val Gln Ser Leu Ile Met
260 265 270

Leu Leu Thr Lys Arg Asn Val Ser Tyr Arg Ala His Glu Leu Leu Gly
275 280 285

Cys Leu Val Phe Ser Ile Trp Tyr Pro Leu Leu Val Ser Cys Leu Pro
290 295 300

Asn Trp Gly Glu Arg Ile Met Phe Val Ile Ala Ser Leu Ser Val Thr
305 310 315 320

Gly Met Gln Gln Val Gln Phe Ser Leu Asn His Phe Ser Ser Ser Val
325 330 335

Tyr Val Gly Lys Pro Lys Gly Asn Asn Trp Phe Glu Lys Gln Thr Asp
340 345 350

Gly Thr Leu Asp Ile Ser Cys Pro Pro Trp Met Asp Trp Phe His Gly
355 360 365

Gly Leu Gln Phe Gln Ile Glu His His Leu Phe Pro Lys Met Pro Arg

```
    370                 375                 380

Cys Asn Leu Arg Lys Ile Ser Pro Tyr Val Ile Glu Leu Cys Lys Lys
385                 390                 395                 400

His Asn Leu Pro Tyr Asn Tyr Ala Ser Phe Ser Lys Ala Asn Glu Met
                405                 410                 415

Thr Leu Arg Thr Leu Arg Asn Thr Ala Leu Gln Ala Arg Asp Ile Thr
            420                 425                 430

Lys Pro Leu Pro Lys Asn Leu Val Trp Glu Ala Leu His Thr His Gly
        435                 440                 445


<210>  19
<211>  2040
<212>  DNA
<213>  Ceratodon purpureus

<220>
<221>  CDS
<222>  (176)..(1627)
<223>  Delta-6-Desaturase


<400>  19
ctcaggcagg tctcagttga tgagacgctg agttctgaat cctttgagct gtgtcaggct      60

cggcacttgt gggatggtga aggagtgatc gatcaggagt gcaggagctg cattagtttc     120

tcagggtcga tcaggttatt ctgaaaaagg ctgcgtctgt gagcagtttg caaaa atg      178
                                                             Met
                                                             1

gcc ctc gtt acc gac ttt ctg aac ttt ctg ggc acg aca tgg agc aag       226
Ala Leu Val Thr Asp Phe Leu Asn Phe Leu Gly Thr Thr Trp Ser Lys
            5                   10                  15

tac agc gtg tac acc cat agc tat gct gga aac tat ggg cct act ttg       274
Tyr Ser Val Tyr Thr His Ser Tyr Ala Gly Asn Tyr Gly Pro Thr Leu
        20                  25                  30

aag cac gcc aaa aag gtt tct gct caa ggt aaa act gcg gga cag aca       322
Lys His Ala Lys Lys Val Ser Ala Gln Gly Lys Thr Ala Gly Gln Thr
    35                  40                  45

ctg aga cag aga tcg gtg cag gac aaa aag cca ggc act tac tct ctg       370
Leu Arg Gln Arg Ser Val Gln Asp Lys Lys Pro Gly Thr Tyr Ser Leu
50                  55                  60                  65

gcc gat gtt gct tct cac gac agg cct gga gac tgc tgg atg atc gtc       418
Ala Asp Val Ala Ser His Asp Arg Pro Gly Asp Cys Trp Met Ile Val
                70                  75                  80

aaa gag aag gtg tat gat att agc cgt ttt gcg gac gac cac cct gga       466
Lys Glu Lys Val Tyr Asp Ile Ser Arg Phe Ala Asp Asp His Pro Gly
            85                  90                  95
```

```
ggg acg gta att agc acc tac ttt ggg cgg gat ggc aca gac gtt ttc       514
Gly Thr Val Ile Ser Thr Tyr Phe Gly Arg Asp Gly Thr Asp Val Phe
        100                 105                 110

gca aca ttc cat cca cct gcc gca tgg aag caa ctc aat gac tac tac       562
Ala Thr Phe His Pro Pro Ala Ala Trp Lys Gln Leu Asn Asp Tyr Tyr
    115                 120                 125

att gga gac ctt gct agg gaa gag ccc ctt gat gaa ttg ctt aaa gac       610
Ile Gly Asp Leu Ala Arg Glu Glu Pro Leu Asp Glu Leu Leu Lys Asp
130                 135                 140                 145

tac aga gat atg aga gcc gag ttt gtt aga gaa ggg ctt ttc aag agt       658
Tyr Arg Asp Met Arg Ala Glu Phe Val Arg Glu Gly Leu Phe Lys Ser
                150                 155                 160

tcc aag gcc tgg ttc ctg ctt cag act ctg att aat gca gct ctc ttt       706
Ser Lys Ala Trp Phe Leu Leu Gln Thr Leu Ile Asn Ala Ala Leu Phe
            165                 170                 175

gct gcg agc att gcg act atc tgt tac gac aag agt tac tgg gct att       754
Ala Ala Ser Ile Ala Thr Ile Cys Tyr Asp Lys Ser Tyr Trp Ala Ile
        180                 185                 190

gtg ctg tca gcc agt ttg atg ggt ctc ttc gtc caa cag tgt gga tgg       802
Val Leu Ser Ala Ser Leu Met Gly Leu Phe Val Gln Gln Cys Gly Trp
    195                 200                 205

ctt gcc cat gat ttc ctt cat caa cag gtc ttt gag aac cgt acc gcg       850
Leu Ala His Asp Phe Leu His Gln Gln Val Phe Glu Asn Arg Thr Ala
210                 215                 220                 225

aac tcc ttc ttt ggc tat ttg ttc ggc aat tgc gtg ctt ggc ttt agt       898
Asn Ser Phe Phe Gly Tyr Leu Phe Gly Asn Cys Val Leu Gly Phe Ser
                230                 235                 240

gta tca tgg tgg agg acg aag cac aac att cat cat act gct ccg aat       946
Val Ser Trp Trp Arg Thr Lys His Asn Ile His His Thr Ala Pro Asn
            245                 250                 255

gag tgc gac gaa cag tac aca cct cta gac gaa gac att gat act ctc       994
Glu Cys Asp Glu Gln Tyr Thr Pro Leu Asp Glu Asp Ile Asp Thr Leu
        260                 265                 270

ccc atc att gcc tgg agc aag gaa att ttg gcc acc gtt gag agc aag      1042
Pro Ile Ile Ala Trp Ser Lys Glu Ile Leu Ala Thr Val Glu Ser Lys
    275                 280                 285

aga att ttg cga gtg ctt caa tat cag cac tac atg att ctg cct cta      1090
Arg Ile Leu Arg Val Leu Gln Tyr Gln His Tyr Met Ile Leu Pro Leu
290                 295                 300                 305

ttg ttc atg gcc cgg tac agt tgg act ttt gga agt ttg ctc ttc aca      1138
Leu Phe Met Ala Arg Tyr Ser Trp Thr Phe Gly Ser Leu Leu Phe Thr
                310                 315                 320

ttc aat cct gat ttg agc acg acc aag gga ttg ata gag aag gga aca      1186
Phe Asn Pro Asp Leu Ser Thr Thr Lys Gly Leu Ile Glu Lys Gly Thr
            325                 330                 335

gtt gct ttt cac tac gcc tgg ttc agt tgg gct gcg ttc cat att ttg      1234
Val Ala Phe His Tyr Ala Trp Phe Ser Trp Ala Ala Phe His Ile Leu
        340                 345                 350
```

```
ccg ggt gtc gct aag cct ctt gcg tgg atg gta gca act gag ctt gtg      1282
Pro Gly Val Ala Lys Pro Leu Ala Trp Met Val Ala Thr Glu Leu Val
    355                 360                 365

gcc ggt ttg ttg ttg gga ttc gtg ttt acg ttg agt cac aat gga aag      1330
Ala Gly Leu Leu Leu Gly Phe Val Phe Thr Leu Ser His Asn Gly Lys
370                 375                 380                 385

gag gtt tac aat gaa tcg aag gac ttc gtg aga gcc cag gtt att acc      1378
Glu Val Tyr Asn Glu Ser Lys Asp Phe Val Arg Ala Gln Val Ile Thr
                390                 395                 400

acc cgt aac acc aag cga ggc tgg ttc aac gat tgg ttc act ggg gga      1426
Thr Arg Asn Thr Lys Arg Gly Trp Phe Asn Asp Trp Phe Thr Gly Gly
            405                 410                 415

ctc gac acc cag att gag cat cac ctg ttt cca aca atg ccc agg cac      1474
Leu Asp Thr Gln Ile Glu His His Leu Phe Pro Thr Met Pro Arg His
        420                 425                 430

aac tac ccc aag atc gca cct cag gtc gag gct ctt tgc aag aag cac      1522
Asn Tyr Pro Lys Ile Ala Pro Gln Val Glu Ala Leu Cys Lys Lys His
    435                 440                 445

ggc ctc gag tac gat aat gtc tcc gtc gtt ggt gcc tct gtc gcg gtt      1570
Gly Leu Glu Tyr Asp Asn Val Ser Val Val Gly Ala Ser Val Ala Val
450                 455                 460                 465

gtg aag gcg ctc aag gaa att gct gat gaa gcg tca att cgg ctt cac      1618
Val Lys Ala Leu Lys Glu Ile Ala Asp Glu Ala Ser Ile Arg Leu His
                470                 475                 480

gct cac taa gaaatcgtcg aactttgact attcattttt ttcgcctggc              1667
Ala His

tacctcaaat gttcgggagc aggtgcttgg cagtgtgttc aaccggagcg cactgaaaat   1727

gtgcagaatc catttccaga aattaccatt cctagctaaa tcttcttttt accaggtcgg   1787

atatatgaaa ctttttttgat gcaacaagta gcattcaatt gaagacattg ttcgagatat  1847

aattcgcagt gtttctattc agcgggcata cgtactagtc catatcggcg gttgccgaga   1907

gtttacatta ttagttggca caacgagtag atctagtgta aatttctatt tccgcatgta   1967

atattactct gaatatatac cgttatctat tttcctaaaa aaaaaaaaaa aaaaaaaaaa   2027

aaaaaaaaaa aaa                                                      2040
```

```
<210>  20
<211>  483
<212>  PRT
<213>  Ceratodon purpureus

<400>  20

Met Ala Leu Val Thr Asp Phe Leu Asn Phe Leu Gly Thr Thr Trp Ser
1               5                   10                  15
```

Lys Tyr Ser Val Tyr Thr His Ser Tyr Ala Gly Asn Tyr Gly Pro Thr
20 25 30

Leu Lys His Ala Lys Lys Val Ser Ala Gln Gly Lys Thr Ala Gly Gln
35 40 45

Thr Leu Arg Gln Arg Ser Val Gln Asp Lys Lys Pro Gly Thr Tyr Ser
50 55 60

Leu Ala Asp Val Ala Ser His Asp Arg Pro Gly Asp Cys Trp Met Ile
65 70 75 80

Val Lys Glu Lys Val Tyr Asp Ile Ser Arg Phe Ala Asp Asp His Pro
85 90 95

Gly Gly Thr Val Ile Ser Thr Tyr Phe Gly Arg Asp Gly Thr Asp Val
100 105 110

Phe Ala Thr Phe His Pro Pro Ala Ala Trp Lys Gln Leu Asn Asp Tyr
115 120 125

Tyr Ile Gly Asp Leu Ala Arg Glu Glu Pro Leu Asp Glu Leu Leu Lys
130 135 140

Asp Tyr Arg Asp Met Arg Ala Glu Phe Val Arg Glu Gly Leu Phe Lys
145 150 155 160

Ser Ser Lys Ala Trp Phe Leu Leu Gln Thr Leu Ile Asn Ala Ala Leu
165 170 175

Phe Ala Ala Ser Ile Ala Thr Ile Cys Tyr Asp Lys Ser Tyr Trp Ala
180 185 190

Ile Val Leu Ser Ala Ser Leu Met Gly Leu Phe Val Gln Gln Cys Gly
195 200 205

Trp Leu Ala His Asp Phe Leu His Gln Gln Val Phe Glu Asn Arg Thr
210 215 220

Ala Asn Ser Phe Phe Gly Tyr Leu Phe Gly Asn Cys Val Leu Gly Phe
225 230 235 240

Ser Val Ser Trp Trp Arg Thr Lys His Asn Ile His His Thr Ala Pro
245 250 255

Asn Glu Cys Asp Glu Gln Tyr Thr Pro Leu Asp Glu Asp Ile Asp Thr
260 265 270

Leu Pro Ile Ile Ala Trp Ser Lys Glu Ile Leu Ala Thr Val Glu Ser
275 280 285

Lys Arg Ile Leu Arg Val Leu Gln Tyr Gln His Tyr Met Ile Leu Pro
290 295 300

Leu Leu Phe Met Ala Arg Tyr Ser Trp Thr Phe Gly Ser Leu Leu Phe
305 310 315 320

Thr Phe Asn Pro Asp Leu Ser Thr Thr Lys Gly Leu Ile Glu Lys Gly
325 330 335

Thr Val Ala Phe His Tyr Ala Trp Phe Ser Trp Ala Ala Phe His Ile
340 345 350

Leu Pro Gly Val Ala Lys Pro Leu Ala Trp Met Val Ala Thr Glu Leu
355 360 365

Val Ala Gly Leu Leu Leu Gly Phe Val Phe Thr Leu Ser His Asn Gly
370 375 380

Lys Glu Val Tyr Asn Glu Ser Lys Asp Phe Val Arg Ala Gln Val Ile
385 390 395 400

Thr Thr Arg Asn Thr Lys Arg Gly Trp Phe Asn Asp Trp Phe Thr Gly
405 410 415

Gly Leu Asp Thr Gln Ile Glu His His Leu Phe Pro Thr Met Pro Arg
420 425 430

His Asn Tyr Pro Lys Ile Ala Pro Gln Val Glu Ala Leu Cys Lys Lys
435 440 445

His Gly Leu Glu Tyr Asp Asn Val Ser Val Val Gly Ala Ser Val Ala
450 455 460

Val Val Lys Ala Leu Lys Glu Ile Ala Asp Glu Ala Ser Ile Arg Leu
465 470 475 480

His Ala His

<210> 21
<211> 1434
<212> DNA
<213> Phaeodactylum tricornutum

<220>

<221> CDS
<222> (1)..(1434)
<223> Delta-6-Desaturase

<400> 21

```
atg ggc aaa gga ggg gac gct cgg gcc tcg aag ggc tca acg gcg gct      48
Met Gly Lys Gly Gly Asp Ala Arg Ala Ser Lys Gly Ser Thr Ala Ala
1               5                   10                  15

cgc aag atc agt tgg cag gaa gtc aag acc cac gcg tct ccg gag gac      96
Arg Lys Ile Ser Trp Gln Glu Val Lys Thr His Ala Ser Pro Glu Asp
            20                  25                  30

gcc tgg atc att cac tcc aat aag gtc tac gac gtg tcc aac tgg cac     144
Ala Trp Ile Ile His Ser Asn Lys Val Tyr Asp Val Ser Asn Trp His
        35                  40                  45

gaa cat ccc gga ggc gcc gtc att ttc acg cac gcc ggt gac gac atg     192
Glu His Pro Gly Gly Ala Val Ile Phe Thr His Ala Gly Asp Asp Met
    50                  55                  60

acg gac att ttc gct gcc ttt cac gca ccc gga tcg cag tcg ctc atg     240
Thr Asp Ile Phe Ala Ala Phe His Ala Pro Gly Ser Gln Ser Leu Met
65                  70                  75                  80

aag aag ttc tac att ggc gaa ttg ctc ccg gaa acc acc ggc aag gag     288
Lys Lys Phe Tyr Ile Gly Glu Leu Leu Pro Glu Thr Thr Gly Lys Glu
                85                  90                  95

ccg cag caa atc gcc ttt gaa aag ggc tac cgc gat ctg cgc tcc aaa     336
Pro Gln Gln Ile Ala Phe Glu Lys Gly Tyr Arg Asp Leu Arg Ser Lys
            100                 105                 110

ctc atc atg atg ggc atg ttc aag tcc aac aag tgg ttc tac gtc tac     384
Leu Ile Met Met Gly Met Phe Lys Ser Asn Lys Trp Phe Tyr Val Tyr
        115                 120                 125

aag tgc ctc agc aac atg gcc att tgg gcc gcc gcc tgt gct ctc gtc     432
Lys Cys Leu Ser Asn Met Ala Ile Trp Ala Ala Ala Cys Ala Leu Val
    130                 135                 140

ttt tac tcg gac cgc ttc tgg gta cac ctg gcc agc gcc gtc atg ctg     480
Phe Tyr Ser Asp Arg Phe Trp Val His Leu Ala Ser Ala Val Met Leu
145                 150                 155                 160

gga aca ttc ttt cag cag tcg gga tgg ttg gca cac gac ttt ctg cac     528
Gly Thr Phe Phe Gln Gln Ser Gly Trp Leu Ala His Asp Phe Leu His
                165                 170                 175

cac cag gtc ttc acc aag cgc aag cac ggg gat ctc gga gga ctc ttt     576
His Gln Val Phe Thr Lys Arg Lys His Gly Asp Leu Gly Gly Leu Phe
            180                 185                 190

tgg ggg aac ctc atg cag ggt tac tcc gta cag tgg tgg aaa aac aag     624
Trp Gly Asn Leu Met Gln Gly Tyr Ser Val Gln Trp Trp Lys Asn Lys
        195                 200                 205

cac aac gga cac cac gcc gtc ccc aac ctc cac tgc tcc tcc gca gtc     672
His Asn Gly His His Ala Val Pro Asn Leu His Cys Ser Ser Ala Val
    210                 215                 220

gcg caa gat ggg gac ccg gac atc gat acc atg ccc ctt ctc gcc tgg     720
```

```
Ala Gln Asp Gly Asp Pro Asp Ile Asp Thr Met Pro Leu Leu Ala Trp
225                 230                 235                 240

tcc gtc cag caa gcc cag tct tac cgg gaa ctc caa gcc gac gga aag      768
Ser Val Gln Gln Ala Gln Ser Tyr Arg Glu Leu Gln Ala Asp Gly Lys
                245                 250                 255

gat tcg ggt ttg gtc aag ttc atg atc cgt aac caa tcc tac ttt tac      816
Asp Ser Gly Leu Val Lys Phe Met Ile Arg Asn Gln Ser Tyr Phe Tyr
            260                 265                 270

ttt ccc atc ttg ttg ctc gcc cgc ctg tcg tgg ttg aac gag tcc ttc      864
Phe Pro Ile Leu Leu Leu Ala Arg Leu Ser Trp Leu Asn Glu Ser Phe
        275                 280                 285

aag tgc gcc ttt ggg ctt gga gct gcg tcg gag aac gct gct ctc gaa      912
Lys Cys Ala Phe Gly Leu Gly Ala Ala Ser Glu Asn Ala Ala Leu Glu
    290                 295                 300

ctc aag gcc aag ggt ctt cag tac ccc ctt ttg gaa aag gct ggc atc      960
Leu Lys Ala Lys Gly Leu Gln Tyr Pro Leu Leu Glu Lys Ala Gly Ile
305                 310                 315                 320

ctg ctg cac tac gct tgg atg ctt aca gtt tcg tcc ggc ttt gga cgc     1008
Leu Leu His Tyr Ala Trp Met Leu Thr Val Ser Ser Gly Phe Gly Arg
                325                 330                 335

ttc tcg ttc gcg tac acc gca ttt tac ttt cta acc gcg acc gcg tcc     1056
Phe Ser Phe Ala Tyr Thr Ala Phe Tyr Phe Leu Thr Ala Thr Ala Ser
            340                 345                 350

tgt gga ttc ttg ctc gcc att gtc ttt ggc ctc ggc cac aac ggc atg     1104
Cys Gly Phe Leu Leu Ala Ile Val Phe Gly Leu Gly His Asn Gly Met
        355                 360                 365

gcc acc tac aat gcc gac gcc cgt ccg gac ttc tgg aag ctc caa gtc     1152
Ala Thr Tyr Asn Ala Asp Ala Arg Pro Asp Phe Trp Lys Leu Gln Val
    370                 375                 380

acc acg act cgc aac gtc acg ggc gga cac ggt ttc ccc caa gcc ttt     1200
Thr Thr Thr Arg Asn Val Thr Gly Gly His Gly Phe Pro Gln Ala Phe
385                 390                 395                 400

gtc gac tgg ttc tgt ggt ggc ctc cag tac caa gtc gac cac cac tta     1248
Val Asp Trp Phe Cys Gly Gly Leu Gln Tyr Gln Val Asp His His Leu
                405                 410                 415

ttc ccc agc ctg ccc cga cac aat ctg gcc aag aca cac gca ctg gtc     1296
Phe Pro Ser Leu Pro Arg His Asn Leu Ala Lys Thr His Ala Leu Val
            420                 425                 430

gaa tcg ttc tgc aag gag tgg ggt gtc cag tac cac gaa gcc gac ctt     1344
Glu Ser Phe Cys Lys Glu Trp Gly Val Gln Tyr His Glu Ala Asp Leu
        435                 440                 445

gtg gac ggg acc atg gaa gtc ttg cac cat ttg ggc agc gtg gcc ggc     1392
Val Asp Gly Thr Met Glu Val Leu His His Leu Gly Ser Val Ala Gly
    450                 455                 460

gaa ttc gtc gtg gat ttt gta cgc gat gga ccc gcc atg taa             1434
Glu Phe Val Val Asp Phe Val Arg Asp Gly Pro Ala Met
465                 470                 475
```

<210> 22
<211> 477
<212> PRT
<213> Phaeodactylum tricornutum

<400> 22

Met Gly Lys Gly Gly Asp Ala Arg Ala Ser Lys Gly Ser Thr Ala Ala
1 5 10 15

Arg Lys Ile Ser Trp Gln Glu Val Lys Thr His Ala Ser Pro Glu Asp
20 25 30

Ala Trp Ile Ile His Ser Asn Lys Val Tyr Asp Val Ser Asn Trp His
35 40 45

Glu His Pro Gly Gly Ala Val Ile Phe Thr His Ala Gly Asp Asp Met
50 55 60

Thr Asp Ile Phe Ala Ala Phe His Ala Pro Gly Ser Gln Ser Leu Met
65 70 75 80

Lys Lys Phe Tyr Ile Gly Glu Leu Leu Pro Glu Thr Thr Gly Lys Glu
85 90 95

Pro Gln Gln Ile Ala Phe Glu Lys Gly Tyr Arg Asp Leu Arg Ser Lys
100 105 110

Leu Ile Met Met Gly Met Phe Lys Ser Asn Lys Trp Phe Tyr Val Tyr
115 120 125

Lys Cys Leu Ser Asn Met Ala Ile Trp Ala Ala Ala Cys Ala Leu Val
130 135 140

Phe Tyr Ser Asp Arg Phe Trp Val His Leu Ala Ser Ala Val Met Leu
145 150 155 160

Gly Thr Phe Phe Gln Gln Ser Gly Trp Leu Ala His Asp Phe Leu His
165 170 175

His Gln Val Phe Thr Lys Arg Lys His Gly Asp Leu Gly Gly Leu Phe
180 185 190

Trp Gly Asn Leu Met Gln Gly Tyr Ser Val Gln Trp Trp Lys Asn Lys
195 200 205

His Asn Gly His His Ala Val Pro Asn Leu His Cys Ser Ser Ala Val
210 215 220

```
Ala Gln Asp Gly Asp Pro Asp Ile Asp Thr Met Pro Leu Leu Ala Trp
225                 230                 235                 240

Ser Val Gln Gln Ala Gln Ser Tyr Arg Glu Leu Gln Ala Asp Gly Lys
                245                 250                 255

Asp Ser Gly Leu Val Lys Phe Met Ile Arg Asn Gln Ser Tyr Phe Tyr
            260                 265                 270

Phe Pro Ile Leu Leu Leu Ala Arg Leu Ser Trp Leu Asn Glu Ser Phe
        275                 280                 285

Lys Cys Ala Phe Gly Leu Gly Ala Ala Ser Glu Asn Ala Ala Leu Glu
    290                 295                 300

Leu Lys Ala Lys Gly Leu Gln Tyr Pro Leu Leu Glu Lys Ala Gly Ile
305                 310                 315                 320

Leu Leu His Tyr Ala Trp Met Leu Thr Val Ser Ser Gly Phe Gly Arg
                325                 330                 335

Phe Ser Phe Ala Tyr Thr Ala Phe Tyr Phe Leu Thr Ala Thr Ala Ser
            340                 345                 350

Cys Gly Phe Leu Leu Ala Ile Val Phe Gly Leu Gly His Asn Gly Met
        355                 360                 365

Ala Thr Tyr Asn Ala Asp Ala Arg Pro Asp Phe Trp Lys Leu Gln Val
    370                 375                 380

Thr Thr Thr Arg Asn Val Thr Gly Gly His Gly Phe Pro Gln Ala Phe
385                 390                 395                 400

Val Asp Trp Phe Cys Gly Gly Leu Gln Tyr Gln Val Asp His His Leu
                405                 410                 415

Phe Pro Ser Leu Pro Arg His Asn Leu Ala Lys Thr His Ala Leu Val
            420                 425                 430

Glu Ser Phe Cys Lys Glu Trp Gly Val Gln Tyr His Glu Ala Asp Leu
        435                 440                 445

Val Asp Gly Thr Met Glu Val Leu His His Leu Gly Ser Val Ala Gly
    450                 455                 460

Glu Phe Val Val Asp Phe Val Arg Asp Gly Pro Ala Met
465                 470                 475
```

<210> 23
<211> 1578
<212> DNA
<213> Physcomitrella patens

<220>
<221> CDS
<222> (1)..(1578)
<223> Delta-6-Desaturase

<400> 23
```
atg gta ttc gcg ggc ggt gga ctt cag cag ggc tct ctc gaa gaa aac      48
Met Val Phe Ala Gly Gly Gly Leu Gln Gln Gly Ser Leu Glu Glu Asn
1               5                   10                  15

atc gac gtc gag cac att gcc agt atg tct ctc ttc agc gac ttc ttc      96
Ile Asp Val Glu His Ile Ala Ser Met Ser Leu Phe Ser Asp Phe Phe
            20                  25                  30

agt tat gtg tct tca act gtt ggt tcg tgg agc gta cac agt ata caa      144
Ser Tyr Val Ser Ser Thr Val Gly Ser Trp Ser Val His Ser Ile Gln
        35                  40                  45

cct ttg aag cgc ctg acg agt aag aag cgt gtt tcg gaa agc gct gcc      192
Pro Leu Lys Arg Leu Thr Ser Lys Lys Arg Val Ser Glu Ser Ala Ala
    50                  55                  60

gtg caa tgt ata tca gct gaa gtt cag aga aat tcg agt acc cag gga      240
Val Gln Cys Ile Ser Ala Glu Val Gln Arg Asn Ser Ser Thr Gln Gly
65                  70                  75                  80

act gcg gag gca ctc gca gaa tca gtc gtg aag ccc acg aga cga agg      288
Thr Ala Glu Ala Leu Ala Glu Ser Val Val Lys Pro Thr Arg Arg Arg
                85                  90                  95

tca tct cag tgg aag aag tcg aca cac ccc cta tca gaa gta gca gta      336
Ser Ser Gln Trp Lys Lys Ser Thr His Pro Leu Ser Glu Val Ala Val
            100                 105                 110

cac aac aag cca agc gat tgc tgg att gtt gta aaa aac aag gtg tat      384
His Asn Lys Pro Ser Asp Cys Trp Ile Val Val Lys Asn Lys Val Tyr
        115                 120                 125

gat gtt tcc aat ttt gcg gac gag cat ccc gga gga tca gtt att agt      432
Asp Val Ser Asn Phe Ala Asp Glu His Pro Gly Gly Ser Val Ile Ser
    130                 135                 140

act tat ttt gga cga gac ggc aca gat gtt ttc tct agt ttt cat gca      480
Thr Tyr Phe Gly Arg Asp Gly Thr Asp Val Phe Ser Ser Phe His Ala
145                 150                 155                 160

gct tct aca tgg aaa att ctt caa gac ttt tac att ggt gac gtg gag      528
Ala Ser Thr Trp Lys Ile Leu Gln Asp Phe Tyr Ile Gly Asp Val Glu
                165                 170                 175

agg gtg gag ccg act cca gag ctg ctg aaa gat ttc cga gaa atg aga      576
Arg Val Glu Pro Thr Pro Glu Leu Leu Lys Asp Phe Arg Glu Met Arg
            180                 185                 190

gct ctt ttc ctg agg gag caa ctt ttc aaa agt tcg aaa ttg tac tat      624
```

```
Ala Leu Phe Leu Arg Glu Gln Leu Phe Lys Ser Ser Lys Leu Tyr Tyr
        195                 200                 205

gtt atg aag ctg ctc acg aat gtt gct att ttt gct gcg agc att gca      672
Val Met Lys Leu Leu Thr Asn Val Ala Ile Phe Ala Ala Ser Ile Ala
    210                 215                 220

ata ata tgt tgg agc aag act att tca gcg gtt ttg gct tca gct tgt      720
Ile Ile Cys Trp Ser Lys Thr Ile Ser Ala Val Leu Ala Ser Ala Cys
225                 230                 235                 240

atg atg gct ctg tgt ttc caa cag tgc gga tgg cta tcc cat gat ttt      768
Met Met Ala Leu Cys Phe Gln Gln Cys Gly Trp Leu Ser His Asp Phe
                245                 250                 255

ctc cac aat cag gtg ttt gag aca cgc tgg ctt aat gaa gtt gtc ggg      816
Leu His Asn Gln Val Phe Glu Thr Arg Trp Leu Asn Glu Val Val Gly
            260                 265                 270

tat gtg atc ggc aac gcc gtt ctg ggg ttt agt aca ggg tgg tgg aag      864
Tyr Val Ile Gly Asn Ala Val Leu Gly Phe Ser Thr Gly Trp Trp Lys
        275                 280                 285

gag aag cat aac ctt cat cat gct gct cca aat gaa tgc gat cag act      912
Glu Lys His Asn Leu His His Ala Ala Pro Asn Glu Cys Asp Gln Thr
    290                 295                 300

tac caa cca att gat gaa gat att gat act ctc ccc ctc att gcc tgg      960
Tyr Gln Pro Ile Asp Glu Asp Ile Asp Thr Leu Pro Leu Ile Ala Trp
305                 310                 315                 320

agc aag gac ata ctg gcc aca gtt gag aat aag aca ttc ttg cga atc     1008
Ser Lys Asp Ile Leu Ala Thr Val Glu Asn Lys Thr Phe Leu Arg Ile
                325                 330                 335

ctc caa tac cag cat ctg ttc ttc atg ggt ctg tta ttt ttc gcc cgt     1056
Leu Gln Tyr Gln His Leu Phe Phe Met Gly Leu Leu Phe Phe Ala Arg
            340                 345                 350

ggt agt tgg ctc ttt tgg agc tgg aga tat acc tct aca gca gtg ctc     1104
Gly Ser Trp Leu Phe Trp Ser Trp Arg Tyr Thr Ser Thr Ala Val Leu
        355                 360                 365

tca cct gtc gac agg ttg ttg gag aag gga act gtt ctg ttt cac tac     1152
Ser Pro Val Asp Arg Leu Leu Glu Lys Gly Thr Val Leu Phe His Tyr
    370                 375                 380

ttt tgg ttc gtc ggg aca gcg tgc tat ctt ctc cct ggt tgg aag cca     1200
Phe Trp Phe Val Gly Thr Ala Cys Tyr Leu Leu Pro Gly Trp Lys Pro
385                 390                 395                 400

tta gta tgg atg gcg gtg act gag ctc atg tcc ggc atg ctg ctg ggc     1248
Leu Val Trp Met Ala Val Thr Glu Leu Met Ser Gly Met Leu Leu Gly
                405                 410                 415

ttt gta ttt gta ctt agc cac aat ggg atg gag gtt tat aat tcg tct     1296
Phe Val Phe Val Leu Ser His Asn Gly Met Glu Val Tyr Asn Ser Ser
            420                 425                 430

aaa gaa ttc gtg agt gca cag atc gta tcc aca cgg gat atc aaa gga     1344
Lys Glu Phe Val Ser Ala Gln Ile Val Ser Thr Arg Asp Ile Lys Gly
        435                 440                 445
```

```
aac ata ttc aac gac tgg ttc act ggt ggc ctt aac agg caa ata gag      1392
Asn Ile Phe Asn Asp Trp Phe Thr Gly Gly Leu Asn Arg Gln Ile Glu
    450                 455                 460

cat cat ctt ttc cca aca atg ccc agg cat aat tta aac aaa ata gca      1440
His His Leu Phe Pro Thr Met Pro Arg His Asn Leu Asn Lys Ile Ala
465                 470                 475                 480

cct aga gtg gag gtg ttc tgt aag aaa cac ggt ctg gtg tac gaa gac      1488
Pro Arg Val Glu Val Phe Cys Lys Lys His Gly Leu Val Tyr Glu Asp
                485                 490                 495

gta tct att gct acc ggc act tgc aag gtt ttg aaa gca ttg aag gaa      1536
Val Ser Ile Ala Thr Gly Thr Cys Lys Val Leu Lys Ala Leu Lys Glu
            500                 505                 510

gtc gcg gag gct gcg gca gag cag cat gct acc acc agt taa              1578
Val Ala Glu Ala Ala Ala Glu Gln His Ala Thr Thr Ser
        515                 520                 525


<210>  24
<211>  525
<212>  PRT
<213>  Physcomitrella patens

<400>  24

Met Val Phe Ala Gly Gly Gly Leu Gln Gln Gly Ser Leu Glu Glu Asn
1               5                   10                  15


Ile Asp Val Glu His Ile Ala Ser Met Ser Leu Phe Ser Asp Phe Phe
            20                  25                  30


Ser Tyr Val Ser Ser Thr Val Gly Ser Trp Ser Val His Ser Ile Gln
        35                  40                  45


Pro Leu Lys Arg Leu Thr Ser Lys Lys Arg Val Ser Glu Ser Ala Ala
    50                  55                  60


Val Gln Cys Ile Ser Ala Glu Val Gln Arg Asn Ser Ser Thr Gln Gly
65                  70                  75                  80


Thr Ala Glu Ala Leu Ala Glu Ser Val Val Lys Pro Thr Arg Arg Arg
                85                  90                  95


Ser Ser Gln Trp Lys Lys Ser Thr His Pro Leu Ser Glu Val Ala Val
            100                 105                 110


His Asn Lys Pro Ser Asp Cys Trp Ile Val Val Lys Asn Lys Val Tyr
        115                 120                 125


Asp Val Ser Asn Phe Ala Asp Glu His Pro Gly Gly Ser Val Ile Ser
    130                 135                 140
```

Thr Tyr Phe Gly Arg Asp Gly Thr Asp Val Phe Ser Ser Phe His Ala
145 150 155 160

Ala Ser Thr Trp Lys Ile Leu Gln Asp Phe Tyr Ile Gly Asp Val Glu
165 170 175

Arg Val Glu Pro Thr Pro Glu Leu Leu Lys Asp Phe Arg Glu Met Arg
180 185 190

Ala Leu Phe Leu Arg Glu Gln Leu Phe Lys Ser Ser Lys Leu Tyr Tyr
195 200 205

Val Met Lys Leu Leu Thr Asn Val Ala Ile Phe Ala Ala Ser Ile Ala
210 215 220

Ile Ile Cys Trp Ser Lys Thr Ile Ser Ala Val Leu Ala Ser Ala Cys
225 230 235 240

Met Met Ala Leu Cys Phe Gln Gln Cys Gly Trp Leu Ser His Asp Phe
245 250 255

Leu His Asn Gln Val Phe Glu Thr Arg Trp Leu Asn Glu Val Val Gly
260 265 270

Tyr Val Ile Gly Asn Ala Val Leu Gly Phe Ser Thr Gly Trp Trp Lys
275 280 285

Glu Lys His Asn Leu His His Ala Ala Pro Asn Glu Cys Asp Gln Thr
290 295 300

Tyr Gln Pro Ile Asp Glu Asp Ile Asp Thr Leu Pro Leu Ile Ala Trp
305 310 315 320

Ser Lys Asp Ile Leu Ala Thr Val Glu Asn Lys Thr Phe Leu Arg Ile
325 330 335

Leu Gln Tyr Gln His Leu Phe Phe Met Gly Leu Leu Phe Phe Ala Arg
340 345 350

Gly Ser Trp Leu Phe Trp Ser Trp Arg Tyr Thr Ser Thr Ala Val Leu
355 360 365

Ser Pro Val Asp Arg Leu Leu Glu Lys Gly Thr Val Leu Phe His Tyr
370 375 380

Phe Trp Phe Val Gly Thr Ala Cys Tyr Leu Leu Pro Gly Trp Lys Pro
385 390 395 400

```
Leu Val Trp Met Ala Val Thr Glu Leu Met Ser Gly Met Leu Leu Gly
                405                 410                 415

Phe Val Phe Val Leu Ser His Asn Gly Met Glu Val Tyr Asn Ser Ser
            420                 425                 430

Lys Glu Phe Val Ser Ala Gln Ile Val Ser Thr Arg Asp Ile Lys Gly
        435                 440                 445

Asn Ile Phe Asn Asp Trp Phe Thr Gly Gly Leu Asn Arg Gln Ile Glu
    450                 455                 460

His His Leu Phe Pro Thr Met Pro Arg His Asn Leu Asn Lys Ile Ala
465                 470                 475                 480

Pro Arg Val Glu Val Phe Cys Lys Lys His Gly Leu Val Tyr Glu Asp
                485                 490                 495

Val Ser Ile Ala Thr Gly Thr Cys Lys Val Leu Lys Ala Leu Lys Glu
            500                 505                 510

Val Ala Glu Ala Ala Ala Glu Gln His Ala Thr Thr Ser
        515                 520                 525


<210>  25
<211>  1332
<212>  DNA
<213>  Caenorhabditis elegans

<220>
<221>  CDS
<222>  (1)..(1332)
<223>  Delta-6-Desaturase


<400>  25
atg gtc gtc gac aag aat gcc tcc ggg ctt cga atg aag gtc gat ggc       48
Met Val Val Asp Lys Asn Ala Ser Gly Leu Arg Met Lys Val Asp Gly
1               5                   10                  15

aaa tgg ctc tac ctt agc gag gaa ttg gtg aag aaa cat cca gga gga       96
Lys Trp Leu Tyr Leu Ser Glu Glu Leu Val Lys Lys His Pro Gly Gly
            20                  25                  30

gct gtt att gaa caa tat aga aat tcg gat gct act cat att ttc cac      144
Ala Val Ile Glu Gln Tyr Arg Asn Ser Asp Ala Thr His Ile Phe His
        35                  40                  45

gct ttc cac gaa gga tct tct cag gct tat aag caa ctt gac ctt ctg      192
Ala Phe His Glu Gly Ser Ser Gln Ala Tyr Lys Gln Leu Asp Leu Leu
    50                  55                  60

aaa aag cac gga gag cac gat gaa ttc ctt gag aaa caa ttg gaa aag      240
```

```
Lys Lys His Gly Glu His Asp Glu Phe Leu Glu Lys Gln Leu Glu Lys
65                  70                  75                  80

aga ctt gac aaa gtt gat atc aat gta tca gca tat gat gtc agt gtt      288
Arg Leu Asp Lys Val Asp Ile Asn Val Ser Ala Tyr Asp Val Ser Val
                85                  90                  95

gca caa gaa aag aaa atg gtt gaa tca ttc gaa aaa cta cga cag aag      336
Ala Gln Glu Lys Lys Met Val Glu Ser Phe Glu Lys Leu Arg Gln Lys
            100                 105                 110

ctt cat gat gat gga tta atg aaa gca aat gaa aca tat ttc ctg ttt      384
Leu His Asp Asp Gly Leu Met Lys Ala Asn Glu Thr Tyr Phe Leu Phe
        115                 120                 125

aaa gcg att tca aca ctt tca att atg gca ttt gca ttt tat ctt cag      432
Lys Ala Ile Ser Thr Leu Ser Ile Met Ala Phe Ala Phe Tyr Leu Gln
    130                 135                 140

tat ctt gga tgg tat att act tct gca tgt tta tta gca ctt gca tgg      480
Tyr Leu Gly Trp Tyr Ile Thr Ser Ala Cys Leu Leu Ala Leu Ala Trp
145                 150                 155                 160

caa caa ttc gga tgg tta aca cat gag ttc tgc cat caa cag cca aca      528
Gln Gln Phe Gly Trp Leu Thr His Glu Phe Cys His Gln Gln Pro Thr
                165                 170                 175

aag aac aga cct ttg aat gat act att tct ttg ttc ttt ggt aat ttc      576
Lys Asn Arg Pro Leu Asn Asp Thr Ile Ser Leu Phe Phe Gly Asn Phe
            180                 185                 190

tta caa gga ttt tca aga gat tgg tgg aag gac aag cat aac act cat      624
Leu Gln Gly Phe Ser Arg Asp Trp Trp Lys Asp Lys His Asn Thr His
        195                 200                 205

cac gct gcc aca aat gta att gat cat gac ggt gat atc gac ttg gca      672
His Ala Ala Thr Asn Val Ile Asp His Asp Gly Asp Ile Asp Leu Ala
    210                 215                 220

cca ctt ttc gca ttt att cca gga gat ttg tgc aag tat aag gcc agc      720
Pro Leu Phe Ala Phe Ile Pro Gly Asp Leu Cys Lys Tyr Lys Ala Ser
225                 230                 235                 240

ttt gaa aaa gca att ctc aag att gta cca tat caa cat ctc tat ttc      768
Phe Glu Lys Ala Ile Leu Lys Ile Val Pro Tyr Gln His Leu Tyr Phe
                245                 250                 255

acc gca atg ctt cca atg ctc cgt ttc tca tgg act ggt cag tca gtt      816
Thr Ala Met Leu Pro Met Leu Arg Phe Ser Trp Thr Gly Gln Ser Val
            260                 265                 270

caa tgg gta ttc aaa gag aat caa atg gag tac aag gtc tat caa aga      864
Gln Trp Val Phe Lys Glu Asn Gln Met Glu Tyr Lys Val Tyr Gln Arg
        275                 280                 285

aat gca ttc tgg gag caa gca aca att gtt gga cat tgg gct tgg gta      912
Asn Ala Phe Trp Glu Gln Ala Thr Ile Val Gly His Trp Ala Trp Val
    290                 295                 300

ttc tat caa ttg ttc tta tta cca aca tgg cca ctt cgg gtt gct tat      960
Phe Tyr Gln Leu Phe Leu Leu Pro Thr Trp Pro Leu Arg Val Ala Tyr
305                 310                 315                 320
```

```
ttc att att tca caa atg gga gga ggc ctt ttg att gct cac gta gtc      1008
Phe Ile Ile Ser Gln Met Gly Gly Gly Leu Leu Ile Ala His Val Val
                325                 330                 335

act ttc aac cat aac tct gtt gat aag tat cca gcc aat tct cga att      1056
Thr Phe Asn His Asn Ser Val Asp Lys Tyr Pro Ala Asn Ser Arg Ile
            340                 345                 350

tta aac aac ttc gcc gct ctt caa att ttg acc aca cgc aac atg act      1104
Leu Asn Asn Phe Ala Ala Leu Gln Ile Leu Thr Thr Arg Asn Met Thr
        355                 360                 365

cca tct cca ttc att gat tgg ctt tgg ggt gga ctc aat tat cag atc      1152
Pro Ser Pro Phe Ile Asp Trp Leu Trp Gly Gly Leu Asn Tyr Gln Ile
    370                 375                 380

gag cac cac ttg ttc cca aca atg cca cgt tgc aat ctg aat gct tgc      1200
Glu His His Leu Phe Pro Thr Met Pro Arg Cys Asn Leu Asn Ala Cys
385                 390                 395                 400

gtg aaa tat gtg aaa gaa tgg tgc aaa gag aat aat ctt cct tac ctc      1248
Val Lys Tyr Val Lys Glu Trp Cys Lys Glu Asn Asn Leu Pro Tyr Leu
                405                 410                 415

gtc gat gac tac ttt gac gga tat gca atg aat ttg caa caa ttg aaa      1296
Val Asp Asp Tyr Phe Asp Gly Tyr Ala Met Asn Leu Gln Gln Leu Lys
            420                 425                 430

aat atg gct gag cac att caa gct aaa gct gcc taa                      1332
Asn Met Ala Glu His Ile Gln Ala Lys Ala Ala
        435                 440


<210>  26
<211>  443
<212>  PRT
<213>  Caenorhabditis elegans

<400>  26

Met Val Val Asp Lys Asn Ala Ser Gly Leu Arg Met Lys Val Asp Gly
1               5                   10                  15


Lys Trp Leu Tyr Leu Ser Glu Glu Leu Val Lys Lys His Pro Gly Gly
            20                  25                  30


Ala Val Ile Glu Gln Tyr Arg Asn Ser Asp Ala Thr His Ile Phe His
        35                  40                  45


Ala Phe His Glu Gly Ser Ser Gln Ala Tyr Lys Gln Leu Asp Leu Leu
    50                  55                  60


Lys Lys His Gly Glu His Asp Glu Phe Leu Glu Lys Gln Leu Glu Lys
65                  70                  75                  80


Arg Leu Asp Lys Val Asp Ile Asn Val Ser Ala Tyr Asp Val Ser Val
                85                  90                  95
```

Ala Gln Glu Lys Lys Met Val Glu Ser Phe Glu Lys Leu Arg Gln Lys
100 105 110

Leu His Asp Asp Gly Leu Met Lys Ala Asn Glu Thr Tyr Phe Leu Phe
115 120 125

Lys Ala Ile Ser Thr Leu Ser Ile Met Ala Phe Ala Phe Tyr Leu Gln
130 135 140

Tyr Leu Gly Trp Tyr Ile Thr Ser Ala Cys Leu Leu Ala Leu Ala Trp
145 150 155 160

Gln Gln Phe Gly Trp Leu Thr His Glu Phe Cys His Gln Gln Pro Thr
165 170 175

Lys Asn Arg Pro Leu Asn Asp Thr Ile Ser Leu Phe Phe Gly Asn Phe
180 185 190

Leu Gln Gly Phe Ser Arg Asp Trp Trp Lys Asp Lys His Asn Thr His
195 200 205

His Ala Ala Thr Asn Val Ile Asp His Asp Gly Asp Ile Asp Leu Ala
210 215 220

Pro Leu Phe Ala Phe Ile Pro Gly Asp Leu Cys Lys Tyr Lys Ala Ser
225 230 235 240

Phe Glu Lys Ala Ile Leu Lys Ile Val Pro Tyr Gln His Leu Tyr Phe
245 250 255

Thr Ala Met Leu Pro Met Leu Arg Phe Ser Trp Thr Gly Gln Ser Val
260 265 270

Gln Trp Val Phe Lys Glu Asn Gln Met Glu Tyr Lys Val Tyr Gln Arg
275 280 285

Asn Ala Phe Trp Glu Gln Ala Thr Ile Val Gly His Trp Ala Trp Val
290 295 300

Phe Tyr Gln Leu Phe Leu Leu Pro Thr Trp Pro Leu Arg Val Ala Tyr
305 310 315 320

Phe Ile Ile Ser Gln Met Gly Gly Gly Leu Leu Ile Ala His Val Val
325 330 335

Thr Phe Asn His Asn Ser Val Asp Lys Tyr Pro Ala Asn Ser Arg Ile
340 345 350

```
Leu Asn Asn Phe Ala Ala Leu Gln Ile Leu Thr Thr Arg Asn Met Thr
        355                 360                 365

Pro Ser Pro Phe Ile Asp Trp Leu Trp Gly Gly Leu Asn Tyr Gln Ile
    370                 375                 380

Glu His His Leu Phe Pro Thr Met Pro Arg Cys Asn Leu Asn Ala Cys
385                 390                 395                 400

Val Lys Tyr Val Lys Glu Trp Cys Lys Glu Asn Asn Leu Pro Tyr Leu
                405                 410                 415

Val Asp Asp Tyr Phe Asp Gly Tyr Ala Met Asn Leu Gln Gln Leu Lys
            420                 425                 430

Asn Met Ala Glu His Ile Gln Ala Lys Ala Ala
        435                 440


<210>  27
<211>  1192
<212>  DNA
<213>  Physcomitrella patens

<220>
<221>  CDS
<222>  (58)..(930)
<223>  Delta-6-Elongase


<400>  27
ctgcttcgtc tcatcttggg ggtgtgattc gggagtgggt tgagttggtg gagcgca         57

atg gag gtc gtg gag aga ttc tac ggt gag ttg gat ggg aag gtc tcg       105
Met Glu Val Val Glu Arg Phe Tyr Gly Glu Leu Asp Gly Lys Val Ser
1               5                   10                  15

cag ggc gtg aat gca ttg ctg ggt agt ttt ggg gtg gag ttg acg gat       153
Gln Gly Val Asn Ala Leu Leu Gly Ser Phe Gly Val Glu Leu Thr Asp
            20                  25                  30

acg ccc act acc aaa ggc ttg ccc ctc gtt gac agt ccc aca ccc atc       201
Thr Pro Thr Thr Lys Gly Leu Pro Leu Val Asp Ser Pro Thr Pro Ile
        35                  40                  45

gtc ctc ggt gtt tct gta tac ttg act att gtc att gga ggg ctt ttg       249
Val Leu Gly Val Ser Val Tyr Leu Thr Ile Val Ile Gly Gly Leu Leu
    50                  55                  60

tgg ata aag gcc agg gat ctg aaa ccg cgc gcc tcg gag cca ttt ttg       297
Trp Ile Lys Ala Arg Asp Leu Lys Pro Arg Ala Ser Glu Pro Phe Leu
65                  70                  75                  80

ctc caa gct ttg gtg ctt gtg cac aac ctg ttc tgt ttt gcg ctc agt       345
Leu Gln Ala Leu Val Leu Val His Asn Leu Phe Cys Phe Ala Leu Ser
                85                  90                  95
```

```
ctg tat atg tgc gtg ggc atc gct tat cag gct att acc tgg cgg tac      393
Leu Tyr Met Cys Val Gly Ile Ala Tyr Gln Ala Ile Thr Trp Arg Tyr
            100                 105                 110

tct ctc tgg ggc aat gca tac aat cct aaa cat aaa gag atg gcg att      441
Ser Leu Trp Gly Asn Ala Tyr Asn Pro Lys His Lys Glu Met Ala Ile
        115                 120                 125

ctg gta tac ttg ttc tac atg tct aag tac gtg gaa ttc atg gat acc      489
Leu Val Tyr Leu Phe Tyr Met Ser Lys Tyr Val Glu Phe Met Asp Thr
    130                 135                 140

gtt atc atg ata ctg aag cgc agc acc agg caa ata agc ttc ctc cac      537
Val Ile Met Ile Leu Lys Arg Ser Thr Arg Gln Ile Ser Phe Leu His
145                 150                 155                 160

gtt tat cat cat tct tca att tcc ctc att tgg tgg gct att gct cat      585
Val Tyr His His Ser Ser Ile Ser Leu Ile Trp Trp Ala Ile Ala His
                165                 170                 175

cac gct cct ggc ggt gaa gca tat tgg tct gcg gct ctg aac tca gga      633
His Ala Pro Gly Gly Glu Ala Tyr Trp Ser Ala Ala Leu Asn Ser Gly
            180                 185                 190

gtg cat gtt ctc atg tat gcg tat tac ttc ttg gct gcc tgc ctt cga      681
Val His Val Leu Met Tyr Ala Tyr Tyr Phe Leu Ala Ala Cys Leu Arg
        195                 200                 205

agt agc cca aag tta aaa aat aag tac ctt ttt tgg ggc agg tac ttg      729
Ser Ser Pro Lys Leu Lys Asn Lys Tyr Leu Phe Trp Gly Arg Tyr Leu
    210                 215                 220

aca caa ttc caa atg ttc cag ttt atg ctg aac tta gtg cag gct tac      777
Thr Gln Phe Gln Met Phe Gln Phe Met Leu Asn Leu Val Gln Ala Tyr
225                 230                 235                 240

tac gac atg aaa acg aat gcg cca tat cca caa tgg ctg atc aag att      825
Tyr Asp Met Lys Thr Asn Ala Pro Tyr Pro Gln Trp Leu Ile Lys Ile
                245                 250                 255

ttg ttc tac tac atg atc tcg ttg ctg ttt ctt ttc ggc aat ttt tac      873
Leu Phe Tyr Tyr Met Ile Ser Leu Leu Phe Leu Phe Gly Asn Phe Tyr
            260                 265                 270

gta caa aaa tac atc aaa ccc tct gac gga aag caa aag gga gct aaa      921
Val Gln Lys Tyr Ile Lys Pro Ser Asp Gly Lys Gln Lys Gly Ala Lys
        275                 280                 285

act gag tga gctgtatcaa gccatagaaa ctctattatg ttagaacctg              970
Thr Glu
    290

aagttggtgc tttcttatct ccacttatct tttaagcagc atcagttttg aaatgatgtg   1030

tgggcgtggt ctgcaagtag tcatcaatat aatcggcctg agcacttcag atggattgtt   1090

agaacatgag taaaagcggt tattacggtg tttattttgt accaaatcac cgcacgggtg   1150

aattgaaata tttcagattt gatcaatttc atctgaaaaa aa                      1192
```

<210> 28

<211> 290
<212> PRT
<213> Physcomitrella patens

<400> 28

Met Glu Val Val Glu Arg Phe Tyr Gly Glu Leu Asp Gly Lys Val Ser
1 5 10 15

Gln Gly Val Asn Ala Leu Leu Gly Ser Phe Gly Val Glu Leu Thr Asp
20 25 30

Thr Pro Thr Thr Lys Gly Leu Pro Leu Val Asp Ser Pro Thr Pro Ile
35 40 45

Val Leu Gly Val Ser Val Tyr Leu Thr Ile Val Ile Gly Gly Leu Leu
50 55 60

Trp Ile Lys Ala Arg Asp Leu Lys Pro Arg Ala Ser Glu Pro Phe Leu
65 70 75 80

Leu Gln Ala Leu Val Leu Val His Asn Leu Phe Cys Phe Ala Leu Ser
85 90 95

Leu Tyr Met Cys Val Gly Ile Ala Tyr Gln Ala Ile Thr Trp Arg Tyr
100 105 110

Ser Leu Trp Gly Asn Ala Tyr Asn Pro Lys His Lys Glu Met Ala Ile
115 120 125

Leu Val Tyr Leu Phe Tyr Met Ser Lys Tyr Val Glu Phe Met Asp Thr
130 135 140

Val Ile Met Ile Leu Lys Arg Ser Thr Arg Gln Ile Ser Phe Leu His
145 150 155 160

Val Tyr His His Ser Ser Ile Ser Leu Ile Trp Trp Ala Ile Ala His
165 170 175

His Ala Pro Gly Gly Glu Ala Tyr Trp Ser Ala Ala Leu Asn Ser Gly
180 185 190

Val His Val Leu Met Tyr Ala Tyr Tyr Phe Leu Ala Ala Cys Leu Arg
195 200 205

Ser Ser Pro Lys Leu Lys Asn Lys Tyr Leu Phe Trp Gly Arg Tyr Leu
210 215 220

Thr Gln Phe Gln Met Phe Gln Phe Met Leu Asn Leu Val Gln Ala Tyr

225 230 235 240

Tyr Asp Met Lys Thr Asn Ala Pro Tyr Pro Gln Trp Leu Ile Lys Ile
245 250 255

Leu Phe Tyr Tyr Met Ile Ser Leu Leu Phe Leu Phe Gly Asn Phe Tyr
260 265 270

Val Gln Lys Tyr Ile Lys Pro Ser Asp Gly Lys Gln Lys Gly Ala Lys
275 280 285

Thr Glu
290

<210> 29
<211> 1054
<212> DNA
<213> Thraustochytrium

<220>
<221> CDS
<222> (43)..(858)
<223> Delta-6-Elongase

<400> 29
gaattcggca cgagagcgcg cggagcggag acctcggccg cg atg atg gag ccg 54
Met Met Glu Pro
1

ctc gac agg tac agg gcg ctg gcg gag ctc gcc gcg agg tac gcc agc 102
Leu Asp Arg Tyr Arg Ala Leu Ala Glu Leu Ala Ala Arg Tyr Ala Ser
5 10 15 20

tcg gcg gcc ttc aag tgg caa gtc acg tac gac gcc aag gac agc ttc 150
Ser Ala Ala Phe Lys Trp Gln Val Thr Tyr Asp Ala Lys Asp Ser Phe
25 30 35

gtc ggg ccc ctg gga atc cgg gag ccg ctc ggg ctc ctg gtg ggc tcc 198
Val Gly Pro Leu Gly Ile Arg Glu Pro Leu Gly Leu Leu Val Gly Ser
40 45 50

gtg gtc ctc tac ctg agc ctg ctg gcc gtg gtc tac gcg ctg cgg aac 246
Val Val Leu Tyr Leu Ser Leu Leu Ala Val Val Tyr Ala Leu Arg Asn
55 60 65

tac ctt ggc ggc ctc atg gcg ctc cgc agc gtg cat aac ctc ggg ctc 294
Tyr Leu Gly Gly Leu Met Ala Leu Arg Ser Val His Asn Leu Gly Leu
70 75 80

tgc ctc ttc tcg ggc gcc gtg tgg atc tac acg agc tac ctc atg atc 342
Cys Leu Phe Ser Gly Ala Val Trp Ile Tyr Thr Ser Tyr Leu Met Ile
85 90 95 100

cag gat ggg cac ttt cgc agc ctc gag gcg gca acg tgc gag ccg ctc 390
Gln Asp Gly His Phe Arg Ser Leu Glu Ala Ala Thr Cys Glu Pro Leu
105 110 115

```
aag cat ccg cac ttc cag ctc atc agc ttg ctc ttt gcg ctg tcc aag      438
Lys His Pro His Phe Gln Leu Ile Ser Leu Leu Phe Ala Leu Ser Lys
            120                 125                 130

atc tgg gag tgg ttc gac acg gtg ctc ctc atc gtc aag ggc aac aag      486
Ile Trp Glu Trp Phe Asp Thr Val Leu Leu Ile Val Lys Gly Asn Lys
        135                 140                 145

ctc cgc ttc ctg cac gtc ttg cac cac gcc acg acc ttt tgg ctc tac      534
Leu Arg Phe Leu His Val Leu His His Ala Thr Thr Phe Trp Leu Tyr
    150                 155                 160

gcc atc gac cac atc ttt ctc tcg tcc atc aag tac ggc gtc gcg gtc      582
Ala Ile Asp His Ile Phe Leu Ser Ser Ile Lys Tyr Gly Val Ala Val
165                 170                 175                 180

aat gct ttc atc cac acc gtc atg tac gcg cac tac ttc cgc cca ttc      630
Asn Ala Phe Ile His Thr Val Met Tyr Ala His Tyr Phe Arg Pro Phe
                185                 190                 195

ccg aag ggc ttg cgc ccg ctt att acg cag ttg cag atc gtc cag ttc      678
Pro Lys Gly Leu Arg Pro Leu Ile Thr Gln Leu Gln Ile Val Gln Phe
            200                 205                 210

att ttc agc atc ggc atc cat acc gcc att tac tgg cac tac gac tgc      726
Ile Phe Ser Ile Gly Ile His Thr Ala Ile Tyr Trp His Tyr Asp Cys
        215                 220                 225

gag ccg ctc gtg cat acc cac ttt tgg gaa tac gtc acg ccc tac ctt      774
Glu Pro Leu Val His Thr His Phe Trp Glu Tyr Val Thr Pro Tyr Leu
    230                 235                 240

ttc gtc gtg ccc ttc ctc atc ctc ttt ttc aat ttt tac ctg cag cag      822
Phe Val Val Pro Phe Leu Ile Leu Phe Phe Asn Phe Tyr Leu Gln Gln
245                 250                 255                 260

tac gtc ctc gcg ccc gca aaa acc aag aag gca tag ccacgtaaca           868
Tyr Val Leu Ala Pro Ala Lys Thr Lys Lys Ala
                265                 270

gtagaccagc agcgccgagg acgcgtgccg cgttatcgcg aagcacgaaa taaagaagat    928

catttgattc aacgaggcta cttgcggcca cgagaaaaaa aaaaaaaaaa aaaaaaaaaa    988

aaaaaaaaaa aaaaaaaaaa aaaaaaaaaa aaaaaaaaaa aaaaaaaaaa aaaaaaaaaa   1048

ctcgag                                                              1054


<210>  30
<211>  271
<212>  PRT
<213>  Thraustochytrium

<400>  30

Met Met Glu Pro Leu Asp Arg Tyr Arg Ala Leu Ala Glu Leu Ala Ala
1               5                   10                  15


Arg Tyr Ala Ser Ser Ala Ala Phe Lys Trp Gln Val Thr Tyr Asp Ala
            20                  25                  30
```

```
Lys Asp Ser Phe Val Gly Pro Leu Gly Ile Arg Glu Pro Leu Gly Leu
        35                  40                  45

Leu Val Gly Ser Val Val Leu Tyr Leu Ser Leu Leu Ala Val Val Tyr
    50                  55                  60

Ala Leu Arg Asn Tyr Leu Gly Gly Leu Met Ala Leu Arg Ser Val His
65                  70                  75                  80

Asn Leu Gly Leu Cys Leu Phe Ser Gly Ala Val Trp Ile Tyr Thr Ser
                85                  90                  95

Tyr Leu Met Ile Gln Asp Gly His Phe Arg Ser Leu Glu Ala Ala Thr
            100                 105                 110

Cys Glu Pro Leu Lys His Pro His Phe Gln Leu Ile Ser Leu Leu Phe
        115                 120                 125

Ala Leu Ser Lys Ile Trp Glu Trp Phe Asp Thr Val Leu Leu Ile Val
    130                 135                 140

Lys Gly Asn Lys Leu Arg Phe Leu His Val Leu His His Ala Thr Thr
145                 150                 155                 160

Phe Trp Leu Tyr Ala Ile Asp His Ile Phe Leu Ser Ser Ile Lys Tyr
                165                 170                 175

Gly Val Ala Val Asn Ala Phe Ile His Thr Val Met Tyr Ala His Tyr
            180                 185                 190

Phe Arg Pro Phe Pro Lys Gly Leu Arg Pro Leu Ile Thr Gln Leu Gln
        195                 200                 205

Ile Val Gln Phe Ile Phe Ser Ile Gly Ile His Thr Ala Ile Tyr Trp
    210                 215                 220

His Tyr Asp Cys Glu Pro Leu Val His Thr His Phe Trp Glu Tyr Val
225                 230                 235                 240

Thr Pro Tyr Leu Phe Val Val Pro Phe Leu Ile Leu Phe Phe Asn Phe
                245                 250                 255

Tyr Leu Gln Gln Tyr Val Leu Ala Pro Ala Lys Thr Lys Lys Ala
            260                 265                 270
```

<210> 31
<211> 837

<212> DNA
<213> Phytophthora infestans

<220>
<221> CDS
<222> (1)..(837)
<223> Delta-6-Elongase

<400> 31

```
atg tcg act gag cta ctg cag agc tac tac gcg tgg gcc aac gcc acg       48
Met Ser Thr Glu Leu Leu Gln Ser Tyr Tyr Ala Trp Ala Asn Ala Thr
1               5                   10                  15

gag gcc aag ctg ctg gac tgg gtc gac cct gag ggc ggc tgg aag gtg       96
Glu Ala Lys Leu Leu Asp Trp Val Asp Pro Glu Gly Gly Trp Lys Val
            20                  25                  30

cat cct atg gca gac tac ccc cta gcc aac ttc tcc agc gtc tac gcc      144
His Pro Met Ala Asp Tyr Pro Leu Ala Asn Phe Ser Ser Val Tyr Ala
        35                  40                  45

atc tgc gtc gga tac ttg ctc ttc gta atc ttc ggc acg gcc ctg atg      192
Ile Cys Val Gly Tyr Leu Leu Phe Val Ile Phe Gly Thr Ala Leu Met
    50                  55                  60

aaa atg gga gtc ccc gcc atc aag acc agt cca tta cag ttt gtg tac      240
Lys Met Gly Val Pro Ala Ile Lys Thr Ser Pro Leu Gln Phe Val Tyr
65                  70                  75                  80

aac ccc atc caa gtc att gcc tgc tct tat atg tgc gtg gag gcc gcc      288
Asn Pro Ile Gln Val Ile Ala Cys Ser Tyr Met Cys Val Glu Ala Ala
                85                  90                  95

atc cag gcc tac cgc aac ggc tac acc gcc gcc ccg tgc aac gcc ttt      336
Ile Gln Ala Tyr Arg Asn Gly Tyr Thr Ala Ala Pro Cys Asn Ala Phe
            100                 105                 110

aag tcc gac gac ccc gtc atg ggc aac gtt ctg tac ctc ttc tat ctc      384
Lys Ser Asp Asp Pro Val Met Gly Asn Val Leu Tyr Leu Phe Tyr Leu
        115                 120                 125

tcc aag atg ctc gac ctg tgc gac aca gtc ttc att atc cta gga aag      432
Ser Lys Met Leu Asp Leu Cys Asp Thr Val Phe Ile Ile Leu Gly Lys
    130                 135                 140

aag tgg aaa cag ctt tcc atc ttg cac gtg tac cac cac ctt acc gtg      480
Lys Trp Lys Gln Leu Ser Ile Leu His Val Tyr His His Leu Thr Val
145                 150                 155                 160

ctt ttc gtc tac tat gtg acg ttc cgc gcc gct cag gac ggg gac tca      528
Leu Phe Val Tyr Tyr Val Thr Phe Arg Ala Ala Gln Asp Gly Asp Ser
                165                 170                 175

tat gct acc atc gtg ctc aac ggc ttc gtg cac acc atc atg tac act      576
Tyr Ala Thr Ile Val Leu Asn Gly Phe Val His Thr Ile Met Tyr Thr
            180                 185                 190

tac tac ttc gtc agc gcc cac acg cgc aac att tgg tgg aag aag tac      624
Tyr Tyr Phe Val Ser Ala His Thr Arg Asn Ile Trp Trp Lys Lys Tyr
        195                 200                 205

ctc acg cgc att cag ctt atc cag ttc gtg acc atg aac gtg cag ggc      672
```

```
Leu Thr Arg Ile Gln Leu Ile Gln Phe Val Thr Met Asn Val Gln Gly
    210                 215                 220

tac ctg acc tac tct cga cag tgc cca ggc atg cct cct aag gtg ccg      720
Tyr Leu Thr Tyr Ser Arg Gln Cys Pro Gly Met Pro Pro Lys Val Pro
225                 230                 235                 240

ctc atg tac ctt gtg tac gtg cag tca ctc ttc tgg ctc ttc atg aat      768
Leu Met Tyr Leu Val Tyr Val Gln Ser Leu Phe Trp Leu Phe Met Asn
                245                 250                 255

ttc tac att cgc gcg tac gtg ttc ggc ccc aag aaa ccg gcc gtg gag      816
Phe Tyr Ile Arg Ala Tyr Val Phe Gly Pro Lys Lys Pro Ala Val Glu
            260                 265                 270

gaa tcg aag aag aag ttg taa                                          837
Glu Ser Lys Lys Lys Leu
        275


<210>  32
<211>  278
<212>  PRT
<213>  Phytophthora infestans

<400>  32

Met Ser Thr Glu Leu Leu Gln Ser Tyr Tyr Ala Trp Ala Asn Ala Thr
1               5                   10                  15


Glu Ala Lys Leu Leu Asp Trp Val Asp Pro Glu Gly Gly Trp Lys Val
            20                  25                  30


His Pro Met Ala Asp Tyr Pro Leu Ala Asn Phe Ser Ser Val Tyr Ala
        35                  40                  45


Ile Cys Val Gly Tyr Leu Leu Phe Val Ile Phe Gly Thr Ala Leu Met
    50                  55                  60


Lys Met Gly Val Pro Ala Ile Lys Thr Ser Pro Leu Gln Phe Val Tyr
65                  70                  75                  80


Asn Pro Ile Gln Val Ile Ala Cys Ser Tyr Met Cys Val Glu Ala Ala
                85                  90                  95


Ile Gln Ala Tyr Arg Asn Gly Tyr Thr Ala Ala Pro Cys Asn Ala Phe
            100                 105                 110


Lys Ser Asp Asp Pro Val Met Gly Asn Val Leu Tyr Leu Phe Tyr Leu
        115                 120                 125


Ser Lys Met Leu Asp Leu Cys Asp Thr Val Phe Ile Ile Leu Gly Lys
    130                 135                 140
```

```
Lys Trp Lys Gln Leu Ser Ile Leu His Val Tyr His His Leu Thr Val
145                 150                 155                 160

Leu Phe Val Tyr Tyr Val Thr Phe Arg Ala Ala Gln Asp Gly Asp Ser
                165                 170                 175

Tyr Ala Thr Ile Val Leu Asn Gly Phe Val His Thr Ile Met Tyr Thr
            180                 185                 190

Tyr Tyr Phe Val Ser Ala His Thr Arg Asn Ile Trp Trp Lys Lys Tyr
        195                 200                 205

Leu Thr Arg Ile Gln Leu Ile Gln Phe Val Thr Met Asn Val Gln Gly
    210                 215                 220

Tyr Leu Thr Tyr Ser Arg Gln Cys Pro Gly Met Pro Pro Lys Val Pro
225                 230                 235                 240

Leu Met Tyr Leu Val Tyr Val Gln Ser Leu Phe Trp Leu Phe Met Asn
                245                 250                 255

Phe Tyr Ile Arg Ala Tyr Val Phe Gly Pro Lys Lys Pro Ala Val Glu
            260                 265                 270

Glu Ser Lys Lys Lys Leu
        275


<210>  33
<211>  954
<212>  DNA
<213>  Mortierella alpina

<220>
<221>  CDS
<222>  (1)..(954)
<223>  Delta-6-Elongase


<400>  33
atg gcc gcc gca atc ttg gac aag gtc aac ttc ggc att gat cag ccc       48
Met Ala Ala Ala Ile Leu Asp Lys Val Asn Phe Gly Ile Asp Gln Pro
1               5                   10                  15

ttc gga atc aag ctc gac acc tac ttt gct cag gcc tat gaa ctc gtc       96
Phe Gly Ile Lys Leu Asp Thr Tyr Phe Ala Gln Ala Tyr Glu Leu Val
            20                  25                  30

acc gga aag tcc atc gac tcc ttc gtc ttc cag gag ggc gtc acg cct      144
Thr Gly Lys Ser Ile Asp Ser Phe Val Phe Gln Glu Gly Val Thr Pro
        35                  40                  45

ctc tcg acc cag aga gag gtc gcc atg tgg act atc act tac ttc gtc      192
Leu Ser Thr Gln Arg Glu Val Ala Met Trp Thr Ile Thr Tyr Phe Val
    50                  55                  60
```

gtc atc ttt ggt ggt cgc cag atc atg aag agc cag gac gcc ttc aag 240
Val Ile Phe Gly Gly Arg Gln Ile Met Lys Ser Gln Asp Ala Phe Lys
65 70 75 80

ctc aag ccc ctc ttc atc ctc cac aac ttc ctc ctg acg atc gcg tcc 288
Leu Lys Pro Leu Phe Ile Leu His Asn Phe Leu Leu Thr Ile Ala Ser
85 90 95

gga tcg ctg ttg ctc ctg ttc atc gag aac ctg gtc ccc atc ctc gcc 336
Gly Ser Leu Leu Leu Leu Phe Ile Glu Asn Leu Val Pro Ile Leu Ala
100 105 110

aga aac gga ctt ttc tac gcc atc tgc gac gac ggt gcc tgg acc cag 384
Arg Asn Gly Leu Phe Tyr Ala Ile Cys Asp Asp Gly Ala Trp Thr Gln
115 120 125

cgc ctc gag ctc ctc tac tac ctc aac tac ctg gtc aag tac tgg gag 432
Arg Leu Glu Leu Leu Tyr Tyr Leu Asn Tyr Leu Val Lys Tyr Trp Glu
130 135 140

ttg gcc gac acc gtc ttt ttg gtc ctc aag aag aag cct ctt gag ttc 480
Leu Ala Asp Thr Val Phe Leu Val Leu Lys Lys Lys Pro Leu Glu Phe
145 150 155 160

ctg cac tac ttc cac cac tcg atg acc atg gtt ctc tgc ttt gtc cag 528
Leu His Tyr Phe His His Ser Met Thr Met Val Leu Cys Phe Val Gln
165 170 175

ctt gga gga tac act tca gtg tcc tgg gtc cct att acc ctc aac ttg 576
Leu Gly Gly Tyr Thr Ser Val Ser Trp Val Pro Ile Thr Leu Asn Leu
180 185 190

act gtc cac gtc ttc atg tac tac tac tac atg cgc tcc gct gcc ggt 624
Thr Val His Val Phe Met Tyr Tyr Tyr Tyr Met Arg Ser Ala Ala Gly
195 200 205

gtt cgc atc tgg tgg aag cag tac ttg acc act ctc cag atc gtc cag 672
Val Arg Ile Trp Trp Lys Gln Tyr Leu Thr Thr Leu Gln Ile Val Gln
210 215 220

ttc gtt ctt gac ctc gga ttc atc tac ttc tgc gcc tac acc tac ttc 720
Phe Val Leu Asp Leu Gly Phe Ile Tyr Phe Cys Ala Tyr Thr Tyr Phe
225 230 235 240

gcc ttc acc tac ttc ccc tgg gct ccc aac gtc ggc aag tgc gcc ggt 768
Ala Phe Thr Tyr Phe Pro Trp Ala Pro Asn Val Gly Lys Cys Ala Gly
245 250 255

acc gag ggt gct gct ctc ttt ggc tgc gga ctc ctc tcc agc tat ctc 816
Thr Glu Gly Ala Ala Leu Phe Gly Cys Gly Leu Leu Ser Ser Tyr Leu
260 265 270

ttg ctc ttt atc aac ttc tac cgc att acc tac aat gcc aag gcc aag 864
Leu Leu Phe Ile Asn Phe Tyr Arg Ile Thr Tyr Asn Ala Lys Ala Lys
275 280 285

gca gcc aag gag cgt gga agc aac ttt acc ccc aag act gtc aag tcc 912
Ala Ala Lys Glu Arg Gly Ser Asn Phe Thr Pro Lys Thr Val Lys Ser
290 295 300

ggc gga tcg ccc aag aag ccc tcc aag agc aag cac atc taa 954
Gly Gly Ser Pro Lys Lys Pro Ser Lys Ser Lys His Ile

305 310 315

<210> 34
<211> 317
<212> PRT
<213> Mortierella alpina

<400> 34

Met Ala Ala Ala Ile Leu Asp Lys Val Asn Phe Gly Ile Asp Gln Pro
1 5 10 15

Phe Gly Ile Lys Leu Asp Thr Tyr Phe Ala Gln Ala Tyr Glu Leu Val
20 25 30

Thr Gly Lys Ser Ile Asp Ser Phe Val Phe Gln Glu Gly Val Thr Pro
35 40 45

Leu Ser Thr Gln Arg Glu Val Ala Met Trp Thr Ile Thr Tyr Phe Val
50 55 60

Val Ile Phe Gly Gly Arg Gln Ile Met Lys Ser Gln Asp Ala Phe Lys
65 70 75 80

Leu Lys Pro Leu Phe Ile Leu His Asn Phe Leu Leu Thr Ile Ala Ser
85 90 95

Gly Ser Leu Leu Leu Leu Phe Ile Glu Asn Leu Val Pro Ile Leu Ala
100 105 110

Arg Asn Gly Leu Phe Tyr Ala Ile Cys Asp Asp Gly Ala Trp Thr Gln
115 120 125

Arg Leu Glu Leu Leu Tyr Tyr Leu Asn Tyr Leu Val Lys Tyr Trp Glu
130 135 140

Leu Ala Asp Thr Val Phe Leu Val Leu Lys Lys Lys Pro Leu Glu Phe
145 150 155 160

Leu His Tyr Phe His His Ser Met Thr Met Val Leu Cys Phe Val Gln
165 170 175

Leu Gly Gly Tyr Thr Ser Val Ser Trp Val Pro Ile Thr Leu Asn Leu
180 185 190

Thr Val His Val Phe Met Tyr Tyr Tyr Tyr Met Arg Ser Ala Ala Gly
195 200 205

Val Arg Ile Trp Trp Lys Gln Tyr Leu Thr Thr Leu Gln Ile Val Gln

```
    210                 215                 220

Phe Val Leu Asp Leu Gly Phe Ile Tyr Phe Cys Ala Tyr Thr Tyr Phe
225                 230                 235                 240

Ala Phe Thr Tyr Phe Pro Trp Ala Pro Asn Val Gly Lys Cys Ala Gly
                245                 250                 255

Thr Glu Gly Ala Ala Leu Phe Gly Cys Gly Leu Leu Ser Ser Tyr Leu
            260                 265                 270

Leu Leu Phe Ile Asn Phe Tyr Arg Ile Thr Tyr Asn Ala Lys Ala Lys
        275                 280                 285

Ala Ala Lys Glu Arg Gly Ser Asn Phe Thr Pro Lys Thr Val Lys Ser
    290                 295                 300

Gly Gly Ser Pro Lys Lys Pro Ser Lys Ser Lys His Ile
305                 310                 315


<210>  35
<211>  957
<212>  DNA
<213>  Mortierella alpina

<220>
<221>  CDS
<222>  (1)..(957)
<223>  Delta-6-Elongase


<400>  35
atg gag tcg att gcg cca ttc ctc cca tca aag atg ccg caa gat ctg        48
Met Glu Ser Ile Ala Pro Phe Leu Pro Ser Lys Met Pro Gln Asp Leu
1               5                   10                  15

ttt atg gac ctt gcc acc gct atc ggt gtc cgg gcc gcg ccc tat gtc        96
Phe Met Asp Leu Ala Thr Ala Ile Gly Val Arg Ala Ala Pro Tyr Val
            20                  25                  30

gat cct ctc gag gcc gcg ctg gtg gcc cag gcc gag aag tac atc ccc       144
Asp Pro Leu Glu Ala Ala Leu Val Ala Gln Ala Glu Lys Tyr Ile Pro
        35                  40                  45

acg att gtc cat cac acg cgt ggg ttc ctg gtc gcg gtg gag tcg cct       192
Thr Ile Val His His Thr Arg Gly Phe Leu Val Ala Val Glu Ser Pro
    50                  55                  60

ttg gcc cgt gag ctg ccg ttg atg aac ccg ttc cac gtg ctg ttg atc       240
Leu Ala Arg Glu Leu Pro Leu Met Asn Pro Phe His Val Leu Leu Ile
65                  70                  75                  80

gtg ctc gct tat ttg gtc acg gtc ttt gtg ggc atg cag atc atg aag       288
Val Leu Ala Tyr Leu Val Thr Val Phe Val Gly Met Gln Ile Met Lys
                85                  90                  95
```

```
aac ttt gag cgg ttc gag gtc aag acg ttt tcg ctc ctg cac aac ttt      336
Asn Phe Glu Arg Phe Glu Val Lys Thr Phe Ser Leu Leu His Asn Phe
            100                 105                 110

tgt ctg gtc tcg atc agc gcc tac atg tgc ggt ggg atc ctg tac gag      384
Cys Leu Val Ser Ile Ser Ala Tyr Met Cys Gly Gly Ile Leu Tyr Glu
        115                 120                 125

gct tat cag gcc aac tat gga ctg ttt gag aac gct gct gat cat acc      432
Ala Tyr Gln Ala Asn Tyr Gly Leu Phe Glu Asn Ala Ala Asp His Thr
    130                 135                 140

ttc aag ggt ctt cct atg gcc aag atg atc tgg ctc ttc tac ttc tcc      480
Phe Lys Gly Leu Pro Met Ala Lys Met Ile Trp Leu Phe Tyr Phe Ser
145                 150                 155                 160

aag atc atg gag ttt gtc gac acc atg atc atg gtc ctc aag aag aac      528
Lys Ile Met Glu Phe Val Asp Thr Met Ile Met Val Leu Lys Lys Asn
                165                 170                 175

aac cgc cag atc tcc ttc ttg cac gtt tac cac cac agc tcc atc ttc      576
Asn Arg Gln Ile Ser Phe Leu His Val Tyr His His Ser Ser Ile Phe
            180                 185                 190

acc atc tgg tgg ttg gtc acc ttt gtt gca ccc aac ggt gaa gcc tac      624
Thr Ile Trp Trp Leu Val Thr Phe Val Ala Pro Asn Gly Glu Ala Tyr
        195                 200                 205

ttc tct gct gcg ttg aac tcg ttc atc cat gtg atc atg tac ggc tac      672
Phe Ser Ala Ala Leu Asn Ser Phe Ile His Val Ile Met Tyr Gly Tyr
    210                 215                 220

tac ttc ttg tcg gcc ttg ggc ttc aag cag gtg tcg ttc atc aag ttc      720
Tyr Phe Leu Ser Ala Leu Gly Phe Lys Gln Val Ser Phe Ile Lys Phe
225                 230                 235                 240

tac atc acg cgc tcg cag atg aca cag ttc tgc atg atg tcg gtc cag      768
Tyr Ile Thr Arg Ser Gln Met Thr Gln Phe Cys Met Met Ser Val Gln
                245                 250                 255

tct tcc tgg gac atg tac gcc atg aag gtc ctt ggc cgc ccc gga tac      816
Ser Ser Trp Asp Met Tyr Ala Met Lys Val Leu Gly Arg Pro Gly Tyr
            260                 265                 270

ccc ttc ttc atc acg gct ctg ctt tgg ttc tac atg tgg acc atg ctc      864
Pro Phe Phe Ile Thr Ala Leu Leu Trp Phe Tyr Met Trp Thr Met Leu
        275                 280                 285

ggt ctc ttc tac aac ttt tac aga aag aac gcc aag ttg gcc aag cag      912
Gly Leu Phe Tyr Asn Phe Tyr Arg Lys Asn Ala Lys Leu Ala Lys Gln
    290                 295                 300

gcc aag gcc gac gct gcc aag gag aag gca agg aag ttg cag taa          957
Ala Lys Ala Asp Ala Ala Lys Glu Lys Ala Arg Lys Leu Gln
305                 310                 315
```

```
<210>  36
<211>  318
<212>  PRT
<213>  Mortierella alpina

<400>  36
```

```
Met Glu Ser Ile Ala Pro Phe Leu Pro Ser Lys Met Pro Gln Asp Leu
1               5                   10                  15

Phe Met Asp Leu Ala Thr Ala Ile Gly Val Arg Ala Ala Pro Tyr Val
            20                  25                  30

Asp Pro Leu Glu Ala Ala Leu Val Ala Gln Ala Glu Lys Tyr Ile Pro
        35                  40                  45

Thr Ile Val His His Thr Arg Gly Phe Leu Val Ala Val Glu Ser Pro
    50                  55                  60

Leu Ala Arg Glu Leu Pro Leu Met Asn Pro Phe His Val Leu Leu Ile
65                  70                  75                  80

Val Leu Ala Tyr Leu Val Thr Val Phe Val Gly Met Gln Ile Met Lys
                85                  90                  95

Asn Phe Glu Arg Phe Glu Val Lys Thr Phe Ser Leu Leu His Asn Phe
            100                 105                 110

Cys Leu Val Ser Ile Ser Ala Tyr Met Cys Gly Gly Ile Leu Tyr Glu
        115                 120                 125

Ala Tyr Gln Ala Asn Tyr Gly Leu Phe Glu Asn Ala Ala Asp His Thr
    130                 135                 140

Phe Lys Gly Leu Pro Met Ala Lys Met Ile Trp Leu Phe Tyr Phe Ser
145                 150                 155                 160

Lys Ile Met Glu Phe Val Asp Thr Met Ile Met Val Leu Lys Lys Asn
                165                 170                 175

Asn Arg Gln Ile Ser Phe Leu His Val Tyr His His Ser Ser Ile Phe
            180                 185                 190

Thr Ile Trp Trp Leu Val Thr Phe Val Ala Pro Asn Gly Glu Ala Tyr
        195                 200                 205

Phe Ser Ala Ala Leu Asn Ser Phe Ile His Val Ile Met Tyr Gly Tyr
    210                 215                 220

Tyr Phe Leu Ser Ala Leu Gly Phe Lys Gln Val Ser Phe Ile Lys Phe
225                 230                 235                 240

Tyr Ile Thr Arg Ser Gln Met Thr Gln Phe Cys Met Met Ser Val Gln
                245                 250                 255
```

Ser Ser Trp Asp Met Tyr Ala Met Lys Val Leu Gly Arg Pro Gly Tyr
260 265 270

Pro Phe Phe Ile Thr Ala Leu Leu Trp Phe Tyr Met Trp Thr Met Leu
275 280 285

Gly Leu Phe Tyr Asn Phe Tyr Arg Lys Asn Ala Lys Leu Ala Lys Gln
290 295 300

Ala Lys Ala Asp Ala Ala Lys Glu Lys Ala Arg Lys Leu Gln
305 310 315

<210> 37
<211> 867
<212> DNA
<213> Caenorhabditis elegans

<220>
<221> CDS
<222> (1)..(867)
<223> Delta-6-Elongase

<400> 37
atg gct cag cat ccg ctc gtt caa cgg ctt ctc gat gtc aaa ttc gac 48
Met Ala Gln His Pro Leu Val Gln Arg Leu Leu Asp Val Lys Phe Asp
1 5 10 15

acg aaa cga ttt gtg gct att gct act cat ggg cca aag aat ttc cct 96
Thr Lys Arg Phe Val Ala Ile Ala Thr His Gly Pro Lys Asn Phe Pro
20 25 30

gac gca gaa ggt cgc aag ttc ttt gct gat cac ttt gat gtt act att 144
Asp Ala Glu Gly Arg Lys Phe Phe Ala Asp His Phe Asp Val Thr Ile
35 40 45

cag gct tca atc ctg tac atg gtc gtt gtg ttc gga aca aaa tgg ttc 192
Gln Ala Ser Ile Leu Tyr Met Val Val Val Phe Gly Thr Lys Trp Phe
50 55 60

atg cgt aat cgt caa cca ttc caa ttg act att cca ctc aac atc tgg 240
Met Arg Asn Arg Gln Pro Phe Gln Leu Thr Ile Pro Leu Asn Ile Trp
65 70 75 80

aat ttc atc ctc gcc gca ttt tcc atc gca gga gct gtc aaa atg acc 288
Asn Phe Ile Leu Ala Ala Phe Ser Ile Ala Gly Ala Val Lys Met Thr
85 90 95

cca gag ttc ttt gga acc att gcc aac aaa gga att gtc gca tcc tac 336
Pro Glu Phe Phe Gly Thr Ile Ala Asn Lys Gly Ile Val Ala Ser Tyr
100 105 110

tgc aaa gtg ttt gat ttc acg aaa gga gag aat gga tac tgg gtg tgg 384
Cys Lys Val Phe Asp Phe Thr Lys Gly Glu Asn Gly Tyr Trp Val Trp
115 120 125

ctc ttc atg gct tcc aaa ctt ttc gaa ctt gtt gac acc atc ttc ttg 432

```
Leu Phe Met Ala Ser Lys Leu Phe Glu Leu Val Asp Thr Ile Phe Leu
    130                 135                 140

gtt ctc cgt aaa cgt cca ctc atg ttc ctt cac tgg tat cac cat att      480
Val Leu Arg Lys Arg Pro Leu Met Phe Leu His Trp Tyr His His Ile
145                 150                 155                 160

ctc acc atg atc tac gcc tgg tac tct cat cca ttg acc cca gga ttc      528
Leu Thr Met Ile Tyr Ala Trp Tyr Ser His Pro Leu Thr Pro Gly Phe
                165                 170                 175

aac aga tac gga att tat ctt aac ttt gtc gtc cac gcc ttc atg tac      576
Asn Arg Tyr Gly Ile Tyr Leu Asn Phe Val Val His Ala Phe Met Tyr
            180                 185                 190

tct tac tac ttc ctt cgc tcg atg aag att cgc gtg cca gga ttc atc      624
Ser Tyr Tyr Phe Leu Arg Ser Met Lys Ile Arg Val Pro Gly Phe Ile
        195                 200                 205

gcc caa gct atc aca tct ctt caa atc gtt caa ttc atc atc tct tgc      672
Ala Gln Ala Ile Thr Ser Leu Gln Ile Val Gln Phe Ile Ile Ser Cys
    210                 215                 220

gcc gtt ctt gct cat ctt ggt tat ctc atg cac ttc acc aat gcc aac      720
Ala Val Leu Ala His Leu Gly Tyr Leu Met His Phe Thr Asn Ala Asn
225                 230                 235                 240

tgt gat ttc gag cca tca gta ttc aag ctc gca gtt ttc atg gac aca      768
Cys Asp Phe Glu Pro Ser Val Phe Lys Leu Ala Val Phe Met Asp Thr
                245                 250                 255

aca tac ttg gct ctt ttc gtc aac ttc ttc ctc caa tca tat gtt ctc      816
Thr Tyr Leu Ala Leu Phe Val Asn Phe Phe Leu Gln Ser Tyr Val Leu
            260                 265                 270

cgc gga gga aaa gac aag tac aag gca gtg cca aag aag aag aac aac      864
Arg Gly Gly Lys Asp Lys Tyr Lys Ala Val Pro Lys Lys Lys Asn Asn
        275                 280                 285

taa                                                                  867


<210>  38
<211>  288
<212>  PRT
<213>  Caenorhabditis elegans

<400>  38

Met Ala Gln His Pro Leu Val Gln Arg Leu Leu Asp Val Lys Phe Asp
1               5                   10                  15


Thr Lys Arg Phe Val Ala Ile Ala Thr His Gly Pro Lys Asn Phe Pro
            20                  25                  30


Asp Ala Glu Gly Arg Lys Phe Phe Ala Asp His Phe Asp Val Thr Ile
        35                  40                  45


Gln Ala Ser Ile Leu Tyr Met Val Val Val Phe Gly Thr Lys Trp Phe
    50                  55                  60
```

```
Met Arg Asn Arg Gln Pro Phe Gln Leu Thr Ile Pro Leu Asn Ile Trp
65                  70                  75                  80

Asn Phe Ile Leu Ala Ala Phe Ser Ile Ala Gly Ala Val Lys Met Thr
                85                  90                  95

Pro Glu Phe Phe Gly Thr Ile Ala Asn Lys Gly Ile Val Ala Ser Tyr
            100                 105                 110

Cys Lys Val Phe Asp Phe Thr Lys Gly Glu Asn Gly Tyr Trp Val Trp
        115                 120                 125

Leu Phe Met Ala Ser Lys Leu Phe Glu Leu Val Asp Thr Ile Phe Leu
    130                 135                 140

Val Leu Arg Lys Arg Pro Leu Met Phe Leu His Trp Tyr His His Ile
145                 150                 155                 160

Leu Thr Met Ile Tyr Ala Trp Tyr Ser His Pro Leu Thr Pro Gly Phe
                165                 170                 175

Asn Arg Tyr Gly Ile Tyr Leu Asn Phe Val Val His Ala Phe Met Tyr
            180                 185                 190

Ser Tyr Tyr Phe Leu Arg Ser Met Lys Ile Arg Val Pro Gly Phe Ile
        195                 200                 205

Ala Gln Ala Ile Thr Ser Leu Gln Ile Val Gln Phe Ile Ile Ser Cys
    210                 215                 220

Ala Val Leu Ala His Leu Gly Tyr Leu Met His Phe Thr Asn Ala Asn
225                 230                 235                 240

Cys Asp Phe Glu Pro Ser Val Phe Lys Leu Ala Val Phe Met Asp Thr
                245                 250                 255

Thr Tyr Leu Ala Leu Phe Val Asn Phe Phe Leu Gln Ser Tyr Val Leu
            260                 265                 270

Arg Gly Gly Lys Asp Lys Tyr Lys Ala Val Pro Lys Lys Lys Asn Asn
        275                 280                 285
```

<210> 39
<211> 1626
<212> DNA
<213> Euglena gracilis

```
<220>
<221>  CDS
<222>  (1)..(1626)
<223>  Delta-4-Desaturase


<400>  39
atg ttg gtg ctg ttt ggc aat ttc tat gtc aag caa tac tcc caa aag        48
Met Leu Val Leu Phe Gly Asn Phe Tyr Val Lys Gln Tyr Ser Gln Lys
1               5                   10                  15

aac ggc aag ccg gag aac gga gcc acc cct gag aac gga gcg aag ccg        96
Asn Gly Lys Pro Glu Asn Gly Ala Thr Pro Glu Asn Gly Ala Lys Pro
            20                  25                  30

caa cct tgc gag aac ggc acg gtg gaa aag cga gag aat gac acc gcc       144
Gln Pro Cys Glu Asn Gly Thr Val Glu Lys Arg Glu Asn Asp Thr Ala
        35                  40                  45

aac gtt cgg ccc acc cgt cca gct gga ccc ccg ccg gcc acg tac tac       192
Asn Val Arg Pro Thr Arg Pro Ala Gly Pro Pro Pro Ala Thr Tyr Tyr
    50                  55                  60

gac tcc ctg gca gtg tcg ggg cag ggc aag gag cgg ctg ttc acc acc       240
Asp Ser Leu Ala Val Ser Gly Gln Gly Lys Glu Arg Leu Phe Thr Thr
65                  70                  75                  80

gat gag gtg agg cgg cac atc ctc ccc acc gat ggc tgg ctg acg tgc       288
Asp Glu Val Arg Arg His Ile Leu Pro Thr Asp Gly Trp Leu Thr Cys
                85                  90                  95

cac gaa gga gtc tac gat gtc act gat ttc ctt gcc aag cac cct ggt       336
His Glu Gly Val Tyr Asp Val Thr Asp Phe Leu Ala Lys His Pro Gly
            100                 105                 110

ggc ggt gtc atc acg ctg ggc ctt gga agg gac tgc aca atc ctc atc       384
Gly Gly Val Ile Thr Leu Gly Leu Gly Arg Asp Cys Thr Ile Leu Ile
        115                 120                 125

gag tca tac cac cct gct ggg cgc ccg gac aag gtg atg gag aag tac       432
Glu Ser Tyr His Pro Ala Gly Arg Pro Asp Lys Val Met Glu Lys Tyr
    130                 135                 140

cgc att ggt acg ctg cag gac ccc aag acg ttc tat gct tgg gga gag       480
Arg Ile Gly Thr Leu Gln Asp Pro Lys Thr Phe Tyr Ala Trp Gly Glu
145                 150                 155                 160

tcc gat ttc tac cct gag ttg aag cgc cgg gcc ctt gca agg ctg aag       528
Ser Asp Phe Tyr Pro Glu Leu Lys Arg Arg Ala Leu Ala Arg Leu Lys
                165                 170                 175

gag gct ggt cag gcg cgg cgc ggc ggc ctt ggg gtg aag gcc ctc ctg       576
Glu Ala Gly Gln Ala Arg Arg Gly Gly Leu Gly Val Lys Ala Leu Leu
            180                 185                 190

gtg ctc acc ctc ttc ttc gtg tcg tgg tac atg tgg gtg gcc cac aag       624
Val Leu Thr Leu Phe Phe Val Ser Trp Tyr Met Trp Val Ala His Lys
        195                 200                 205

tcc ttc ctc tgg gcc gcc gtc tgg ggc ttc gcc ggc tcc cac gtc ggg       672
Ser Phe Leu Trp Ala Ala Val Trp Gly Phe Ala Gly Ser His Val Gly
    210                 215                 220
```

```
ctg agc atc cag cac gat ggc aac cac ggc gcg ttc agc cgc aac aca       720
Leu Ser Ile Gln His Asp Gly Asn His Gly Ala Phe Ser Arg Asn Thr
225                 230                 235                 240

ctg gtg aac cgc ctg gcg ggg tgg ggc atg gac ttg atc ggc gcg tcg       768
Leu Val Asn Arg Leu Ala Gly Trp Gly Met Asp Leu Ile Gly Ala Ser
                245                 250                 255

tcc acg gtg tgg gag tac cag cac gtc atc ggc cac cac cag tac acc       816
Ser Thr Val Trp Glu Tyr Gln His Val Ile Gly His His Gln Tyr Thr
            260                 265                 270

aac ctc gtg tcg gac acg cta ttc agt ctg cct gag aac gat ccg gac       864
Asn Leu Val Ser Asp Thr Leu Phe Ser Leu Pro Glu Asn Asp Pro Asp
        275                 280                 285

gtc ttc tcc agc tac ccg ctg atg cgc atg cac ccg gat acg gcg tgg       912
Val Phe Ser Ser Tyr Pro Leu Met Arg Met His Pro Asp Thr Ala Trp
    290                 295                 300

cag ccg cac cac cgc ttc cag cac ctg ttc gcg ttc cca ctg ttc gcc       960
Gln Pro His His Arg Phe Gln His Leu Phe Ala Phe Pro Leu Phe Ala
305                 310                 315                 320

ctg atg aca atc agc aag gtg ctg acc agc gat ttc gct gtc tgc ctc      1008
Leu Met Thr Ile Ser Lys Val Leu Thr Ser Asp Phe Ala Val Cys Leu
                325                 330                 335

agc atg aag aag ggg tcc atc gac tgc tcc tcc agg ctc gtc cca ctg      1056
Ser Met Lys Lys Gly Ser Ile Asp Cys Ser Ser Arg Leu Val Pro Leu
            340                 345                 350

gag ggg cag ctg ctg ttc tgg ggg gcc aag ctg gcg aac ttc ctg ttg      1104
Glu Gly Gln Leu Leu Phe Trp Gly Ala Lys Leu Ala Asn Phe Leu Leu
        355                 360                 365

cag att gtg ttg cca tgc tac ctc cac ggg aca gct atg ggc ctg gcc      1152
Gln Ile Val Leu Pro Cys Tyr Leu His Gly Thr Ala Met Gly Leu Ala
    370                 375                 380

ctc ttc tct gtt gct cac ctt gtg tcg ggg gag tac ctc gcg atc tgc      1200
Leu Phe Ser Val Ala His Leu Val Ser Gly Glu Tyr Leu Ala Ile Cys
385                 390                 395                 400

ttc atc atc aac cac atc agc gag tct tgt gag ttt atg aat aca agc      1248
Phe Ile Ile Asn His Ile Ser Glu Ser Cys Glu Phe Met Asn Thr Ser
                405                 410                 415

ttt caa acc gcc gcc cgg agg aca gag atg ctt cag gca gca cat cag      1296
Phe Gln Thr Ala Ala Arg Arg Thr Glu Met Leu Gln Ala Ala His Gln
            420                 425                 430

gca gcg gag gcc aag aag gtg aag ccc acc cct cca ccg aac gat tgg      1344
Ala Ala Glu Ala Lys Lys Val Lys Pro Thr Pro Pro Pro Asn Asp Trp
        435                 440                 445

gct gtg aca cag gtc caa tgc tgc gtg aat tgg aga tca ggt ggc gtg      1392
Ala Val Thr Gln Val Gln Cys Cys Val Asn Trp Arg Ser Gly Gly Val
    450                 455                 460

ttg gcc aat cac ctc tct gga ggc ttg aac cac cag atc gag cat cat      1440
Leu Ala Asn His Leu Ser Gly Gly Leu Asn His Gln Ile Glu His His
465                 470                 475                 480
```

```
ctg ttc ccc agc atc tcg cat gcc aac tac ccc acc atc gcc cct gtt      1488
Leu Phe Pro Ser Ile Ser His Ala Asn Tyr Pro Thr Ile Ala Pro Val
                485                 490                 495

gtg aag gag gtg tgc gag gag tac ggg ttg ccg tac aag aat tac gtc      1536
Val Lys Glu Val Cys Glu Glu Tyr Gly Leu Pro Tyr Lys Asn Tyr Val
            500                 505                 510

acg ttc tgg gat gca gtc tgt ggc atg gtt cag cac ctc cgg ttg atg      1584
Thr Phe Trp Asp Ala Val Cys Gly Met Val Gln His Leu Arg Leu Met
        515                 520                 525

ggt gct cca ccg gtg cca acg aac ggg gac aaa aag tca taa              1626
Gly Ala Pro Pro Val Pro Thr Asn Gly Asp Lys Lys Ser
    530                 535                 540


<210>  40
<211>  541
<212>  PRT
<213>  Euglena gracilis

<400>  40

Met Leu Val Leu Phe Gly Asn Phe Tyr Val Lys Gln Tyr Ser Gln Lys
1               5                   10                  15


Asn Gly Lys Pro Glu Asn Gly Ala Thr Pro Glu Asn Gly Ala Lys Pro
            20                  25                  30


Gln Pro Cys Glu Asn Gly Thr Val Glu Lys Arg Glu Asn Asp Thr Ala
        35                  40                  45


Asn Val Arg Pro Thr Arg Pro Ala Gly Pro Pro Pro Ala Thr Tyr Tyr
    50                  55                  60


Asp Ser Leu Ala Val Ser Gly Gln Gly Lys Glu Arg Leu Phe Thr Thr
65                  70                  75                  80


Asp Glu Val Arg Arg His Ile Leu Pro Thr Asp Gly Trp Leu Thr Cys
                85                  90                  95


His Glu Gly Val Tyr Asp Val Thr Asp Phe Leu Ala Lys His Pro Gly
            100                 105                 110


Gly Gly Val Ile Thr Leu Gly Leu Gly Arg Asp Cys Thr Ile Leu Ile
        115                 120                 125


Glu Ser Tyr His Pro Ala Gly Arg Pro Asp Lys Val Met Glu Lys Tyr
    130                 135                 140


Arg Ile Gly Thr Leu Gln Asp Pro Lys Thr Phe Tyr Ala Trp Gly Glu
145                 150                 155                 160
```

```
Ser Asp Phe Tyr Pro Glu Leu Lys Arg Arg Ala Leu Ala Arg Leu Lys
                165                 170                 175

Glu Ala Gly Gln Ala Arg Arg Gly Gly Leu Gly Val Lys Ala Leu Leu
            180                 185                 190

Val Leu Thr Leu Phe Phe Val Ser Trp Tyr Met Trp Val Ala His Lys
        195                 200                 205

Ser Phe Leu Trp Ala Ala Val Trp Gly Phe Ala Gly Ser His Val Gly
    210                 215                 220

Leu Ser Ile Gln His Asp Gly Asn His Gly Ala Phe Ser Arg Asn Thr
225                 230                 235                 240

Leu Val Asn Arg Leu Ala Gly Trp Gly Met Asp Leu Ile Gly Ala Ser
                245                 250                 255

Ser Thr Val Trp Glu Tyr Gln His Val Ile Gly His His Gln Tyr Thr
            260                 265                 270

Asn Leu Val Ser Asp Thr Leu Phe Ser Leu Pro Glu Asn Asp Pro Asp
        275                 280                 285

Val Phe Ser Ser Tyr Pro Leu Met Arg Met His Pro Asp Thr Ala Trp
    290                 295                 300

Gln Pro His His Arg Phe Gln His Leu Phe Ala Phe Pro Leu Phe Ala
305                 310                 315                 320

Leu Met Thr Ile Ser Lys Val Leu Thr Ser Asp Phe Ala Val Cys Leu
                325                 330                 335

Ser Met Lys Lys Gly Ser Ile Asp Cys Ser Ser Arg Leu Val Pro Leu
            340                 345                 350

Glu Gly Gln Leu Leu Phe Trp Gly Ala Lys Leu Ala Asn Phe Leu Leu
        355                 360                 365

Gln Ile Val Leu Pro Cys Tyr Leu His Gly Thr Ala Met Gly Leu Ala
    370                 375                 380

Leu Phe Ser Val Ala His Leu Val Ser Gly Glu Tyr Leu Ala Ile Cys
385                 390                 395                 400

Phe Ile Ile Asn His Ile Ser Glu Ser Cys Glu Phe Met Asn Thr Ser
```

```
                405                 410                 415

Phe Gln Thr Ala Ala Arg Arg Thr Glu Met Leu Gln Ala Ala His Gln
            420                 425                 430

Ala Ala Glu Ala Lys Lys Val Lys Pro Thr Pro Pro Pro Asn Asp Trp
        435                 440                 445

Ala Val Thr Gln Val Gln Cys Cys Val Asn Trp Arg Ser Gly Gly Val
    450                 455                 460

Leu Ala Asn His Leu Ser Gly Gly Leu Asn His Gln Ile Glu His His
465                 470                 475                 480

Leu Phe Pro Ser Ile Ser His Ala Asn Tyr Pro Thr Ile Ala Pro Val
                485                 490                 495

Val Lys Glu Val Cys Glu Glu Tyr Gly Leu Pro Tyr Lys Asn Tyr Val
            500                 505                 510

Thr Phe Trp Asp Ala Val Cys Gly Met Val Gln His Leu Arg Leu Met
        515                 520                 525

Gly Ala Pro Pro Val Pro Thr Asn Gly Asp Lys Lys Ser
    530                 535                 540


<210>  41
<211>  1548
<212>  DNA
<213>  Thraustochytrium

<220>
<221>  CDS
<222>  (1)..(1548)
<223>  Delta-4-Desaturase


<400>  41
atg acg gtc ggg ttt gac gaa acg gtg act atg gac acg gtc cgc aac      48
Met Thr Val Gly Phe Asp Glu Thr Val Thr Met Asp Thr Val Arg Asn
1               5                   10                  15

cac aac atg ccg gac gac gcc tgg tgc gcg atc cac ggc acc gtg tac      96
His Asn Met Pro Asp Asp Ala Trp Cys Ala Ile His Gly Thr Val Tyr
            20                  25                  30

gac atc acc aag ttc agc aag gtg cac ccc ggc ggg gac atc atc atg     144
Asp Ile Thr Lys Phe Ser Lys Val His Pro Gly Gly Asp Ile Ile Met
        35                  40                  45

ctg gcc gct ggc aag gag gcc acc atc ctg ttc gag acc tac cac atc     192
Leu Ala Ala Gly Lys Glu Ala Thr Ile Leu Phe Glu Thr Tyr His Ile
    50                  55                  60
```

```
aag ggc gtc ccg gac gcg gtg ctg cgc aag tac aag gtc ggc aag ctc      240
Lys Gly Val Pro Asp Ala Val Leu Arg Lys Tyr Lys Val Gly Lys Leu
65                  70                  75                  80

ccc cag ggc aag aag ggc gaa acg agc cac atg ccc acc ggg ctc gac      288
Pro Gln Gly Lys Lys Gly Glu Thr Ser His Met Pro Thr Gly Leu Asp
                85                  90                  95

tcg gcc tcc tac tac tcg tgg gac agc gag ttt tac agg gtg ctc cgc      336
Ser Ala Ser Tyr Tyr Ser Trp Asp Ser Glu Phe Tyr Arg Val Leu Arg
            100                 105                 110

gag cgc gtc gcc aag aag ctg gcc gag ccc ggc ctc atg cag cgc gcg      384
Glu Arg Val Ala Lys Lys Leu Ala Glu Pro Gly Leu Met Gln Arg Ala
        115                 120                 125

cgc atg gag ctc tgg gcc aag gcg atc ttc ctc ctg gca ggt ttc tgg      432
Arg Met Glu Leu Trp Ala Lys Ala Ile Phe Leu Leu Ala Gly Phe Trp
    130                 135                 140

ggc tcc ctt tac gcc atg tgc gtg cta gac ccg cac ggc ggt gcc atg      480
Gly Ser Leu Tyr Ala Met Cys Val Leu Asp Pro His Gly Gly Ala Met
145                 150                 155                 160

gta gcc gcc gtt acg ctc ggc gtg ttc gct gcc ttt gtc gga act tgc      528
Val Ala Ala Val Thr Leu Gly Val Phe Ala Ala Phe Val Gly Thr Cys
                165                 170                 175

atc cag cac gac ggc agc cac ggc gcc ttc tcc aag tcg cga ttc atg      576
Ile Gln His Asp Gly Ser His Gly Ala Phe Ser Lys Ser Arg Phe Met
            180                 185                 190

aac aag gcg gcg ggc tgg acc ctc gac atg atc ggc gcg agt gcg atg      624
Asn Lys Ala Ala Gly Trp Thr Leu Asp Met Ile Gly Ala Ser Ala Met
        195                 200                 205

acc tgg gag atg cag cac gtt ctt ggc cac cac ccg tac acc aac ctc      672
Thr Trp Glu Met Gln His Val Leu Gly His His Pro Tyr Thr Asn Leu
    210                 215                 220

atc gag atg gag aac ggt ttg gcc aag gtc aag ggc gcc gac gtc gac      720
Ile Glu Met Glu Asn Gly Leu Ala Lys Val Lys Gly Ala Asp Val Asp
225                 230                 235                 240

ccg aag aag gtc gac cag gag agc gac ccg gac gtc ttc agt acg tac      768
Pro Lys Lys Val Asp Gln Glu Ser Asp Pro Asp Val Phe Ser Thr Tyr
                245                 250                 255

ccg atg ctt cgc ctg cac ccg tgg cac cgc cag cgg ttt tac cac aag      816
Pro Met Leu Arg Leu His Pro Trp His Arg Gln Arg Phe Tyr His Lys
            260                 265                 270

ttc cag cac ctg tac gcc ccg ttt atc ttt ggg tct atg acg att aac      864
Phe Gln His Leu Tyr Ala Pro Phe Ile Phe Gly Ser Met Thr Ile Asn
        275                 280                 285

aag gtg att tcc cag gat gtc ggg gtt gtg ctg cgc aag cgc ctg ttc      912
Lys Val Ile Ser Gln Asp Val Gly Val Val Leu Arg Lys Arg Leu Phe
    290                 295                 300

cag atc gac gcc aac tgc cgg tat ggc agc ccc tgg tac gtg gcc cgc      960
Gln Ile Asp Ala Asn Cys Arg Tyr Gly Ser Pro Trp Tyr Val Ala Arg
305                 310                 315                 320
```

```
ttc tgg atc atg aag ctc ctc acc acg ctc tac atg gtg gcg ctt ccc      1008
Phe Trp Ile Met Lys Leu Leu Thr Thr Leu Tyr Met Val Ala Leu Pro
                325                 330                 335

atg tac atg cag ggg cct gct cag ggc ttg aag ctt ttc ttc atg gcc      1056
Met Tyr Met Gln Gly Pro Ala Gln Gly Leu Lys Leu Phe Phe Met Ala
            340                 345                 350

cac ttc acc tgc gga gag gtc ctc gcc acc atg ttt att gtc aac cac      1104
His Phe Thr Cys Gly Glu Val Leu Ala Thr Met Phe Ile Val Asn His
        355                 360                 365

atc atc gag ggc gtc agc tac gct tcc aag gac gcg gtc aag ggc gtc      1152
Ile Ile Glu Gly Val Ser Tyr Ala Ser Lys Asp Ala Val Lys Gly Val
    370                 375                 380

atg gct ccg ccg cgc act gtg cac ggt gtc acc ccg atg cag gtg acg      1200
Met Ala Pro Pro Arg Thr Val His Gly Val Thr Pro Met Gln Val Thr
385                 390                 395                 400

caa aag gcg ctc agt gcg gcc gag tcg gcc aag tcg gac gcc gac aag      1248
Gln Lys Ala Leu Ser Ala Ala Glu Ser Ala Lys Ser Asp Ala Asp Lys
                405                 410                 415

acg acc atg atc ccc ctc aac gac tgg gcc gct gtg cag tgc cag acc      1296
Thr Thr Met Ile Pro Leu Asn Asp Trp Ala Ala Val Gln Cys Gln Thr
            420                 425                 430

tct gtg aac tgg gct gtc ggg tcg tgg ttt tgg aac cac ttt tcg ggc      1344
Ser Val Asn Trp Ala Val Gly Ser Trp Phe Trp Asn His Phe Ser Gly
        435                 440                 445

ggc ctc aac cac cag att gag cac cac tgc ttc ccc caa aac ccc cac      1392
Gly Leu Asn His Gln Ile Glu His His Cys Phe Pro Gln Asn Pro His
    450                 455                 460

acg gtc aac gtc tac atc tcg ggc atc gtc aag gag acc tgc gaa gaa      1440
Thr Val Asn Val Tyr Ile Ser Gly Ile Val Lys Glu Thr Cys Glu Glu
465                 470                 475                 480

tac ggc gtg ccg tac cag gct gag atc agc ctc ttc tct gcc tat ttc      1488
Tyr Gly Val Pro Tyr Gln Ala Glu Ile Ser Leu Phe Ser Ala Tyr Phe
                485                 490                 495

aag atg ctg tcg cac ctc cgc acg ctc ggc aac gag gac ctc acg gcc      1536
Lys Met Leu Ser His Leu Arg Thr Leu Gly Asn Glu Asp Leu Thr Ala
            500                 505                 510

tgg tcc acg tga                                                      1548
Trp Ser Thr
        515


<210>  42
<211>  515
<212>  PRT
<213>  Thraustochytrium

<400>  42

Met Thr Val Gly Phe Asp Glu Thr Val Thr Met Asp Thr Val Arg Asn
1               5                   10                  15
```

```
His Asn Met Pro Asp Asp Ala Trp Cys Ala Ile His Gly Thr Val Tyr
            20                  25                  30

Asp Ile Thr Lys Phe Ser Lys Val His Pro Gly Gly Asp Ile Ile Met
        35                  40                  45

Leu Ala Ala Gly Lys Glu Ala Thr Ile Leu Phe Glu Thr Tyr His Ile
    50                  55                  60

Lys Gly Val Pro Asp Ala Val Leu Arg Lys Tyr Lys Val Gly Lys Leu
65                  70                  75                  80

Pro Gln Gly Lys Lys Gly Glu Thr Ser His Met Pro Thr Gly Leu Asp
                85                  90                  95

Ser Ala Ser Tyr Tyr Ser Trp Asp Ser Glu Phe Tyr Arg Val Leu Arg
            100                 105                 110

Glu Arg Val Ala Lys Lys Leu Ala Glu Pro Gly Leu Met Gln Arg Ala
        115                 120                 125

Arg Met Glu Leu Trp Ala Lys Ala Ile Phe Leu Leu Ala Gly Phe Trp
    130                 135                 140

Gly Ser Leu Tyr Ala Met Cys Val Leu Asp Pro His Gly Gly Ala Met
145                 150                 155                 160

Val Ala Ala Val Thr Leu Gly Val Phe Ala Ala Phe Val Gly Thr Cys
                165                 170                 175

Ile Gln His Asp Gly Ser His Gly Ala Phe Ser Lys Ser Arg Phe Met
            180                 185                 190

Asn Lys Ala Ala Gly Trp Thr Leu Asp Met Ile Gly Ala Ser Ala Met
        195                 200                 205

Thr Trp Glu Met Gln His Val Leu Gly His His Pro Tyr Thr Asn Leu
    210                 215                 220

Ile Glu Met Glu Asn Gly Leu Ala Lys Val Lys Gly Ala Asp Val Asp
225                 230                 235                 240

Pro Lys Lys Val Asp Gln Glu Ser Asp Pro Asp Val Phe Ser Thr Tyr
                245                 250                 255

Pro Met Leu Arg Leu His Pro Trp His Arg Gln Arg Phe Tyr His Lys
```

260 265 270

Phe Gln His Leu Tyr Ala Pro Phe Ile Phe Gly Ser Met Thr Ile Asn
275 280 285

Lys Val Ile Ser Gln Asp Val Gly Val Val Leu Arg Lys Arg Leu Phe
290 295 300

Gln Ile Asp Ala Asn Cys Arg Tyr Gly Ser Pro Trp Tyr Val Ala Arg
305 310 315 320

Phe Trp Ile Met Lys Leu Leu Thr Thr Leu Tyr Met Val Ala Leu Pro
325 330 335

Met Tyr Met Gln Gly Pro Ala Gln Gly Leu Lys Leu Phe Phe Met Ala
340 345 350

His Phe Thr Cys Gly Glu Val Leu Ala Thr Met Phe Ile Val Asn His
355 360 365

Ile Ile Glu Gly Val Ser Tyr Ala Ser Lys Asp Ala Val Lys Gly Val
370 375 380

Met Ala Pro Pro Arg Thr Val His Gly Val Thr Pro Met Gln Val Thr
385 390 395 400

Gln Lys Ala Leu Ser Ala Ala Glu Ser Ala Lys Ser Asp Ala Asp Lys
405 410 415

Thr Thr Met Ile Pro Leu Asn Asp Trp Ala Ala Val Gln Cys Gln Thr
420 425 430

Ser Val Asn Trp Ala Val Gly Ser Trp Phe Trp Asn His Phe Ser Gly
435 440 445

Gly Leu Asn His Gln Ile Glu His His Cys Phe Pro Gln Asn Pro His
450 455 460

Thr Val Asn Val Tyr Ile Ser Gly Ile Val Lys Glu Thr Cys Glu Glu
465 470 475 480

Tyr Gly Val Pro Tyr Gln Ala Glu Ile Ser Leu Phe Ser Ala Tyr Phe
485 490 495

Lys Met Leu Ser His Leu Arg Thr Leu Gly Asn Glu Asp Leu Thr Ala
500 505 510

```
Trp Ser Thr
        515


<210>  43
<211>  960
<212>  DNA
<213>  Thallasiosira pseudonana

<220>
<221>  CDS
<222>  (1)..(960)
<223>  Delta-5-Elongase


<400>  43
atg gtg ttg tac aat gtg gcg caa gtg ctg ctc aat ggg tgg acg gtg        48
Met Val Leu Tyr Asn Val Ala Gln Val Leu Leu Asn Gly Trp Thr Val
1               5                   10                  15

tat gcg att gtg gat gcg gtg atg aat aga gac cat ccg ttt att gga        96
Tyr Ala Ile Val Asp Ala Val Met Asn Arg Asp His Pro Phe Ile Gly
            20                  25                  30

agt aga agt ttg gtt ggg gcg gcg ttg cat agt ggg agc tcg tat gcg       144
Ser Arg Ser Leu Val Gly Ala Ala Leu His Ser Gly Ser Ser Tyr Ala
        35                  40                  45

gtg tgg gtt cat tat tgt gat aag tat ttg gag ttc ttt gat acg tat       192
Val Trp Val His Tyr Cys Asp Lys Tyr Leu Glu Phe Phe Asp Thr Tyr
    50                  55                  60

ttt atg gtg ttg agg ggg aaa atg gac cag atg gta ctt ggt gaa gtt       240
Phe Met Val Leu Arg Gly Lys Met Asp Gln Met Val Leu Gly Glu Val
65                  70                  75                  80

ggt ggc agt gtg tgg tgt ggc gtt gga tat atg gat atg gag aag atg       288
Gly Gly Ser Val Trp Cys Gly Val Gly Tyr Met Asp Met Glu Lys Met
                85                  90                  95

ata cta ctc agc ttt gga gtg cat cgg tct gct cag gga acg ggg aag       336
Ile Leu Leu Ser Phe Gly Val His Arg Ser Ala Gln Gly Thr Gly Lys
            100                 105                 110

gct ttc acc aac aac gtt acc aat cca cat ctc acg ctt cca cct cat       384
Ala Phe Thr Asn Asn Val Thr Asn Pro His Leu Thr Leu Pro Pro His
        115                 120                 125

tct aca aaa aca aaa aaa cag gtc tcc ttc ctc cac atc tac cac cac       432
Ser Thr Lys Thr Lys Lys Gln Val Ser Phe Leu His Ile Tyr His His
    130                 135                 140

acg acc ata gcg tgg gca tgg tgg atc gcc ctc cgc ttc tcc ccc ggt       480
Thr Thr Ile Ala Trp Ala Trp Trp Ile Ala Leu Arg Phe Ser Pro Gly
145                 150                 155                 160

gga gac att tac ttc ggg gca ctc ctc aac tcc atc atc cac gtc ctc       528
Gly Asp Ile Tyr Phe Gly Ala Leu Leu Asn Ser Ile Ile His Val Leu
                165                 170                 175

atg tat tcc tac tac gcc ctt gcc cta ctc aag gtc agt tgt cca tgg       576
Met Tyr Ser Tyr Tyr Ala Leu Ala Leu Leu Lys Val Ser Cys Pro Trp
            180                 185                 190
```

```
aaa cga tac ctg act caa gct caa tta ttg caa ttc aca agt gtg gtg      624
Lys Arg Tyr Leu Thr Gln Ala Gln Leu Leu Gln Phe Thr Ser Val Val
        195                 200                 205

gtt tat acg ggg tgt acg ggt tat act cat tac tat cat acg aag cat      672
Val Tyr Thr Gly Cys Thr Gly Tyr Thr His Tyr Tyr His Thr Lys His
    210                 215                 220

gga gcg gat gag aca cag cct agt tta gga acg tat tat ttc tgt tgt      720
Gly Ala Asp Glu Thr Gln Pro Ser Leu Gly Thr Tyr Tyr Phe Cys Cys
225                 230                 235                 240

gga gtg cag gtg ttt gag atg gtt agt ttg ttt gta ctc ttt tcc atc      768
Gly Val Gln Val Phe Glu Met Val Ser Leu Phe Val Leu Phe Ser Ile
                245                 250                 255

ttt tat aaa cga tcc tat tcg aag aag aac aag tca gga gga aag gat      816
Phe Tyr Lys Arg Ser Tyr Ser Lys Lys Asn Lys Ser Gly Gly Lys Asp
            260                 265                 270

agc aag aag aat gat gat ggg aat aat gag gat caa tgt cac aag gct      864
Ser Lys Lys Asn Asp Asp Gly Asn Asn Glu Asp Gln Cys His Lys Ala
        275                 280                 285

atg aag gat ata tcg gag ggt gcg aag gag gtt gtg ggg cat gca gcg      912
Met Lys Asp Ile Ser Glu Gly Ala Lys Glu Val Val Gly His Ala Ala
    290                 295                 300

aag gat gct gga aag ttg gtg gct acg aga gta agg tgt aag gtg taa      960
Lys Asp Ala Gly Lys Leu Val Ala Thr Arg Val Arg Cys Lys Val
305                 310                 315


<210>  44
<211>  319
<212>  PRT
<213>  Thallasiosira pseudonana

<400>  44

Met Val Leu Tyr Asn Val Ala Gln Val Leu Leu Asn Gly Trp Thr Val
1               5                   10                  15


Tyr Ala Ile Val Asp Ala Val Met Asn Arg Asp His Pro Phe Ile Gly
            20                  25                  30


Ser Arg Ser Leu Val Gly Ala Ala Leu His Ser Gly Ser Ser Tyr Ala
        35                  40                  45


Val Trp Val His Tyr Cys Asp Lys Tyr Leu Glu Phe Phe Asp Thr Tyr
    50                  55                  60


Phe Met Val Leu Arg Gly Lys Met Asp Gln Met Val Leu Gly Glu Val
65                  70                  75                  80


Gly Gly Ser Val Trp Cys Gly Val Gly Tyr Met Asp Met Glu Lys Met
                85                  90                  95
```

Ile Leu Leu Ser Phe Gly Val His Arg Ser Ala Gln Gly Thr Gly Lys
100 105 110

Ala Phe Thr Asn Asn Val Thr Asn Pro His Leu Thr Leu Pro Pro His
115 120 125

Ser Thr Lys Thr Lys Lys Gln Val Ser Phe Leu His Ile Tyr His His
130 135 140

Thr Thr Ile Ala Trp Ala Trp Trp Ile Ala Leu Arg Phe Ser Pro Gly
145 150 155 160

Gly Asp Ile Tyr Phe Gly Ala Leu Leu Asn Ser Ile Ile His Val Leu
165 170 175

Met Tyr Ser Tyr Tyr Ala Leu Ala Leu Leu Lys Val Ser Cys Pro Trp
180 185 190

Lys Arg Tyr Leu Thr Gln Ala Gln Leu Leu Gln Phe Thr Ser Val Val
195 200 205

Val Tyr Thr Gly Cys Thr Gly Tyr Thr His Tyr Tyr His Thr Lys His
210 215 220

Gly Ala Asp Glu Thr Gln Pro Ser Leu Gly Thr Tyr Tyr Phe Cys Cys
225 230 235 240

Gly Val Gln Val Phe Glu Met Val Ser Leu Phe Val Leu Phe Ser Ile
245 250 255

Phe Tyr Lys Arg Ser Tyr Ser Lys Lys Asn Lys Ser Gly Gly Lys Asp
260 265 270

Ser Lys Lys Asn Asp Asp Gly Asn Asn Glu Asp Gln Cys His Lys Ala
275 280 285

Met Lys Asp Ile Ser Glu Gly Ala Lys Glu Val Val Gly His Ala Ala
290 295 300

Lys Asp Ala Gly Lys Leu Val Ala Thr Arg Val Arg Cys Lys Val
305 310 315

<210> 45
<211> 819
<212> DNA
<213> Thallasiosira pseudonana

```
<220>
<221>  CDS
<222>  (1)..(819)
<223>  Delta-5-Elongase


<400>  45
atg gac gcc tac aac gct gca atg gat aag atc ggt gcc gcc atc atc      48
Met Asp Ala Tyr Asn Ala Ala Met Asp Lys Ile Gly Ala Ala Ile Ile
1               5                   10                  15

gat tgg tct gat ccc gat ggc aag ttc cgt gcc gat aga gag gac tgg      96
Asp Trp Ser Asp Pro Asp Gly Lys Phe Arg Ala Asp Arg Glu Asp Trp
            20                  25                  30

tgg ctc tgc gac ttc cgt agc gcc atc acc atc gcy ctc atc tac atc     144
Trp Leu Cys Asp Phe Arg Ser Ala Ile Thr Ile Ala Leu Ile Tyr Ile
        35                  40                  45

gcc ttc gtc atc ctc ggt tcc gcc gtc atg caa tcc ctc ccc gca atg     192
Ala Phe Val Ile Leu Gly Ser Ala Val Met Gln Ser Leu Pro Ala Met
    50                  55                  60

gat ccc tac ccc atc aaa ttc ctc tac aac gtc tcc caa atc ttc ctt     240
Asp Pro Tyr Pro Ile Lys Phe Leu Tyr Asn Val Ser Gln Ile Phe Leu
65                  70                  75                  80

tgt gcc tac atg act gtc gag gcg gga ttt ttg gcc tac cgc aat gga     288
Cys Ala Tyr Met Thr Val Glu Ala Gly Phe Leu Ala Tyr Arg Asn Gly
                85                  90                  95

ttt acc gtc atg cct tgc aat cat ttc aat gtg aat gat cct ccc gtg     336
Phe Thr Val Met Pro Cys Asn His Phe Asn Val Asn Asp Pro Pro Val
            100                 105                 110

gcg aat ctt ctt tgg ttg ttt tat att tcc aag gtg tgg gac ttt tgg     384
Ala Asn Leu Leu Trp Leu Phe Tyr Ile Ser Lys Val Trp Asp Phe Trp
        115                 120                 125

gat acc att ttc att gtg ttg ggg aag aag tgg cgt caa tta tct ttc     432
Asp Thr Ile Phe Ile Val Leu Gly Lys Lys Trp Arg Gln Leu Ser Phe
    130                 135                 140

ttg cat gta tac cat cac acc acc atc ttt cta ttc tat tgg ctg aat     480
Leu His Val Tyr His His Thr Thr Ile Phe Leu Phe Tyr Trp Leu Asn
145                 150                 155                 160

gcc aat gtc ttg tac gat ggt gac atc ttc ctt acc atc ttg ctc aat     528
Ala Asn Val Leu Tyr Asp Gly Asp Ile Phe Leu Thr Ile Leu Leu Asn
                165                 170                 175

gga ttc atc cac acg gtg atg tac acg tat tac ttc atc tgt atg cat     576
Gly Phe Ile His Thr Val Met Tyr Thr Tyr Tyr Phe Ile Cys Met His
            180                 185                 190

acc aaa gat tcc aag acg ggc aag agt ctt cct ata tgg tgg aag tcg     624
Thr Lys Asp Ser Lys Thr Gly Lys Ser Leu Pro Ile Trp Trp Lys Ser
        195                 200                 205

agt ttg acg gcg ttt cag ttg ttg caa ttc act atc atg atg agt cag     672
Ser Leu Thr Ala Phe Gln Leu Leu Gln Phe Thr Ile Met Met Ser Gln
    210                 215                 220
```

```
gct acc tac ctt gtc ttc cac ggg tgt gat aag gtg tcg ctt cgt atc       720
Ala Thr Tyr Leu Val Phe His Gly Cys Asp Lys Val Ser Leu Arg Ile
225                 230                 235                 240

acg att gtg tac ttt gtg tac att ttg agt ttg ttc ttc ctt ttt gct       768
Thr Ile Val Tyr Phe Val Tyr Ile Leu Ser Leu Phe Phe Leu Phe Ala
                245                 250                 255

cag ttc ttt gtg caa tca tac atg gca ccc aaa aag aag aag agt gct       816
Gln Phe Phe Val Gln Ser Tyr Met Ala Pro Lys Lys Lys Lys Ser Ala
            260                 265                 270

tag                                                                   819


<210>  46
<211>  272
<212>  PRT
<213>  Thallasiosira pseudonana

<400>  46

Met Asp Ala Tyr Asn Ala Ala Met Asp Lys Ile Gly Ala Ala Ile Ile
1               5                   10                  15


Asp Trp Ser Asp Pro Asp Gly Lys Phe Arg Ala Asp Arg Glu Asp Trp
            20                  25                  30


Trp Leu Cys Asp Phe Arg Ser Ala Ile Thr Ile Ala Leu Ile Tyr Ile
        35                  40                  45


Ala Phe Val Ile Leu Gly Ser Ala Val Met Gln Ser Leu Pro Ala Met
    50                  55                  60


Asp Pro Tyr Pro Ile Lys Phe Leu Tyr Asn Val Ser Gln Ile Phe Leu
65                  70                  75                  80


Cys Ala Tyr Met Thr Val Glu Ala Gly Phe Leu Ala Tyr Arg Asn Gly
                85                  90                  95


Phe Thr Val Met Pro Cys Asn His Phe Asn Val Asn Asp Pro Pro Val
            100                 105                 110


Ala Asn Leu Leu Trp Leu Phe Tyr Ile Ser Lys Val Trp Asp Phe Trp
        115                 120                 125


Asp Thr Ile Phe Ile Val Leu Gly Lys Lys Trp Arg Gln Leu Ser Phe
    130                 135                 140


Leu His Val Tyr His His Thr Thr Ile Phe Leu Phe Tyr Trp Leu Asn
145                 150                 155                 160


Ala Asn Val Leu Tyr Asp Gly Asp Ile Phe Leu Thr Ile Leu Leu Asn
```

165 170 175

Gly Phe Ile His Thr Val Met Tyr Thr Tyr Tyr Phe Ile Cys Met His
180 185 190

Thr Lys Asp Ser Lys Thr Gly Lys Ser Leu Pro Ile Trp Trp Lys Ser
195 200 205

Ser Leu Thr Ala Phe Gln Leu Leu Gln Phe Thr Ile Met Met Ser Gln
210 215 220

Ala Thr Tyr Leu Val Phe His Gly Cys Asp Lys Val Ser Leu Arg Ile
225 230 235 240

Thr Ile Val Tyr Phe Val Tyr Ile Leu Ser Leu Phe Phe Leu Phe Ala
245 250 255

Gln Phe Phe Val Gln Ser Tyr Met Ala Pro Lys Lys Lys Lys Ser Ala
260 265 270

<210> 47
<211> 936
<212> DNA
<213> Crypthecodinium cohnii

<220>
<221> CDS
<222> (1)..(936)
<223> Delta-5-Elongase

<400> 47
atg tct gcc ttc atg act ctc cca cag gct ctc tcc gat gtg acc tcg 48
Met Ser Ala Phe Met Thr Leu Pro Gln Ala Leu Ser Asp Val Thr Ser
1 5 10 15

gcc ttg gtc acg ctg gga aag gat gtc tcc agc cct tca gct ttt caa 96
Ala Leu Val Thr Leu Gly Lys Asp Val Ser Ser Pro Ser Ala Phe Gln
20 25 30

gct gtc act ggc ttc tgc agg gag cag tgg ggg att ccg aca gta ttc 144
Ala Val Thr Gly Phe Cys Arg Glu Gln Trp Gly Ile Pro Thr Val Phe
35 40 45

tgc ctg ggc tac ttg gcc atg gtc tac gcg gcc aga aga ccc ctc ccg 192
Cys Leu Gly Tyr Leu Ala Met Val Tyr Ala Ala Arg Arg Pro Leu Pro
50 55 60

cag cac ggc tac atg gtt gcg gtg gac cgt tgc ttc gct gct tgg aac 240
Gln His Gly Tyr Met Val Ala Val Asp Arg Cys Phe Ala Ala Trp Asn
65 70 75 80

ttg gct ctc tct gtc ttc agc act tgg ggc ttc tac cac atg gct gtc 288
Leu Ala Leu Ser Val Phe Ser Thr Trp Gly Phe Tyr His Met Ala Val
85 90 95

```
ggg ctc tac aac atg aca gag acg agg ggc ttg caa ttc acc atc tgc       336
Gly Leu Tyr Asn Met Thr Glu Thr Arg Gly Leu Gln Phe Thr Ile Cys
            100                 105                 110

ggt tcg act ggg gag ctc gtg cag aac ctt cag act ggc cca acc gct       384
Gly Ser Thr Gly Glu Leu Val Gln Asn Leu Gln Thr Gly Pro Thr Ala
        115                 120                 125

ctg gcg ctc tgc ctc ttc tgc ttc agc aag atc ccc gag ttg atg gac       432
Leu Ala Leu Cys Leu Phe Cys Phe Ser Lys Ile Pro Glu Leu Met Asp
    130                 135                 140

acg gtg ttt ctc atc ctg aag gcc aag aag gtc cgc ttc ttg cag tgg       480
Thr Val Phe Leu Ile Leu Lys Ala Lys Lys Val Arg Phe Leu Gln Trp
145                 150                 155                 160

tac cac cat gcc aca gtc atg ctc ttc tgt tgg ctc gcc ctc gcg acg       528
Tyr His His Ala Thr Val Met Leu Phe Cys Trp Leu Ala Leu Ala Thr
                165                 170                 175

gag tac act cct ggc ttg tgg ttt gcg gcg acg aac tac ttc gtg cac       576
Glu Tyr Thr Pro Gly Leu Trp Phe Ala Ala Thr Asn Tyr Phe Val His
            180                 185                 190

tcc atc atg tac atg tac ttc ttc ctc atg acc ttc aag tcg gcc gcg       624
Ser Ile Met Tyr Met Tyr Phe Phe Leu Met Thr Phe Lys Ser Ala Ala
        195                 200                 205

aag gtg gtg aag ccc atc gcc cct ctc atc aca gtt atc cag att gct       672
Lys Val Val Lys Pro Ile Ala Pro Leu Ile Thr Val Ile Gln Ile Ala
    210                 215                 220

cag atg gtc tgg ggc ctc atc gtc aac ggc atc gcc atc acc acc ttc       720
Gln Met Val Trp Gly Leu Ile Val Asn Gly Ile Ala Ile Thr Thr Phe
225                 230                 235                 240

ttc acg act ggt gcc tgc cag atc cag tct gtg act gtg tat tcg gcc       768
Phe Thr Thr Gly Ala Cys Gln Ile Gln Ser Val Thr Val Tyr Ser Ala
                245                 250                 255

atc atc atg tac gct tcg tac ttc tac ctg ttc tcc cag ctc ttc ttc       816
Ile Ile Met Tyr Ala Ser Tyr Phe Tyr Leu Phe Ser Gln Leu Phe Phe
            260                 265                 270

gag gcc cat ggt gcc gct ggc aag aac aag aag aag ttg acc cgc gag       864
Glu Ala His Gly Ala Ala Gly Lys Asn Lys Lys Lys Leu Thr Arg Glu
        275                 280                 285

ctc tct cga aaa atc tcg gag gct ctc ctg aac acc ggt gac gag gtt       912
Leu Ser Arg Lys Ile Ser Glu Ala Leu Leu Asn Thr Gly Asp Glu Val
    290                 295                 300

tcc aag cac ctg aag gtg aat tga                                       936
Ser Lys His Leu Lys Val Asn
305                 310
```

<210> 48
<211> 311
<212> PRT
<213> Crypthecodinium cohnii

<400> 48

```
Met Ser Ala Phe Met Thr Leu Pro Gln Ala Leu Ser Asp Val Thr Ser
1               5                   10                  15

Ala Leu Val Thr Leu Gly Lys Asp Val Ser Ser Pro Ser Ala Phe Gln
            20                  25                  30

Ala Val Thr Gly Phe Cys Arg Glu Gln Trp Gly Ile Pro Thr Val Phe
        35                  40                  45

Cys Leu Gly Tyr Leu Ala Met Val Tyr Ala Ala Arg Arg Pro Leu Pro
    50                  55                  60

Gln His Gly Tyr Met Val Ala Val Asp Arg Cys Phe Ala Ala Trp Asn
65                  70                  75                  80

Leu Ala Leu Ser Val Phe Ser Thr Trp Gly Phe Tyr His Met Ala Val
                85                  90                  95

Gly Leu Tyr Asn Met Thr Glu Thr Arg Gly Leu Gln Phe Thr Ile Cys
            100                 105                 110

Gly Ser Thr Gly Glu Leu Val Gln Asn Leu Gln Thr Gly Pro Thr Ala
        115                 120                 125

Leu Ala Leu Cys Leu Phe Cys Phe Ser Lys Ile Pro Glu Leu Met Asp
    130                 135                 140

Thr Val Phe Leu Ile Leu Lys Ala Lys Lys Val Arg Phe Leu Gln Trp
145                 150                 155                 160

Tyr His His Ala Thr Val Met Leu Phe Cys Trp Leu Ala Leu Ala Thr
                165                 170                 175

Glu Tyr Thr Pro Gly Leu Trp Phe Ala Ala Thr Asn Tyr Phe Val His
            180                 185                 190

Ser Ile Met Tyr Met Tyr Phe Phe Leu Met Thr Phe Lys Ser Ala Ala
        195                 200                 205

Lys Val Val Lys Pro Ile Ala Pro Leu Ile Thr Val Ile Gln Ile Ala
    210                 215                 220

Gln Met Val Trp Gly Leu Ile Val Asn Gly Ile Ala Ile Thr Thr Phe
225                 230                 235                 240

Phe Thr Thr Gly Ala Cys Gln Ile Gln Ser Val Thr Val Tyr Ser Ala
                245                 250                 255
```

```
Ile Ile Met Tyr Ala Ser Tyr Phe Tyr Leu Phe Ser Gln Leu Phe Phe
            260                 265                 270

Glu Ala His Gly Ala Ala Gly Lys Asn Lys Lys Lys Leu Thr Arg Glu
        275                 280                 285

Leu Ser Arg Lys Ile Ser Glu Ala Leu Leu Asn Thr Gly Asp Glu Val
    290                 295                 300

Ser Lys His Leu Lys Val Asn
305                 310


<210>  49
<211>  927
<212>  DNA
<213>  Crypthecodinium cohnii

<220>
<221>  CDS
<222>  (1)..(927)
<223>  Delta-5-Elongase


<400>  49
atg gct tcc tac caa caa gca ttc tcc gaa ttg gct aga gct ttg tcc       48
Met Ala Ser Tyr Gln Gln Ala Phe Ser Glu Leu Ala Arg Ala Leu Ser
1               5                   10                  15

act ttg aac cac gac ttc tcc agc gtc gag cca ttc aaa gtc gtg acg       96
Thr Leu Asn His Asp Phe Ser Ser Val Glu Pro Phe Lys Val Val Thr
            20                  25                  30

cag ttc tgc agg gac cag tgg gcg atc ccg aca gtc ttt tgc atc ggt      144
Gln Phe Cys Arg Asp Gln Trp Ala Ile Pro Thr Val Phe Cys Ile Gly
        35                  40                  45

tac ttg gca atg gtc tac gcc acg cga aga cct atc gcg aag cac ccc      192
Tyr Leu Ala Met Val Tyr Ala Thr Arg Arg Pro Ile Ala Lys His Pro
    50                  55                  60

tac atg tct ctc gtg gat cgc tgc ttt gcg gcc tgg aac ttg ggc ctc      240
Tyr Met Ser Leu Val Asp Arg Cys Phe Ala Ala Trp Asn Leu Gly Leu
65                  70                  75                  80

tcg ctc ttc agt tgc tgg ggc ttc tac cac atg gca gtg gga ctc tcc      288
Ser Leu Phe Ser Cys Trp Gly Phe Tyr His Met Ala Val Gly Leu Ser
                85                  90                  95

cac acc act tgg aat ttc ggg ctc cag ttc acc atc tgc ggc agc acc      336
His Thr Thr Trp Asn Phe Gly Leu Gln Phe Thr Ile Cys Gly Ser Thr
            100                 105                 110

acg gag ctt gtg aat ggc ttc cag aag ggc ccg gcg gcc ctc gcc ctc      384
Thr Glu Leu Val Asn Gly Phe Gln Lys Gly Pro Ala Ala Leu Ala Leu
        115                 120                 125

atc ctg ttc tgc ttc tcc aag atc ccg gag ttg ggc gac acc gtc ttc      432
```

```
Ile Leu Phe Cys Phe Ser Lys Ile Pro Glu Leu Gly Asp Thr Val Phe
    130                 135                 140

ttg atc ttg aag gga aag aag gtc cgc ttc ttg cag tgg tac cac cac        480
Leu Ile Leu Lys Gly Lys Lys Val Arg Phe Leu Gln Trp Tyr His His
145                 150                 155                 160

acg acc gtg atg ctc ttc tgt tgg atg gcc ttg gcg act gag tac act        528
Thr Thr Val Met Leu Phe Cys Trp Met Ala Leu Ala Thr Glu Tyr Thr
                165                 170                 175

cct gga ttg tgg ttc gcg gcc acg aac tac ttc gtg cac tcc atc atg        576
Pro Gly Leu Trp Phe Ala Ala Thr Asn Tyr Phe Val His Ser Ile Met
            180                 185                 190

tac atg tac ttc ttc ctc atg acc ttc aag acg gcc gcc ggc atc atc        624
Tyr Met Tyr Phe Phe Leu Met Thr Phe Lys Thr Ala Ala Gly Ile Ile
        195                 200                 205

aag ccc atc gcg cct ctc atc acc atc atc cag atc tcc cag atg gtc        672
Lys Pro Ile Ala Pro Leu Ile Thr Ile Ile Gln Ile Ser Gln Met Val
    210                 215                 220

tgg ggc ttg gtc gtg aac gcc atc gcc gtc ggc acc ttc ttc acc aca        720
Trp Gly Leu Val Val Asn Ala Ile Ala Val Gly Thr Phe Phe Thr Thr
225                 230                 235                 240

ggc aac tgc cag atc cag gca gtg aca gtc tac tcc gcc atc gtg atg        768
Gly Asn Cys Gln Ile Gln Ala Val Thr Val Tyr Ser Ala Ile Val Met
                245                 250                 255

tac gcc tcc tac ttc tac ctc ttc ggc cag ctc ttc ttc gag gcc cag        816
Tyr Ala Ser Tyr Phe Tyr Leu Phe Gly Gln Leu Phe Phe Glu Ala Gln
            260                 265                 270

ggt tcg gct gga aag gac aag aag aag ttg gcc cga gag ctg agc cga        864
Gly Ser Ala Gly Lys Asp Lys Lys Lys Leu Ala Arg Glu Leu Ser Arg
        275                 280                 285

aag gtc tcg cgg gct ctc aca gca acg ggc gaa gag gtg tcg aag cac        912
Lys Val Ser Arg Ala Leu Thr Ala Thr Gly Glu Glu Val Ser Lys His
    290                 295                 300

atg aag gtg aat tga                                                    927
Met Lys Val Asn
305


<210>  50
<211>  308
<212>  PRT
<213>  Crypthecodinium cohnii

<400>  50

Met Ala Ser Tyr Gln Gln Ala Phe Ser Glu Leu Ala Arg Ala Leu Ser
1               5                   10                  15


Thr Leu Asn His Asp Phe Ser Ser Val Glu Pro Phe Lys Val Val Thr
            20                  25                  30
```

```
Gln Phe Cys Arg Asp Gln Trp Ala Ile Pro Thr Val Phe Cys Ile Gly
        35                  40                  45

Tyr Leu Ala Met Val Tyr Ala Thr Arg Arg Pro Ile Ala Lys His Pro
    50                  55                  60

Tyr Met Ser Leu Val Asp Arg Cys Phe Ala Ala Trp Asn Leu Gly Leu
65                  70                  75                  80

Ser Leu Phe Ser Cys Trp Gly Phe Tyr His Met Ala Val Gly Leu Ser
                85                  90                  95

His Thr Thr Trp Asn Phe Gly Leu Gln Phe Thr Ile Cys Gly Ser Thr
            100                 105                 110

Thr Glu Leu Val Asn Gly Phe Gln Lys Gly Pro Ala Ala Leu Ala Leu
        115                 120                 125

Ile Leu Phe Cys Phe Ser Lys Ile Pro Glu Leu Gly Asp Thr Val Phe
    130                 135                 140

Leu Ile Leu Lys Gly Lys Lys Val Arg Phe Leu Gln Trp Tyr His His
145                 150                 155                 160

Thr Thr Val Met Leu Phe Cys Trp Met Ala Leu Ala Thr Glu Tyr Thr
                165                 170                 175

Pro Gly Leu Trp Phe Ala Ala Thr Asn Tyr Phe Val His Ser Ile Met
            180                 185                 190

Tyr Met Tyr Phe Phe Leu Met Thr Phe Lys Thr Ala Ala Gly Ile Ile
        195                 200                 205

Lys Pro Ile Ala Pro Leu Ile Thr Ile Ile Gln Ile Ser Gln Met Val
    210                 215                 220

Trp Gly Leu Val Val Asn Ala Ile Ala Val Gly Thr Phe Phe Thr Thr
225                 230                 235                 240

Gly Asn Cys Gln Ile Gln Ala Val Thr Val Tyr Ser Ala Ile Val Met
                245                 250                 255

Tyr Ala Ser Tyr Phe Tyr Leu Phe Gly Gln Leu Phe Phe Glu Ala Gln
            260                 265                 270

Gly Ser Ala Gly Lys Asp Lys Lys Lys Leu Ala Arg Glu Leu Ser Arg
        275                 280                 285
```

Lys Val Ser Arg Ala Leu Thr Ala Thr Gly Glu Glu Val Ser Lys His
    290                 295                 300

Met Lys Val Asn
305

<210> 51
<211> 795
<212> DNA
<213> Oncorhynchus mykiss

<220>
<221> CDS
<222> (1)..(795)
<223> Delta-5-Elongase

<400> 51
atg gct tca aca tgg caa agc gtt cag tcc atg cgc cag tgg att tta 48
Met Ala Ser Thr Trp Gln Ser Val Gln Ser Met Arg Gln Trp Ile Leu
1               5                   10                  15

gag aat gga gat aaa agg aca gac cca tgg cta ctg gtc tac tcc cct 96
Glu Asn Gly Asp Lys Arg Thr Asp Pro Trp Leu Leu Val Tyr Ser Pro
            20                  25                  30

atg cca gtg gcc att ata ttc ctc ctc tat ctt ggt gtg gtc tgg gct 144
Met Pro Val Ala Ile Ile Phe Leu Leu Tyr Leu Gly Val Val Trp Ala
        35                  40                  45

ggg ccc aag ctg atg aaa cgc agg gaa cca gtt gat ctc aag gct gta 192
Gly Pro Lys Leu Met Lys Arg Arg Glu Pro Val Asp Leu Lys Ala Val
    50                  55                  60

ctc att gtc tac aac ttc gcc atg gtc tgc ctg tct gtc tac atg ttc 240
Leu Ile Val Tyr Asn Phe Ala Met Val Cys Leu Ser Val Tyr Met Phe
65                  70                  75                  80

cat gag ttc ttg gtc acg tcc ttg ctg tct aac tac agt tac ctg tgt 288
His Glu Phe Leu Val Thr Ser Leu Leu Ser Asn Tyr Ser Tyr Leu Cys
                85                  90                  95

caa cct gtg gat tac agc act agt cca ctg gcg atg agg atg gcc aaa 336
Gln Pro Val Asp Tyr Ser Thr Ser Pro Leu Ala Met Arg Met Ala Lys
            100                 105                 110

gta tgc tgg tgg ttt ttc ttc tcc aag gtc ata gaa ttg gct gac acg 384
Val Cys Trp Trp Phe Phe Phe Ser Lys Val Ile Glu Leu Ala Asp Thr
        115                 120                 125

gtg ttc ttc atc ctg agg aag aag aac agt cag ctg act ttc ctg cat 432
Val Phe Phe Ile Leu Arg Lys Lys Asn Ser Gln Leu Thr Phe Leu His
    130                 135                 140

gtc tat cac cat ggc acc atg atc ttc aac tgg tgg gca ggg gtc aag 480
Val Tyr His His Gly Thr Met Ile Phe Asn Trp Trp Ala Gly Val Lys
145                 150                 155                 160

tat ctg gct gga ggc caa tcg ttc ttc atc ggc ctg ctc aat acc ttt 528
Tyr Leu Ala Gly Gly Gln Ser Phe Phe Ile Gly Leu Leu Asn Thr Phe

```
                165                 170                 175

gtg cac atc gtg atg tac tct tac tac gga ctg gct gcc ctg ggg cct      576
Val His Ile Val Met Tyr Ser Tyr Tyr Gly Leu Ala Ala Leu Gly Pro
            180                 185                 190

cac acg cag aag tac tta tgg tgg aag cgc tat ctg acc tca ctg cag      624
His Thr Gln Lys Tyr Leu Trp Trp Lys Arg Tyr Leu Thr Ser Leu Gln
        195                 200                 205

ctg ctc cag ttt gtc ctg ttg acc act cac act ggc tac aac ctc ttc      672
Leu Leu Gln Phe Val Leu Leu Thr Thr His Thr Gly Tyr Asn Leu Phe
    210                 215                 220

act gag tgt gac ttc ccg gac tcc atg aac gct gtg gtg ttt gcc tac      720
Thr Glu Cys Asp Phe Pro Asp Ser Met Asn Ala Val Val Phe Ala Tyr
225                 230                 235                 240

tgt gtc agt ctc att gct ctc ttc agc aac ttc tac tat cag agc tac      768
Cys Val Ser Leu Ile Ala Leu Phe Ser Asn Phe Tyr Tyr Gln Ser Tyr
                245                 250                 255

ctc aac agg aag agc aag aag aca taa                                  795
Leu Asn Arg Lys Ser Lys Lys Thr
            260


<210>  52
<211>  264
<212>  PRT
<213>  Oncorhynchus mykiss

<400>  52

Met Ala Ser Thr Trp Gln Ser Val Gln Ser Met Arg Gln Trp Ile Leu
1               5                   10                  15


Glu Asn Gly Asp Lys Arg Thr Asp Pro Trp Leu Leu Val Tyr Ser Pro
            20                  25                  30


Met Pro Val Ala Ile Ile Phe Leu Leu Tyr Leu Gly Val Val Trp Ala
        35                  40                  45


Gly Pro Lys Leu Met Lys Arg Arg Glu Pro Val Asp Leu Lys Ala Val
    50                  55                  60


Leu Ile Val Tyr Asn Phe Ala Met Val Cys Leu Ser Val Tyr Met Phe
65                  70                  75                  80


His Glu Phe Leu Val Thr Ser Leu Leu Ser Asn Tyr Ser Tyr Leu Cys
                85                  90                  95


Gln Pro Val Asp Tyr Ser Thr Ser Pro Leu Ala Met Arg Met Ala Lys
            100                 105                 110


Val Cys Trp Trp Phe Phe Phe Ser Lys Val Ile Glu Leu Ala Asp Thr
```

115 120 125

Val Phe Phe Ile Leu Arg Lys Lys Asn Ser Gln Leu Thr Phe Leu His
130 135 140

Val Tyr His His Gly Thr Met Ile Phe Asn Trp Trp Ala Gly Val Lys
145 150 155 160

Tyr Leu Ala Gly Gly Gln Ser Phe Phe Ile Gly Leu Leu Asn Thr Phe
165 170 175

Val His Ile Val Met Tyr Ser Tyr Tyr Gly Leu Ala Ala Leu Gly Pro
180 185 190

His Thr Gln Lys Tyr Leu Trp Trp Lys Arg Tyr Leu Thr Ser Leu Gln
195 200 205

Leu Leu Gln Phe Val Leu Leu Thr Thr His Thr Gly Tyr Asn Leu Phe
210 215 220

Thr Glu Cys Asp Phe Pro Asp Ser Met Asn Ala Val Val Phe Ala Tyr
225 230 235 240

Cys Val Ser Leu Ile Ala Leu Phe Ser Asn Phe Tyr Tyr Gln Ser Tyr
245 250 255

Leu Asn Arg Lys Ser Lys Lys Thr
260

<210> 53
<211> 885
<212> DNA
<213> Oncorhynchus mykiss

<220>
<221> CDS
<222> (1)..(885)
<223> Delta-5-Elongase

<400> 53
atg gag act ttt aat tat aaa cta aac atg tac ata gac tca tgg atg 48
Met Glu Thr Phe Asn Tyr Lys Leu Asn Met Tyr Ile Asp Ser Trp Met
1 5 10 15

ggt ccc aga gat gag cgg gta cag gga tgg ctg ctt ctg gac aac tac 96
Gly Pro Arg Asp Glu Arg Val Gln Gly Trp Leu Leu Leu Asp Asn Tyr
20 25 30

cct cca acc ttt gca cta aca gtc atg tac ctg ctg atc gta tgg atg 144
Pro Pro Thr Phe Ala Leu Thr Val Met Tyr Leu Leu Ile Val Trp Met
35 40 45

```
ggg ccc aag tac atg aga cac aga cag ccg gtg tct tgc cgg ggt ctc      192
Gly Pro Lys Tyr Met Arg His Arg Gln Pro Val Ser Cys Arg Gly Leu
    50                  55                  60

ctc ttg gtc tac aat ctg ggc ctc acg atc ttg tcc ttc tat atg ttc      240
Leu Leu Val Tyr Asn Leu Gly Leu Thr Ile Leu Ser Phe Tyr Met Phe
65                  70                  75                  80

tat gag atg gtg tct gct gtg tgg cac ggg gat tat aac ttc ttt tgc      288
Tyr Glu Met Val Ser Ala Val Trp His Gly Asp Tyr Asn Phe Phe Cys
                85                  90                  95

caa gac aca cac agt gca gga gaa acc gat acc aag atc ata aat gtg      336
Gln Asp Thr His Ser Ala Gly Glu Thr Asp Thr Lys Ile Ile Asn Val
            100                 105                 110

ctg tgg tgg tac tac ttc tcc aag ctc ata gag ttt atg gat acc ttc      384
Leu Trp Trp Tyr Tyr Phe Ser Lys Leu Ile Glu Phe Met Asp Thr Phe
        115                 120                 125

ttc ttc atc ctg cgg aag aac aac cat caa atc acg ttt ctg cac atc      432
Phe Phe Ile Leu Arg Lys Asn Asn His Gln Ile Thr Phe Leu His Ile
    130                 135                 140

tac cac cat gct agc atg ctc aac atc tgg tgg ttc gtc atg aac tgg      480
Tyr His His Ala Ser Met Leu Asn Ile Trp Trp Phe Val Met Asn Trp
145                 150                 155                 160

gtg ccc tgt ggt cac tcc tac ttt ggt gcc tcc ctg aac agc ttc atc      528
Val Pro Cys Gly His Ser Tyr Phe Gly Ala Ser Leu Asn Ser Phe Ile
                165                 170                 175

cat gtc ctg atg tac tct tac tat ggg ctc tct gct gtc ccg gcc ttg      576
His Val Leu Met Tyr Ser Tyr Tyr Gly Leu Ser Ala Val Pro Ala Leu
            180                 185                 190

cgg ccc tat cta tgg tgg aag aaa tac atc aca caa gta cag ctg att      624
Arg Pro Tyr Leu Trp Trp Lys Lys Tyr Ile Thr Gln Val Gln Leu Ile
        195                 200                 205

cag ttc ttt ttg acc atg tcc cag acg ata tgt gca gtc att tgg cca      672
Gln Phe Phe Leu Thr Met Ser Gln Thr Ile Cys Ala Val Ile Trp Pro
    210                 215                 220

tgt gat ttc ccc aga ggg tgg ctg tat ttc cag ata ttc tat gtc atc      720
Cys Asp Phe Pro Arg Gly Trp Leu Tyr Phe Gln Ile Phe Tyr Val Ile
225                 230                 235                 240

aca ctt att gcc ctt ttc tca aac ttc tac att cag act tac aag aaa      768
Thr Leu Ile Ala Leu Phe Ser Asn Phe Tyr Ile Gln Thr Tyr Lys Lys
                245                 250                 255

cac ctt gtt tca caa aag aag gag tat cat cag aat ggc tct gtt gct      816
His Leu Val Ser Gln Lys Lys Glu Tyr His Gln Asn Gly Ser Val Ala
            260                 265                 270

tca ttg aat ggc cat gtg aat ggg gtg aca ccc acg gaa acc att aca      864
Ser Leu Asn Gly His Val Asn Gly Val Thr Pro Thr Glu Thr Ile Thr
        275                 280                 285

cac agg aaa gtg agg ggg gac                                          885
His Arg Lys Val Arg Gly Asp
    290                 295
```

<210> 54
<211> 295
<212> PRT
<213> Oncorhynchus mykiss

<400> 54

Met Glu Thr Phe Asn Tyr Lys Leu Asn Met Tyr Ile Asp Ser Trp Met
1 5 10 15

Gly Pro Arg Asp Glu Arg Val Gln Gly Trp Leu Leu Leu Asp Asn Tyr
20 25 30

Pro Pro Thr Phe Ala Leu Thr Val Met Tyr Leu Leu Ile Val Trp Met
35 40 45

Gly Pro Lys Tyr Met Arg His Arg Gln Pro Val Ser Cys Arg Gly Leu
50 55 60

Leu Leu Val Tyr Asn Leu Gly Leu Thr Ile Leu Ser Phe Tyr Met Phe
65 70 75 80

Tyr Glu Met Val Ser Ala Val Trp His Gly Asp Tyr Asn Phe Phe Cys
85 90 95

Gln Asp Thr His Ser Ala Gly Glu Thr Asp Thr Lys Ile Ile Asn Val
100 105 110

Leu Trp Trp Tyr Tyr Phe Ser Lys Leu Ile Glu Phe Met Asp Thr Phe
115 120 125

Phe Phe Ile Leu Arg Lys Asn Asn His Gln Ile Thr Phe Leu His Ile
130 135 140

Tyr His His Ala Ser Met Leu Asn Ile Trp Trp Phe Val Met Asn Trp
145 150 155 160

Val Pro Cys Gly His Ser Tyr Phe Gly Ala Ser Leu Asn Ser Phe Ile
165 170 175

His Val Leu Met Tyr Ser Tyr Tyr Gly Leu Ser Ala Val Pro Ala Leu
180 185 190

Arg Pro Tyr Leu Trp Trp Lys Lys Tyr Ile Thr Gln Val Gln Leu Ile
195 200 205

Gln Phe Phe Leu Thr Met Ser Gln Thr Ile Cys Ala Val Ile Trp Pro
210 215 220

```
Cys Asp Phe Pro Arg Gly Trp Leu Tyr Phe Gln Ile Phe Tyr Val Ile
225                 230                 235                 240

Thr Leu Ile Ala Leu Phe Ser Asn Phe Tyr Ile Gln Thr Tyr Lys Lys
                245                 250                 255

His Leu Val Ser Gln Lys Lys Glu Tyr His Gln Asn Gly Ser Val Ala
            260                 265                 270

Ser Leu Asn Gly His Val Asn Gly Val Thr Pro Thr Glu Thr Ile Thr
        275                 280                 285

His Arg Lys Val Arg Gly Asp
    290                 295


<210>  55
<211>  6753
<212>  DNA
<213>  Oncorhynchus mykiss

<220>
<221>  CDS
<222>  (513)..(1397)
<223>  Delta-5-Elongase


<400>  55
acggattaga agccgccgag cgggtgacag ccctccgaag gaagactctc ctccgtgcgt      60

cctcgtcctc accggtcgcg ttcctgaaac gcagatgtgc ctcgcgccgc actgctccga     120

acaataaaga ttctacaata ctagctttta tggttatgaa gaggaaaaat tggcagtaac     180

ctggccccac aaaccttcaa atgaacgaat caaattaaca accataggat gataatgcga     240

ttagtttttt agccttattt ctggggtaat taatcagcga agcgatgatt tttgatctat     300

taacagatat ataaatgcaa aaactgcatt aaccacttta actaatactt tcaacatttt     360

cggtttgtat tacttcttat tcaaatgtaa taaaagtatc aacaaaaaat tgttaatata     420

cctctatact ttaacgtcaa ggagaaaaaa ccccggatcg gactactagc agctgtaata     480

cgactcacta tagggaatat taagcttaca ta atg gag act ttt aat tat aaa      533
                                    Met Glu Thr Phe Asn Tyr Lys
                                    1               5

cta aac atg tac ata gac tca tgg atg ggt ccc aga gat gag cgg gta      581
Leu Asn Met Tyr Ile Asp Ser Trp Met Gly Pro Arg Asp Glu Arg Val
        10                  15                  20

cag gga tgg ctg ctt ctg gac aac tac cct cca acc ttt gca cta aca      629
Gln Gly Trp Leu Leu Leu Asp Asn Tyr Pro Pro Thr Phe Ala Leu Thr
    25                  30                  35

gtc atg tac ctg ctg atc gta tgg atg ggg ccc aag tac atg aga cac      677
```

Val Met Tyr Leu Leu Ile Val Trp Met Gly Pro Lys Tyr Met Arg His
40 45 50 55

aga cag ccg gtg tct tgc cgg ggt ctc ctc ttg gtc tac aat ctg ggc 725
Arg Gln Pro Val Ser Cys Arg Gly Leu Leu Leu Val Tyr Asn Leu Gly
60 65 70

ctc acg atc ttg tcc ttc tat atg ttc tat gag atg gtg tct gct gtg 773
Leu Thr Ile Leu Ser Phe Tyr Met Phe Tyr Glu Met Val Ser Ala Val
75 80 85

tgg cac ggg gat tat aac ttc ttt tgc caa gac aca cac agt gca gga 821
Trp His Gly Asp Tyr Asn Phe Phe Cys Gln Asp Thr His Ser Ala Gly
90 95 100

gaa acc gat acc aag atc ata aat gtg ctg tgg tgg tac tac ttc tcc 869
Glu Thr Asp Thr Lys Ile Ile Asn Val Leu Trp Trp Tyr Tyr Phe Ser
105 110 115

aag ctc ata gag ttt atg gat acc ttc ttc ttc atc ctg cgg aag aac 917
Lys Leu Ile Glu Phe Met Asp Thr Phe Phe Phe Ile Leu Arg Lys Asn
120 125 130 135

aac cat caa atc acg ttt ctg cac atc tac cac cat gct agc atg ctc 965
Asn His Gln Ile Thr Phe Leu His Ile Tyr His His Ala Ser Met Leu
140 145 150

aac atc tgg tgg ttc gtc atg aac tgg gtg ccc tgt ggt cac tcc tac 1013
Asn Ile Trp Trp Phe Val Met Asn Trp Val Pro Cys Gly His Ser Tyr
155 160 165

ttt ggt gcc tcc ctg aac agc ttc atc cat gtc ctg atg tac tct tac 1061
Phe Gly Ala Ser Leu Asn Ser Phe Ile His Val Leu Met Tyr Ser Tyr
170 175 180

tat ggg ctc tct gct gtc ccg gcc ttg cgg ccc tat cta tgg tgg aag 1109
Tyr Gly Leu Ser Ala Val Pro Ala Leu Arg Pro Tyr Leu Trp Trp Lys
185 190 195

aaa tac atc aca caa gta cag ctg att cag ttc ttt ttg acc atg tcc 1157
Lys Tyr Ile Thr Gln Val Gln Leu Ile Gln Phe Phe Leu Thr Met Ser
200 205 210 215

cag acg ata tgt gca gtc att tgg cca tgt gat ttc ccc aga ggg tgg 1205
Gln Thr Ile Cys Ala Val Ile Trp Pro Cys Asp Phe Pro Arg Gly Trp
220 225 230

ctg tat ttc cag ata ttc tat gtc atc aca ctt att gcc ctt ttc tca 1253
Leu Tyr Phe Gln Ile Phe Tyr Val Ile Thr Leu Ile Ala Leu Phe Ser
235 240 245

aac ttc tac att cag act tac aag aaa cac ctt gtt tca caa aag aag 1301
Asn Phe Tyr Ile Gln Thr Tyr Lys Lys His Leu Val Ser Gln Lys Lys
250 255 260

gag tat cat cag aat ggc tct gtt gct tca ttg aat ggc cat gtg aat 1349
Glu Tyr His Gln Asn Gly Ser Val Ala Ser Leu Asn Gly His Val Asn
265 270 275

ggg gtg aca ccc acg gaa acc att aca cac agg aaa gtg agg ggg gac 1397
Gly Val Thr Pro Thr Glu Thr Ile Thr His Arg Lys Val Arg Gly Asp
280 285 290 295

tgaaggatcc actagtaacg gccgccagtg tgctggaatt ctgcagatat ccagcacagt 1457
ggcggccgct cgagtctaga gggcccttcg aaggtaagcc tatccctaac cctctcctcg 1517
gtctcgattc tacgcgtacc ggtcatcatc accatcacca ttgagtttaa acccgctgat 1577
cctagagggc cgcatcatgt aattagttat gtcacgctta cattcacgcc ctccccccac 1637
atccgctcta accgaaaagg aaggagttag acaacctgaa gtctaggtcc ctatttattt 1697
ttttatagtt atgttagtat taagaacgtt atttatattt caaatttttc tttttttct 1757
gtacagacgc gtgtacgcat gtaacattat actgaaaacc ttgcttgaga aggttttggg 1817
acgctcgaag gctttaattt gcaagctgcg gccctgcatt aatgaatcgg ccaacgcgcg 1877
gggagaggcg gtttgcgtat tgggcgctct tccgcttcct cgctcactga ctcgctgcgc 1937
tcggtcgttc ggctgcggcg agcggtatca gctcactcaa aggcggtaat acggttatcc 1997
acagaatcag gggataacgc aggaaagaac atgtgagcaa aaggccagca aaagcccagg 2057
aaccgtaaaa aggccgcgtt gctggcgttt ttccataggc tccgcccccc tgacgagcat 2117
cacaaaaatc gacgctcaag tcagaggtgg cgaaacccga caggactata aagataccag 2177
gcgtttcccc ctggaagctc cctcgtgcgc tctcctgttc cgaccctgcc gcttaccgga 2237
tacctgtccg cctttctccc ttcgggaagc gtggcgcttt ctcatagctc acgctgtagg 2297
tatctcagtt cggtgtaggt cgttcgctcc aagctgggct gtgtgcacga accccccgtt 2357
cagcccgacc gctgcgcctt atccggtaac tatcgtcttg agtccaaccc ggtaagacac 2417
gacttatcgc cactggcagc agccactggt aacaggatta gcagagcgag gtatgtaggc 2477
ggtgctacag agttcttgaa gtggtggcct aactacggct acactagaag gacagtattt 2537
ggtatctgcg ctctgctgaa gccagttacc ttcggaaaaa gagttggtag ctcttgatcc 2597
ggcaaacaaa ccaccgctgg tagcggtggt ttttttgttt gcaagcagca gattacgcgc 2657
agaaaaaaag gatctcaaga agatcctttg atcttttcta cggggtctga cgctcagtgg 2717
aacgaaaact cacgttaagg gattttggtc atgagattat caaaaaggat cttcacctag 2777
atccttttaa attaaaaatg aagttttaaa tcaatctaaa gtatatatga gtaaacttgg 2837
tctgacagtt accaatgctt aatcagtgag gcacctatct cagcgatctg tctatttcgt 2897
tcatccatag ttgcctgact ccccgtcgtg tagataacta cgatacggga gcgcttacca 2957
tctggcccca gtgctgcaat gataccgcga gacccacgct caccggctcc agatttatca 3017
gcaataaacc agccagccgg aagggccgag cgcagaagtg gtcctgcaac tttatccgcc 3077
tccatccagt ctattaattg ttgccgggaa gctagagtaa gtagttcgcc agttaatagt 3137
ttgcgcaacg ttgttgccat tgctacaggc atcgtggtgt cacgctcgtc gtttggtatg 3197
gcttcattca gctccggttc ccaacgatca aggcgagtta catgatcccc catgttgtgc 3257
aaaaaagcgg ttagctcctt cggtcctccg atcgttgtca gaagtaagtt ggccgcagtg 3317

ttatcactca tggttatggc agcactgcat aattctctta ctgtcatgcc atccgtaaga 3377

tgcttttctg tgactggtga gtactcaacc aagtcattct gagaatagtg tatgcggcga 3437

ccgagttgct cttgcccggc gtcaacacgg gataataccg cgccacatag cagaacttta 3497

aaagtgctca tcattggaaa acgttcttcg gggcgaaaac tctcaaggat cttaccgctg 3557

ttgagatcca gttcgatgta acccactcgt gcacccaact gatcttcagc atcttttact 3617

ttcaccagcg tttctgggtg agcaaaaaca ggaaggcaaa atgccgcaaa aaagggaata 3677

agggcgacac ggaaatgttg aatactcata ctcttccttt ttcaatatta ttgaagcatt 3737

tatcagggtt attgtctcat gagcggatac atatttgaat gtatttagaa aaataaacaa 3797

ataggggttc cgcgcacatt tccccgaaaa gtgccacctg acgtctaaga aaccattatt 3857

atcatgacat taacctataa aaataggcgt atcacgaggc cctttcgtct tcaagaaatt 3917

cggtcgaaaa aagaaaagga gagggccaag agggagggca ttggtgacta ttgagcacgt 3977

gagtatacgt gattaagcac acaaaggcag cttggagtat gtctgttatt aatttcacag 4037

gtagttctgg tccattggtg aaagtttgcg gcttgcagag cacagaggcc gcagaatgtg 4097

ctctagattc cgatgctgac ttgctgggta ttatatgtgt gcccaataga aagagaacaa 4157

ttgacccggt tattgcaagg aaaatttcaa gtcttgtaaa agcatataaa aatagttcag 4217

gcactccgaa atacttggtt ggcgtgtttc gtaatcaacc taaggaggat gttttggctc 4277

tggtcaatga ttacggcatt gatatcgtcc aactgcacgg agatgagtcg tggcaagaat 4337

accaagagtt cctcggtttg ccagttatta aaagactcgt atttccaaaa gactgcaaca 4397

tactactcag tgcagcttca cagaaacctc attcgtttat tcccttgttt gattcagaag 4457

caggtgggac aggtgaactt ttggattgga actcgatttc tgactgggtt ggaaggcaag 4517

agagccccga gagcttacat tttatgttag ctggtggact gacgccagaa aatgttggtg 4577

atgcgcttag attaaatggc gttattggtg ttgatgtaag cggaggtgtg gagacaaatg 4637

gtgtaaaaga ctctaacaaa atagcaaatt tcgtcaaaaa tgctaagaaa taggttatta 4697

ctgagtagta tttatttaag tattgtttgt gcacttgccc tagcttatcg atgataagct 4757

gtcaaagatg agaattaatt ccacggacta tagactatac tagatactcc gtctactgta 4817

cgatacactt ccgctcaggt ccttgtcctt taacgaggcc ttaccactct tttgttactc 4877

tattgatcca gctcagcaaa ggcagtgtga tctaagattc tatcttcgcg atgtagtaaa 4937

actagctaga ccgagaaaga gactagaaat gcaaaaggca cttctacaat ggctgccatc 4997

attattatcc gatgtgacgc tgcagcttct caatgatatt cgaatacgct ttgaggagat 5057

acagcctaat atccgacaaa ctgttttaca gatttacgat cgtacttgtt acccatcatt 5117

gaattttgaa catccgaacc tgggagtttt ccctgaaaca gatagtatat ttgaacctgt 5177

ataataatat atagtctagc gctttacgga agacaatgta tgtatttcgg ttcctggaga 5237

aactattgca tctattgcat aggtaatctt gcacgtcgca tccccggttc attttctgcg 5297

tttccatctt gcacttcaat agcatatctt tgttaacgaa gcatctgtgc ttcattttgt 5357

agaacaaaaa tgcaacgcga gagcgctaat ttttcaaaca aagaatctga gctgcatttt 5417

tacagaacag aaatgcaacg cgaaagcgct attttaccaa cgaagaatct gtgcttcatt 5477

tttgtaaaac aaaaatgcaa cgcgacgaga gcgctaattt ttcaaacaaa gaatctgagc 5537

tgcattttta cagaacagaa atgcaacgcg agagcgctat tttaccaaca aagaatctat 5597

acttcttttt tgttctacaa aaatgcatcc cgagagcgct atttttctaa caaagcatct 5657

tagattactt tttttctcct ttgtgcgctc tataatgcag tctcttgata actttttgca 5717

ctgtaggtcc gttaaggtta gaagaaggct actttggtgt ctattttctc ttccataaaa 5777

aaagcctgac tccacttccc gcgtttactg attactagcg aagctgcggg tgcatttttt 5837

caagataaag gcatccccga ttatattcta taccgatgtg gattgcgcat actttgtgaa 5897

cagaaagtga tagcgttgat gattcttcat tggtcagaaa attatgaacg gtttcttcta 5957

ttttgtctct atatactacg tataggaaat gtttacattt tcgtattgtt ttcgattcac 6017

tctatgaata gttcttacta caattttttt gtctaaagag taatactaga gataaacata 6077

aaaaatgtag aggtcgagtt tagatgcaag ttcaaggagc gaaaggtgga tgggtaggtt 6137

atatagggat atagcacaga gatatatagc aaagagatac ttttgagcaa tgtttgtgga 6197

agcggtattc gcaatgggaa gctccacccc ggttgataat cagaaaagcc ccaaaaacag 6257

gaagattgta taagcaaata tttaaattgt aaacgttaat attttgttaa aattcgcgtt 6317

aaatttttgt taaatcagct cattttttaa cgaatagccc gaaatcggca aaatccctta 6377

taaatcaaaa gaatagaccg agatagggtt gagtgttgtt ccagtttcca acaagagtcc 6437

actattaaag aacgtggact ccaacgtcaa agggcgaaaa agggtctatc agggcgatgg 6497

cccactacgt gaaccatcac cctaatcaag ttttttgggg tcgaggtgcc gtaaagcagt 6557

aaatcggaag ggtaaacgga tgcccccatt tagagcttga cggggaaagc cggcgaacgt 6617

ggcgagaaag gaagggaaga aagcgaaagg agcgggggct agggcggtgg gaagtgtagg 6677

ggtcacgctg ggcgtaacca ccacacccgc cgcgcttaat ggggcgctac agggcgcgtg 6737

gggatgatcc actagt 6753

<210> 56
<211> 295
<212> PRT
<213> Oncorhynchus mykiss

<400> 56

Met Glu Thr Phe Asn Tyr Lys Leu Asn Met Tyr Ile Asp Ser Trp Met

```
1               5                   10                  15

Gly Pro Arg Asp Glu Arg Val Gln Gly Trp Leu Leu Leu Asp Asn Tyr
            20                  25                  30

Pro Pro Thr Phe Ala Leu Thr Val Met Tyr Leu Leu Ile Val Trp Met
        35                  40                  45

Gly Pro Lys Tyr Met Arg His Arg Gln Pro Val Ser Cys Arg Gly Leu
    50                  55                  60

Leu Leu Val Tyr Asn Leu Gly Leu Thr Ile Leu Ser Phe Tyr Met Phe
65                  70                  75                  80

Tyr Glu Met Val Ser Ala Val Trp His Gly Asp Tyr Asn Phe Phe Cys
                85                  90                  95

Gln Asp Thr His Ser Ala Gly Glu Thr Asp Thr Lys Ile Ile Asn Val
            100                 105                 110

Leu Trp Trp Tyr Tyr Phe Ser Lys Leu Ile Glu Phe Met Asp Thr Phe
        115                 120                 125

Phe Phe Ile Leu Arg Lys Asn Asn His Gln Ile Thr Phe Leu His Ile
    130                 135                 140

Tyr His His Ala Ser Met Leu Asn Ile Trp Trp Phe Val Met Asn Trp
145                 150                 155                 160

Val Pro Cys Gly His Ser Tyr Phe Gly Ala Ser Leu Asn Ser Phe Ile
                165                 170                 175

His Val Leu Met Tyr Ser Tyr Tyr Gly Leu Ser Ala Val Pro Ala Leu
            180                 185                 190

Arg Pro Tyr Leu Trp Trp Lys Lys Tyr Ile Thr Gln Val Gln Leu Ile
        195                 200                 205

Gln Phe Phe Leu Thr Met Ser Gln Thr Ile Cys Ala Val Ile Trp Pro
    210                 215                 220

Cys Asp Phe Pro Arg Gly Trp Leu Tyr Phe Gln Ile Phe Tyr Val Ile
225                 230                 235                 240

Thr Leu Ile Ala Leu Phe Ser Asn Phe Tyr Ile Gln Thr Tyr Lys Lys
                245                 250                 255
```

```
His Leu Val Ser Gln Lys Lys Glu Tyr His Gln Asn Gly Ser Val Ala
            260                 265                 270

Ser Leu Asn Gly His Val Asn Gly Val Thr Pro Thr Glu Thr Ile Thr
        275                 280                 285

His Arg Lys Val Arg Gly Asp
    290                 295
```

```
<210>  57
<211>  6645
<212>  DNA
<213>  Oncorhynchus mykiss

<220>
<221>  CDS
<222>  (513)..(1304)
<223>  Delta-5-Elongase

<400>  57
acggattaga agccgccgag cgggtgacag ccctccgaag gaagactctc ctccgtgcgt      60

cctcgtcctc accggtcgcg ttcctgaaac gcagatgtgc ctcgcgccgc actgctccga     120

acaataaaga ttctacaata ctagctttta tggttatgaa gaggaaaaat tggcagtaac     180

ctggccccac aaaccttcaa atgaacgaat caaattaaca accataggat gataatgcga     240

ttagtttttt agccttattt ctggggtaat taatcagcga agcgatgatt tttgatctat     300

taacagatat ataaatgcaa aaactgcatt aaccacttta actaatactt tcaacatttt     360

cggtttgtat tacttcttat tcaaatgtaa taaaagtatc aacaaaaaat tgttaatata     420

cctctatact ttaacgtcaa ggagaaaaaa ccccggatcg gactactagc agctgtaata     480

cgactcacta tagggaatat taagcttaca ta atg gct tca aca tgg caa agc      533
                                    Met Ala Ser Thr Trp Gln Ser
                                    1               5

gtt cag tcc atg cgc cag tgg att tta gag aat gga gat aaa agg aca      581
Val Gln Ser Met Arg Gln Trp Ile Leu Glu Asn Gly Asp Lys Arg Thr
        10                  15                  20

gac cca tgg cta ctg gtc tac tcc cct atg cca gtg gcc att ata ttc      629
Asp Pro Trp Leu Leu Val Tyr Ser Pro Met Pro Val Ala Ile Ile Phe
    25                  30                  35

ctc ctc tat ctt ggt gtg gtc tgg gct ggg ccc aag ctg atg aaa cgc      677
Leu Leu Tyr Leu Gly Val Val Trp Ala Gly Pro Lys Leu Met Lys Arg
40                  45                  50                  55

agg gaa cca gtt gat ctc aag gct gta ctc att gtc tac aac ttc gcc      725
Arg Glu Pro Val Asp Leu Lys Ala Val Leu Ile Val Tyr Asn Phe Ala
                60                  65                  70

atg gtc tgc ctg tct gtc tac atg ttc cat gag ttc ttg gtc acg tcc      773
Met Val Cys Leu Ser Val Tyr Met Phe His Glu Phe Leu Val Thr Ser
            75                  80                  85
```

```
ttg ctg tct aac tac agt tac ctg tgt caa cct gtg gat tac agc act      821
Leu Leu Ser Asn Tyr Ser Tyr Leu Cys Gln Pro Val Asp Tyr Ser Thr
        90                  95                  100

agt cca ctg gcg atg agg atg gcc aaa gta tgc tgg tgg ttt ttc ttc      869
Ser Pro Leu Ala Met Arg Met Ala Lys Val Cys Trp Trp Phe Phe Phe
    105                 110                 115

tcc aag gtc ata gaa ttg gct gac acg gtg ttc ttc atc ctg agg aag      917
Ser Lys Val Ile Glu Leu Ala Asp Thr Val Phe Phe Ile Leu Arg Lys
120                 125                 130                 135

aag aac agt cag ctg act ttc ctg cat gtc tat cac cat ggc acc atg      965
Lys Asn Ser Gln Leu Thr Phe Leu His Val Tyr His His Gly Thr Met
                140                 145                 150

atc ttc aac tgg tgg gca ggg gtc aag tat ctg gct gga ggc caa tcg     1013
Ile Phe Asn Trp Trp Ala Gly Val Lys Tyr Leu Ala Gly Gly Gln Ser
            155                 160                 165

ttc ttc atc ggc ctg ctc aat acc ttt gtg cac atc gtg atg tac tct     1061
Phe Phe Ile Gly Leu Leu Asn Thr Phe Val His Ile Val Met Tyr Ser
        170                 175                 180

tac tac gga ctg gct gcc ctg ggg cct cac acg cag aag tac tta tgg     1109
Tyr Tyr Gly Leu Ala Ala Leu Gly Pro His Thr Gln Lys Tyr Leu Trp
    185                 190                 195

tgg aag cgc tat ctg acc tca ctg cag ctg ctc cag ttt gtc ctg ttg     1157
Trp Lys Arg Tyr Leu Thr Ser Leu Gln Leu Leu Gln Phe Val Leu Leu
200                 205                 210                 215

acc act cac act ggc tac aac ctc ttc act gag tgt gac ttc ccg gac     1205
Thr Thr His Thr Gly Tyr Asn Leu Phe Thr Glu Cys Asp Phe Pro Asp
                220                 225                 230

tcc atg aac gct gtg gtg ttt gcc tac tgt gtc agt ctc att gct ctc     1253
Ser Met Asn Ala Val Val Phe Ala Tyr Cys Val Ser Leu Ile Ala Leu
            235                 240                 245

ttc agc aac ttc tac tat cag agc tac ctc aac agg aag agc aag aag     1301
Phe Ser Asn Phe Tyr Tyr Gln Ser Tyr Leu Asn Arg Lys Ser Lys Lys
        250                 255                 260

aca taaggatcca ctagtaacgg ccgccagtgt gctggaattc tgcagatatc          1354
Thr

catcacactg gcggccgctc gagcatgcat ctagagggcc gcatcatgta attagttatg   1414

tcacgcttac attcacgccc tccccccaca tccgctctaa ccgaaaagga aggagttaga   1474

caacctgaag tctaggtccc tatttatttt tttatagtta tgttagtatt aagaacgtta   1534

tttatatttc aaatttttct tttttttctg tacagacgcg tgtacgcatg taacattata   1594

ctgaaaacct tgcttgagaa ggttttggga cgctcgaagg ctttaatttg cggccctgca   1654

ttaatgaatc ggccaacgcg cggggagagg cggtttgcgt attgggcgct cttccgcttc   1714

ctcgctcact gactcgctgc gctcggtcgt tcggctgcgg cgagcggtat cagctcactc   1774
```

aaaggcggta atacggttat ccacagaatc aggggataac gcaggaaaga acatgtgagc 1834

aaaaggccag caaaagccca ggaaccgtaa aaaggccgcg ttgctggcgt ttttccatag 1894

gctccgcccc cctgacgagc atcacaaaaa tcgacgctca agtcagaggt ggcgaaaccc 1954

gacaggacta taaagatacc aggcgtttcc ccctggaagc tccctcgtgc gctctcctgt 2014

tccgaccctg ccgcttaccg gatacctgtc cgcctttctc ccttcgggaa gcgtggcgct 2074

ttctcatagc tcacgctgta ggtatctcag ttcggtgtag gtcgttcgct ccaagctggg 2134

ctgtgtgcac gaaccccccg ttcagcccga ccgctgcgcc ttatccggta actatcgtct 2194

tgagtccaac ccggtaagac acgacttatc gccactggca gcagccactg gtaacaggat 2254

tagcagagcg aggtatgtag gcggtgctac agagttcttg aagtggtggc ctaactacgg 2314

ctacactaga aggacagtat ttggtatctg cgctctgctg aagccagtta ccttcggaaa 2374

aagagttggt agctcttgat ccggcaaaca aaccaccgct ggtagcggtg gtttttttgt 2434

ttgcaagcag cagattacgc gcagaaaaaa aggatctcaa gaagatcctt tgatcttttc 2494

tacggggtct gacgctcagt ggaacgaaaa ctcacgttaa gggattttgg tcatgagatt 2554

atcaaaaagg atcttcacct agatcctttt aaattaaaaa tgaagtttta aatcaatcta 2614

aagtatatat gagtaaactt ggtctgacag ttaccaatgc ttaatcagtg aggcacctat 2674

ctcagcgatc tgtctatttc gttcatccat agttgcctga ctccccgtcg tgtagataac 2734

tacgatacgg gagcgcttac catctggccc cagtgctgca atgataccgc gagacccacg 2794

ctcaccggct ccagatttat cagcaataaa ccagccagcc ggaagggccg agcgcagaag 2854

tggtcctgca actttatccg cctccattca gtctattaat tgttgccggg aagctagagt 2914

aagtagttcg ccagttaata gtttgcgcaa cgttgttggc attgctacag gcatcgtggt 2974

gtcactctcg tcgtttggta tggcttcatt cagctccggt tcccaacgat caaggcgagt 3034

tacatgatcc cccatgttgt gcaaaaaagc ggttagctcc ttcggtcctc cgatcgttgt 3094

cagaagtaag ttggccgcag tgttatcact catggttatg gcagcactgc ataattctct 3154

tactgtcatg ccatccgtaa gatgcttttc tgtgactggt gagtactcaa ccaagtcatt 3214

ctgagaatag tgtatgcggc gaccgagttg ctcttgcccg gcgtcaatac gggataatag 3274

tgtatcacat agcagaactt taaaagtgct catcattgga aaacgttctt cggggcgaaa 3334

actctcaagg atcttaccgc tgttgagatc cagttcgatg taacccactc gtgcacccaa 3394

ctgatcttca gcatctttta ctttcaccag cgtttctggg tgagcaaaaa caggaaggca 3454

aaatgccgca aaaaagggaa taagggcgac acggaaatgt tgaatactca tactcttcct 3514

ttttcaatgg gtaataactg atataattaa attgaagctc taatttgtga gtttagtata 3574

catgcattta cttataatac agttttttag ttttgctggc cgcatcttct caaatatgct 3634

tcccagcctg cttttctgta acgttcaccc tctaccttag catcccttcc ctttgcaaat 3694

agtcctcttc caacaataat aatgtcagat cctgtagaga ccacatcatc cacggttcta 3754

tactgttgac ccaatgcgtc tcccttgtca tctaaaccca caccgggtgt cataatcaac 3814

caatcgtaac cttcatctct tccacccatg tctctttgag caataaagcc gataacaaaa 3874

tctttgtcgc tcttcgcaat gtcaacagta cccttagtat attctccagt agatagggag 3934

cccttgcatg acaattctgc taacatcaaa aggcctctag gttcctttgt tacttcttct 3994

gccgcctgct tcaaaccgct aacaatacct gggcccacca caccgtgtgc attcgtaatg 4054

tctgcccatt ctgctattct gtatacaccc gcagagtact gcaatttgac tgtattacca 4114

atgtcagcaa attttctgtc ttcgaagagt aaaaaattgt acttggcgga taatgccttt 4174

agcggcttaa ctgtgccctc catggaaaaa tcagtcaaga tatccacatg tgtttttagt 4234

aaacaaattt tgggacctaa tgcttcaact aactccagta attccttggt ggtacgaaca 4294

tccaatgaag cacacaagtt tgtttgcttt tcgtgcatga tattaaatag cttggcagca 4354

acaggactag gatgagtagc agcacgttcc ttatatgtag ctttcgacat gatttatctt 4414

cgtttcctgc aggtttttgt tctgtgcagt tgggttaaga atactgggca atttcatgtt 4474

tcttcaacac tacatatgcg tatatatacc aatctaagtc tgtgctcctt ccttcgttct 4534

tccttctgtt cggagattac cgaatcaaaa aaatttcaaa gaaaccgaaa tcaaaaaaaa 4594

gaataaaaaa aaaatgatga attgaattga aaagctagct tatcgatgat aagctgtcaa 4654

agatgagaat taattccacg gactatagac tatactagat actccgtcta ctgtacgata 4714

cacttccgct caggtccttg tcctttaacg aggccttacc actcttttgt tactctattg 4774

atccagctca gcaaaggcag tgtgatctaa gattctatct tcgcgatgta gtaaaactag 4834

ctagaccgag aaagagacta gaaatgcaaa aggcacttct acaatggctg ccatcattat 4894

tatccgatgt gacgctgcag cttctcaatg atattcgaat acgctttgag gagatacagc 4954

ctaatatccg acaaactgtt ttacagattt acgatcgtac ttgttaccca tcattgaatt 5014

ttgaacatcc gaacctggga gttttccctg aaacagatag tatatttgaa cctgtataat 5074

aatatatagt ctagcgcttt acggaagaca atgtatgtat ttcggttcct ggagaaacta 5134

ttgcatctat tgcataggta atcttgcacg tcgcatcccc ggttcatttt ctgcgtttcc 5194

atcttgcact tcaatagcat atctttgtta acgaagcatc tgtgcttcat tttgtagaac 5254

aaaaatgcaa cgcgagagcg ctaatttttc aaacaaagaa tctgagctgc atttttacag 5314

aacagaaatg caacgcgaaa gcgctatttt accaacgaag aatctgtgct tcatttttgt 5374

aaaacaaaaa tgcaacgcga cgagagcgct aatttttcaa acaaagaatc tgagctgcat 5434

ttttacagaa cagaaatgca acgcgagagc gctattttac caacaaagaa tctatacttc 5494

ttttttgttc tacaaaaatg catcccgaga gcgctatttt tctaacaaag catcttagat 5554

tactttttt ctcctttgtg cgctctataa tgcagtctct tgataacttt ttgcactgta 5614

ggtccgttaa ggttagaaga aggctacttt ggtgtctatt ttctcttcca taaaaaaagc 5674

ctgactccac ttcccgcgtt tactgattac tagcgaagct gcgggtgcat tttttcaaga 5734

taaaggcatc cccgattata ttctataccg atgtggattg cgcatacttt gtgaacagaa 5794

agtgatagcg ttgatgattc ttcattggtc agaaaattat gaacggtttc ttctattttg 5854

tctctatata ctacgtatag gaaatgttta cattttcgta ttgttttcga ttcactctat 5914

gaatagttct tactacaatt tttttgtcta aagagtaata ctagagataa acataaaaaa 5974

tgtagaggtc gagtttagat gcaagttcaa ggagcgaaag gtggatgggt aggttatata 6034

gggatatagc acagagatat atagcaaaga gatacttttg agcaatgttt gtggaagcgg 6094

tattcgcaat gggaagctcc accccggttg ataatcagaa aagccccaaa aacaggaaga 6154

ttgtataagc aaatatttaa attgtaaacg ttaatatttt gttaaaattc gcgttaaatt 6214

tttgttaaat cagctcattt tttaacgaat agcccgaaat cggcaaaatc ccttataaat 6274

caaaagaata gaccgagata gggttgagtg ttgttccagt ttccaacaag agtccactat 6334

taaagaacgt ggactccaac gtcaaagggc gaaaaagggt ctatcagggc gatggcccac 6394

tacgtgaacc atcaccctaa tcaagttttt tggggtcgag gtgccgtaaa gcagtaaatc 6454

ggaagggtaa acggatgccc ccatttagag cttgacgggg aaagccggcg aacgtggcga 6514

gaaaggaagg gaagaaagcg aaaggagcgg gggctagggc ggtgggaagt gtaggggtca 6574

cgctgggcgt aaccaccaca cccgccgcgc ttaatggggc gctacagggc gcgtggggat 6634

gatccactag t 6645

<210> 58
<211> 264
<212> PRT
<213> Oncorhynchus mykiss

<400> 58

Met Ala Ser Thr Trp Gln Ser Val Gln Ser Met Arg Gln Trp Ile Leu
1 5 10 15

Glu Asn Gly Asp Lys Arg Thr Asp Pro Trp Leu Leu Val Tyr Ser Pro
20 25 30

Met Pro Val Ala Ile Ile Phe Leu Leu Tyr Leu Gly Val Val Trp Ala
35 40 45

Gly Pro Lys Leu Met Lys Arg Arg Glu Pro Val Asp Leu Lys Ala Val
50 55 60

Leu Ile Val Tyr Asn Phe Ala Met Val Cys Leu Ser Val Tyr Met Phe

```
65                  70                  75                  80

His Glu Phe Leu Val Thr Ser Leu Leu Ser Asn Tyr Ser Tyr Leu Cys
                85                  90                  95

Gln Pro Val Asp Tyr Ser Thr Ser Pro Leu Ala Met Arg Met Ala Lys
            100                 105                 110

Val Cys Trp Trp Phe Phe Phe Ser Lys Val Ile Glu Leu Ala Asp Thr
        115                 120                 125

Val Phe Phe Ile Leu Arg Lys Lys Asn Ser Gln Leu Thr Phe Leu His
    130                 135                 140

Val Tyr His His Gly Thr Met Ile Phe Asn Trp Trp Ala Gly Val Lys
145                 150                 155                 160

Tyr Leu Ala Gly Gly Gln Ser Phe Phe Ile Gly Leu Leu Asn Thr Phe
                165                 170                 175

Val His Ile Val Met Tyr Ser Tyr Tyr Gly Leu Ala Ala Leu Gly Pro
            180                 185                 190

His Thr Gln Lys Tyr Leu Trp Trp Lys Arg Tyr Leu Thr Ser Leu Gln
        195                 200                 205

Leu Leu Gln Phe Val Leu Leu Thr Thr His Thr Gly Tyr Asn Leu Phe
    210                 215                 220

Thr Glu Cys Asp Phe Pro Asp Ser Met Asn Ala Val Val Phe Ala Tyr
225                 230                 235                 240

Cys Val Ser Leu Ile Ala Leu Phe Ser Asn Phe Tyr Tyr Gln Ser Tyr
                245                 250                 255

Leu Asn Arg Lys Ser Lys Lys Thr
            260
```